# EUROPEAN PATENT APPLICATION

(11) **EP 4 534 659 A2**
(43) Date of publication of application: **09.04.2025**
(21) Application number: 24213677.8
(22) Date of filing: 02.08.2011
(51) Int. Cl.: C12N 9/02

(54) **MUTATED PROTOPORPHYRINOGEN IX OXIDASE (PPX) GENES**

(30) Priority: 03.08.2010 US 37043610 P
(62) Divisional of application: 18184232.9
(71) Applicant: Cibus US LLC, San Diego, California 92121 (US); Cibus Europe B.V., 4811 CA Breda (NL)
(72) Inventor: Gocal, Gregory F.W., San Diego, CA 92129 (US); Beetham, Peter R., Carlsbad, CA 92011 (US); De Schopke, Aura, San Diego, CA 92126 (US); Dumm, Sarah, San Diego, CA 92109 (US); Pearce, James, La Jolla, CA 92037 (US); Schopke, Christian, Vista, CA 92081 (US); Walker, Keith A., San Diego, CA 92130 (US)
(74) Representative: Zimmermann & Partner Patentanwälte mbB

(57) **Abstract**

Provided are compositions and methods relating to gene and/or protein mutations in transgenic or non-transgenic plants. In certain embodiments, the disclosure relates to mutations in the protoporphyrinogen IX (PPX) gene. In some embodiments the disclosure relates to plants that are herbicide resitant.

## Description

### FIELD OF THE INVENTION

This disclosure relates, at least in part, to gene and/or protein mutations in plants.

### BACKGROUND OF THE INVENTION

The following description is provided simply as an aid in understanding the invention and is not admitted to describe or constitute prior art.

Examples of certain mutations in the PPX genes of plants have been reported. For example, US Pat. No. 5,767,373 discloses "eukaryotic DNA sequences coding for native protoporphyrinogen oxidase (protox) or modified forms of the enzyme which are herbicide tolerant;" US Pat. No. 6,282,837 discloses "eukaryotic DNA sequences coding for native protoporphyrinogen oxidase (protox) or modified forms of the enzyme which are herbicide tolerant and a method for controlling weeds using plants having altered protox activity which confers tolerance to herbicides;" US Pat No. 6,308,458 discloses "methods for controlling the growth of undesired vegetation comprising applying an effective amount of a protox-inhibiting herbicide to a population of transgenic plants or plant seed transformed with a DNA sequence coding for a modified protox enzyme that is tolerant to a protox-inhibiting herbicide or to the locus where a population of the transgenic plants or plant seeds is cultivated;" US Patent No. 6,905,852 discloses "[a] protoporphyrinogen oxidase tolerant to photobleaching herbicide and derivatives thereof, comprising a polypeptide having the amino acid sequence represented by SEQ ID No. 2 [a PPX protein] or mutated peptides derived therefrom by deletion, addition, substitution, etc. of one or more amino acids in the above amino acid sequence and having an activity substantially equivalent to that of the protoporphyrinogen oxidase;" US Patent No. discloses "methods to confer resistance to protoporphyrinogen-inhibiting herbicides onto crop plants. Resistance is conferred by genetically engineering the plants to express cloned DNA encoding a protoporphyrinogen oxidase resistant to porphyric herbicides;" US Patent Application Publication No. 20020086395 discloses "[a] method for evaluating the ability of a compound to inhibit the protoporphyrinogen oxidase activity, which comprises the steps of: (1) culturing a transformant expressing a protoporphyrinogen oxidase gene present in a DNA fragment in a medium containing substantially no protoheme compounds in each comparative system of the presence and absence of a test compound to measure a growth rate of the transformant under each condition, said transformant being resulted from a host cell deficient in the growing ability based on the protoporphyrinogen oxidase activity transformed with the DNA fragment in which a promoter functionable in the host cell and a protoporphyrinogen oxidase gene are operatively linked, and (2) determining the ability of the compound to inhibit the protoporphyrinogen oxidase activity by comparing the growth rates; and the like;" Patzoldt WL, et al., PNAS USA 103:12329-34 (2006) discloses a "3-bp deletion corresponding to the G210 codon" of PPX; and Li X, et al., Plant Physiology 133:736-47 (2003) discloses "isolation of plant protoporphyrinogen oxidase (PPO) genes and the isolation of herbicide-resistant mutants." The terms PPO and PPX are used interchangeably herein.

### SUMMARY OF THE INVENTION

The present disclosure relates, at least, in part to methods and compositions relating to gene and protein mutations in plants. In some aspects and embodiments, the present disclosure may also relate to compositions and methods for producing herbicide-resistant plants. The present disclosure methods and compositions relate, at least in part to mutations in a protoporphyrinogen IX oxidase (PPX) gene.

In one aspect, there is provided a plant or a plant cell including a mutated PPX gene. In certain embodiments, the mutated PPX gene encodes a mutated PPX protein. In certain embodiments, a plant having a plant cell that includes a mutated PPX gene may be herbicide-resistant; for example, resistant to a PPX-inhibiting herbicide. In certain embodiments, the plant or the plant cell is non-transgenic. In certain embodiments, the plant or the plant cell is transgenic. The disclosure also provides recombinant vectors including such mutated PPX genes, as well as transgenic plants containing such mutated PPX genes.

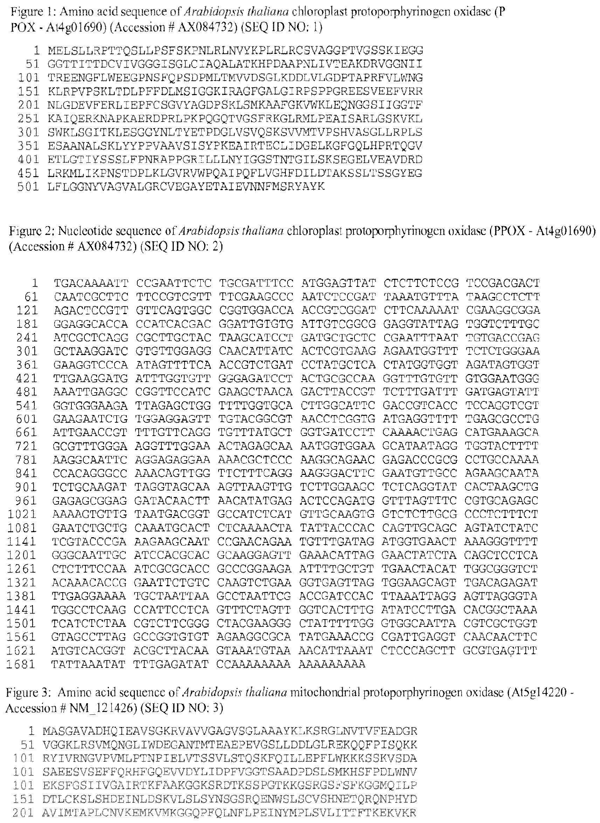

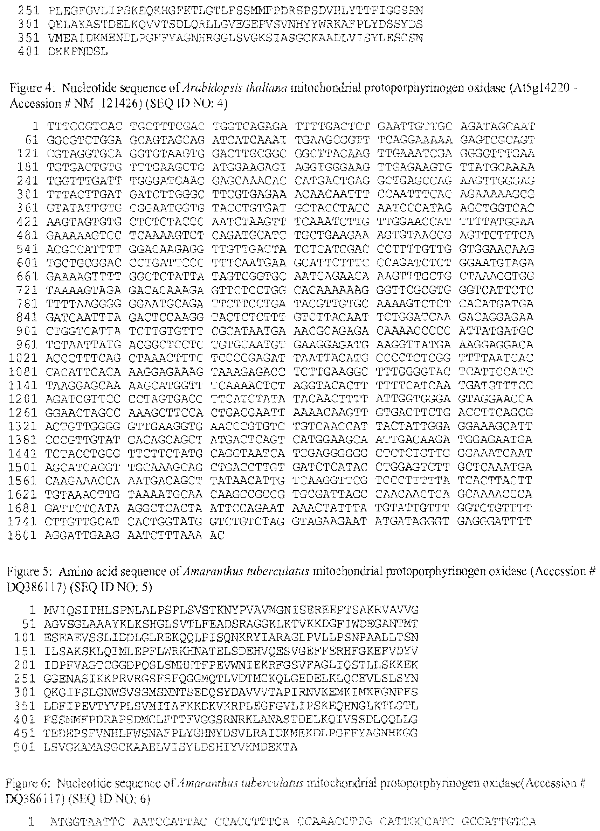

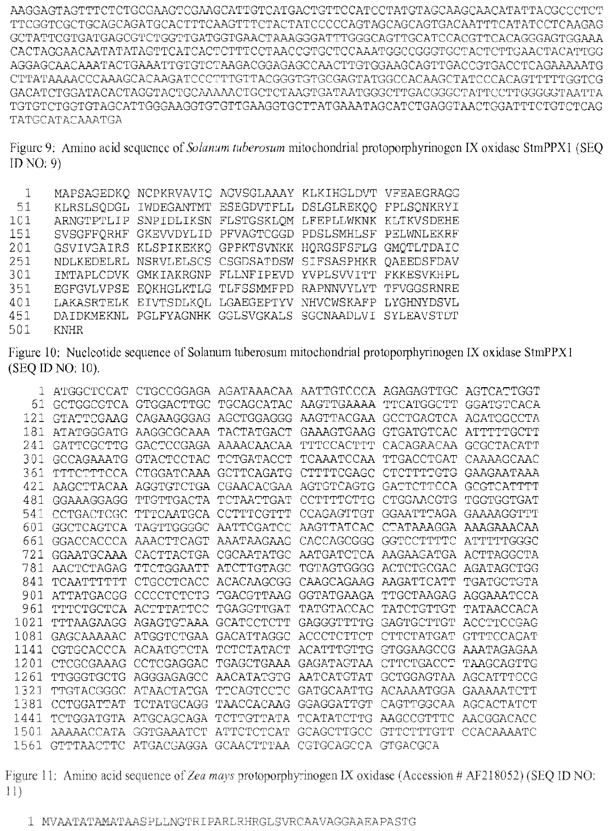

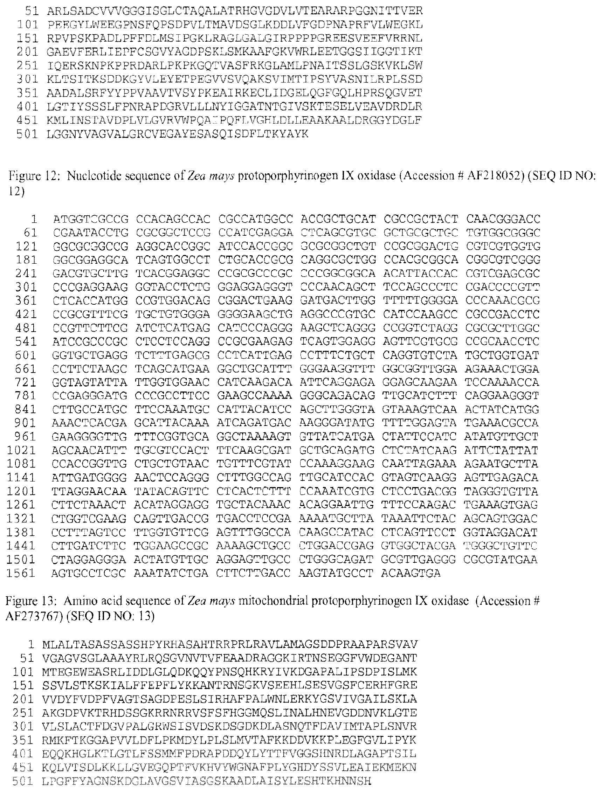

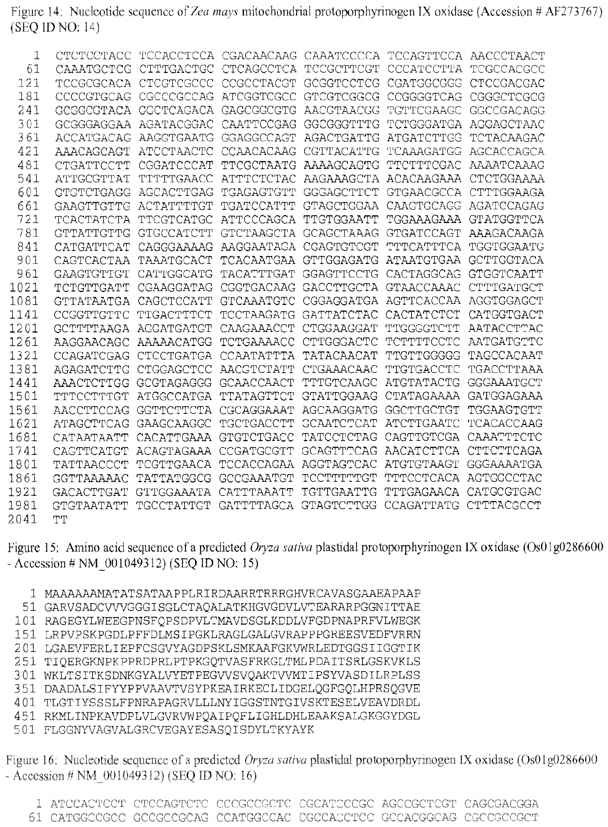

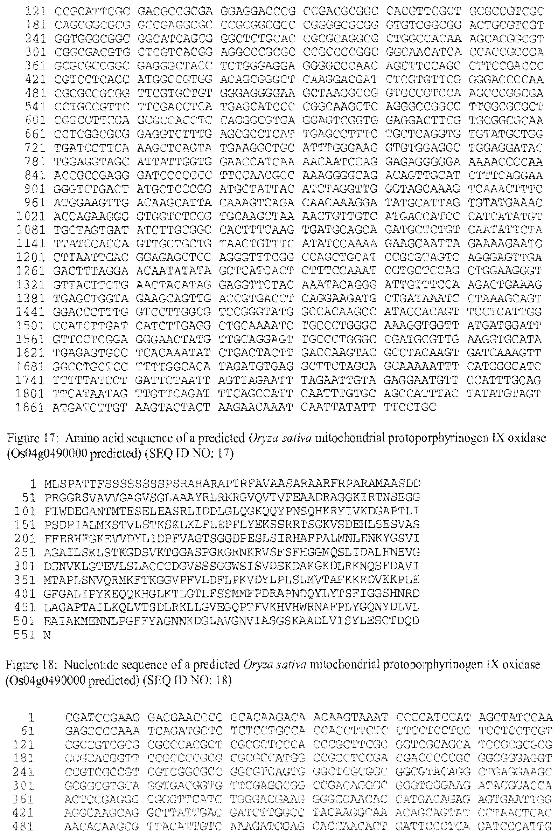

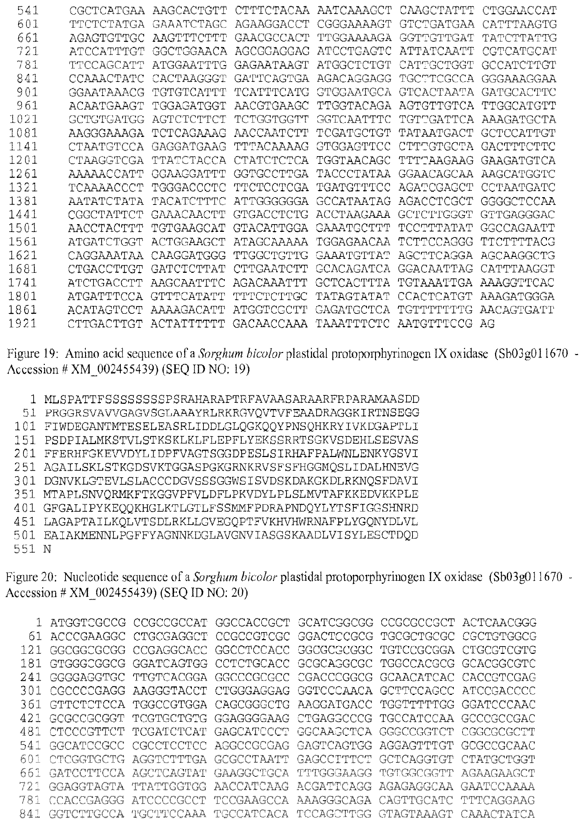

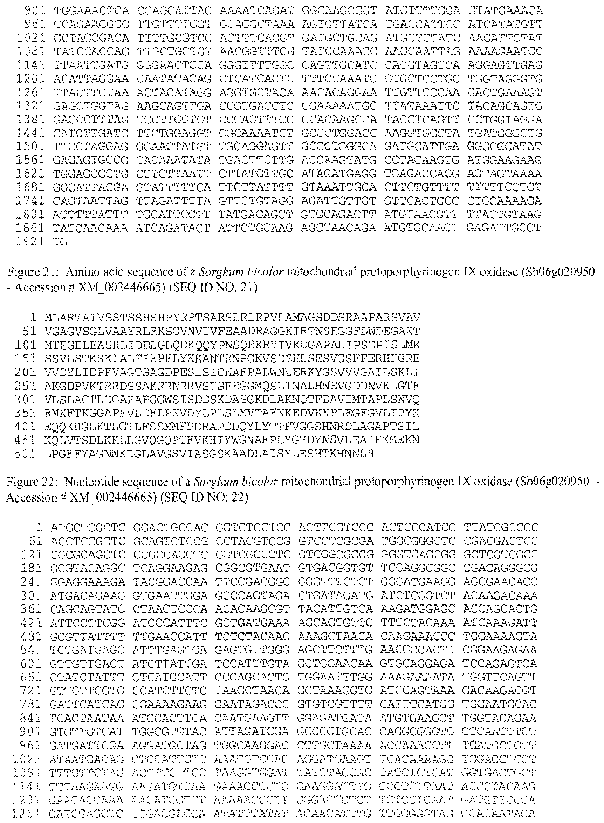

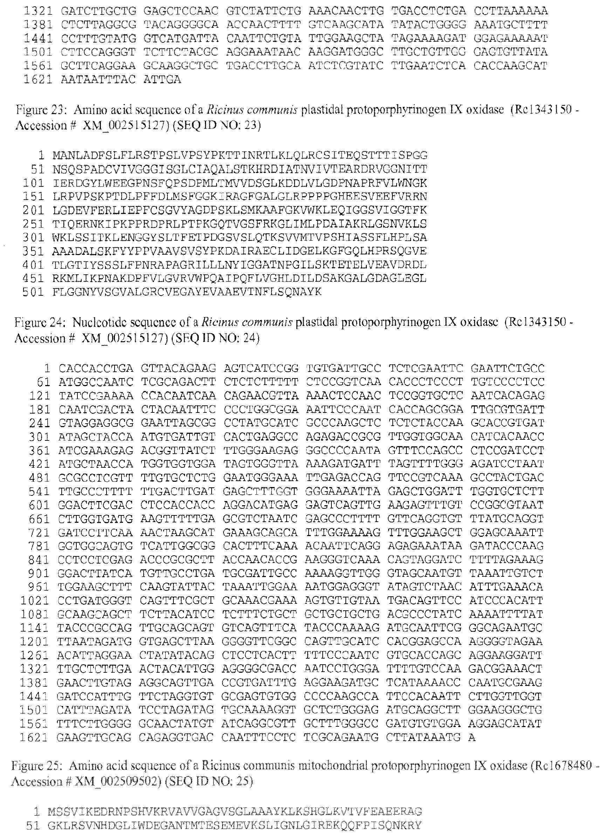

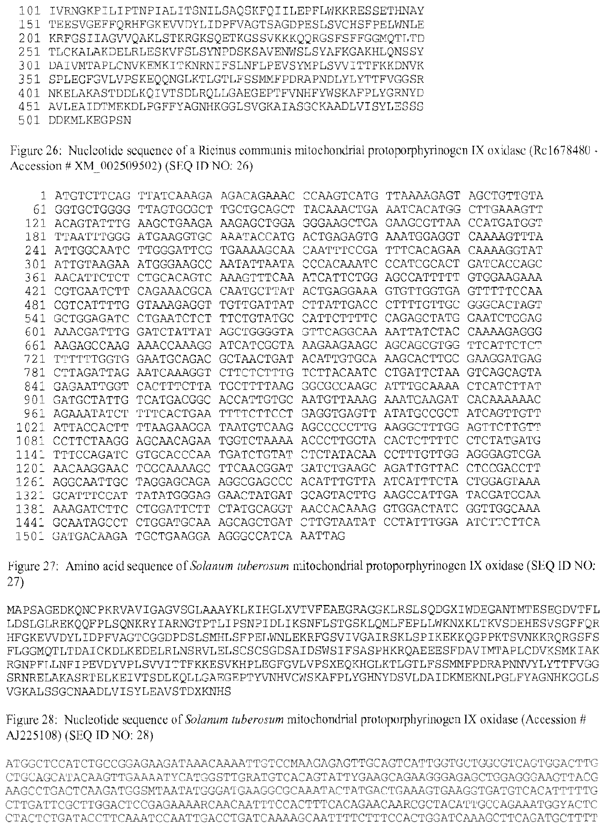

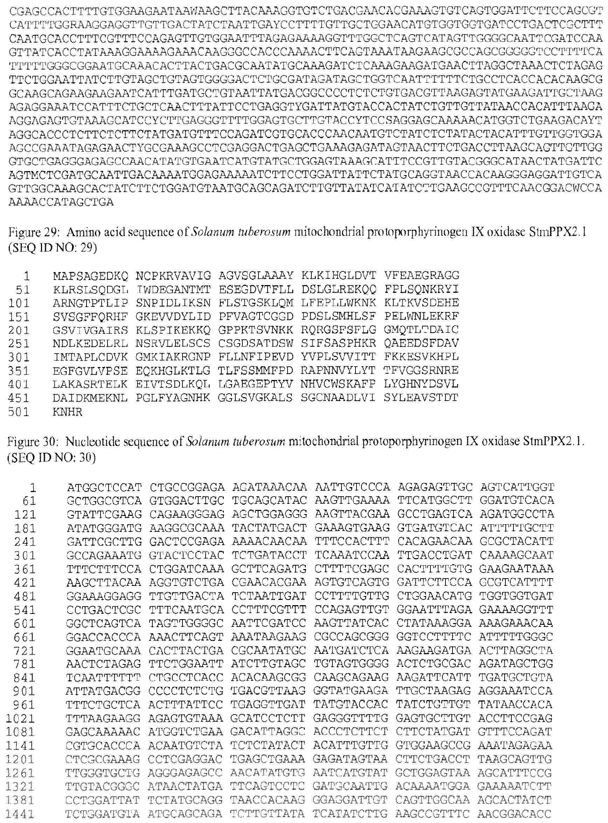

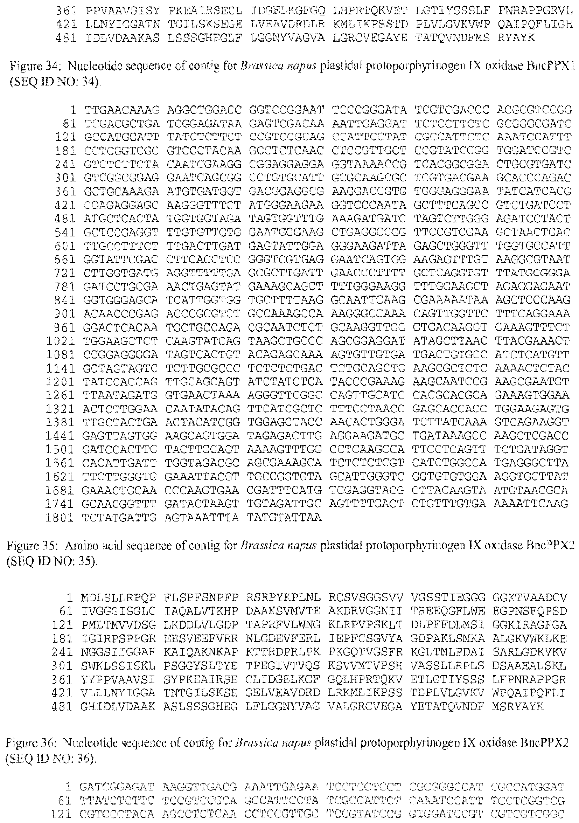

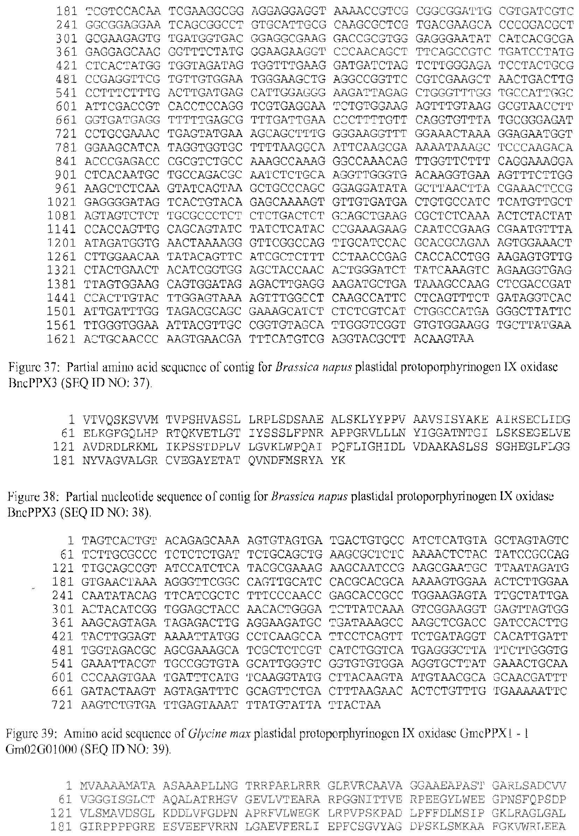

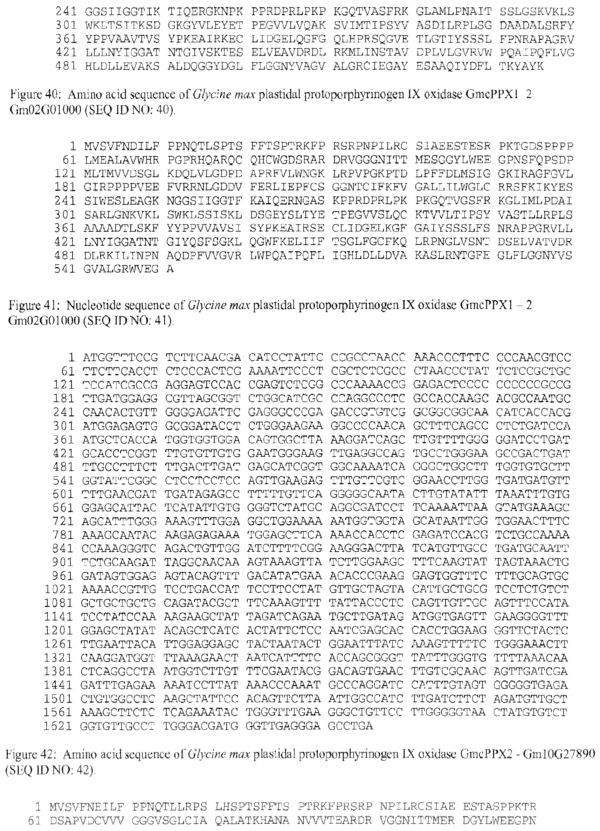

As used herein, the term "PPX gene" refers to a DNA sequence capable of generating a PPX polypeptide that shares homology and/or amino acid identity with amino acid sequence SEQ ID NO: 1, and/or encodes a protein that demonstrates PPX activity. In certain embodiments, the PPX gene has 70%; 75%; 80%; 85%; 90%; 95%; 96%; 97%; 98%; 99%; or 100% identity to a specific PPX gene; for example, the mitochondrial Russet Burbank PPX genes, for example, StmPPX1 or StmPPX2; or for example, a plastidal Russet Burbank PPX gene, for example, StcPPX1. In certain embodiments, the PPX gene has 60%; 70%; 75%; 80%; 85%; 90%; 95%; 96%; 97%; 98%; 99%; or 100% identity to a sequence selected from the sequences in Figures 2, 4, 6, 8, 10, 12, 14, 16, 18, 20, 22, 24, 26, 28, 30, 32, 34, 36, 38, 41 43 and 45. In some embodiments, a PPX gene is a mitochondrial PPX gene; for example, StmPPX1 or StmPPX2. In some embodiments, a PPX gene is a plastidal PPX gene; for example, StcPPX1. In some embodiments, a PPX gene is a mitochondrial PPX gene allele; for example, StmPPX2.1 or StmPPX2.2. In some embodiments, a PPX gene is a plastidal PPX gene allele; for example, StcPPX1 or StcPPX1.1. In some plants, such as water hemp, the protein product of a single PPX gene is both mitochondrial and plastidal as disclosed in Patzoldt WL, et al., PNAS USA 103:12329-34 (2006).

As used herein, the term "mutation" refers to at least a single nucleotide variation in a nucleic acid sequence and/or a single amino acid variation in a polypeptide relative to the normal sequence or wild-type sequence or a reference sequence, e.g., SEQ ID NO: 1 or SEQ ID NO: 2. In some embodiments a mutation refers to at least a single nucleotide variation in a nucleic acid sequence and/or a single amino acid variation in a polypeptide relative to a nucleotide or amino acid sequence of a PPX protein that is not herbicide resistant. In certain embodiments, a mutation may include a substitution, a deletion, an inversion or an insertion. In some embodiments, a substitution, deletion, insertion, or inversion may include a variation at 1, 2, 3, 4, 5, 6, 7, 8, 9, 10, 11, 12, 13, 14, 15, 16, 17, 18, 19, 20, 21, 22, 23 or 24 nucleotides. In some embodiments, a substitution, deletion, insertion, or inversion may include a variation at 1, 2, 3, 4, 5, 6, 7 or 8 amino acid positions. The term "nucleic acid" or "nucleic acid sequence" refers to an oligonucleotide, nucleotide or polynucleotide, and fragments or portions thereof, which may be single or double stranded, and represent the sense or antisense strand. A nucleic acid may include DNA or RNA, and may be of natural or synthetic origin. For example, a nucleic acid may include mRNA or cDNA. Nucleic acid may include nucleic acid that has been amplified (*e.g.,* using polymerase chain reaction). The convention "NTwt###NTmut" is used to indicate a mutation that results in the wild-type nucleotide NTwt at position ### in the nucleic acid being replaced with mutant NTmut. The single letter code for nucleotides is as described in the U.S. Patent Office Manual of Patent Examining Procedure, section 2422, table 1. In this regard, the nucleotide designation "R" means purine such as guanine or adenine, "Y" means pyrimidine such as cytosine or thymine (uracil if RNA); "M" means adenine or cytosine; "K" means guanine or thymine; and "W" means adenine or thymine.

As used herein, the term "mutated PPX gene" refers to a PPX gene having one or more mutations at nucleotide positions relative to a reference PPX nucleic acid sequence. In certain embodiments a mutated PPX gene has one or more mutations relative to a corresponding wild type PPX sequence. As used herein, the term "wild-type" may be used to designate the standard allele at a locus, or the allele having the highest frequency in a particular population. In some instances, wild-type allele may be represented by a particular amino acid or nucleic acid sequence. For example, a wild-type potato plastidal PPX protein may be represented by SEQ ID NO: 7. For example, a wild-type potato mitochondrial PPX protein may be represented by SEQ ID NO: 9. In some embodiments a mutated PPX gene has one or more mutations relative to a reference PPX nucleic acid sequence, for example SEQ ID NOs: 2, 4, 6, 8, 10, 12, 14, 16, 18, 20, 22, 24, 26, 28, 30, 32, 34, 36, 38, 41 43 or 45 or at homologous positions of paralogs thereof. In some embodiments, the mutated PPX gene is modified with at least one mutation. In other embodiments, the mutated PPX gene is modified with at least two mutations. In other embodiments, the mutated PPX gene is modified with at least three mutations. In some embodiments, a mutated PPX gene encodes a mutated PPX protein. In some embodiments, a mutated PPX gene includes two or more nucleic acid sequence mutations selected from Tables 2, 3a and 3b. In some embodiments, a mutated PPX gene encodes one or more mutated mitochondrial PPX proteins. In other embodiments, a mutated PPX gene encodes one or more mutated plastidal PPX proteins. In some embodiments, a mutated PPX gene is a mutated mitochondrial PPX gene; for example, mutated StmPPX1. In some embodiments, a mutated PPX gene is a mutated mitochondrial PPX gene; for example, mutated StmPPX2. In some embodiments, a mutated PPX gene is a mutated plastidal PPX gene; for example, mutated StcPPX1. In some embodiments, a mutated PPX gene is a mutated mitochondrial PPX gene allele; for example, mutated StmPPX2.1 or mutated StmPPX2.2. In some embodiments, a mutated PPX gene is a mutated plastidal PPX gene allele; for example, mutated StcPPX1 or mutated StcPPX1.1. In some embodiments, there is at least one mutation in a plastid PPX gene and at least one mutation in a mitochondrial PPX gene. In some embodiments, one or more mutations in a PPX gene leads to herbicide resistance; for example, resistance to a PPX-inhibiting herbicide. In some embodiments, the mutated PPX gene encodes a mutated PPX protein that has increased resistance to one or more herbicides as compared to a reference PPX protein.

In some embodiments, the mutations in a mutated PPX gene encodes a protein having a combination of two or more mutations. In certain embodiments, at least one mutation is in the plastid PPX gene and at least one mutation is in a mitochondrial PPX gene. In certain embodiments, the combinations are selected from Tables 4a and 4b. In some embodiments, the mutations in a mutated PPX gene encode a protein having a combination of three or more mutations; for example, combinations selected from Tables 4a and 4b. In some embodiments, the at least one mutation in the plastidal PPX gene and the at least one mutation in the mitochondrial PPX gene are at the same corresponding position. In other embodiments, the at least one mutation in the plastid PPX gene and the at least one mutation in the mitochondrial PPX gene are at different corresponding positions.

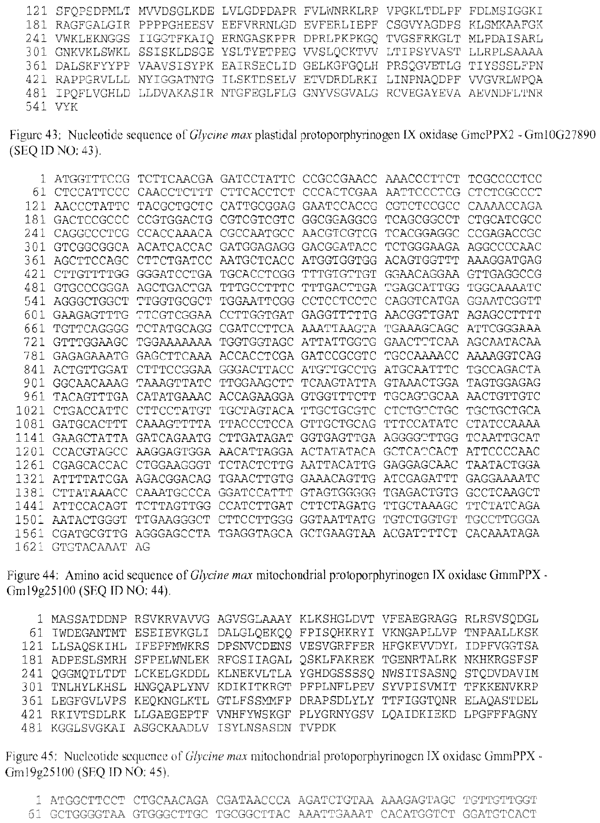

As used herein, the term "PPX protein" refers to a protein that has homology and/or amino acid identity to a PPX protein of SEQ ID NOs: 1, 3, 5, 7, 9, 11, 13, 15, 17, 19, 21, 23, 25, 27, 29, 31, 33, 35, 37, 39, 40, 42 or 44 and/or demonstrates PPX activity. In certain embodiments, the PPX protein has 70%; 75%; 80%; 85%; 90%; 95%; 96%; 97%; 98%; 99%; or 100% identity to a specific PPX protein, such as for example, the mitochondrial Russet Burbank PPX protein or the plastidal Russet Burbank PPX proteins. In certain embodiments, the PPX protein has 70%; 75%; 80%; 85%; 90%; 95%; 96%; 97%; 98%; 99%; or 100% identity to a sequence selected from the sequences in Figures 1, 3, 5, 7, 9, 11, 13, 15, 17, 19, 21, 23, 25, 27, 29, 31, 33, 35, 37, 39, 40, 42 or 44.

As used herein, the term "mutated PPX protein" refers to a PPX protein having one or more mutations at positions of amino acids relative to a reference PPX amino acid sequence, or at homologous positions of paralogs thereof. In some embodiments, a mutated PPX protein has one or more mutations relative to a reference PPX amino acid sequence, for example, a reference PPX amino acid sequence having SEQ ID NOs: 1, 3, 5, 7, 9, 11, 13, 15, 17, 19, 21, 23, 25, 27, 29, 31, 33, 35, 37, 39, 40, 42 or 44, or portions thereof. In certain embodiments a mutated PPX protein has one or more mutations relative to a corresponding wild type protein. In some embodiments a mutated PPX protein has one or more mutations relative to a corresponding protein that is not herbicide resistant. In some embodiments, the PPX protein is modified with at least one mutation. In other embodiments, the PPX protein is modified with at least two mutations. In other embodiments, the PPX protein is modified with at least three mutations. In some embodiments, one or more mitochondrial PPX proteins are mutated. In other embodiments, one or more plastidal PPX proteins are mutated. In yet another embodiment one or more mitochondrial PPX proteins and one or more plastidal PPX proteins are mutated. In some embodiments, the term mutated PPX protein refers to a PPX protein that has increased resistance to one or more herbicides as compared to a reference protein.

In some embodiments, a mutated PPX protein includes a mutation at one or more amino acid positions corresponding to a position selected from the group consisting of 52, 85, 105, 111, 130, 139, 143, 144, 145, 147, 165, 167, 170, 180, 185, 192, 193, 199, 206, 212, 219, 220, 221, 226, 228, 229, 230, 237, 244, 256, 257, 270, 271, 272, 305, 311, 316, 318, 332, 343, 354, 357, 359, 360, 366, 393, 403, 424, 426, 430, 438, 440, 444, 455, 457, 470, 478, 483, 484, 485, 487, 490, 503, 508 and 525 of SEQ ID NO: 1. In some embodiments, a mutated PPX protein includes a mutation at one or more amino acid positions corresponding to a position selected from the group consisting of 58, 64, 74, 84, 93, 97, 98, 101, 119, 121, 124, 139, 150 151, 157, 164, 170, 177, 187, 188, 195, 214, 215, 229, 230, 271, 274, 278, 283, 292, 296, 307, 324, 330, 396, 404, 406, 410, 421, 423, 434, 447, 448, 449, 451, 454, 465, 470 and 500 of SEQ ID NO: 9. In some embodiments, a plant or plant cell may include a mutated protoporphyrinogen IX oxidase (PPX) gene wherein the gene encodes a protein including a mutation at one or more amino acid positions corresponding to a position selected from the group consisting of G52, N85, N105, E111, G130, D139, P143, R144, F145, L147, F165, L167, I170, A180, P185, E192, S193, R199, V206, E212, Y219, A220, G221, L226, M228, K229, A230, K237, S244, R256, R257, K270, P271, Q272, S305, E311, T316, T318,S332, S343, A354, L357, K359, L360, A366, L393, L403, L424, Y426, S430 , K438, E440, V444, L455, K457, V470, F478, F483, D484, I485, D487, K490, L503, V508 and I525 of SEQ ID NO: 1. In some embodiments, a plant or plant cell may include a mutated protoporphyrinogen IX oxidase (PPX) gene wherein the gene encodes a protein including a mutation at one or more amino acid positions corresponding to a position selected from the group consisting of D58, E64, G74, G84, L93, K97, K98, A101, S119, F121, T124, N139, E150, S151, Q157, V164, D170, C177, H187, L188, N195, P214, I215, K229, K230, C271, D274, F283, A292, S296, C307, N324, D330, S396, A404, R406, K410, L421, A423, C434, D447, S448, V449, D451, D454, Y465, K470 and T500 of SEQ ID NO: 9. In some embodiments, a PPX protein is a paralog of *Arabidopsis thaliana* PPX protein (for example the PPX protein may be a potato plastidal PPX protein) and the PPX protein may have an N at the position corresponding to position 52 of SEQ ID NO:1, wherein the N is substituted with an amino acid other than an N; a K at the position corresponding to position 272 of SEQ ID NO:1, wherein the K is substituted with an amino acid other than a K; an S at the position corresponding to position 359 of SEQ ID NO:1, wherein the S is substituted with an amino acid other than an S; and/or an S at the position corresponding to position 525 of SEQ ID NO:1, wherein the S is substituted with an amino acid other than an S. In some embodiments, a mutated PPX protein includes two or more mutations, at least one mutation of which is at the amino acid position corresponding to a position selected from the group consisting of G52, N85, N105, E111, G130, D139, P143, R144, F145, L147, F165, L167, I170, A180, P185, E192, S193, R199, V206, E212, Y219, A220, G221, L226, M228, K229, A230, K237, S244, R256, R257, K270, P271, Q272, S305, E311, T316, T318,S332, S343, A354, L357, K359, L360, A366, L393, L403, L424, Y426, S430 , K438, E440, V444, L455, K457, V470, F478, F483, D484, I485, D487, K490, L503, V508, and I525 of SEQ ID NO: 1. In some embodiments, a mutated PPX protein includes two or more mutations, at least one mutation of which is at the amino acid position corresponding to a position selected from the group consisting of D58, E64, G74, G84, L93, K97, K98, A101, S119, F121, T124, N139, E150, S151, Q157, V164, D170, C177, H187, L188, N195, P214, I215, K229, K230, C271, D274, F283, A292, S296, C307, N324, D330, S396, A404, R406, K410, L421, A423, C434, D447, S448, V449, D451, D454, Y465, K470 and T500 of SEQ ID NO: 9. In some embodiments, a PPX protein is a paralog of *Arabidopsis thaliana* PPX protein (for example the PPX protein may be a potato plastidal PPX protein) and the PPX protein has two or more mutations and has one or more of: (1) an N at the position corresponding to position 52 of SEQ ID NO:1, wherein the N is substituted with an amino acid other than an N; (2) a K at the position corresponding to position 272 of SEQ ID NO:1, wherein the K is substituted with an amino acid other than a K; (3) an S at the position corresponding to position 359 of SEQ ID NO:1, wherein the S is substituted with an amino acid other than an S; and/or (4) an S at the position corresponding to position 525 of SEQ ID NO:1, wherein the S is substituted with an amino acid other than an S. In some embodiments, a mutated PPX protein includes three or more mutations, at least one mutation of which is at the amino acid position corresponding to a position selected from the group consisting of G52, N85, N105, E111, G130, D139, P143, R144, F145, L147, F165, L167, I170, A180, P185, E192, S193, R199, V206, E212, Y219, A220, G221, L226, M228, K229, A230, K237, S244, R256, R257, K270, P271, Q272, S305, E311, T316, T318,S332, S343, A354, L357, K359, L360, A366, L393, L403, L424, Y426, S430 , K438, E440, V444, L455, K457, V470, F478, F483, D484, I485, D487, K490, L503, V508 and I525 of SEQ ID NO: 1. In some embodiments, a mutated PPX protein includes three or more mutations, at least one mutation of which is at the amino acid position corresponding to a position selected from the group consisting of D58, E64, G74, G84, L93, K97, K98, A101, S119, F121, T124, N139, E150, S151, Q157, V164, D170, C177, H187, L188, N195, P214, I215, K229, K230, C271, D274, F283, A292, S296, C307, N324, D330, S396, A404, R406, K410, L421, A423, C434, D447, S448, V449, D451, D454, Y465, K470 and T500 of SEQ ID NO: 9. In some embodiments, a PPX protein is a paralog of *Arabidopsis thaliana* PPX protein (for example the PPX protein may be a potato PPX protein) and the PPX protein has three or more mutations and has one or more of: (1) an N at the position corresponding to position 52 of SEQ ID NO:1, wherein the N is substituted with an amino acid other than an N; (2) a K at the position corresponding to position 272 of SEQ ID NO:1, wherein the K is substituted with an amino acid other than a K; (3) an S at the position corresponding to position 359 of SEQ ID NO:1, wherein the S is substituted with an amino acid other than an S; and/or (4) an S at the position corresponding to position 525 of SEQ ID NO:1, wherein the S is substituted with an amino acid other than an S.

In conjunction with the various aspects, embodiments, compositions and methods disclosed herein, a mutated PPX protein includes one or more amino acid mutations selected from Tables 1, 2, 3a, 3b, 4a, 4b, 8a-f, 9a-d and 10. In some embodiments, a mutated PPX protein includes two or more amino acid mutations selected from Tables 1, 2, 3a, 3b, 4a, 4b, 8a-f, 9a-d and 10. In some embodiments, a mutated PPX protein includes three or more amino acid mutations selected from Tables 1, 2, 3a, 3b, 4a, 4b, 8a-f, 9a-d and 10. In some embodiments, a mutated PPX protein includes one or more nucleic acid sequence mutations selected from Tables 2, 3a and 3b. In some embodiments, the one or more mutations in a mutated PPX protein includes one or more mutations, two or more mutations, or three or more mutations selected from the group consisting of a glycine to lysine at a position corresponding to position 52 of SEQ ID NO: 1; an asparagine to aspartic acid at a position corresponding to position 85 of SEQ ID NO: 1; a glutamic acid to valine at a position corresponding to position 111 of SEQ ID NO: 1; a glycine to asparagine at a position corresponding to position 130 of SEQ ID NO: 1; an aspartic acid to histidine at a position corresponding to position 139 of SEQ ID NO: 1; a proline to arginine at a position corresponding to position 143 of SEQ ID NO: 1; an arginine to cysteine at a position corresponding to position 144 of SEQ ID NO: 1; an arginine to leucine at a position corresponding to position 144 of SEQ ID NO: 1; an arginine to histidine at a position corresponding to position 144 of SEQ ID NO: 1; a phenylalanine to leucine at a position corresponding to position 145 of SEQ ID NO: 1; a phenylalanine to tyrosine at a position corresponding to position 145 of SEQ ID NO: 1; a leucine to valine at a position corresponding to position 147 of SEQ ID NO: 1; a phenylalanine to asparagine at a position corresponding to position 165 of SEQ ID NO: 1; an alanine to threonine at a position corresponding to position 180 of SEQ ID NO: 1; a proline to arginine at a position corresponding to position 185 of SEQ ID NO: 1; a proline to histidine at a position corresponding to position 185 of SEQ ID NO: 1; a proline to tyrosine at a position corresponding to position 185 of SEQ ID NO: 1; a glutamic acid to aspartic acid at a position corresponding to position 192 of SEQ ID NO: 1; a glutamic acid to lysine at a position corresponding to position 192 of SEQ ID NO: 1; a serine to threonine at a position corresponding to position 193 of SEQ ID NO: 1; an arginine to leucine at a position corresponding to position 199 of SEQ ID NO: 1; a valine to phenylalanine at a position corresponding to position 206 of SEQ ID NO: 1; a tyrosine to serine at a position corresponding to position 219 of SEQ ID NO: 1; an alanine to cysteine at a position corresponding to position 220 of SEQ ID NO: 1; an alanine to isoleucine at a position corresponding to position 220 of SEQ ID NO: 1; an alanine to leucine at a position corresponding to position 220 of SEQ ID NO: 1; an alanine to threonine at a position corresponding to position 220 of SEQ ID NO: 1; an alanine to valine at a position corresponding to position 220 of SEQ ID NO: 1; a leucine to methionine at a position corresponding to position 226 of SEQ ID NO: 1; a methionine to leucine at a position corresponding to position 228 of SEQ ID NO: 1; a lysine to glutamine at a position corresponding to position 229 of SEQ ID NO: 1; an alanine to phenylalanine at a position corresponding to position 230 of SEQ ID NO: 1; a serine to glycine at a position corresponding to position 244 of SEQ ID NO: 1; a serine to threonine at a position corresponding to position 244 of SEQ ID NO: 1; an arginine to histidine at a position corresponding to position 256 of SEQ ID NO: 1; an arginine to serine at a position corresponding to position 256 of SEQ ID NO: 1; a lysine to glutamic acid at a position corresponding to position 270 of SEQ ID NO: 1; a lysine to glutamine at a position corresponding to position 270 of SEQ ID NO: 1; a proline to arginine at a position corresponding to position 271 of SEQ ID NO: 1; a glutamine to phenylalanine at a position corresponding to position 272 of SEQ ID NO: 1; a serine to leucine at a position corresponding to position 305 of SEQ ID NO: 1; a glutamic acid arginine at a position corresponding to position 311 of SEQ ID NO: 1; a threonine to glycine at a position corresponding to position 316 of SEQ ID NO: 1; a threonine to glycine at a position corresponding to position 318 of SEQ ID NO: 1; a serine to cysteine at a position corresponding to position 332 of SEQ ID NO: 1; a leucine to isoleucine at a position corresponding to position 357 of SEQ ID NO: 1; a lysine to arginine at a position corresponding to position 359 of SEQ ID NO: 1; a lysine to threonine at a position corresponding to position 359 of SEQ ID NO: 1; a leucine to aspartic acid at a position corresponding to position 360 of SEQ ID NO: 1; a leucine to lysine at a position corresponding to position 360 of SEQ ID NO: 1; an alanine to glutamic acid at a position corresponding to position 366 of SEQ ID NO: 1; a leucine to methionine at a position corresponding to position 393 of SEQ ID NO: 1; a leucine to serine at a position corresponding to position 393 of SEQ ID NO: 1; a leucine to valine at a position corresponding to position 393 of SEQ ID NO: 1; a leucine to arginine at a position corresponding to position 403 of SEQ ID NO: 1; a leucine to serine at a position corresponding to position 403 of SEQ ID NO: 1; a leucine to serine at a position corresponding to position 424 of SEQ ID NO: 1; a tyrosine to cysteine at a position corresponding to position 426 of SEQ ID NO: 1; a tyrosine to phenylalanine at a position corresponding to position 426 of SEQ ID NO: 1; a tyrosine to histidine at a position corresponding to position 426 of SEQ ID NO: 1; a tyrosine to isoleucine at a position corresponding to position 426 of SEQ ID NO: 1; a tyrosine to leucine at a position corresponding to position 426 of SEQ ID NO: 1; a tyrosine to arginine at a position corresponding to position 426 of SEQ ID NO: 1; a tyrosine to threonine at a position corresponding to position 426 of SEQ ID NO: 1; a tyrosine to valine at a position corresponding to position 426 of SEQ ID NO: 1; a serine to leucine at a position corresponding to position 430 of SEQ ID NO: 1; a lysine to serine at a position corresponding to position 438 of SEQ ID NO: 1; a glutamic acid to lysine at a position corresponding to position 440 of SEQ ID NO: 1; a valine to isoleucine at a position corresponding to position 444 of SEQ ID NO: 1; a leucine to valine at a position corresponding to position 455 of SEQ ID NO: 1; a lysine to valine at a position corresponding to position 457 of SEQ ID NO: 1; a valine to serine at a position corresponding to position 470 of SEQ ID NO: 1; a valine to tyrosine at a position corresponding to position 470 of SEQ ID NO: 1; a phenylalanine to serine at a position corresponding to position 478 of SEQ ID NO: 1; a phenylalanine to glycine at a position corresponding to position 483 of SEQ ID NO: 1; an aspartic acid to alanine at a position corresponding to position 484 of SEQ ID NO: 1; an isoleucine to glutamic acid at a position corresponding to position 485 of SEQ ID NO: 1; an aspartic acid to glycine at a position corresponding to position 487 of SEQ ID NO: 1; a lysine to asparagine at a position corresponding to position 490 of SEQ ID NO: 1; a leucine to phenylalanine at a position corresponding to position 503 of SEQ ID NO: 1; a valine to threonine at a position corresponding to position 508 of SEQ ID NO: 1; and an isoleucine to threonine at a position corresponding to position 525 of SEQ ID NO: 1. In some embodiments, a PPX protein is a paralog of *Arabidopsis thaliana* PPX protein (for example the PPX protein may be a potato PPX protein) and the PPX protein may have an N at the position corresponding to position 52 of SEQ ID NO: 1, wherein the N is substituted with an amino acid other than an N; a K at the position corresponding to position 272 of SEQ ID NO: 1, wherein the K is substituted with an amino acid other than a K; an S at the position corresponding to position 359 of SEQ ID NO: 1, wherein the S is substituted with an amino acid other than an S; and/or an S at the position corresponding to position 525 of SEQ ID NO: 1, wherein the S is substituted with an amino acid other than an S. In such embodiments, the one or more mutations in a mutated PPX protein includes one or more mutations, two or more mutations, or three or more mutations selected from the group consisting of an asparagine to lysine at a position corresponding to position 52 of SEQ ID NO: 1; an asparagine to aspartic acid at a position corresponding to position 85 of SEQ ID NO: 1; an arginine to cysteine at a position corresponding to position 144 of SEQ ID NO: 1; an arginine to histidine at a position corresponding to position 144 of SEQ ID NO: 1; a phenylalanine to leucine at a position corresponding to position 145 of SEQ ID NO: 1; a phenylalanine to tyrosine at a position corresponding to position 145 of SEQ ID NO: 1; an alanine to threonine at a position corresponding to position 180 of SEQ ID NO: 1; a proline to arginine at a position corresponding to position 185 of SEQ ID NO: 1; a proline to histidine at a position corresponding to position 185 of SEQ ID NO: 1; an alanine to cysteine at a position corresponding to position 220 of SEQ ID NO: 1; an alanine to isoleucine at a position corresponding to position 220 of SEQ ID NO: 1; an alanine to leucine at a position corresponding to position 220 of SEQ ID NO: 1; an alanine to threonine at a position corresponding to position 220 of SEQ ID NO: 1; an alanine to valine at a position corresponding to position 220 of SEQ ID NO: 1; a leucine to methionine at a position corresponding to position 226 of SEQ ID NO: 1; a methionine to leucine at a position corresponding to position 228 of SEQ ID NO: 1; a serine to glycine at a position corresponding to position 244 of SEQ ID NO: 1; a serine to threonine at a position corresponding to position 244 of SEQ ID NO: 1; a lysine to phenylalanine at a position corresponding to position 272 of SEQ ID NO: 1; a serine to leucine at a position corresponding to position 305 of SEQ ID NO: 1; a serine to cysteine at a position corresponding to position 332 of SEQ ID NO: 1; a leucine to isoleucine at a position corresponding to position 357 of SEQ ID NO: 1; a serine to arginine at a position corresponding to position 359 of SEQ ID NO: 1; a serine to threonine at a position corresponding to position 359 of SEQ ID NO: 1; a leucine to methionine at a position corresponding to position 393 of SEQ ID NO: 1; a leucine to serine at a position corresponding to position 393 of SEQ ID NO: 1; a leucine to valine at a position corresponding to position 393 of SEQ ID NO: 1; a leucine to arginine at a position corresponding to position 403 of SEQ ID NO: 1; a leucine to serine at a position corresponding to position 403 of SEQ ID NO: 1; a leucine to serine at a position corresponding to position 424 of SEQ ID NO: 1; a tyrosine to cysteine at a position corresponding to position 426 of SEQ ID NO: 1; a tyrosine to phenylalanine at a position corresponding to position 426 of SEQ ID NO: 1; a tyrosine to histidine at a position corresponding to position 426 of SEQ ID NO: 1; a tyrosine to isoleucine at a position corresponding to position 426 of SEQ ID NO: 1; a tyrosine to leucine at a position corresponding to position 426 of SEQ ID NO: 1; a tyrosine to arginine at a position corresponding to position 426 of SEQ ID NO: 1; a tyrosine to threonine at a position corresponding to position 426 of SEQ ID NO: 1; a tyrosine to valine at a position corresponding to position 426 of SEQ ID NO: 1; a phenylalanine to serine at a position corresponding to position 478 of SEQ ID NO: 1; an isoleucine to threonine at a position corresponding to position 525 of SEQ ID NO: 1; an aspartic acid to asparagine at a position corresponding to position 58 of SEQ ID NO: 9; a glutamic acid to valine at a position corresponding to position 64 of SEQ ID NO: 9; a glycine to cysteine at a position corresponding to position 74 of SEQ ID NO: 9; a glycine to asparagine at a position corresponding to position 84 of SEQ ID NO: 9; a leucine to histidine at a position corresponding to position 93 of SEQ ID NO: 9; a lysine to arginine at a position corresponding to position 97 of SEQ ID NO: 9; an arginine to histidine at a position corresponding to position 98 of SEQ ID NO: 9; an arginine to cysteine at a position corresponding to position 98 of SEQ ID NO: 9; an arginine to leucine at a position corresponding to position 98 of SEQ ID NO:9; an alanine to valine at a position corresponding to position 101 of SEQ ID NO: 9; a serine to asparagine at a position corresponding to position 119 of SEQ ID NO: 9; a phenylalanine to leucine at a position corresponding to position 121 of SEQ ID NO: 9; a threonine to isoleucine at a position corresponding to position 124 of SEQ ID NO: 9; an asparagine to tyrosine at a position corresponding to position 139 of SEQ ID NO: 9; an asparagine to arginine at a position corresponding to position 139 of SEQ ID NO: 9; an asparagine to histidine at a position corresponding to position 139 of SEQ ID NO: 9; a glutamic acid to aspartic acid at a position corresponding to position 150 of SEQ ID NO: 9; a glutamic acid to lysine at a position corresponding to position 150 of SEQ ID NO: 9; a serine to threonine at a position corresponding to position 151 of SEQ ID NO: 9; a glutamine to leucine at a position corresponding to position 157 of SEQ ID NO: 9; a valine to phenylalanine at a position corresponding to position 164 of SEQ ID NO: 9; a valine to alanine at a position corresponding to position 164 of SEQ ID NO: 9; an aspartic acid to glutamic acid at a position corresponding to position 170 of SEQ ID NO: 9; a cysteine to serine at a position corresponding to position 177 of SEQ ID NO: 9; a histidine to glutamine at a position corresponding to position 187 of SEQ ID NO: 9; a leucine to phenylalanine at a position corresponding to position 188 of SEQ ID NO: 9; an asparagine to lysine at a position corresponding to position 195 of SEQ ID NO: 9; a proline to serine at a position corresponding to position 214 of SEQ ID NO: 9; a proline to histidine at a position corresponding to position 214 of SEQ ID NO: 9; an isoleucine to serine at a position corresponding to position 215 of SEQ ID NO: 9; an isoleucine to histidine at a position corresponding to position 215 of SEQ ID NO: 9; a lysine to glutamic acid at a position corresponding to position 229 of SEQ ID NO: 9; a lysine to glutamine at a position corresponding to position 229 of SEQ ID NO: 9; a lysine to arginine at a position corresponding to position 230 of SEQ ID NO: 9; a cysteine to arginine at a position corresponding to position 271 of SEQ ID NO: 9; an aspartic acid to glycine at a position corresponding to position 274 of SEQ ID NO: 9; a phenylalanine to glycine at a position corresponding to position 283 of SEQ ID NO: 9; an alanine to glycine at a position corresponding to position 292 of SEQ ID NO: 9; a serine to leucine at a position corresponding to position 296 of SEQ ID NO: 9; a cysteine to serine at a position corresponding to position 307 of SEQ ID NO: 9; an asparagine to aspartic acid at a position corresponding to position 324 of SEQ ID NO: 9; an asparagine to lysine at a position corresponding to position 324 of SEQ ID NO: 9; an aspartic acid to glutamic acid at a position corresponding to position 330 of SEQ ID NO: 9; a serine to leucine at a position corresponding to position 396 of SEQ ID NO: 9; an alanine to serine at a position corresponding to position 404 of SEQ ID NO: 9; an arginine to lysine at a position corresponding to position 406 of SEQ ID NO: 9; a lysine to isoleucine at a position corresponding to position 410 of SEQ ID NO: 9; a leucine to valine at a position corresponding to position 421 of SEQ ID NO: 9; an alanine to valine at a position corresponding to position 423 of SEQ ID NO: 9; a cysteine to serine at a position corresponding to position 434 of SEQ ID NO: 9; a cysteine to tyrosine at a position corresponding to position 434 of SEQ ID NO: 9; an aspartic acid to glycine at a position corresponding to position 447 of SEQ ID NO: 9; a serine to alanine at a position corresponding to position 448 of SEQ ID NO: 9; a valine to glutamic acid at a position corresponding to position 449 of SEQ ID NO: 9; an aspartic acid to glycine at a position corresponding to position 451 of SEQ ID NO: 9; an aspartic acid to asparagine at a position corresponding to position 454 of SEQ ID NO: 9; a tyrosine to phenylalanine at a position corresponding to position 465 of SEQ ID NO: 9; a lysine to threonine at a position corresponding to position 470 of SEQ ID NO: 9; and a threonine to serine at a position corresponding to position 500 of SEQ ID NO: 9.

In conjunction with any of the aspects, embodiments, methods and/or compositions disclosed herein, In certain embodiments, a mutated PPX protein may include a combination of mutations; for example a combination of mutations selected from Tables 4a and 4b. In some embodiments, the mutated PPX protein includes a combination of two or more mutations; for example, combinations selected from Tables 4a and 4b. In some embodiments, the mutated PPX protein includes a combination of three or more mutations; for example, combinations selected from Tables 4a and 4b. In some embodiments, the combination of mutations in a mutated PPX gene encode a protein having a mutation at a position corresponding to Y426 of SEQ ID NO: 1 and a mutation at one or more amino acid positions corresponding to a position selected from the group consisting of: N85, R144, F145, A180, A220, L226, and S244 of SEQ ID NO: 1. In some embodiments, the combination of mutations in a mutated PPX gene encode a protein having a mutation at a position corresponding to L393 of SEQ ID NO: 1 and a mutation at one or more amino acid positions corresponding to a position selected from the group consisting of: R144, F145, A220, S224 and S244 of SEQ ID NO: 1. In some embodiments, the combination of mutations encode a protein having a mutation at a position corresponding to L403 of SEQ ID NO: 1 and a mutation at one or more amino acid positions corresponding to a position selected from the group consisting of: F145, A220 and L226 of SEQ ID NO: 1. In some embodiments, the combination of mutations encode a protein having a mutation at a position corresponding to R144 of SEQ ID NO: 1 and a mutation at one or more amino acid positions corresponding to a position selected from the group consisting of: G52, N85, A220, S244, L226, M228, K272, S332, L393, L424, Y426 and I525 of SEQ ID NO: 1. In some embodiments, the combination of mutations encode a protein having a mutation at a position corresponding to N85 of SEQ ID NO: 1 and a mutation at one or more amino acid positions corresponding to a position selected from the group consisting of: R144, F145, A180, A220, L226, M228, and Q272 of SEQ ID NO: 1. In some embodiments, the combination of mutations encode a protein having a mutation at a position corresponding to L424 of SEQ ID NO: 1 and a mutation at the amino acid position corresponding to a position selected from the group consisting of: R144, F145, A220, L226 and L393 of SEQ ID NO: 1. In some embodiments, the combination of mutations encode a protein having a mutation at a position corresponding to I525 of SEQ ID NO: 1 and a mutation at the amino acid position corresponding to a position N85, F144, F145, A180, L226 and S244 of SEQ ID NO: 1. In some embodiments, the combination of mutations encode a protein having a mutation at a position corresponding to R144 of SEQ ID NO: 1 and a mutation at the amino acid position corresponding to a position A220 of SEQ ID NO: 1. In some embodiments, a PPX protein is a paralog of *Arabidopsis thaliana* PPX protein (for example the PPX protein may be a potato PPX protein) and the PPX protein may have an N at the position corresponding to position 52 of SEQ ID NO: 1, wherein the N is substituted with an amino acid other than an N; a K at the position corresponding to position 272 of SEQ ID NO: 1, wherein the K is substituted with an amino acid other than a K; an S at the position corresponding to position 359 of SEQ ID NO: 1, wherein the S is substituted with an amino acid other than an S; and/or an S at the position corresponding to position 525 of SEQ ID NO: 1, wherein the S is substituted with an amino acid other than an S. In such embodiments, the mutated PPX protein includes a combination of two or more mutations; for example, combinations selected from Tables 4a and 4b. In such embodiments, the mutated PPX protein includes a combination of three or more mutations; for example, combinations selected from Tables 4a and 4b. In some embodiments, the combination of mutations in a mutated PPX gene encode a protein having a mutation at a position corresponding to Y426 of SEQ ID NO: 1 and a mutation at one or more amino acid positions corresponding to a position selected from the group consisting of: N85, R144, F145, A180, A220, L226, and S244 of SEQ ID NO: 1. In some embodiments, the combination of mutations in a mutated PPX gene encode a protein having a mutation at a position corresponding to L393 of SEQ ID NO: 1 and a mutation at one or more amino acid positions corresponding to a position selected from the group consisting of: R144, F145, A220, S244 and S224 of SEQ ID NO: 1. In some embodiments, the combination of mutations encode a protein having a mutation at a position corresponding to L403 of SEQ ID NO: 1 and a mutation at one or more amino acid positions corresponding to a position selected from the group consisting of: F145, A220 and L226 of SEQ ID NO: 1. In some embodiments, the combination of mutations encode a protein having a mutation at a position corresponding to R144 of SEQ ID NO: 1 and a mutation at one or more amino acid positions corresponding to a position selected from the group consisting of: N52, N85, A220, S244, L226, M228, K272, S332, L393, L424, Y426 and S525 of SEQ ID NO: 1. In some embodiments, the combination of mutations encode a protein having a mutation at a position corresponding to N85 of SEQ ID NO: 1 and a mutation at one or more amino acid positions corresponding to a position selected from the group consisting of: R144, F145, A180, A220, L226, M228, and K272 of SEQ ID NO: 1. In some embodiments, the combination of mutations encode a protein having a mutation at a position corresponding to L424 of SEQ ID NO: 1 and a mutation at the amino acid position corresponding to a position selected from the group consisting of: R144, F145, A220, L226 and L393 of SEQ ID NO: 1. In some embodiments, the combination of mutations encode a protein having a mutation at a position corresponding to S525 of SEQ ID NO: 1 and a mutation at the amino acid position corresponding to a position N85, F144, F145, A180, L226 and S244 of SEQ ID NO: 1. In some embodiments, the combination of mutations encode a protein having a mutation at a position corresponding to 98 of SEQ ID NO: 9 and a mutation at the amino acid position corresponding to a position selected from the group consisting of 74, 93, 97, 98, 119, 121, 124, 139, 150, 151, 164, 188, 214, 229, 230, 271, 274, 292, 307, 324, 396, 410, 423, 434, 447, 448, 451, 465, 470 and 500 of SEQ ID NO: 9. In certain embodiments, the combination of mutations encode a protein having a mutation at a position corresponding to 98 of SEQ ID NO: 9 and a mutation at the amino acid position corresponding to a position selected from the group consisting of 271, 274, 292, 307, 324, 330, 396, 404, 406, 410, 423, 434, 447, 448, 454, 465, 470 and 500 of SEQ ID NO: 9. In certain embodiments, the combination of mutations encode a protein having a mutation at a position corresponding to 98 of SEQ ID NO: 9 and a mutation at the amino acid position corresponding to a position selected from the group consisting of 307 and 423 of SEQ ID NO: 9. In certain embodiments, the combination of mutations encode a protein having a mutation at a position corresponding to 98 of SEQ ID NO: 9 and a mutation at the amino acid position corresponding to a position selected from the group consisting 124, 188, 214 and 229 of SEQ ID NO: 9.

In conjunction with any of the aspects, embodiments, methods and/or compositions disclosed herein the PPX protein may be a paralog of *Arabidopsis thaliana* PPX protein (for example the PPX protein may be a potato mitochondrial PPX protein) and the PPX protein may have one or more corresponding PPX amino acids to SEQ ID NO: 9. In conjunction with any of the aspects, embodiments, methods and/or compositions disclosed herein, the one or more mutations in a mutated PPX gene may encode a mutated PPX protein having one or more mutations, two or more mutations, three or more mutations selected from the group consisting of a mutated PPX protein may include one or more mutation at the amino acid position corresponding to one or more positions selected from the group consisting of positions 58, 64, 74, 84, 93, 97, 98, 101, 119, 121, 124, 139, 150 151, 157, 164, 170, 177, 187, 188, 195, 214, 215, 229, 230, 271, 274, 278, 283, 292, 296, 307, 324, 330, 396, 404, 406, 410, 421, 423, 434, 447, 448, 449, 451, 454, 465, 470 and 500 of SEQ ID NO: 9.

In conjunction with any of the aspects, embodiments, methods and/or compositions disclosed herein, the plant cell may have a mutated PPX gene. In certain embodiments, the mutated PPX gene encodes a mutated PPX protein. In certain embodiments, the plant cell may be part of a herbicide-resistant plant. The method may include introducing into a plant cell a gene repair oligonucleobase (GRON); for example, using a GRON with a targeted mutation in a PPX gene. In certain embodiments, the plant cell produced by the method may include a PPX gene capable of expressing a mutated PPX protein. The method may further include identifying a plant cell or a plant including a plant cell that includes (1) a mutated PPX gene and/or (2) normal growth and/or catalytic activity as compared to a corresponding wild-type plant cell. The herbicide-resistant plant having a plant cell such as described herein may be identified in the presence of a PPX-inhibiting herbicide. In some embodiments, the plant cell is non-transgenic. In some embodiments, the plant cell is transgenic. A plant that includes a plant cell such as described herein may be a non-transgenic or transgenic herbicide-resistant plant; for example, the plant and/or plant cell may have a mutated PPX gene that results in resistance to at least one herbicide. In some embodiments, a plant having a plant cell as described herein may be produced asexually; for example, from one or more plant cells or from plant tissue made up of one or more plant cells; for example, from a tuber. In other embodiments, a plant having a plant cell such as described herein may be produced sexually.

In another aspect, there is provided a method for producing a herbicide-resistant plant. The method may include introducing into a plant cell a gene repair oligonucleobase (GRON); for example, using a GRON designed with a targeted mutation in a PPX gene. The mutated PPX gene may express a mutated PPX protein. The method may further include identifying a plant that has normal growth and/or catalytic activity as compared to a corresponding wild-type plant cell. The plant may be identified in the presence of a PPX-inhibiting herbicide. In some embodiments, the plant is non-transgenic. The plant may in some embodiments be a non-transgenic herbicide-resistant plant; for example, the plant may include a mutated PPX gene that results in resistance or tolerance to at least one herbicide.

In another aspect there is provided a seed including a mutated PPX gene. In some embodiments, the seed has a mutated PPX gene. In some embodiments, the mutated PPX encodes a mutated PPX protein. In some embodiments the mutated PPX protein may be resistant to a herbicide; for example, a PPX-inhibiting herbicide. In some embodiments, a plant grown from the seed is resistant to at least one herbicide; for example, a PPX-inhibiting herbicide.

In another aspect, there is provided a method for increasing the herbicide-resistance of a plant by: (a) crossing a first plant to a second plant, in which the first plant that includes a mutated PPX gene, in which the gene encodes a mutated PPX protein; (b) screening a population resulting from the cross for increased herbicide-resistance; for example, increased resistance to a PPX-inhibiting herbicide (c) selecting a member resulting from the cross having increased herbicide-resistance; and/or (d) producing seeds resulting from the cross. In some embodiments, a hybrid seed is produced by any of the methods such as described herein. In some embodiments, plants are grown from seeds produced by any of the methods such as described herein. In some embodiments, the plants and/or seeds are non-transgenic. In some embodiments, the plants and/or seeds are transgenic.

In another aspect, there is provided a method of controlling weeds in a field containing plants by applying an effective amount of at least one herbicide to a field containing weeds and plants. In some embodiments of the method, the at least one herbicide is a PPX-inhibiting herbicide. In some embodiments of the method, one or more of the plants in the field includes a mutated PPX gene; for example such as described herein. In some embodiments of the method one or more of the plants in the field includes a non-transgenic or transgenic plant having a mutated PPX gene such as described herein. In some embodiments, the mutated PPX gene encodes a mutated PPX protein. In some embodiments, one more of the plants in the field is herbicide resistant; for example, resistant to a PPX-inhibiting herbicide.

In another aspect, there is provided an isolated nucleic acid encoding a PPX protein or portion thereof. In some embodiments the isolated nucleic acid includes one or more of the PPX gene mutations such as described herein. In some embodiments, the isolated nucleic acid encodes a mutated PPX protein as disclosed herein. In certain embodiments, the isolated nucleic acid encodes a PPX protein that is herbicide resistant; for example, resistant to a PPX-inhibiting herbicide.

In another aspect, there is provided an expression vector containing an isolated nucleic acid of a mutated PPX gene. In some embodiments, the expression vector contains an isolated nucleic acid encoding a PPX protein. In some embodiments, the isolated nucleic acid encodes a protein having a mutation selected from the mutations shown in Tables 1, 2, 3a, 3b, 4a, 4b, 8a-f, 9a-d and 10. In certain embodiments, the isolated nucleic acid encodes a protein having two or more mutations. In some embodiments, the two or more mutations are selected from Tables 1, 2, 3a, 3b, 4a, 4b, 8a-f, 9a-d and 10. In certain embodiments, the isolated nucleic acid encodes a PPX protein that is herbicide resistant; for example, resistant to a PPX-inhibiting herbicide.

As used herein, the term "herbicide" refers to any chemical or substance that can kill a plant or that can halt or reduce growth and/or viability of a plant. In some embodiments, herbicide resistance is the genetically heritable ability of a plant to survive and reproduce following treatment with a concentration of herbicide that would normally kill or severely injure an unmodified wildtype plant. In some embodiments, in conjunction with any of the aspects, embodiments, methods and/or compositions disclosed herein, the herbicide is a PPX-inhibiting herbicide. In some embodiments, a PPX-inhibiting herbicide is a herbicide from a chemical family selected from the group of chemical families listed in Table 5. In some embodiments, a PPX-inhibiting herbicide is a herbicide from a chemical family selected from the group of chemical families consisting of N-phenylphthalimides, triazolinones, and pyrimidindiones. In some embodiments, a PPX-inhibiting herbicide is selected from the group of herbicides listed in Table 5. In some embodiments, PPX-inhibiting herbicide is selected from the group of herbicides consisting of flumoioxazin, sulfentrazone, and saflufenacil. In other embodiments, the PPX-inhibiting herbicide is a flumioxazin herbicide. In other embodiments, the PPX-inhibiting herbicide is a sulfentrazone herbicide. In other embodiments, the PPX-inhibiting herbicide is a saflufenacil herbicide.

In conjunction with any of the aspects, embodiments, methods and/or compositions disclosed herein, the plant or plant cell is from a plant crop selected from the group consisting of potato, sunflower, sugar beet, maize, cotton, soybean, wheat, rye, oats, rice, canola, fruits, vegetables, tobacco, barley, sorghum, tomato, mango, peach, apple, pear, strawberry, banana, melon, carrot, lettuce, onion, soya spp, sugar cane, pea, field beans, poplar, grape, citrus, alfalfa, rye, oats, turf and forage grasses, flax, oilseed rape, cucumber, morning glory, balsam, pepper, eggplant, marigold, lotus, cabbage, daisy, carnation, petunia, tulip, iris, lily, and nut-producing plants insofar as they are not already specifically mentioned. In some embodiments, the plant or plant cell is of a species selected from Table 6. In some embodiments, the plant or plant cell is of a species selected from the group consisting of *Arabidopsis thaliana, Solanum tuberosum, Solanum phureja*, *Oryza sativa, Amaranthus tuberculatus, Zea mays, Brassica napus,* and *Glycine max.* In some embodiments, the plant or plant cell is a Russet Burbank potato cultivar. In some embodiments, a mutated PPX gene encodes a Russet Burbank PPX protein. In some embodiments, a mutated PPX gene encodes an *Arabidopsis thaliana* PPX protein. In some embodiments, a mutated PPX gene encodes a *Solanum tuberosum* PPX protein. In some embodiments, a mutated PPX gene encodes a *Solanum phureja* PPX protein. In some embodiments, a mutated PPX gene encodes a *Zea mays* PPX protein. In some embodiments, a mutated PPX gene encodes an *Oryza sativa* PPX protein. In some embodiments, a mutated PPX gene encodes an *Amaranthus tuberculatus* PPX protein. In some embodiments, a mutated PPX gene encodes a *Sorghum bicolor* PPX protein. In some embodiments, a mutated PPX gene encodes a *Ricinus communis* PPX protein. In some embodiments, a mutated PPX gene encodes a *Brassica napus* PPX protein. In some embodiments, a mutated PPX gene encodes a *Glycine max* PPX protein. In some embodiments, a mutated PPX gene At4g01690 encodes an *Arabidopsis thaliana* PPX protein. In some embodiments a mutated PPX gene At5g14220 encodes an *Arabidopsis thaliana* PPX protein.

In any of the aspects, embodiments, methods or compositions disclosed herein may include one or more mutated PPX genes. In some embodiments, the methods and compositions involve one or more mutated PPX genes that encode one or more mitochondrial PPX proteins. In other embodiments, the methods and compositions include one or more mutated PPX genes which encode one or more plastidal PPX proteins. In some embodiments, the methods and compositions include one or more mutated PPX genes which encode one or more plastidal PPX proteins and mitochondrial PPX proteins. In some embodiments, the methods and compositions include a mitochondrial mutated PPX gene StmPPX1. In some embodiments, the methods and compositions include a mitochondrial mutated PPX gene StmPPX2. In some embodiments, the plant has the plastidal mutated PPX gene StcPPX1. In some embodiments, the methods and compositions include a mitochondrial mutated PPX gene allele StcPPX2.1. In some embodiments, the methods and compositions include a mitochondrial mutated PPX gene allele StcPPX2.2. In some embodiments, the methods and compositions include a plastidal mutated PPX gene allele StcPPX1. In some embodiments, the methods and compositions include a plastidal mutated PPX gene allele StcPPX1.1.

As used herein, the term "gene" refers to a DNA sequence that includes control and coding sequences necessary for the production of an RNA, which may have a non-coding function (*e.g.,* a ribosomal or transfer RNA) or which may encode a polypeptide or a polypeptide precursor. The RNA or polypeptide may be encoded by a full length coding sequence or by any portion of the coding sequence so long as the desired activity or function is retained.

As used herein, the term "coding sequence" refers to a sequence of a nucleic acid or its complement, or a part thereof, that can be transcribed and/or translated to produce the mRNA for and/or the polypeptide or a fragment thereof. Coding sequences include exons in a genomic DNA or immature primary RNA transcripts, which are joined together by the cell's biochemical machinery to provide a mature mRNA. The anti-sense strand is the complement of such a nucleic acid, and the encoding sequence can be deduced therefrom.

As used herein, the term "non-coding sequence" refers to a sequence of a nucleic acid or its complement, or a part thereof, that is not transcribed into amino acid *in vivo,* or where tRNA does not interact to place or attempt to place an amino acid. Non-coding sequences include both intron sequences in genomic DNA or immature primary RNA transcripts, and gene-associated sequences such as promoters, enhancers, silencers, *etc.*

A nucleobase is a base, which in certain preferred embodiments is a purine, pyrimidine, or a derivative or analog thereof. Nucleosides are nucleobases that contain a pentosefuranosyl moiety, e.g., an optionally substituted riboside or 2'-deoxyriboside. The moiety may be any group that increases DNA binding and/or decreases nuclease degradation as compared to a nucleoside not having the moiety. Nucleosides can be linked by one of several linkage moieties, which may or may not contain phosphorus. Nucleosides that are linked by unsubstituted phosphodiester linkages are termed nucleotides. As used herein, the term "nucleobase" includes peptide nucleobases, the subunits of peptide nucleic acids, and morpholine nucleobases as well as nucleosides and nucleotides.

An oligonucleobase is a polymer comprising nucleobases; preferably at least a portion of which can hybridize by Watson-Crick base pairing to a DNA having the complementary sequence. An oligonucleobase chain may have a single 5' and 3' terminus, which are the ultimate nucleobases of the polymer. A particular oligonucleobase chain can contain nucleobases of all types. An oligonucleobase compound is a compound comprising one or more oligonucleobase chains that may be complementary and hybridized by Watson-Crick base pairing. Ribo-type nucleobases include pentosefuranosyl containing nucleobases wherein the 2' carbon is a methylene substituted with a hydroxyl, alkyloxy or halogen. Deoxyribo-type nucleobases are nucleobases other than ribo-type nucleobases and include all nucleobases that do not contain a pentosefuranosyl moiety.

In conjunction with any of the aspects, embodiments, methods and/or compositions disclosed herein, an oligonucleobase strand may include both oligonucleobase chains and segments or regions of oligonucleobase chains. An oligonucleobase strand may have a 5' end and a 3' end, and when an oligonucleobase strand is coextensive with a chain, the 5' and 3' ends of the strand are also 5' and 3' termini of the chain.

As used herein, the term "gene repair oligonucleobase" or "GRON" refers to oligonucleobases, including mixed duplex oligonucleotides, non-nucleotide containing molecules, single stranded oligodeoxynucleotides and other gene repair molecules.

As used herein, the term "transgenic" refers to an organism or cell that has DNA derived from another organism inserted into its genome. For example, in some embodiments, a transgenic organism or cell includes inserted DNA that includes a foreign promoter and/or coding region.

As used herein, the term "non-transgenic" refers to an organism or cell that does not have DNA derived from another organism inserted into its genome although a non-transgenic plant or cell may have one or more artificially introduced targeted mutations.

As used herein, the term "isolated", when referring to a nucleic acid (e.g., an oligonucleotide such as RNA, DNA, or a mixed polymer) refers to a nucleic acid that is apart from a substantial portion of the genome in which it naturally occurs and/or is substantially separated from other cellular components which naturally accompany such nucleic acid. For example, any nucleic acid that has been produced synthetically (e.g., by serial base condensation) is considered to be isolated. Likewise, nucleic acids that are recombinantly expressed, cloned, produced by a primer extension reaction (e.g., PCR), or otherwise excised from a genome are also considered to be isolated.

As used herein, the term "amino acid sequence" refers to a polypeptide or protein sequence. The convention "AAwt###AAmut" is used to indicate a mutation that results in the wild-type amino acid AAwt at position ### in the polypeptide being replaced with mutant AAmut.

As used herein, the term "complement" refers to the complementary sequence to a nucleic acid according to standard Watson/Crick pairing rules. A complement sequence can also be a sequence of RNA complementary to the DNA sequence or its complementary sequence, and can also be a cDNA.

As used herein, the term "substantially complementary" refers to two sequences that hybridize under near stringent hybridization conditions. The skilled artisan will understand that substantially complementary sequences need not hybridize along their entire length.

As used herein the term "codon" refers to a sequence of three adjacent nucleotides (either RNA or DNA) constituting the genetic code that determines the insertion of a specific amino acid in a polypeptide chain during protein synthesis or the signal to stop protein synthesis. The term "codon" is also used to refer to the corresponding (and complementary) sequences of three nucleotides in the messenger RNA into which the original DNA is transcribed.

As used herein, the term "homology" refers to sequence similarity among proteins and DNA. The term "homology" or "homologous" refers to a degree of identity. There may be partial homology or complete homology. A partially homologous sequence is one that has less than 100% sequence identity when compared to another sequence.

As used herein, the term "about" in quantitative terms refers to plus or minus 10%. For example, "about 3%" would encompass 2.7-3.3% and "about 10%" would encompass 9-11%. Moreover, where "about" is used herein in conjunction with a quantitative term it is understood that in addition to the value plus or minus 10%, the exact value of the quantitative term is also contemplated and described. For example, the term "about 3%" expressly contemplates, describes and includes exactly 3%.

### BRIEF DESCRIPTION OF THE FIGURES

Figure 1 is the amino acid sequence of an *Arabidopsis thaliana* chloroplast (plastid) PPX protein (SEQ ID NO: 1).
Figure 2 is the nucleic acid sequence of an *Arabidopsis thaliana* chloroplast (plastid) PPX cDNA (SEQ ID NO: 2).
Figure 3 is the amino acid sequence of *Arabidopsis thaliana* mitochondrial PPX protein (SEQ ID NO: 3).
Figure 4 is the nucleic acid sequence of an *Arabidopsis thaliana* mitochondrial PPX cDNA (SEQ ID NO: 4).
Figure 5 is the amino acid sequence of *Amaranthus tuberculatus* mitochondrial PPX protein (SEQ ID NO: 5).
Figure 6 is the nucleic acid sequence of *Amaranthus tuberculatus* mitochondrial PPX cDNA (SEQ ID NO: 6).
Figure 7 is the amino acid sequence of *Solanum tuberosum* plastidal PPX protein StcPPX (SEQ ID NO: 7).
Figure 8 is the nucleic acid sequence of *Solanum tuberosum* plastidal PPX cDNA (SEQ ID NO: 8).
Figure 9 is the amino acid sequence of *Solanum tuberosum* mitochondrial PPX protein (SEQ ID NO: 9).
Figure 10 is the nucleic acid sequence of *Solanum tuberosum* mitochondrial PPX cDNA (SEQ ID NO: 10).
Figure 11 is the amino acid sequence of *Zea mays* plastidal PPX protein (SEQ ID NO: 11).
Figure 12 is the nucleic acid sequence of *Zea mays* plastidal PPX cDNA (SEQ ID NO: 12).
Figure 13 is the amino acid sequence of *Zea mays* mitochondrial PPX protein (SEQ ID NO: 13).
Figure 14 is the nucleic acid sequence of *Zea mays* mitochondrial PPX cDNA (SEQ ID NO: 14).
Figure 15 is the amino acid sequence of *Oryza sativa* plastidal PPX protein (SEQ ID NO: 15).
Figure 16 is the nucleic acid sequence of *Oryza sativa* plastidal PPX cDNA (SEQ ID NO: 16).
Figure 17 is the amino acid sequence of *Oryza sativa* mitochondrial PPX protein cDNA (SEQ ID NO: 17).
Figure 18 is the nucleic acid sequence of *Oryza sativa* mitochondrial PPX cDNA (SEQ ID NO: 18).
Figure 19 is the amino acid sequence of *Sorghum bicolor* plastidal PPX protein (SEQ ID NO: 19).
Figure 20 is the nucleic acid sequence of *Sorghum bicolor* plastidal PPX cDNA (SEQ ID NO: 20).
Figure 21 is the amino acid sequence of *Sorghum bicolor* mitochondrial PPX protein (SEQ ID NO: 21).
Figure 22 is the nucleic acid sequence of *Sorghum bicolor* mitochondrial PPX cDNA (SEQ ID NO: 22).
Figure 23 is the amino acid sequence of *Ricinus communis* plastidal PPX protein (SEQ ID NO: 23).
Figure 24 is the nucleic acid sequence of *Ricinus communis* plastidal PPX cDNA (SEQ ID NO: 24).
Figure 25 is the amino acid sequence of *Ricinus communis* mitochondrial PPX protein (SEQ ID NO: 25).
Figure 26 is the nucleic acid sequence of *Ricinus communis* mitochondrial PPX cDNA (SEQ ID NO: 26).
Figure 27 is the amino acid sequence of *Solanum tuberosum* mitochondrial PPX protein StmPPX1 (SEQ ID NO: 27).
Figure 28 is the nucleic acid sequence of *Solanum tuberosum* mitochondrial PPX cDNA StmPPX1 (SEQ ID NO: 28).
Figure 29 is the amino acid sequence of *Solanum tuberosum* mitochondrial PPX protein StmPPX2.1 (SEQ ID NO: 29).
Figure 30 is the nucleic acid sequence of *Solanum tuberosum* mitochondrial PPX cDNA StmPPX2.1 (SEQ ID NO: 30).
Figure 31 is the amino acid sequence of *Solanum tuberosum* mitochondrial PPX protein StmPPX2.2 (SEQ ID NO: 31).
Figure 32 is the nucleic acid sequence of *Solanum tuberosum* mitochondrial PPX cDNAStmPPX2.2 (SEQ ID NO: 32).
Figure 33 is the amino acid sequence of *Brassica napus* plastidal PPX protein BncPPX1 (SEQ ID NO: 33).
Figure 34 is the nucleic acid sequence of *Brassica napus* PPX cDNA BncPPX1 (SEQ ID NO: 34).
Figure 35 is the amino acid sequence of *Brassica napus* plastidal PPX protein BncPPX2 (SEQ ID NO: 35).
Figure 36 is the nucleic acid sequence of *Brassica napus* PPX cDNA BncPPX2 (SEQ ID NO: 36).
Figure 37 is the partial amino acid sequence of *Brassica napus* plastidal PPX protein BncPPX3 (SEQ ID NO: 37).
Figure 38 is the partial nucleic acid sequence of *Brassica napus* PPX cDNA BncPPX3 (SEQ ID NO: 38).
Figure 39 is the amino acid sequence of *Glycine max* plastidal PPX protein GmcPPX1-1 (SEQ ID NO: 39).
Figure 40 is the amino acid sequence of *Glycine max* plastidal PPX protein GmcPPX1-2 (SEQ ID NO: 40).
Figure 41 is the nucleic acid sequence of *Glycine max* plastidal PPX protein GmcPPX1 (SEQ ID NO: 41).
Figure 42 is the amino acid sequence of *Glycine max* plastidal PPX protein GmcPPX2 (SEQ ID NO: 42).
Figure 43 is the nucleic acid sequence of *Glycine max* plastidal PPX protein GmcPPX2 (SEQ ID NO: 43).
Figure 44 is the amino acid sequence of *Glycine max* mitochondrial PPX protein GmcPPX (SEQ ID NO: 44).

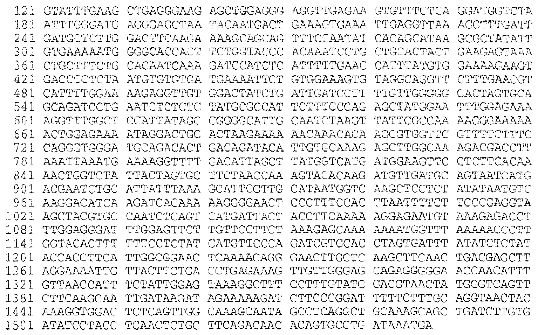

Figure 45 is the nucleic acid sequence of *Glycine max* mitochondrial PPX protein GmcPPX (SEQ ID NO: 45).

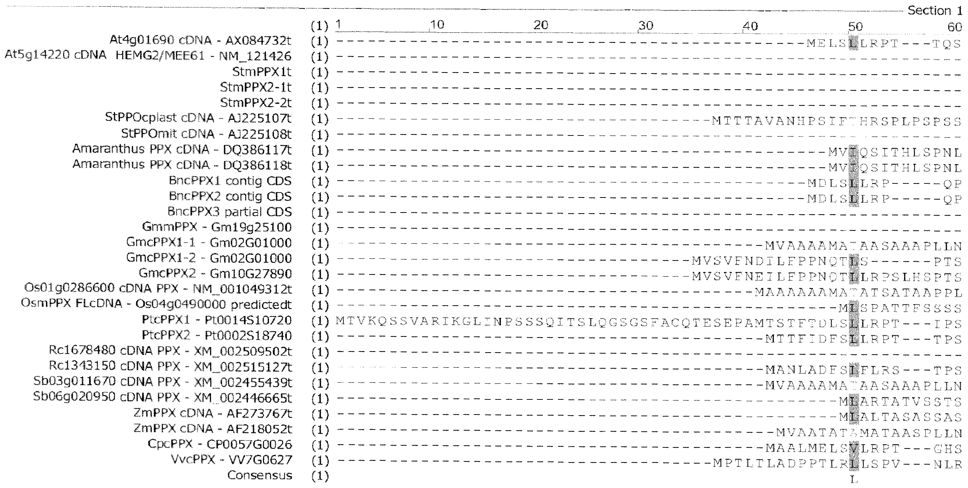

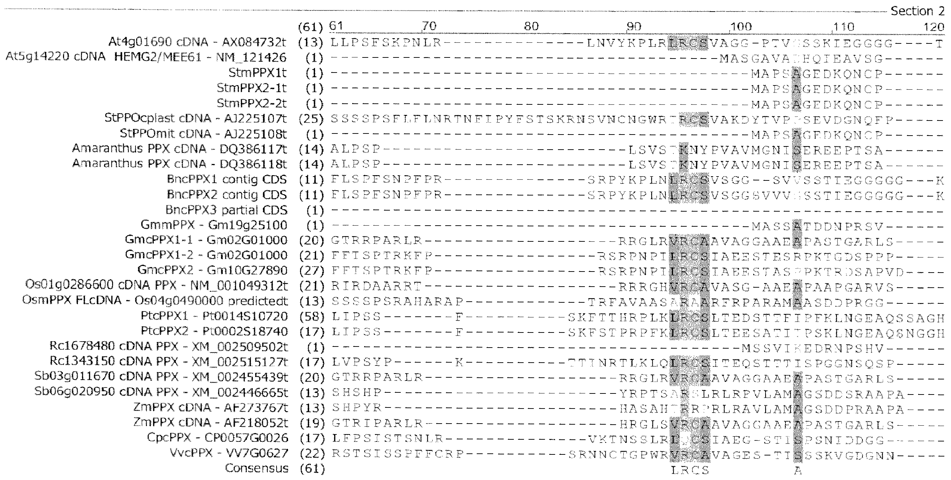

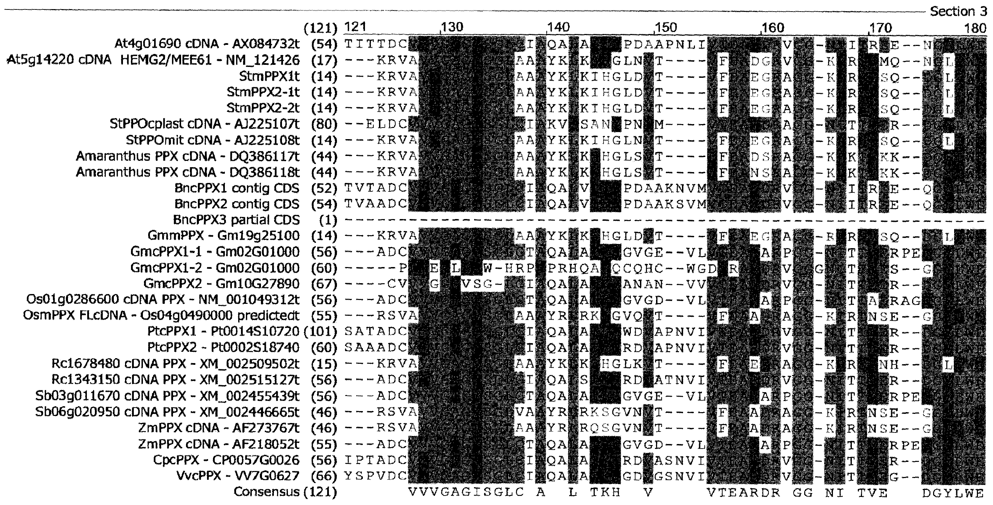

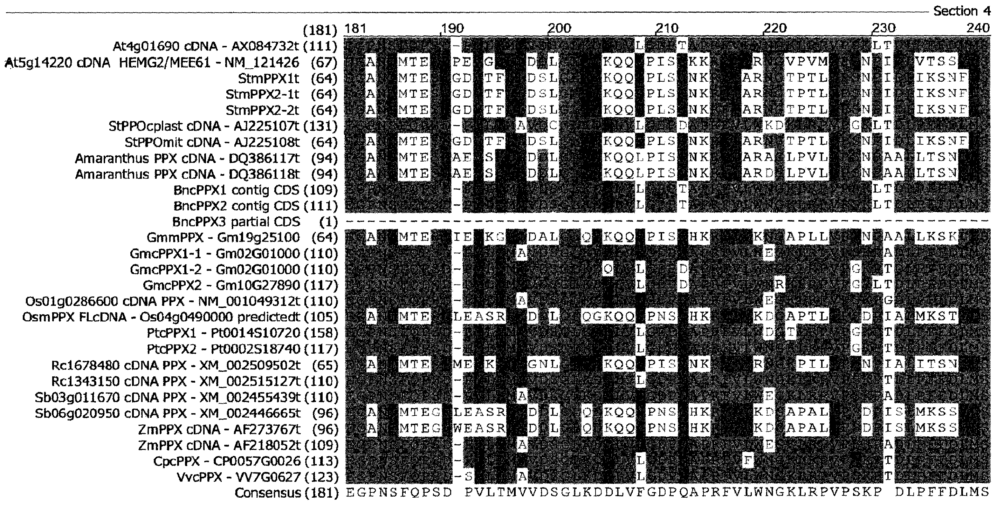

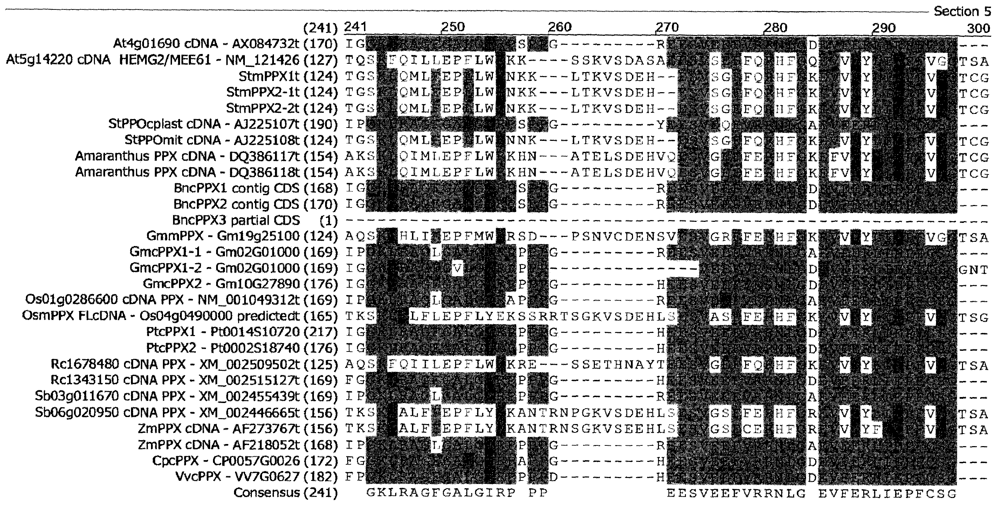

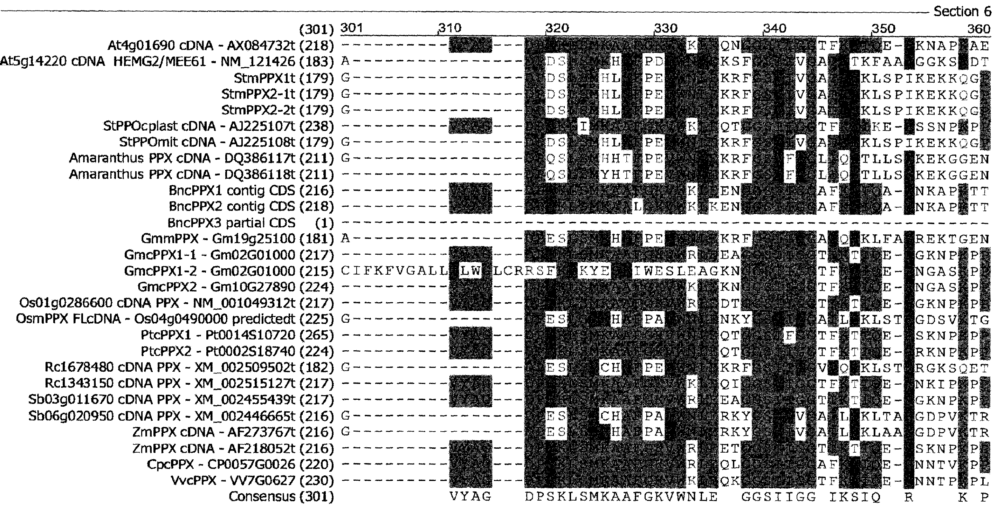

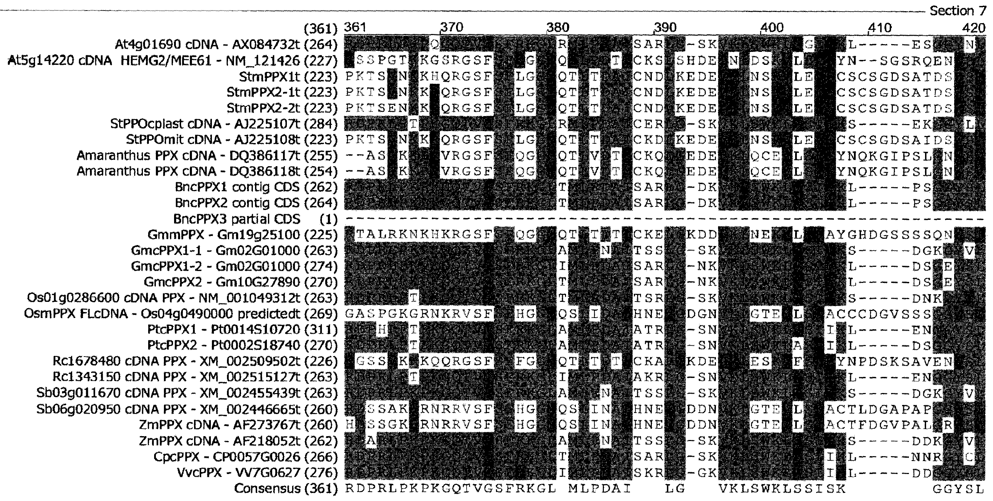

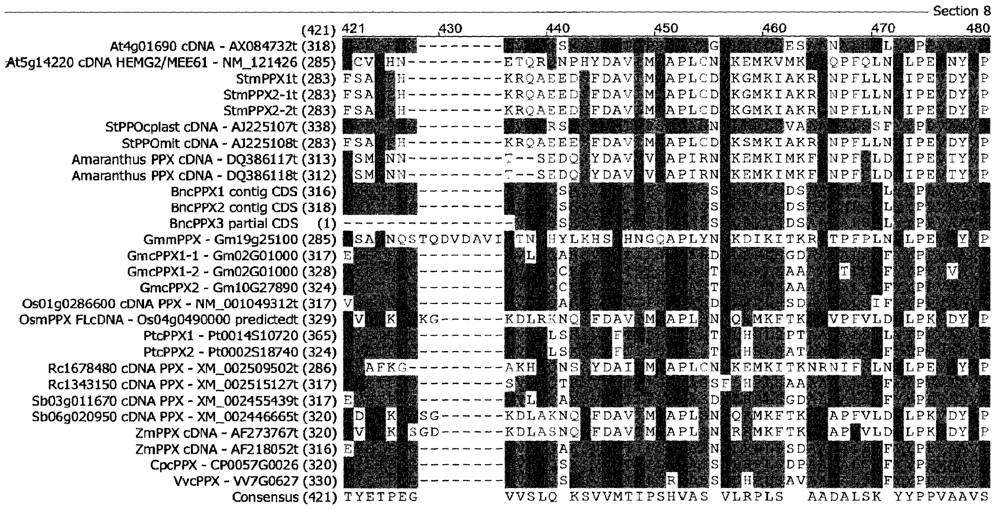

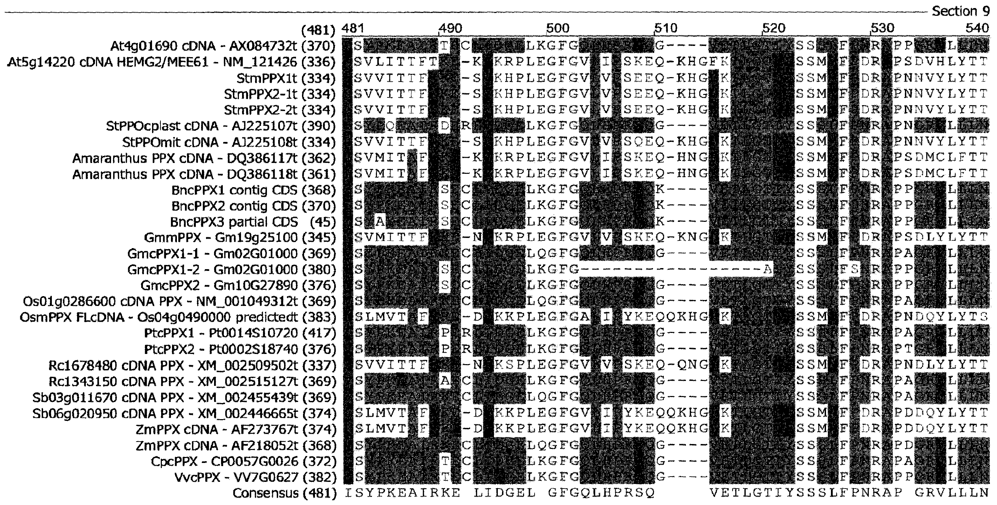

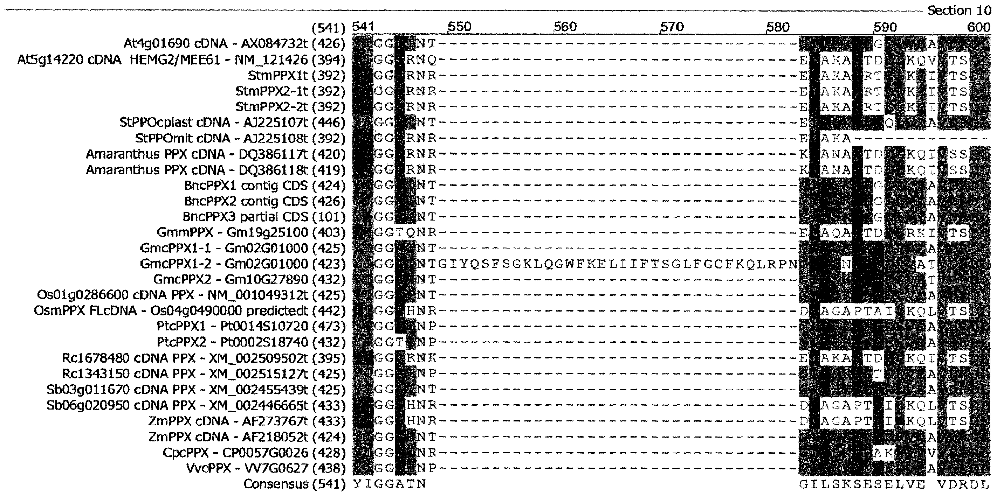

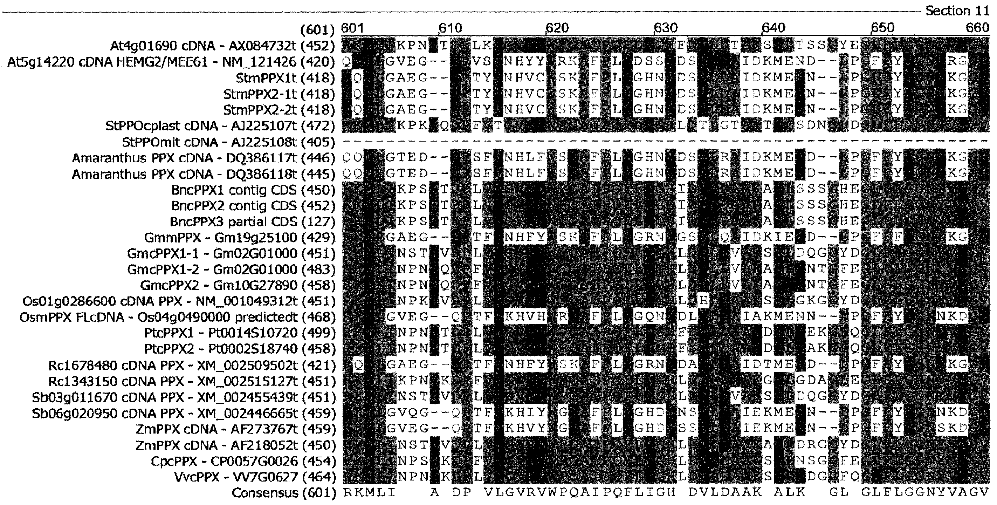

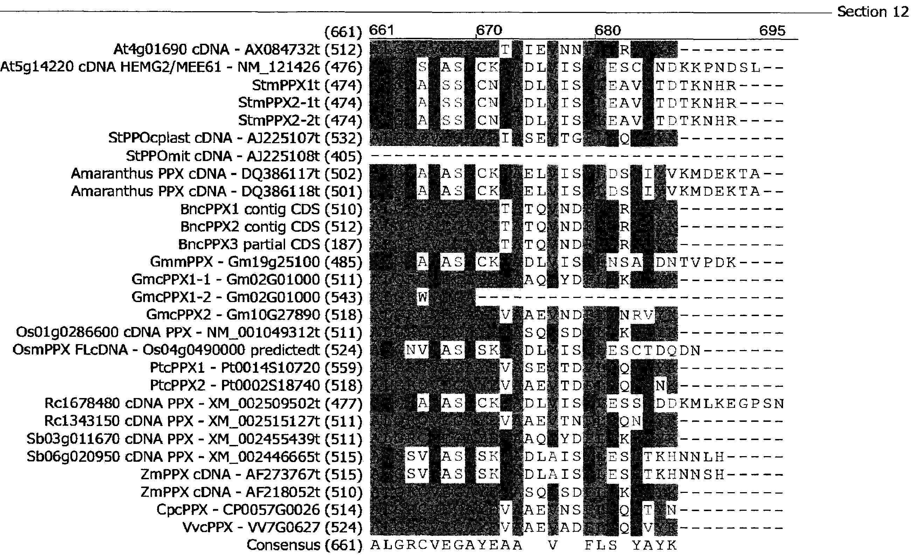

Figure 46 is an alignment of PPX proteins of various plant species.
Figure 47 is a table of homologous amino acid positions in plant PPX amino acid sequences of various species.
Figure 48 is a table of homologous amino acid positions in plant PPX amino acid sequences of various species.

### DETAILED DESCRIPTION OF THE INVENTION

### Rapid Trait Development System (RTDS^{™})

In any of the various aspects and embodiments of the compositions and methods disclosed herein, mutations in genes and proteins may be made using, for example, the Rapid Trait Development System (***RTDS***^{™}) technology developed by Cibus. In combination or alone, plants containing any of the mutations disclosed herein can form the basis of new herbicide-resistant products. Also provided are seeds produced from the mutated plants in which the PPX genes are either homozygous or heterozygous for the mutations. The mutations disclosed herein can be in combination with any other mutation known or with mutations discovered in the future.

As used herein, the term "heterozygous" refers to having different alleles at one or more genetic loci in homologous chromosome segments. As used herein "heterozygous" may also refer to a sample, a cell, a cell population or an organism in which different alleles at one or more genetic loci may be detected. Heterozygous samples may also be determined via methods known in the art such as, for example, nucleic acid sequencing. For example, if a sequencing electropherogram shows two peaks at a single locus and both peaks are roughly the same size, the sample may be characterized as heterozygous. Or, if one peak is smaller than another, but is at least about 25% the size of the larger peak, the sample may be characterized as heterozygous. In some embodiments, the smaller peak is at least about 15% of the larger peak. In other embodiments, the smaller peak is at least about 10% of the larger peak. In other embodiments, the smaller peak is at least about 5% of the larger peak. In other embodiments, a minimal amount of the smaller peak is detected.

As used herein, "homozygous" refers to having identical alleles at one or more genetic loci in homologous chromosome segments. "Homozygous" may also refer to a sample, a cell, a cell population or an organism in which the same alleles at one or more genetic loci may be detected. Homozygous samples may be determined via methods known in the art, such as, for example, nucleic acid sequencing. For example, if a sequencing electropherogram shows a single peak at a particular locus, the sample may be termed "homozygous" with respect to that locus.

The term "hemizygous" refers to a gene or gene segment being present only once in the genotype of a cell or an organism because the second allele is deleted. As used herein "hemizygous" may also refer to a sample, a cell, a cell population or an organism in which an allele at one or more genetic loci may be detected only once in the genotype.

In some embodiments, *RTDS* is based on altering a targeted gene by utilizing the cell's own gene repair system to specifically modify the gene sequence in situ and not insert foreign DNA and/or gene expression control sequences. This procedure may effect a precise change in the genetic sequence while the rest of the genome is left unaltered. In contrast to conventional transgenic GMOs, there is no integration of foreign genetic material, nor is any foreign genetic material left in the plant. In many embodiments, the changes in the genetic sequence introduced by *RTDS* are not randomly inserted. Since affected genes remain in their native location, no random, uncontrolled or adverse pattern of expression occurs.

The *RTDS* that effects this change is a chemically synthesized oligonucleotide (e.g., using a gene repair oligonucleobase (GRON)) which may be composed of both DNA and modified RNA bases as well as other chemical moieties, and is designed to hybridize at the targeted gene location to create a mismatched base-pair(s). This mismatched base-pair acts as a signal to attract the cell's own natural gene repair system to that site and correct (replace, insert or delete) the designated nucleotide(s) within the gene. Once the correction process is complete the *RTDS* molecule is degraded and the now-modified or repaired gene is expressed under that gene's normal endogenous control mechanisms.

### Gene Repair Oligonucleobases ("GRON")

The methods and compositions disclosed herein can be practiced or made with "gene repair oligonucleobases" for example, having the conformations and chemistries as described in detail below. The "gene repair oligonucleobases" as contemplated herein have also been described in published scientific and patent literature using other names including "recombinagenic oligonucleobases;" "RNA/DNA chimeric oligonucleotides;" "chimeric oligonucleotides;" "mixed duplex oligonucleotides" (MDONs); "RNA DNA oligonucleotides (RDOs);" "gene targeting oligonucleotides;" "genoplasts;" "single stranded modified oligonucleotides;" "Single stranded oligodeoxynucleotide mutational vectors" (SSOMVs); "duplex mutational vectors;" and "heteroduplex mutational vectors."

Oligonucleobases having the conformations and chemistries described in U.S. Pat. No. 5,565,350 by Kmiec (Kmiec I) and U.S. Pat. No. 5,731,181 by Kmiec (Kmiec II), hereby incorporated by reference, are suitable for use as "gene repair oligonucleobases" of the present disclosure. The gene repair oligonucleobases in Kmiec I and/or Kmiec II contain two complementary strands, one of which contains at least one segment of RNA-type nucleotides (an "RNA segment") that are base paired to DNA-type nucleotides of the other strand.

Kmiec II discloses that purine and pyrimidine base-containing non-nucleotides can be substituted for nucleotides. Additional gene repair molecules that can be used for the present disclosure include, but are not limited to, those described in U.S. Pat. Nos. 5,756,325; 5,871,984; 5,760,012; 5,888,983; 5,795,972; 5,780,296; 5,945,339; 6,004,804; and 6,010,907 and in International Patent No. PCT/US00/23457; and in International Patent Publication Nos. WO 98/49350; WO 99/07865; WO 99/58723; WO 99/58702; and WO 99/40789, which are each hereby incorporated in their entirety.

In conjunction with any of the aspects, embodiments, methods and/or compositions disclosed herein, the gene repair oligonucleobase may be a mixed duplex oligonucleotides (MDON) in which the RNA-type nucleotides of the mixed duplex oligonucleotide are made RNase resistant by replacing the 2'-hydroxyl with a fluoro, chloro or bromo functionality or by placing a substituent on the 2'-O. Suitable substituents include the substituents taught by the Kmiec II. Alternative substituents may include, but are not limited to the substituents taught by U.S. Pat. No. 5,334,711 (Sproat) and the substituents taught by patent publications EP 629 387 and EP 679 657 (collectively, the Martin Applications), which are hereby incorporated by reference. As used herein, a 2'-fluoro, chloro or bromo derivative of a ribonucleotide or a ribonucleotide having a 2'-OH substituted with a substituent described in the Martin Applications or Sproat is termed a "2'-Substituted Ribonucleotide." As used herein the term "RNA-type nucleotide" means a 2'-hydroxyl or 2'-Substituted Nucleotide that is linked to other nucleotides of a mixed duplex oligonucleotide by an unsubstituted phosphodiester linkage or any of the non-natural linkages taught by Kmiec I or Kmiec II. As used herein the term "deoxyribo-type nucleotide" means a nucleotide having a 2'-H, which can be linked to other nucleotides of a gene repair oligonucleobase by an unsubstituted phosphodiester linkage or any of the non-natural linkages taught by Kmiec I or Kmiec II.

In conjunction with any of the aspects, embodiments, methods and/or compositions disclosed herein, the gene repair oligonucleobase may be a mixed duplex oligonucleotides (MDON) that is linked solely by unsubstituted phosphodiester bonds. In alternative embodiments, the linkage is by substituted phosphodiesters, phosphodiester derivatives and non-phosphorus-based linkages as taught by Kmiec II. In yet another embodiment, each RNA-type nucleotide in the mixed duplex oligonucleotide is a 2'-Substituted Nucleotide. Particular preferred embodiments of 2'-Substituted Ribonucleotides include, but are not limited to, 2'-fluoro, 2'-methoxy, 2'-propyloxy, 2'-allyloxy, 2'-hydroxylethyloxy, 2'-methoxyethyloxy, 2'-fluoropropyloxy and 2'-trifluoropropyloxy substituted ribonucleotides. More preferred embodiments of 2'-Substituted Ribonucleotides are 2'-fluoro, 2'-methoxy, 2'-methoxyethyloxy, and 2'-allyloxy substituted nucleotides. In another embodiment the mixed duplex oligonucleotide is linked by unsubstituted phosphodiester bonds.

Although mixed duplex oligonucleotides (MDONs) having only a single type of 2'-substituted RNA-type nucleotide are more conveniently synthesized, the methods of the invention can also be practiced with mixed duplex oligonucleotides having two or more types of RNA-type nucleotides. The function of an RNA segment may not be affected by an interruption caused by the introduction of a deoxynucleotide between two RNA-type trinucleotides, accordingly, the term RNA segment encompasses terms such as "interrupted RNA segment." An uninterrupted RNA segment is termed a contiguous RNA segment. In an alternative embodiment an RNA segment can contain alternating RNase-resistant and unsubstituted 2'-OH nucleotides. The mixed duplex oligonucleotides preferably have fewer than 100 nucleotides and more preferably fewer than 85 nucleotides, but more than 50 nucleotides. The first and second strands are Watson-Crick base paired. In one embodiment the strands of the mixed duplex oligonucleotide are covalently bonded by a linker, such as a single stranded hexa, penta or tetranucleotide so that the first and second strands are segments of a single oligonucleotide chain having a single 3' and a single 5' end. The 3' and 5' ends can be protected by the addition of a "hairpin cap" whereby the 3' and 5' terminal nucleotides are Watson-Crick paired to adjacent nucleotides. A second hairpin cap can, additionally, be placed at the junction between the first and second strands distant from the 3' and 5' ends, so that the Watson-Crick pairing between the first and second strands is stabilized.

The first and second strands contain two regions that are homologous with two fragments of the target gene, i.e., have the same sequence as the target gene. A homologous region contains the nucleotides of an RNA segment and may contain one or more DNA-type nucleotides of connecting DNA segment and may also contain DNA-type nucleotides that are not within the intervening DNA segment. The two regions of homology are separated by, and each is adjacent to, a region having a sequence that differs from the sequence of the target gene, termed a "heterologous region." The heterologous region can contain one, two or three mismatched nucleotides. The mismatched nucleotides can be contiguous or alternatively can be separated by one or two nucleotides that are homologous with the target gene. Alternatively, the heterologous region can also contain an insertion or one, two, three or of five or fewer nucleotides. Alternatively, the sequence of the mixed duplex oligonucleotide may differ from the sequence of the target gene only by the deletion of one, two, three, or five or fewer nucleotides from the mixed duplex oligonucleotide. The length and position of the heterologous region is, in this case, deemed to be the length of the deletion, even though no nucleotides of the mixed duplex oligonucleotide are within the heterologous region. The distance between the fragments of the target gene that are complementary to the two homologous regions is identical to the length of the heterologous region where a substitution or substitutions is intended. When the heterologous region contains an insertion, the homologous regions are thereby separated in the mixed duplex oligonucleotide farther than their complementary homologous fragments are in the gene, and the converse is applicable when the heterologous region encodes a deletion.

The RNA segments of the mixed duplex oligonucleotides are each a part of a homologous region, i.e., a region that is identical in sequence to a fragment of the target gene, which segments together preferably contain at least 13 RNA-type nucleotides and preferably from 16 to 25 RNA-type nucleotides or yet more preferably 18-22 RNA-type nucleotides or most preferably 20 nucleotides. In one embodiment, RNA segments of the homology regions are separated by and adjacent to, i.e., "connected by" an intervening DNA segment. In one embodiment, each nucleotide of the heterologous region is a nucleotide of the intervening DNA segment. An intervening DNA segment that contains the heterologous region of a mixed duplex oligonucleotide is termed a "mutator segment."

In conjunction with any of the aspects, embodiments, methods and/or compositions disclosed herein, the gene repair oligonucleobase (GRON) may be a single stranded oligodeoxynucleotide mutational vector (SSOMV), for example, such as disclosed in International Patent Application PCT/US00/23457, U.S. Pat. Nos. 6,271,360, 6,479,292, and 7,060,500 which are incorporated by reference in their entirety. The sequence of the SSOMV is based on the same principles as the mutational vectors described for example in U.S. Pat. Nos. 5,756,325; 5,871,984; 5,760,012; 5,888,983; 5,795,972; 5,780,296; 5,945,339; 6,004,804; and 6,010,907 and in International Publication Nos. WO 98/49350; WO 99/07865; WO 99/58723; WO 99/58702; and WO 99/40789. The sequence of the SSOMV contains two regions that are homologous with the target sequence separated by a region that contains the desired genetic alteration termed the mutator region. The mutator region can have a sequence that is the same length as the sequence that separates the homologous regions in the target sequence, but having a different sequence. Such a mutator region can cause a substitution. Alternatively, the homologous regions in the SSOMV can be contiguous to each other, while the regions in the target gene having the same sequence are separated by one, two or more nucleotides. Such an SSOMV causes a deletion from the target gene of the nucleotides that are absent from the SSOMV. Lastly, the sequence of the target gene that is identical to the homologous regions may be adjacent in the target gene but separated by one, two, or more nucleotides in the sequence of the SSOMV. Such an SSOMV causes an insertion in the sequence of the target gene.

The nucleotides of the SSOMV are deoxyribonucleotides that are linked by unmodified phosphodiester bonds except that the 3' terminal and/or 5' terminal internucleotide linkage or alternatively the two 3' terminal and/or 5' terminal internucleotide linkages can be a phosphorothioate or phosphoamidate. As used herein an internucleotide linkage is the linkage between nucleotides of the SSOMV and does not include the linkage between the 3' end nucleotide or 5' end nucleotide and a blocking substituent. In a specific embodiment the length of the SSOMV is between 21 and 55 deoxynucleotides and the lengths of the homology regions are, accordingly, a total length of at least 20 deoxynucleotides and at least two homology regions should each have lengths of at least 8 deoxynucleotides.

The SSOMV can be designed to be complementary to either the coding or the non-coding strand of the target gene. When the desired mutation is a substitution of a single base, it is preferred that both the mutator nucleotide and the targeted nucleotide be a pyrimidine. To the extent that is consistent with achieving the desired functional result, it is preferred that both the mutator nucleotide and the targeted nucleotide in the complementary strand be pyrimidines. Particularly preferred are SSOMVs that encode transversion mutations, i.e., a C or T mutator nucleotide is mismatched, respectively, with a C or T nucleotide in the complementary strand.

In addition to the oligodeoxynucleotide, the SSOMV can contain a 5' blocking substituent that is attached to the 5' terminal carbons through a linker. The chemistry of the linker is not critical other than its length, which should preferably be at least 6 atoms long and that the linker should be flexible. A variety of non-toxic substituents such as biotin, cholesterol or other steroids or a non-intercalating cationic fluorescent dye can be used. Particularly preferred reagents to make SSOMVs are the reagents sold as Cy3^{™} and Cy5^{™} by Glen Research, Sterling Va. (now GE Healthcare), which are blocked phosphoroamidites that upon incorporation into an oligonucleotide yield 3,3,3',3'-tetramethyl N,N'-isopropyl substituted indomonocarbocyanine and indodicarbocyanine dyes, respectively. Cy3 is particularly preferred. When the indocarbocyanine is N-oxyalkyl substituted it can be conveniently linked to the 5' terminal of the oligodeoxynucleotide as a phosphodiester with a 5' terminal phosphate. The chemistry of the dye linker between the dye and the oligodeoxynucleotide is not critical and is chosen for synthetic convenience. When the commercially available Cy3 phosphoramidite is used as directed, the resulting 5' modification consists of a blocking substituent and linker together which are a N-hydroxypropyl, N'-phosphatidylpropyl 3,3,3',3'-tetramethyl indomonocarbocyanine.

In a preferred embodiment the indocarbocyanine dye is tetra substituted at the 3 and 3' positions of the indole rings. Without limitations as to theory these substitutions prevent the dye from being an intercalating dye. The identity of the substituents at these positions is not critical. The SSOMV can in addition have a 3' blocking substituent. Again the chemistry of the 3' blocking substituent is not critical.

The mutations herein described might also be obtained by mutagenesis (random, somatic or directed) and any other DNA editing or recombination technologies including, but not limited to, gene targeting using site-specific homologous recombination by zinc finger nucleases.

### Delivery of Gene Repair Oligonucleobases into Plant Cells

Any commonly known method used to transform a plant cell can be used for delivering the gene repair oligonucleobases. Illustrative methods are described below.

### Microcarriers and Microfibers

The use of metallic microcarriers (microspheres) for introducing large fragments of DNA into plant cells having cellulose cell walls by projectile penetration is well known to those skilled in the relevant art (henceforth biolistic delivery). U.S. Pat. Nos. 4,945,050; 5,100,792 and 5,204,253 describe general techniques for selecting microcarriers and devices for projecting them.

Specific conditions for using microcarriers in the methods disclosed herein are described in International Publication WO 99/07865. In an illustrative technique, ice cold microcarriers (60 mg/mL), mixed duplex oligonucleotide (60 mg/mL) 2.5 M CaCl₂ and 0.1 M spermidine are added in that order; the mixture gently agitated, e.g., by vortexing, for 10 minutes and then left at room temperature for 10 minutes, whereupon the microcarriers are diluted in 5 volumes of ethanol, centrifuged and resuspended in 100% ethanol. Good results can be obtained with a concentration in the adhering solution of 8-10 µg/µL microcarriers, 14-17 µg/mL mixed duplex oligonucleotide, 1.1-1.4 M CaCl₂ and 18-22 mM spermidine. Optimal results were observed under the conditions of 8 µg/µL microcarriers, 16.5 µg/mL mixed duplex oligonucleotide, 1.3 M CaCl₂ and 21 mM spermidine.

Gene repair oligonucleobases can also be introduced into plant cells for the practice of the present disclosure using microfibers to penetrate the cell wall and cell membrane. U.S. Pat. No. 5,302,523 to Coffee et al. describes the use of 30.times.0.5 µm and 10.times.0.3 µm silicon carbide fibers to facilitate transformation of suspension maize cultures of Black Mexican Sweet. Any mechanical technique that can be used to introduce DNA for transformation of a plant cell using microfibers can be used to deliver gene repair oligonucleobases for transmutation.

An illustrative technique for microfiber delivery of a gene repair oligonucleobase is as follows: Sterile microfibers (2 µg) are suspended in 150 µL of plant culture medium containing about 10 µg of a mixed duplex oligonucleotide. A suspension culture is allowed to settle and equal volumes of packed cells and the sterile fiber/nucleotide suspension are vortexed for 10 minutes and plated. Selective media are applied immediately or with a delay of up to about 120 h as is appropriate for the particular trait.

### Protoplast Electroporation

In an alternative embodiment, the gene repair oligonucleobases can be delivered to the plant cell by electroporation of a protoplast derived from a plant part. The protoplasts are formed by enzymatic treatment of a plant part, particularly a leaf, according to techniques well known to those skilled in the art. See, e.g., Gallois et al., 1996, in Methods in Molecular Biology 55:89-107, Humana Press, Totowa, N.J.; Kipp et al., 1999, in Methods in Molecular Biology 133:213-221, Humana Press, Totowa, N.J. The protoplasts need not be cultured in growth media prior to electroporation. Illustrative conditions for electroporation are 3.times.10.sup.5 protoplasts in a total volume of 0.3 mL with a concentration of gene repair oligonucleobase of between 0.6-4 µg/mL.

### Protoplast PEG-mediated DNA uptake

In an alternative embodiment, nucleic acids are taken up by plant protoplasts in the presence of the membrane-modifying agent polyethylene glycol, according to techniques well known to those skilled in the art (see, e.g., Gharti-Chhetri *et al.*, 1992; Datta *et al.*, 1992).

### Microinjection

In an alternative embodiment, the gene repair oligonucleobases can be delivered by injecting it with a microcapillary into plant cells or into protoplasts (see, e.g., Miki *et al.*, 1989; Schnorf *et al.*, 1991).
Transgenics

In any of the various aspects and embodiments of the compositions and methods disclosed herein, mutations in genes and proteins may be made using, for example, transgenic technology. In some embodiments, the compositions and methods include a plant or plant cell having a transformed nucleic acid construct including a promoter operably linked to a PPX nucleotide disclosed herein. The methods disclosed herein may include introducing a PPX nucleic acid construct disclosed herein into at least one plant cell and regenerating a transformed plant therefrom. The nucleic acid construct comprises at least one nucleotide that encodes a herbicide-resistant PPX protein as disclosed herein, particularly the nucleotide sequences of set forth in Figures 2, 4, 6, 8, 10 and 12, and fragments and variants thereof. The methods further involve the use of a promoter that is capable of driving gene expression in a plant cell. In one embodiment, such a promoter is a constitutive promoter or a tissue-preferred promoter. A plant produced by these methods may have increased PPX activity, and/or particularly herbicide-tolerant PPX activity, when compared to an untransformed plant. Thus, the methods find use in enhancing or increasing the resistance of a plant to at least one herbicide that increases the activity of the PPX enzyme, particularly in the presence of a PPX-inhibiting herbicide.

In conjunction with any of the aspects, embodiments, methods and/or compositions disclosed herein, the methods for producing a herbicide-resistant plant may include transforming a plant cell with a nucleic acid construct comprising a nucleotide sequence operably linked to a promoter that drives expression in a plant cell and regenerating a transformed plant from said transformed plant cell. The nucleotide sequence is selected from those nucleotide sequences that encode the herbicide-resistant PPX disclosed herein, particularly the nucleotide sequences set forth in Figures 2, 4, 6, 8, 10 and 12, and fragments and variants thereof. A herbicide-resistant plant produced by this method comprises enhanced resistance, compared to an untransformed plant, to at least one herbicide, particularly a herbicide that interferes with the activity of the PPX enzyme such as, for example, a PPX-inhibiting herbicide.

The disclosed nucleic acid molecules can be used in nucleic acid constructs for the transformation of plants, for example, crop plants, such as *Solanum tuberosum.* In one embodiment, such nucleic acid constructs containing the nucleic acid molecules of the present disclosure can be used to produce transgenic plants to provide for resistance to herbicides, such as herbicides that are known to inhibit PPX activity, such as PPX-inhibiting herbicides. The nucleic acid constructs can be used in expression cassettes, expression vectors, transformation vectors, plasmids and the like. The transgenic plants obtained following transformation with such constructs demonstrate increased resistance to PPX-inhibiting herbicides such as, for example, flumioxazin and sulfentrazone herbicides.

### Constructs

The nucleic acid molecules disclosed herein (e.g., mutated PPX genes) can be used in the production of recombinant nucleic acid constructs. In one embodiment, the nucleic acid molecules of the present disclosure can be used in the preparation of nucleic acid constructs, for example, expression cassettes for expression in the plant of interest.

Expression cassettes may include regulatory sequences operably linked to the PPX nucleic acid sequences disclosed herein. The cassette may additionally contain at least one additional gene to be co-transformed into the organism. Alternatively, the additional gene(s) can be provided on multiple expression cassettes.

The nucleic acid constructs may be provided with a plurality of restriction sites for insertion of the PPX nucleic acid sequence to be under the transcriptional regulation of the regulatory regions. The nucleic acid constructs may additionally contain nucleic acid molecules encoding for selectable marker genes.

Any promoter can be used in the production of the nucleic acid constructs. The promoter may be native or analogous, or foreign or heterologous, to the plant host and/or to the PPX nucleic acid sequences disclosed herein. Additionally, the promoter may be the natural sequence or alternatively a synthetic sequence. Where the promoter is "foreign" or "heterologous" to the plant host, it is intended that the promoter is not found in the native plant into which the promoter is introduced. Where the promoter is "foreign" or "heterologous" to the PPX nucleic acid sequences disclosed herein, it is intended that the promoter is not the native or naturally occurring promoter for the operably linked PPX nucleic acid sequences disclosed herein. As used herein, a chimeric gene comprises a coding sequence operably linked to a transcription initiation region that is heterologous to the coding sequence.

In conjunction with any of the aspects, embodiments, methods and/or compositions disclosed herein, the PPX nucleic acid sequences disclosed herein may be expressed using heterologous promoters, the native promoter sequences may be used in the preparation of the constructs. Such constructs would change expression levels of the PPX protein in the plant or plant cell. Thus, the phenotype of the plant or plant cell is altered.

Any promoter can be used in the preparation of constructs to control the expression of the PPX coding sequence, such as promoters providing for constitutive, tissue-preferred, inducible, or other promoters for expression in plants. Constitutive promoters include, for example, the core promoter of the Rsyn7 promoter and other constitutive promoters disclosed in WO 99/43 838 and U.S. Patent No. 6,072,050; the core CaMV 35S promoter (Odell et al. (1985) Nature 313:810-812); rice actin (McElroy et al. (1990) Plant Cell 2:163-171); ubiquitin (Christensen et al. (1989) Plant Mol. Biol. 12:619-632 and Christensen et al. (1992) Plant Mol. Biol. 18:675-689); pEMU (Last et al. (1991) Theor. Appl. Genet. 81:581-588); MAS (Velten et al. (1984) EMBO J. 3:2723-2730); ALS promoter (U.S. Patent No. 5,659,026), and the like. Other constitutive promoters include, for example, U.S. Patent Nos. 5,608,149; 5,608,144; 5,604,121; 5,569,597; 5,466,785; 5,399,680; 5,268,463; 5,608,142; and 6,177,611.

Tissue-preferred promoters can be utilized to direct PPX expression within a particular plant tissue. Such tissue-preferred promoters include, but are not limited to, leaf-preferred promoters, root-preferred promoters, seed-preferred promoters, and stem-preferred promoters. Tissue-preferred promoters include Yamamoto et al. (1997) Plant J. 12(2):255-265; Kawamata et al. (1997) Plant Cell Physiol. 38(7):792-803; Hansen et al. (1997) Mol. Gen Genet. 254(3):337-343; Russell et al. (1997) Transgenic Res. 6(2):157-168; Rinehart et al. (1996) Plant Physiol. 1 12(3):1331-1341; Van Camp et al. (1996) Plant Physiol. 1 12(2):525-535; Canevascini et al. (1996) Plant Physiol. 112(2): 513-524; Yamamoto et al. (1994) Plant Cell Physiol. 35(5):773-778; Lam (1994) Results Probl. Cell Differ. 20:181-196; Orozco et al. (1993) Plant Mol Biol. 23(6):1129-1138; Matsuoka et al. (1993) Proc Natl. Acad. Sci. USA 90(20):9586- 9590; and Guevara-Garcia et al. (1993) Plant J. 4(3):495-505.

The nucleic acid constructs may also include transcription termination regions. Where transcription terminations regions are used, any termination region may be used in the preparation of the nucleic acid constructs. For example, the termination region may be native to the transcriptional initiation region, may be native to the operably linked PPX sequence of interest, may be native to the plant host, or may be derived from another source (i.e., foreign or heterologous to the promoter, the PPX nucleic acid molecule of interest, the plant host, or any combination thereof). Examples of termination regions that are available for use in the constructs of the present disclosure include those from the Ti-plasmid of *A. tumefaciens*, such as the octopine synthase and nopaline synthase termination regions. See also Guerineau et al. (1991) Mol. Gen. Genet. 262:141-144; Proudfoot (1991) Cell 64:671-674; Sanfacon et al. (1991) Genes Dev. 5:141-149; Mogen et al. (1990) Plant Cell 2:1261-1272; Munroe et al. (1990) Gene 91:151-158; Ballas et al. (1989) Nucleic Acids Res. 17:7891-7903; and Joshi et al. (1987) Nucleic Acid Res. 15:9627-9639.

In conjunction with any of the aspects, embodiments, methods and/or compositions disclosed herein, the nucleic acids may be optimized for increased expression in the transformed plant. That is, the nucleic acids encoding the mutant PPX proteins can be synthesized using plant-preferred codons for improved expression. See, for example, Campbell and Gowri (1990) Plant Physiol. 92:1-11 for a discussion of host-preferred codon usage. Methods are available in the art for synthesizing plant-preferred genes. See, for example, U.S. Patent Nos. 5,380,831, and 5,436,391, and Murray et al. (1989) Nucleic Acids Res. 17:477-498.

In addition, other sequence modifications can be made to the nucleic acid sequences disclosed herein. For example, additional sequence modifications are known to enhance gene expression in a cellular host. These include elimination of sequences encoding spurious polyadenylation signals, exon/intron splice site signals, transposon-like repeats, and other such well-characterized sequences that may be deleterious to gene expression. The G-C content of the sequence may also be adjusted to levels average for a target cellular host, as calculated by reference to known genes expressed in the host cell. In addition, the sequence can be modified to avoid predicted hairpin secondary mRNA structures.

Other nucleic acid sequences may also be used in the preparation of the constructs of the present disclosure, for example to enhance the expression of the PPX coding sequence. Such nucleic acid sequences include the introns of the maize AdhI, intronl gene (Callis et al. (1987) Genes and Development 1:1183-1200), and leader sequences, (W-sequence) from the Tobacco Mosaic virus (TMV), Maize Chlorotic Mottle Virus and Alfalfa Mosaic Virus (Gallie et al.(1987) Nucleic Acid Res. 15:8693-8711, and Skuzeski et al. (1990) Plant Mol. Biol. 15:65-79, 1990). The first intron from the shrunken-1 locus of maize has been shown to increase expression of genes in chimeric gene constructs. U.S. Pat. Nos. 5,424,412 and 5,593,874 disclose the use of specific introns in gene expression constructs, and Gallie et al. ((1994) Plant Physiol. 106:929-939) also have shown that introns are useful for regulating gene expression on a tissue specific basis. To further enhance or to optimize PPX gene expression, the plant expression vectors disclosed herein may also contain DNA sequences containing matrix attachment regions (MARs). Plant cells transformed with such modified expression systems, then, may exhibit overexpression or constitutive expression of a nucleotide sequence of the disclosure.

The expression constructs disclosed herein can also include nucleic acid sequences capable of directing the expression of the PPX sequence to the chloroplast. Such nucleic acid sequences include chloroplast targeting sequences that encodes a chloroplast transit peptide to direct the gene product of interest to plant cell chloroplasts. Such transit peptides are known in the art. With respect to chloroplast-targeting sequences, "operably linked" means that the nucleic acid sequence encoding a transit peptide (i.e., the chloroplast-targeting sequence) is linked to the PPX nucleic acid molecules disclosed herein such that the two sequences are contiguous and in the same reading frame. See, for example, Von Heijne et al. (1991) Plant Mol. Biol. Rep. 9:104-126; Clark et al. (1989) J. Biol. Chem. 264:17544-17550; Della-Cioppa et al. (1987) Plant Physiol. 84:965-968; Romer et al. (1993) Biochem. Biophys. Res. Commun. 196:1414-1421; and Shah et al. (1986) Science 233:478-481. While the PPX proteins disclosed herein may include a native chloroplast transit peptide, any chloroplast transit peptide known in the art can be fused to the amino acid sequence of a mature PPX protein by operably linking a choloroplast-targeting sequence to the 5'-end of a nucleotide sequence encoding a mature PPX protein.

Chloroplast targeting sequences are known in the art and include the chloroplast small subunit of ribulose-1,5-bisphosphate carboxylase (Rubisco) (de Castro Silva Filho et al. (1996) Plant Mol. Biol. 30:769-780; Schnell et al. (1991) J. Biol. Chem. 266(5):3335-3342); 5-(enolpyruvyl)shikimate-3-phosphate synthase (EPSPS) (Archer et al. (1990) J. Bioenerg. Biomemb. 22(6):789-810); tryptophan synthase (Zhao et al. (1995) J. Biol. Chem. 270(1 1):6081- 6087); plastocyanin (Lawrence et al. (1997) J. Biol. Chem. 272(33):20357-20363); chorismate synthase (Schmidt et al. (1993) J. Biol. Chem. 268(36):27447-27457); and the light harvesting chlorophyll a/b binding protein (LHBP) (Lamppa et al. (1988) J. Biol. Chem. 263:14996-14999). See also Von Heijne et al. (1991) Plant Mol. Biol. Rep. 9:104-126; Clark et al. (1989) J. Biol. Chem. 264:17544-17550; Della-Cioppa et al. (1987) Plant Physiol. 84:965-968; Romer et al. (1993) Biochem. Biophys. Res. Commun. 196:1414-1421; and Shah et al. (1986) Science 233 :478-481.

In conjunction with any of the aspects, embodiments, methods and/or compositions disclosed herein, the nucleic acid constructs may be prepared to direct the expression of the mutant PPX coding sequence from the plant cell chloroplast. Methods for transformation of chloroplasts are known in the art. See, for example, Svab et al. (1990) Proc. Natl. Acad. Sci. USA 87:8526-8530; Svab and Maliga (1993) Proc. Natl. Acad. Sci. USA 90:913-917; Svab and Maliga (1993) EMBO J. 12:601-606. The method relies on particle gun delivery of DNA containing a selectable marker and targeting of the DNA to the plastid genome through homologous recombination. Additionally, plastid transformation can be accomplished by transactivation of a silent plastid-borne transgene by tissue-preferred expression of a nuclear-encoded and plastid-directed RNA polymerase. Such a system has been reported in McBride et al. (1994) Proc. Natl. Acad. Sci. USA 91:7301-7305.

The nucleic acids of interest to be targeted to the chloroplast may be optimized for expression in the chloroplast to account for differences in codon usage between the plant nucleus and this organelle. In this manner, the nucleic acids of interest may be synthesized using chloroplast-preferred codons. See, for example, U.S. Patent No. 5,380,831, herein incorporated by reference.

The nucleic acid constructs can be used to transform plant cells and regenerate transgenic plants comprising the mutant PPX coding sequences. Numerous plant transformation vectors and methods for transforming plants are available. See, for example, U.S. Patent No. 6,753,458, An, G. et al. (1986) Plant Physiol., 81:301-305; Fry, J. et al. (1987) Plant Cell Rep. 6:321-325; Block, M. (1988) Theor. Appl Genet.76:767-774; Hinchee et al. (1990) Stadler. Genet. Symp.203212.203-212; Cousins et al. (1991) Aust. J. Plant Physiol. 18:481-494; Chee, P. P. and Slightom, J. L. (1992) Gene.118:255-260; Christou et al. (1992) Trends. Biotechnol. 10:239-246; D'Halluin et al. (1992) Bio/Technol. 10:309-3 14; Dhir et al. (1992) Plant Physiol. 99:81-88; Casas et al. (1993) Proc. Nat. Acad Sci. USA 90:11212-11216; Christou, P. (1993) In Vitro Cell. Dev. Biol.-Plant; 29P:1 19-124; Davies, et al. (1993) Plant Cell Rep. 12:180-183; Dong, J. A. and Mc Hughen, A. (1993) Plant Sci. 91:139-148; Franklin, C. I. and Trieu, T. N. (1993) Plant. Physiol. 102:167; Golovkin et al. (1993) Plant Sci. 90:41-52; Guo Chin Sci. Bull. 38:2072-2078; Asano, et al. (1994) Plant Cell Rep. 13; Ayeres N. M. and Park, W. D. (1994) Crit. Rev. Plant. Sci. 13:219-239; Barcelo et al. (1994) Plant. J. 5:583-592; Becker, et al. (1994) Plant. J. 5:299-307; Borkowska et al. (1994) Acta. Physiol Plant. 16:225- 230; Christou, P. (1994) Agro. Food. Ind. Hi Tech. 5: 17-27; Eapen et al. (1994) Plant Cell Rep. 13:582-586; Hartman et al. (1994) Bio-Technology 12: 919923; Ritala et al. (1994) Plant. Mol. Biol. 24:317-325; and Wan, Y. C. and Lemaux, P. G. (1994) Plant Physiol. 104:3748. The constructs may also be transformed into plant cells using homologous recombination.

The disclosed constructs comprising the PPX nucleic acid sequences disclosed herein can be used in various methods to produce transgenic host cells, such as bacteria, yeast, and to transform plant cells and in some cases regenerate transgenic plants. For example, methods of producing a transgenic crop plant containing the PPX mutant proteins disclosed herein, where expression of the nucleic acid(s) in the plant results in herbicide tolerance as compared to wild-type plants or to known PPX mutant type plants comprising: (a) introducing into a plant cell an expression vector comprising nucleic acid encoding a mutant PPX protein, and (b) generating from the plant cell a transgenic plant which is herbicide tolerant.

### PPX Mutations

The compositions and methods may relate at least in part to mutations in a PPX gene, for example mutations that render a plant resistant or tolerant to a herbicide of the PPX-inhibiting family of herbicides. The compositions and methods also in certain embodiments relate to the use of a gene repair oligonucleobase to make a desired mutation in the chromosomal or episomal sequences of a plant in the gene encoding for a PPX protein. The mutated protein, which may in some embodiments substantially maintain the catalytic activity of the wild-type protein, allowing for increased resistance or tolerance of the plant to a herbicide of the PPX-inhibiting family, and thus in some embodiments allowing for substantially normal growth or development of the plant, its organs, tissues, or cells as compared to the wild-type plant irrespective of the presence or absence of the herbicide. The compositions and methods also relate to a non-transgenic or transgenic plant cell in which a PPX gene has been mutated, a non-transgenic plant or transgenic regenerated therefrom, as well as a plant resulting from a cross using a regenerated non-transgenic or transgenic plant to a plant having a mutation in a different PPX gene, for example. These mutations may also be applied to target tolerance to these inhibitors in plants including crop plants, algae, bacteria, fungi and mammalian systems.

In conjunction with any of the aspects, embodiments, methods and/or compositions disclosed herein, at least one mutation of a mutated PPX protein may be at the amino acid position corresponding to a position selected from the group consisting of 52, 85, 105, 111, 130, 139, 143, 144, 145, 147, 165, 167, 170, 180, 185, 192, 193, 199, 206, 212, 219, 220, 221, 226, 228, 229, 230, 237, 244, 256, 257, 270, 271, 272, 305, 311, 316, 318, 332, 343, 354, 357, 359, 360, 366, 393, 403, 424, 426, 430, 438, 440, 444, 455, 457, 470, 478, 483, 484, 485, 487, 490, 503, 508, and 525 of SEQ ID NO: 1. In some embodiments, a mutated PPX protein includes a mutation at one or more amino acid positions corresponding to a position selected from the group consisting of 58, 64, 74, 84, 93, 97, 98, 101, 119, 121, 124, 139, 150 151, 157, 164, 170, 177, 187, 188, 195, 214, 215, 229, 230, 271, 274, 278, 283, 292, 296, 307, 324, 330, 396, 404, 406, 410, 421, 423, 434, 447, 448, 449, 451, 454, 465, 470 and 50 of SEQ ID NO: 9. In some embodiments, a mutated PPX protein includes a mutation at the amino acid position corresponding to position 52 of SEQ ID NO: 1. In some embodiments, a mutated PPX protein includes a mutation at the amino acid position corresponding to position 85 of SEQ ID NO: 1. In some embodiments, a mutated PPX protein includes a mutation at the amino acid position corresponding to position 111 of SEQ ID NO: 1. In some embodiments, a mutated PPX protein includes a mutation at the amino acid position corresponding to position 130 of SEQ ID NO: 1. In some embodiments, a mutated PPX protein includes a mutation at the amino acid position corresponding to position 139 of SEQ ID NO: 1. In some embodiments, a mutated PPX protein includes a mutation at the amino acid position corresponding to position 143 of SEQ ID NO: 1. In some embodiments, a mutated PPX protein includes a mutation at the amino acid position corresponding to position 144 of SEQ ID NO: 1. In some embodiments, a mutated PPX protein includes a mutation at the amino acid position corresponding to position 145 of SEQ ID NO: 1. In some embodiments, a mutated PPX protein includes a mutation at the amino acid position corresponding to position 147 of SEQ ID NO: 1. In some embodiments, a mutated PPX protein includes a mutation at the amino acid position corresponding to position 165 of SEQ ID NO: 1. In some embodiments, a mutated PPX protein includes a mutation at the amino acid position corresponding to position 180 of SEQ ID NO: 1. In some embodiments, a mutated PPX protein includes a mutation at the amino acid position corresponding to position 185 of SEQ ID NO: 1. In some embodiments, a mutated PPX protein includes a mutation at the amino acid position corresponding to position 192 of SEQ ID NO: 1. In some embodiments, a mutated PPX protein includes a mutation at the amino acid position corresponding to position 193 of SEQ ID NO: 1. In some embodiments, a mutated PPX protein includes a mutation at the amino acid position corresponding to position 199 of SEQ ID NO: 1. In some embodiments, a mutated PPX protein includes a mutation at the amino acid position corresponding to position 206 of SEQ ID NO: 1. In some embodiments, a mutated PPX protein includes a mutation at the amino acid position corresponding to position 219 of SEQ ID NO: 1. In some embodiments, a mutated PPX protein includes a mutation at the amino acid position corresponding to position 220 of SEQ ID NO: 1. In some embodiments, a mutated PPX protein includes a mutation at the amino acid position corresponding to position 226 of SEQ ID NO: 1. In some embodiments, a mutated PPX protein includes a mutation at the amino acid position corresponding to position 228 of SEQ ID NO: 1. In some embodiments, a mutated PPX protein includes a mutation at the amino acid position corresponding to position 229 of SEQ ID NO: 1. In some embodiments, a mutated PPX protein includes a mutation at the amino acid position corresponding to position 230 of SEQ ID NO: 1. In some embodiments, a mutated PPX protein includes a mutation at the amino acid position corresponding to position 244 of SEQ ID NO: 1. In some embodiments, a mutated PPX protein includes a mutation at the amino acid position corresponding to position 256 of SEQ ID NO: 1. In some embodiments, a mutated PPX protein includes a mutation at the amino acid position corresponding to position 270 of SEQ ID NO: 1. In some embodiments, a mutated PPX protein includes a mutation at the amino acid position corresponding to position 271 of SEQ ID NO: 1. In some embodiments, a mutated PPX protein includes a mutation at the amino acid position corresponding to position 272 of SEQ ID NO: 1. In some embodiments, a mutated PPX protein includes a mutation at the amino acid position corresponding to position 305 of SEQ ID NO: 1. In some embodiments, a mutated PPX protein includes a mutation at the amino acid position corresponding to position 311 of SEQ ID NO: 1. In some embodiments, a mutated PPX protein includes a mutation at the amino acid position corresponding to position 316 of SEQ ID NO: 1. In some embodiments, a mutated PPX protein includes a mutation at the amino acid position corresponding to position 318 of SEQ ID NO: 1. In some embodiments, a mutated PPX protein includes a mutation at the amino acid position corresponding to position 332 of SEQ ID NO: 1. In some embodiments, a mutated PPX protein includes a mutation at the amino acid position corresponding to position 357 of SEQ ID NO: 1. In some embodiments, a mutated PPX protein includes a mutation at the amino acid position corresponding to position 359 of SEQ ID NO: 1. In some embodiments, a mutated PPX protein includes a mutation at the amino acid position corresponding to position 360 of SEQ ID NO: 1. In some embodiments, a mutated PPX protein includes a mutation at the amino acid position corresponding to position 366 of SEQ ID NO: 1. In some embodiments, a mutated PPX protein includes a mutation at the amino acid position corresponding to position 393 of SEQ ID NO: 1. In some embodiments, a mutated PPX protein includes a mutation at the amino acid position corresponding to position 403 of SEQ ID NO: 1. In some embodiments, a mutated PPX protein includes a mutation at the amino acid position corresponding to position 424 of SEQ ID NO: 1. In some embodiments, a mutated PPX protein includes a mutation at the amino acid position corresponding to position 426 of SEQ ID NO: 1. In some embodiments, a mutated PPX protein includes a mutation at the amino acid position corresponding to position 438 of SEQ ID NO: 1. In some embodiments, a mutated PPX protein includes a mutation at the amino acid position corresponding to position 440 of SEQ ID NO: 1. In some embodiments, a mutated PPX protein includes a mutation at the amino acid position corresponding to position 444 of SEQ ID NO: 1. In some embodiments, a mutated PPX protein includes a mutation at the amino acid position corresponding to position 455 of SEQ ID NO: 1. In some embodiments, a mutated PPX protein includes a mutation at the amino acid position corresponding to position 457 of SEQ ID NO: 1. In some embodiments, a mutated PPX protein includes a mutation at the amino acid position corresponding to position 470 of SEQ ID NO: 1. In some embodiments, a mutated PPX protein includes a mutation at the amino acid position corresponding to position 478 of SEQ ID NO: 1. In some embodiments, a mutated PPX protein includes a mutation at the amino acid position corresponding to position a phenylalanine to glycine at a position corresponding to position 483 of SEQ ID NO: 1. In some embodiments, a mutated PPX protein includes a mutation at the amino acid position corresponding to position 484 of SEQ ID NO: 1. In some embodiments, a mutated PPX protein includes a mutation at the amino acid position corresponding to position 485 of SEQ ID NO: 1. In some embodiments, a mutated PPX protein includes a mutation at the amino acid position corresponding to position 487 of SEQ ID NO: 1. In some embodiments, a mutated PPX protein includes a mutation at the amino acid position corresponding to position 490 of SEQ ID NO: 1. In some embodiments, a mutated PPX protein includes a mutation at the amino acid position corresponding to position 503 of SEQ ID NO: 1. In some embodiments, a mutated PPX protein includes a mutation at the amino acid position corresponding to position 508 of SEQ ID NO: 1. In some embodiments, a mutated PPX protein includes a mutation at the amino acid position corresponding to position 525 of SEQ ID NO: 1. In some embodiments, a mutated PPX protein includes a mutation at the amino acid position corresponding to position 58 of SEQ ID NO: 9. In some embodiments, a mutated PPX protein includes a mutation at the amino acid position corresponding to position 64 of SEQ ID NO: 9. In some embodiments, a mutated PPX protein includes a mutation at the amino acid position corresponding to position 74 of SEQ ID NO: 9. In some embodiments, a mutated PPX protein includes a mutation at the amino acid position corresponding to position 84 of SEQ ID NO: 9. In some embodiments, a mutated PPX protein includes a mutation at the amino acid position corresponding to position 93 of SEQ ID NO: 9. In some embodiments, a mutated PPX protein includes a mutation at the amino acid position corresponding to position 97 of SEQ ID NO: 9. In some embodiments, a mutated PPX protein includes a mutation at the amino acid position corresponding to position 98 of SEQ ID NO: 9. In some embodiments, a mutated PPX protein includes a mutation at the amino acid position corresponding to position 101 of SEQ ID NO: 9. In some embodiments, a mutated PPX protein includes a mutation at the amino acid position corresponding to position 119 of SEQ ID NO: 9. In some embodiments, a mutated PPX protein includes a mutation at the amino acid position corresponding to position 121 of SEQ ID NO: 9. In some embodiments, a mutated PPX protein includes a mutation at the amino acid position corresponding to position 124 of SEQ ID NO: 9. In some embodiments, a mutated PPX protein includes a mutation at the amino acid position corresponding to position 139 of SEQ ID NO: 9. In some embodiments, a mutated PPX protein includes a mutation at the amino acid position corresponding to position 150 of SEQ ID NO: 9. In some embodiments, a mutated PPX protein includes a mutation at the amino acid position corresponding to position 151 of SEQ ID NO: 9. In some embodiments, a mutated PPX protein includes a mutation at the amino acid position corresponding to position 157 of SEQ ID NO: 9. In some embodiments, a mutated PPX protein includes a mutation at the amino acid position corresponding to position 164 of SEQ ID NO: 9. In some embodiments, a mutated PPX protein includes a mutation at the amino acid position corresponding to position 170 of SEQ ID NO: 9. In some embodiments, a mutated PPX protein includes a mutation at the amino acid position corresponding to position 177 of SEQ ID NO: 9. In some embodiments, a mutated PPX protein includes a mutation at the amino acid position corresponding to position 187 of SEQ ID NO: 9. In some embodiments, a mutated PPX protein includes a mutation at the amino acid position corresponding to position 188 of SEQ ID NO: 9. In some embodiments, a mutated PPX protein includes a mutation at the amino acid position corresponding to position 195 of SEQ ID NO: 9. In some embodiments, a mutated PPX protein includes a mutation at the amino acid position corresponding to position 214 of SEQ ID NO: 9. In some embodiments, a mutated PPX protein includes a mutation at the amino acid position corresponding to position 215 of SEQ ID NO: 9. In some embodiments, a mutated PPX protein includes a mutation at the amino acid position corresponding to position 229 of SEQ ID NO: 9. In some embodiments, a mutated PPX protein includes a mutation at the amino acid position corresponding to position 230 of SEQ ID NO: 9. In some embodiments, a mutated PPX protein includes a mutation at the amino acid position corresponding to position 271 of SEQ ID NO: 9. In some embodiments, a mutated PPX protein includes a mutation at the amino acid position corresponding to position 274 of SEQ ID NO: 9. In some embodiments, a mutated PPX protein includes a mutation at the amino acid position corresponding to position 278 of SEQ ID NO: 9. In some embodiments, a mutated PPX protein includes a mutation at the amino acid position corresponding to position 283 of SEQ ID NO: 9. In some embodiments, a mutated PPX protein includes a mutation at the amino acid position corresponding to position 292 of SEQ ID NO: 9. In some embodiments, a mutated PPX protein includes a mutation at the amino acid position corresponding to position 296 of SEQ ID NO: 9. In some embodiments, a mutated PPX protein includes a mutation at the amino acid position corresponding to position 307 of SEQ ID NO: 9. In some embodiments, a mutated PPX protein includes a mutation at the amino acid position corresponding to position 324 of SEQ ID NO: 9. In some embodiments, a mutated PPX protein includes a mutation at the amino acid position corresponding to position 330 of SEQ ID NO: 9. In some embodiments, a mutated PPX protein includes a mutation at the amino acid position corresponding to position 396 of SEQ ID NO: 9. In some embodiments, a mutated PPX protein includes a mutation at the amino acid position corresponding to position 404 of SEQ ID NO: 9. In some embodiments, a mutated PPX protein includes a mutation at the amino acid position corresponding to position 406 of SEQ ID NO: 9. In some embodiments, a mutated PPX protein includes a mutation at the amino acid position corresponding to position 410 of SEQ ID NO: 9. In some embodiments, a mutated PPX protein includes a mutation at the amino acid position corresponding to position 421 of SEQ ID NO: 9. In some embodiments, a mutated PPX protein includes a mutation at the amino acid position corresponding to position 423 of SEQ ID NO: 9. In some embodiments, a mutated PPX protein includes a mutation at the amino acid position corresponding to position 434 of SEQ ID NO: 9. In some embodiments, a mutated PPX protein includes a mutation at the amino acid position corresponding to position 447 of SEQ ID NO: 9. In some embodiments, a mutated PPX protein includes a mutation at the amino acid position corresponding to position 448 of SEQ ID NO: 9. In some embodiments, a mutated PPX protein includes a mutation at the amino acid position corresponding to position 449 of SEQ ID NO: 9. In some embodiments, a mutated PPX protein includes a mutation at the amino acid position corresponding to position 451 of SEQ ID NO: 9. In some embodiments, a mutated PPX protein includes a mutation at the amino acid position corresponding to position 454 of SEQ ID NO: 9. In some embodiments, a mutated PPX protein includes a mutation at the amino acid position corresponding to position 465 of SEQ ID NO: 9. In some embodiments, a mutated PPX protein includes a mutation at the amino acid position corresponding to position 470 of SEQ ID NO: 9. In some embodiments, a mutated PPX protein includes a mutation at the amino acid position corresponding to position 500 of SEQ ID NO: 9. In some embodiments, a mutated PPX protein includes two or more mutations, at least one mutation of which is at the amino acid position corresponding to a position selected from the group consisting of 52, 85, 105, 111, 130, 139, 143, 144, 145, 147, 165, 167, 170, 180, 185, 192, 193, 199, 206, 212, 219, 220, 221, 226, 228, 229, 230, 237, 244, 256, 257, 270, 271, 272, 305, 311, 316, 318, 332, 343, 354, 357, 359, 360, 366, 393, 403, 424, 426, 430, 438, 440, 444, 455, 457, 470, 478, 483, 484, 485, 487, 490, 503, 508 and 525 of SEQ ID NO: 1. In some embodiments, a mutated PPX protein includes two or more mutations, at least one mutation of which is at the amino acid position corresponding to a position selected from the group consisting of 58, 64, 74, 84, 93, 97, 98, 101, 119, 121, 124, 139, 150 151, 157, 164, 170, 177, 187, 188, 195, 214, 215, 229, 230, 271, 274, 278, 283, 292, 296, 307, 324, 330, 396, 404, 406, 410, 421, 423, 434, 447, 448, 449, 451, 454, 465, 470 and 500 of SEQ ID NO: 9. In some embodiments, a mutated PPX protein includes three or more mutations, at least one mutation of which is at the amino acid position corresponding to a position selected from the group consisting of 52, 85, 105, 111, 130, 139, 143, 144, 145, 147, 165, 167, 170, 180, 185, 192, 193, 199, 206, 212, 219, 220, 221, 226, 228, 229, 230, 237, 244, 256, 257, 270, 271, 272, 305, 311, 316, 318, 332, 343, 354, 357, 359, 360, 366, 393, 403, 424, 426, 430, 438, 440, 444, 455, 457, 470, 478, 483, 484, 485, 487, 490, 503, 508 and 525 of SEQ ID NO: 1. In some embodiments, a mutated PPX protein includes three or more mutations, at least one mutation of which is at the amino acid position corresponding to a position selected from the group consisting of 58, 64, 74, 84, 93, 97, 98, 101, 119, 121, 124, 139, 150 151, 157, 164, 170, 177, 187, 188, 195, 214, 215, 229, 230, 271, 274, 278, 283, 292, 296, 307, 324, 330, 396, 404, 406, 410, 421, 423, 434, 447, 448, 449, 451, 454, 465, 470 and 500 of SEQ ID NO: 9.

In conjunction with any of the aspects, embodiments, methods and/or compositions disclosed herein, at least one mutation of a mutated PPX protein may be at the amino acid position corresponding to a position selected from the group consisting of G52, N85, N105, E111, G130, D139, P143, R144, F145, L147, F165, L167, I170, A180, P185, E192, S193, R199, V206, E212, Y219, A220, G221, L226, M228, K229, A230, K237, S244, R256, R257, K270, P271, Q272, S305, E311, T316, T318,S332, S343, A354, L357, K359, L360, A366, L393, L403, L424, Y426, S430 , K438, E440, V444, L455, K457, V470, F478, F483, D484, I485, D487, K490, L503, V508 and I525 of SEQ ID NO: 1. In conjunction with any of the aspects, embodiments, methods and/or compositions disclosed herein, at least one mutation of a mutated PPX protein may be at the amino acid position corresponding to a position selected from the group consisting of D58, E64, G74, G84, L93, K97, K98, A101, S119, F121, T124, N139, E150, S151, Q157, V164, D170, C177, H187, L188, N195, P214, I215, K229, K230, C271, D274, F283, A292, S296, C307, N324, D330, S396, A404, R406, K410, L421, A423, C434, D447, S448, V449, D451, D454, Y465, K470and T500 of SEQ ID NO: 9. In some embodiments, a mutated PPX protein includes a mutation at the amino acid position corresponding to position G52 of SEQ ID NO: 1. In some embodiments, a mutated PPX protein includes a mutation at the amino acid position corresponding to position N85 of SEQ ID NO: 1. In some embodiments, a mutated PPX protein includes a mutation at the amino acid position corresponding to position E111 of SEQ ID NO: 1. In some embodiments, a mutated PPX protein includes a mutation at the amino acid position corresponding to position G130 of SEQ ID NO: 1. In some embodiments, a mutated PPX protein includes a mutation at the amino acid position corresponding to position D139 of SEQ ID NO: 1. In some embodiments, a mutated PPX protein includes a mutation at the amino acid position corresponding to position P143 of SEQ ID NO: 1. In some embodiments, a mutated PPX protein includes a mutation at the amino acid position corresponding to position R144 of SEQ ID NO: 1. In some embodiments, a mutated PPX protein includes a mutation at the amino acid position corresponding to position F145 of SEQ ID NO: 1. In some embodiments, a mutated PPX protein includes a mutation at the amino acid position corresponding to position L147 of SEQ ID NO: 1. In some embodiments, a mutated PPX protein includes a mutation at the amino acid position corresponding to position F165 of SEQ ID NO: 1. In some embodiments, a mutated PPX protein includes a mutation at the amino acid position corresponding to position L167 of SEQ ID NO: 1. In some embodiments, a mutated PPX protein includes a mutation at the amino acid position corresponding to position I170 of SEQ ID NO: 1. In some embodiments, a mutated PPX protein includes a mutation at the amino acid position corresponding to position A180 of SEQ ID NO: 1. In some embodiments, a mutated PPX protein includes a mutation at the amino acid position corresponding to position P185 of SEQ ID NO: 1. In some embodiments, a mutated PPX protein includes a mutation at the amino acid position corresponding to position E192 of SEQ ID NO: 1. In some embodiments, a mutated PPX protein includes a mutation at the amino acid position corresponding to position S193 of SEQ ID NO: 1. In some embodiments, a mutated PPX protein includes a mutation at the amino acid position corresponding to position R199 of SEQ ID NO: 1. In some embodiments, a mutated PPX protein includes a mutation at the amino acid position corresponding to position V206 of SEQ ID NO: 1. In some embodiments, a mutated PPX protein includes a mutation at the amino acid position corresponding to position E212 of SEQ ID NO: 1. In some embodiments, a mutated PPX protein includes a mutation at the amino acid position corresponding to position Y219 of SEQ ID NO: 1. In some embodiments, a mutated PPX protein includes a mutation at the amino acid position corresponding to position A220 of SEQ ID NO: 1. In some embodiments, a mutated PPX protein includes a mutation at the amino acid position corresponding to position G221 of SEQ ID NO: 1. In some embodiments, a mutated PPX protein includes a mutation at the amino acid position corresponding to position L226 of SEQ ID NO: 1. In some embodiments, a mutated PPX protein includes a mutation at the amino acid position corresponding to position M228 of SEQ ID NO: 1. In some embodiments, a mutated PPX protein includes a mutation at the amino acid position corresponding to position K229 of SEQ ID NO: 1. In some embodiments, a mutated PPX protein includes a mutation at the amino acid position corresponding to position A230 of SEQ ID NO: 1. In some embodiments, a mutated PPX protein includes a mutation at the amino acid position corresponding to position K237 of SEQ ID NO: 1. In some embodiments, a mutated PPX protein includes a mutation at the amino acid position corresponding to position S244 of SEQ ID NO: 1. In some embodiments, a mutated PPX protein includes a mutation at the amino acid position corresponding to position R256 of SEQ ID NO: 1. In some embodiments, a mutated PPX protein includes a mutation at the amino acid position corresponding to position R257 of SEQ ID NO: 1. In some embodiments, a mutated PPX protein includes a mutation at the amino acid position corresponding to position K270 of SEQ ID NO: 1. In some embodiments, a mutated PPX protein includes a mutation at the amino acid position corresponding to position P271 of SEQ ID NO: 1. In some embodiments, a mutated PPX protein includes a mutation at the amino acid position corresponding to position Q272 of SEQ ID NO: 1. In some embodiments, a mutated PPX protein includes a mutation at the amino acid position corresponding to position S305 of SEQ ID NO: 1. In some embodiments, a mutated PPX protein includes a mutation at the amino acid position corresponding to position E311 of SEQ ID NO: 1. In some embodiments, a mutated PPX protein includes a mutation at the amino acid position corresponding to position T316 of SEQ ID NO: 1. In some embodiments, a mutated PPX protein includes a mutation at the amino acid position corresponding to position T318 of SEQ ID NO: 1. In some embodiments, a mutated PPX protein includes a mutation at the amino acid position corresponding to position S332 of SEQ ID NO: 1. In some embodiments, a mutated PPX protein includes a mutation at the amino acid position corresponding to position S343 of SEQ ID NO: 1. In some embodiments, a mutated PPX protein includes a mutation at the amino acid position corresponding to position A354 of SEQ ID NO: 1. In some embodiments, a mutated PPX protein includes a mutation at the amino acid position corresponding to position L357 of SEQ ID NO: 1. In some embodiments, a mutated PPX protein includes a mutation at the amino acid position corresponding to position K359 of SEQ ID NO: 1. In some embodiments, a mutated PPX protein includes a mutation at the amino acid position corresponding to position L360 of SEQ ID NO: 1. In some embodiments, a mutated PPX protein includes a mutation at the amino acid position corresponding to position A366 of SEQ ID NO: 1. In some embodiments, a mutated PPX protein includes a mutation at the amino acid position corresponding to position L393 of SEQ ID NO: 1. In some embodiments, a mutated PPX protein includes a mutation at the amino acid position corresponding to position L403 of SEQ ID NO: 1. In some embodiments, a mutated PPX protein includes a mutation at the amino acid position corresponding to position L424 of SEQ ID NO: 1. In some embodiments, a mutated PPX protein includes a mutation at the amino acid position corresponding to position Y426 of SEQ ID NO: 1. In some embodiments, a mutated PPX protein includes a mutation at the amino acid position corresponding to position S430 of SEQ ID NO: 1. In some embodiments, a mutated PPX protein includes a mutation at the amino acid position corresponding to position K438 of SEQ ID NO: 1. In some embodiments, a mutated PPX protein includes a mutation at the amino acid position corresponding to position E440 of SEQ ID NO: 1. In some embodiments, a mutated PPX protein includes a mutation at the amino acid position corresponding to position V444 of SEQ ID NO: 1. In some embodiments, a mutated PPX protein includes a mutation at the amino acid position corresponding to position L455 of SEQ ID NO: 1. In some embodiments, a mutated PPX protein includes a mutation at the amino acid position corresponding to position K457 of SEQ ID NO: 1. In some embodiments, a mutated PPX protein includes a mutation at the amino acid position corresponding to position V470 of SEQ ID NO: 1. In some embodiments, a mutated PPX protein includes a mutation at the amino acid position corresponding to position F478 of SEQ ID NO: 1. In some embodiments, a mutated PPX protein includes a mutation at the amino acid position corresponding to position F483 of SEQ ID NO: 1. In some embodiments, a mutated PPX protein includes a mutation at the amino acid position corresponding to position D484 of SEQ ID NO: 1. In some embodiments, a mutated PPX protein includes a mutation at the amino acid position corresponding to position I485 of SEQ ID NO: 1. In some embodiments, a mutated PPX protein includes a mutation at the amino acid position corresponding to position D487 of SEQ ID NO: 1. In some embodiments, a mutated PPX protein includes a mutation at the amino acid position corresponding to position K490 of SEQ ID NO: 1. In some embodiments, a mutated PPX protein includes a mutation at the amino acid position corresponding to position L503 of SEQ ID NO: 1. In some embodiments, a mutated PPX protein includes a mutation at the amino acid position corresponding to position V508 of SEQ ID NO: 1. In some embodiments, a mutated PPX protein includes a mutation at the amino acid position corresponding to position I525 of SEQ ID NO: 1. In some embodiments, a mutated PPX protein includes a mutation at the amino acid position corresponding to position D58 of SEQ ID NO: 9. In some embodiments, a mutated PPX protein includes a mutation at the amino acid position corresponding to position E64 of SEQ ID NO: 9. In some embodiments, a mutated PPX protein includes a mutation at the amino acid position corresponding to position G74 of SEQ ID NO: 9. In some embodiments, a mutated PPX protein includes a mutation at the amino acid position corresponding to position G84 of SEQ ID NO: 9. In some embodiments, a mutated PPX protein includes a mutation at the amino acid position corresponding to position L93 of SEQ ID NO: 9. In some embodiments, a mutated PPX protein includes a mutation at the amino acid position corresponding to position K97 of SEQ ID NO: 9. In some embodiments, a mutated PPX protein includes a mutation at the amino acid position corresponding to position K98 of SEQ ID NO: 9. In some embodiments, a mutated PPX protein includes a mutation at the amino acid position corresponding to position A101 of SEQ ID NO: 9. In some embodiments, a mutated PPX protein includes a mutation at the amino acid position corresponding to position S119 of SEQ ID NO: 9. In some embodiments, a mutated PPX protein includes a mutation at the amino acid position corresponding to position F121 of SEQ ID NO: 9. In some embodiments, a mutated PPX protein includes a mutation at the amino acid position corresponding to position T124 of SEQ ID NO: 9. In some embodiments, a mutated PPX protein includes a mutation at the amino acid position corresponding to position N139 of SEQ ID NO: 9. In some embodiments, a mutated PPX protein includes a mutation at the amino acid position corresponding to position E150 of SEQ ID NO: 9. In some embodiments, a mutated PPX protein includes a mutation at the amino acid position corresponding to position S151 of SEQ ID NO: 9. In some embodiments, a mutated PPX protein includes a mutation at the amino acid position corresponding to position Q157 of SEQ ID NO: 9. In some embodiments, a mutated PPX protein includes a mutation at the amino acid position corresponding to position V164 of SEQ ID NO: 9. In some embodiments, a mutated PPX protein includes a mutation at the amino acid position corresponding to position D170 of SEQ ID NO: 9. In some embodiments, a mutated PPX protein includes a mutation at the amino acid position corresponding to position C177 of SEQ ID NO: 9. In some embodiments, a mutated PPX protein includes a mutation at the amino acid position corresponding to position H187 of SEQ ID NO: 9. In some embodiments, a mutated PPX protein includes a mutation at the amino acid position corresponding to position L188 of SEQ ID NO: 9. In some embodiments, a mutated PPX protein includes a mutation at the amino acid position corresponding to position N195 of SEQ ID NO: 9. In some embodiments, a mutated PPX protein includes a mutation at the amino acid position corresponding to position P214 of SEQ ID NO: 9. In some embodiments, a mutated PPX protein includes a mutation at the amino acid position corresponding to position I215 of SEQ ID NO: 9. In some embodiments, a mutated PPX protein includes a mutation at the amino acid position corresponding to position K229 of SEQ ID NO: 9. In some embodiments, a mutated PPX protein includes a mutation at the amino acid position corresponding to position K230 of SEQ ID NO: 9. In some embodiments, a mutated PPX protein includes a mutation at the amino acid position corresponding to position C271 of SEQ ID NO: 9. In some embodiments, a mutated PPX protein includes a mutation at the amino acid position corresponding to position D274 of SEQ ID NO: 9. In some embodiments, a mutated PPX protein includes a mutation at the amino acid position corresponding to position F283 of SEQ ID NO: 9. In some embodiments, a mutated PPX protein includes a mutation at the amino acid position corresponding to position A292 of SEQ ID NO: 9. In some embodiments, a mutated PPX protein includes a mutation at the amino acid position corresponding to position S296 of SEQ ID NO: 9. In some embodiments, a mutated PPX protein includes a mutation at the amino acid position corresponding to position C307 of SEQ ID NO: 9. In some embodiments, a mutated PPX protein includes a mutation at the amino acid position corresponding to position N324 of SEQ ID NO: 9. In some embodiments, a mutated PPX protein includes a mutation at the amino acid position corresponding to position D330 of SEQ ID NO: 9. In some embodiments, a mutated PPX protein includes a mutation at the amino acid position corresponding to position S396 of SEQ ID NO: 9. In some embodiments, a mutated PPX protein includes a mutation at the amino acid position corresponding to position A404 of SEQ ID NO: 9. In some embodiments, a mutated PPX protein includes a mutation at the amino acid position corresponding to position R406 of SEQ ID NO: 9. In some embodiments, a mutated PPX protein includes a mutation at the amino acid position corresponding to position K410 of SEQ ID NO: 9. In some embodiments, a mutated PPX protein includes a mutation at the amino acid position corresponding to position L421 of SEQ ID NO: 9. In some embodiments, a mutated PPX protein includes a mutation at the amino acid position corresponding to position A423 of SEQ ID NO: 9. In some embodiments, a mutated PPX protein includes a mutation at the amino acid position corresponding to position C434 of SEQ ID NO: 9. In some embodiments, a mutated PPX protein includes a mutation at the amino acid position corresponding to position D447 of SEQ ID NO: 9. In some embodiments, a mutated PPX protein includes a mutation at the amino acid position corresponding to position S448 of SEQ ID NO: 9. In some embodiments, a mutated PPX protein includes a mutation at the amino acid position corresponding to position V449 of SEQ ID NO: 9. In some embodiments, a mutated PPX protein includes a mutation at the amino acid position corresponding to position D451 of SEQ ID NO: 9. In some embodiments, a mutated PPX protein includes a mutation at the amino acid position corresponding to position D454 of SEQ ID NO: 9. In some embodiments, a mutated PPX protein includes a mutation at the amino acid position corresponding to position Y465 of SEQ ID NO: 9. In some embodiments, a mutated PPX protein includes a mutation at the amino acid position corresponding to position K470 of SEQ ID NO: 9. In some embodiments, a mutated PPX protein includes a mutation at the amino acid position corresponding to position T500 of SEQ ID NO: 9. In some embodiments, a PPX protein is a paralog of *Arabidopsis thaliana* PPX protein (for example the PPX protein may be a potato plastidal PPX protein) and the PPX protein may have an N at the position corresponding to position 52 of SEQ ID NO:1, wherein the N is substituted with an amino acid other than an N; a K at the position corresponding to position 272 of SEQ ID NO:1, wherein the K is substituted with an amino acid other than a K; an S at the position corresponding to position 359 of SEQ ID NO: 1, wherein the S is substituted with an amino acid other than an S; and/or an S at the position corresponding to position 525 of SEQ ID NO: 1, wherein the S is substituted with an amino acid other than an S. In such embodiments, a mutated PPX protein includes a mutation at the amino acid position corresponding to position N52 of SEQ ID NO: 1. In some embodiments, a mutated PPX protein includes a mutation at the amino acid position corresponding to position N85 of SEQ ID NO: 1. In some embodiments, a mutated PPX protein includes a mutation at the amino acid position corresponding to position R144 of SEQ ID NO: 1. In some embodiments, a mutated PPX protein includes a mutation at the amino acid position corresponding to position F145 of SEQ ID NO: 1. In some embodiments, a mutated PPX protein includes a mutation at the amino acid position corresponding to position A180 of SEQ ID NO: 1. In some embodiments, a mutated PPX protein includes a mutation at the amino acid position corresponding to position P185 of SEQ ID NO: 1. In some embodiments, a mutated PPX protein includes a mutation at the amino acid position corresponding to position A220 of SEQ ID NO: 1. In some embodiments, a mutated PPX protein includes a mutation at the amino acid position corresponding to position L226 of SEQ ID NO: 1. In some embodiments, a mutated PPX protein includes a mutation at the amino acid position corresponding to position M228 of SEQ ID NO: 1. In some embodiments, a mutated PPX protein includes a mutation at the amino acid position corresponding to position S244 of SEQ ID NO: 1. In some embodiments, a mutated PPX protein includes a mutation at the amino acid position corresponding to position K272 of SEQ ID NO: 1. In some embodiments, a mutated PPX protein includes a mutation at the amino acid position correposnding to S305 of SEQ ID NO: 1. In some embodiments, a mutated PPX protein includes a mutation at the amino acid position corresponding to position S332 of SEQ ID NO: 1. In some embodiments, a mutated PPX protein includes a mutation at the amino acid position corresponding to position L357 of SEQ ID NO: 1. In some embodiments, a mutated PPX protein includes a mutation at the amino acid position corresponding to position S359 of SEQ ID NO: 1. In some embodiments, a mutated PPX protein includes a mutation at the amino acid position corresponding to position L393 of SEQ ID NO: 1. In some embodiments, a mutated PPX protein includes a mutation at the amino acid position corresponding to position L403 of SEQ ID NO: 1. In some embodiments, a mutated PPX protein includes a mutation at the amino acid position corresponding to position L424 of SEQ ID NO: 1. In some embodiments, a mutated PPX protein includes a mutation at the amino acid position corresponding to position Y426 of SEQ ID NO: 1. In some embodiments, a mutated PPX protein includes a mutation at the amino acid position corresponding to position F478 of SEQ ID NO: 1. In some embodiments, a mutated PPX protein includes a mutation at the amino acid position corresponding to position S525 of SEQ ID NO: 1.

In some embodiments, a mutated PPX protein includes two or more mutations, at least one mutation of which is at the amino acid position corresponding to a position selected from the group consisting of G52, N85, R144, F145, A180, P185, A220, L226, M228, S244, Q272, S305, S332, L357, K359, L393, L403, L424, Y426, F478 and I525 of SEQ ID NO: 1. In some embodiments, a PPX protein is a paralog of *Arabidopsis thaliana* PPX protein (for example the PPX protein may be a potato PPX protein) and the PPX protein has two or more mutations and has one or more of: (1) an N at the position corresponding to position 52 of SEQ ID NO: 1, wherein the N is substituted with an amino acid other than an N; (2) a K at the position corresponding to position 272 of SEQ ID NO: 1, wherein the K is substituted with an amino acid other than a K; (3) an S at the position corresponding to position 359 of SEQ ID NO: 1, wherein the S is substituted with an amino acid other than an S; and/or (4) an S at the position corresponding to position 525 of SEQ ID NO: 1, wherein the S is substituted with an amino acid other than an S. In such embodiments, a mutated PPX protein includes two or more mutations, at least one mutation of which is at the amino acid position corresponding to a position selected from the group consisting of N52, N85, R144, F145, A180, P185, A220, L226, M228, S244, K272, S305, S332, L357, S359, L393, L403, L424, Y426, F478 and S525 of SEQ ID NO: 1. In some embodiments, a mutated PPX protein includes three or more mutations, at least one mutation of which is at the amino acid position corresponding to a position selected from the group consisting of G52, N85, R144, F145, A180, P185, A220, L226, M228, S244, Q272, S305, S332, L357, K359, L393, L403, L424, Y426, F478 and I525 of SEQ ID NO: 1. In some embodiments, a PPX protein is a paralog of *Arabidopsis thaliana* PPX protein (for example the PPX protein may be a potato PPX protein) and the PPX protein has three or more mutations and has one or more of: (1) an N at the position corresponding to position 52 of SEQ ID NO: 1, wherein the N is substituted with an amino acid other than an N; (2) a K at the position corresponding to position 272 of SEQ ID NO: 1, wherein the K is substituted with an amino acid other than a K; (3) an S at the position corresponding to position 359 of SEQ ID NO: 1, wherein the S is substituted with an amino acid other than an S; and/or (4) an S at the position corresponding to position 525 of SEQ ID NO: 1, wherein the S is substituted with an amino acid other than an S. In such embodiments, a mutated PPX protein includes three or more mutations, at least one mutation of which is at the amino acid position corresponding to a position selected from the group consisting of N52, N85, R144, F145, A180, P185, A220, L226, M228, S244, K272, S305, S332, L357, S359, L393, L403, L424, Y426, F478 and S525 of SEQ ID NO: 1.

In conjunction with any of the aspects, embodiments, methods and/or compositions disclosed herein, the mutated PPX protein may include one or more mutations selected from the mutations shown in Table 1.

**Table 1: Amino acid mutations in the Arabidopsis thaliana PPX protein.**

| | | | | | | | |
|---|---|---|---|---|---|---|---|
| G52K | F145Y | A220I | M228L | S332C | L393S | Y426C | Y426R |
| N85D | A180T | A220L | S244G | L3571 | L393V | Y426F | Y426T |
| R144C | P185H | A220T | S244T | K359R | L403R | Y426H | Y426V |
| R144H | P185R | A220V | Q272F | K359T | L403S | Y4261 | F478S |
| F145L | A220C | L226M | S305L | L393M | L424S | Y426L | I525T |
| E111V | L147V | S193T | A230F | P271R | L360K | L455V | I485E |
| G130N | F165N | R199L | R256H | E311R | A366E | K457V | K490N |
| D139H | P185Y | V206F | R256S | T318G | K438S | V470S | L503F |
| P143R | E192D | Y219S | K270E | S332L | E440K | V470Y | V508T |
| R144L | E192K | K229Q | K270Q | L360D | V444I | D484A | |

In conjunction with any of the aspects, embodiments, methods and/or compositions disclosed herein, the one or more mutations in a mutated PPX gene may encode a mutated PPX protein having one or more mutations, two or more mutations, or three or more mutations selected from the group consisting of a glycine to lysine at a position corresponding to position 52 of SEQ ID NO: 1; an asparagine to aspartic acid at a position corresponding to position 85 of SEQ ID NO: 1; a glutamic acid to valine at a position corresponding to position 111 of SEQ ID NO: 1;a glycine to asparagine at a position corresponding to position 130 of SEQ ID NO: 1; an aspartic acid to histidine at a position corresponding to position 139 of SEQ ID NO: 1; a proline to arginine at a position corresponding to position 143 of SEQ ID NO: 1; an arginine to cysteine at a position corresponding to position 144 of SEQ ID NO: 1; an arginine to histidine at a position corresponding to position 144 of SEQ ID NO: 1; an arginine to leucine at a position corresponding to position 144 of SEQ ID NO: 1; a phenylalanine to leucine at a position corresponding to position 145 of SEQ ID NO: 1, a phenylalanine to tyrosine at a position corresponding to position 145 of SEQ ID NO: 1; a leucine to valine at a position corresponding to position 147 of SEQ ID NO: 1; a phenylalanine to asparagine at a position corresponding to position 165 of SEQ ID NO: 1; an alanine to threonine at a position corresponding to position 180 of SEQ ID NO: 1; a proline to histidine at a position corresponding to position 185 of SEQ ID NO: 1; a proline to arginine at a position corresponding to position 185 of SEQ ID NO: 1; a proline to tyrosine at a position corresponding to position 185 of SEQ ID NO: 1; a glutamic acid to aspartic acid at a position corresponding to position 192 of SEQ ID NO: 1; a glutamic acid to lysine at a position corresponding to position 192 of SEQ ID NO: 1; a serine to threonine at a position corresponding to position 193 of SEQ ID NO: 1; an arginine to leucine at a position corresponding to position 199 of SEQ ID NO: 1; a valine to phenylalanine at a position corresponding to position 206 of SEQ ID NO: 1; a tyrosine to serine at a position corresponding to position 219 of SEQ ID NO: 1; an alanine to cysteine at a position corresponding to position 220 of SEQ ID NO: 1; an alanine to isoleucine at a position corresponding to position 220 of SEQ ID NO: 1; an alanine to leucine at a position corresponding to position 220 of SEQ ID NO: 1; an alanine to threonine at a position corresponding to position 220 of SEQ ID NO: 1; an alanine to valine at a position corresponding to position 220 of SEQ ID NO: 1; a leucine to methionine at a position corresponding to position 226 of SEQ ID NO: 1; a methionine to leucine at a position corresponding to position 228 of SEQ ID NO: 1; a lysine to glutamine at a position corresponding to position 229 of SEQ ID NO: 1; an alanine to phenylalanine at a position corresponding to position 230 of SEQ ID NO: 1; a serine to glycine at a position corresponding to position 244 of SEQ ID NO: 1; a serine to threonine at a position corresponding to position 244 of SEQ ID NO: 1; an arginine to histidine at a position corresponding to position 256 of SEQ ID NO: 1; an arginine to serine at a position corresponding to position 256 of SEQ ID NO: 1; a lysine to glutamic acid at a position corresponding to position 270; a lysine to glutamine at a position corresponding to position 270; a proline to arginine at a position corresponding to position 271 of SEQ ID NO: 1; a glutamine to phenylalanine at a position corresponding to position 272 of SEQ ID NO: 1; a serine to leucine at a position corresponding to position 305 of SEQ ID NO: 1; a glutamic acid to arginine at a position corresponding to position 311 of SEQ ID NO: 1; a threonine to glycine at a position corresponding to position 316 of SEQ ID NO: 1; a threonine to glycine at a position corresponding to position 318 of SEQ ID NO: 1; a serine to cysteine at a position corresponding to position 332 of SEQ ID NO: 1; a serine to leucine at a position corresponding to position 332 of SEQ ID NO: 1; a leucine to isoleucine at a position corresponding to position 357 of SEQ ID NO: 1; a lysine to arginine at a position corresponding to position 359 of SEQ ID NO: 1; a lysine to threonine at a position corresponding to position 359 of SEQ ID NO: 1; a leucine to lysine at a position corresponding to position 360 of SEQ ID NO 1; a leucine to aspartic acid at a position corresponding to position 360 of SEQ ID NO: 1; an alanine to glutamic acid at a position corresponding to position 366 of SEQ ID NO: 1; a leucine to methionine at a position corresponding to position 393 of SEQ ID NO: 1; a leucine to serine at a position corresponding to position 393 of SEQ ID NO: 1; a leucine to valine at a position corresponding to position 393 of SEQ ID NO: 1; a leucine to arginine at a position corresponding to position 403 of SEQ ID NO: 1; a leucine to serine at a position corresponding to position 403 of SEQ ID NO: 1; a leucine to serine at a position corresponding to position 424 of SEQ ID NO: 1; a tyrosine to cysteine at a position corresponding to position 426 of SEQ ID NO: 1; a tyrosine to phenylalanine at a position corresponding to position 426 of SEQ ID NO: 1; a tyrosine to histidine at a position corresponding to position 426 of SEQ ID NO: 1; a tyrosine to isoleucine at a position corresponding to position 426 of SEQ ID NO: 1; a tyrosine to leucine at a position corresponding to position 426 of SEQ ID NO: 1; a tyrosine to arginine at a position corresponding to position 426 of SEQ ID NO: 1; a tyrosine to threonine at a position corresponding to position 426 of SEQ ID NO: 1; a tyrosine to valine at a position corresponding to position 426 of SEQ ID NO: 1; a lysine to serine at a position corresponding to position 438 of SEQ ID NO: 1; a glutamic acid to lysine at a position corresponding to position 440 of SEQ ID NO: 1; a valine to isoleucine at a position corresponding to position 444 of SEQ ID NO: 1; a leucine to valine at a position corresponding to position 455 of SEQ ID NO: 1; a lysine to valine at a position corresponding to position 457 of SEQ ID NO: 1; a valine to serine at a position corresponding to position 470 of SEQ ID NO: 1; a valine to tyrosine at a position corresponding to position 470 of SEQ ID NO: 1; a phenylalanine to serine at a position corresponding to position 478 of SEQ ID NO: 1; a phenylalanine to glycine at a position corresponding to position 483 of SEQ ID NO: 1; an aspartic acid to alanine at a position corresponding to position 484 of SEQ ID NO: 1; an isoleucine to glutamic acid at a position corresponding to position 485 of SEQ ID NO: 1; a lysine to asparagine at a position corresponding to position 490 of SEQ ID NO: 1; a leucine to phenylalanine at a position corresponding to position 503 of SEQ ID NO: 1; a valine to threonine at a position corresponding to position 508 of SEQ ID NO: 1; and an isoleucine to threonine at a position corresponding to position 525 of SEQ ID NO: 1.

In conjunction with any of the aspects, embodiments, methods and/or compositions disclosed herein, a mutated PPX gene may encode a mutated PPX protein that includes an glycine to lysine at a position corresponding to position 52 of SEQ ID NO: 1. In some embodiments, a mutated PPX gene encodes a mutated PPX protein that includes an asparagine to aspartic acid at a position corresponding to position 85 of SEQ ID NO: 1. In some embodiments, a mutated PPX gene encodes a mutated PPX protein that includes a glutamic acid to valine at a position corresponding to position 111 of SEQ ID NO: 1. In some embodiments, a mutated PPX gene encodes a mutated PPX protein that includes a glycine to asparagine at a position corresponding to position 130 of SEQ ID NO: 1. In some embodiments, a mutated PPX gene encodes a mutated PPX protein that includes an aspartic acid to histidine at a position corresponding to position 139 of SEQ ID NO: 1. In some embodiments, a mutated PPX gene encodes a mutated PPX protein that includes a proline to arginine at a position corresponding to position 143 of SEQ ID NO: 1. In some embodiments, a mutated PPX gene encodes a mutated PPX protein that includes an arginine to cysteine at a position corresponding to position 144 of SEQ ID NO: 1. In some embodiments, a mutated PPX gene encodes a mutated PPX protein that includes an arginine to leucine at a position corresponding to position 144 of SEQ ID NO: 1. In some embodiments, a mutated PPX gene encodes a mutated PPX protein that includes an arginine to histidine at a position corresponding to position 144 of SEQ ID NO: 1. In some embodiments, a mutated PPX gene encodes a mutated PPX protein that includes a phenylalanine to leucine at a position corresponding to position 145 of SEQ ID NO: 1. In some embodiments, a mutated PPX gene encodes a mutated PPX protein that includes a phenylalanine to tyrosine at a position corresponding to position 145 of SEQ ID NO: 1. In some embodiments, a mutated PPX gene encodes a mutated PPX protein that includes a leucine to valine at a position corresponding to position 147 of SEQ ID NO: 1. In some embodiments, a mutated PPX gene encodes a mutated PPX protein that includes a phenylalanine to asparagine at a position corresponding to position 165 of SEQ ID NO: 1. In some embodiments, a mutated PPX gene encodes a mutated PPX protein that includes an alanine to threonine at a position corresponding to position 180 of SEQ ID NO: 1. In some embodiments, a mutated PPX gene encodes a mutated PPX protein that includes a proline to histidine at a position corresponding to position 185 of SEQ ID NO: 1. In some embodiments, a mutated PPX gene encodes a mutated PPX protein that includes a proline to arginine at a position corresponding to position 185 of SEQ ID NO: 1. In some embodiments, a mutated PPX gene encodes a mutated PPX protein that includes a proline to tyrosine at a position corresponding to position 185 of SEQ ID NO: 1. In some embodiments, a mutated PPX gene encodes a mutated PPX protein that includes a glutamic acid to aspartic acid at a position corresponding to position 192 of SEQ ID NO: 1. In some embodiments, a mutated PPX gene encodes a mutated PPX protein that includes a glutamic acid to lysine at a position corresponding to position 192 of SEQ ID NO: 1. In some embodiments, a mutated PPX gene encodes a mutated PPX protein that includes a serine to threonine at a position corresponding to position 193 of SEQ ID NO: 1. In some embodiments, a mutated PPX gene encodes a mutated PPX protein that includes an arginine to leucine at a position corresponding to position 199 of SEQ ID NO: 1. In some embodiments, a mutated PPX gene encodes a mutated PPX protein that includes a valine to phenylalanine at a position corresponding to position 206 of SEQ ID NO: 1. In some embodiments, a mutated PPX gene encodes a mutated PPX protein that includes a tyrosine to serine at a position corresponding to position 219 of SEQ ID NO: 1. In some embodiments, a mutated PPX gene encodes a mutated PPX protein that includes an alanine to cysteine at a position corresponding to position 220 of SEQ ID NO: 1. In some embodiments, a mutated PPX gene encodes a mutated PPX protein that includes an alanine to isoleucine at a position corresponding to position 220 of SEQ ID NO: 1. In some embodiments, a mutated PPX gene encodes a mutated PPX protein that includes an alanine to leucine at a position corresponding to position 220 of SEQ ID NO: 1. In some embodiments, a mutated PPX gene encodes a mutated PPX protein that includes an alanine to threonine at a position corresponding to position 220 of SEQ ID NO: 1. In some embodiments, a mutated PPX gene encodes a mutated PPX protein that includes an alanine to valine at a position corresponding to position 220 of SEQ ID NO: 1. In some embodiments, a mutated PPX gene encodes a mutated PPX protein that includes a leucine to methionine at a position corresponding to position 226 of SEQ ID NO: 1. In some embodiments, a mutated PPX gene encodes a mutated PPX protein that includes a methionine to leucine at a position corresponding to position 228 of SEQ ID NO: 1. In some embodiments, a mutated PPX gene encodes a mutated PPX protein that includes a lysine to glutamine at a position corresponding to position 229 of SEQ ID NO: 1. In some embodiments, a mutated PPX gene encodes a mutated PPX protein that includes an alanine to phenylalanine at a position corresponding to position 230 of SEQ ID NO: 1. In some embodiments, a mutated PPX gene encodes a mutated PPX protein that includes a serine to glycine at a position corresponding to position 244 of SEQ ID NO: 1. In some embodiments, a mutated PPX gene encodes a mutated PPX protein that includes a serine to threonine at a position corresponding to position 244 of SEQ ID NO: 1. In some embodiments, a mutated PPX gene encodes a mutated PPX protein that includes an arginine to histidine at a position corresponding to position 256 of SEQ ID NO: 1. In some embodiments, a mutated PPX gene encodes a mutated PPX protein that includes an arginine to serine at a position corresponding to position 256 of SEQ ID NO: 1. In some embodiments, a mutated PPX gene encodes a mutated PPX protein that includes a lysine to glutamic acid at a position corresponding to position 270. In some embodiments, a mutated PPX gene encodes a mutated PPX protein that includes a lysine to glutamine at a position corresponding to position 270. In some embodiments, a mutated PPX gene encodes a mutated PPX protein that includes a proline to arginine at a position corresponding to position 271 of SEQ ID NO: 1. In some embodiments, a mutated PPX gene encodes a mutated PPX protein that includes a glutamine to phenylalanine at a position corresponding to position 272 of SEQ ID NO: 1. In some embodiments, a mutated PPX gene encodes a mutated PPX protein that includes a serine to threonine at a position corresponding to position 244 of SEQ ID NO: 1. In some embodiments, a mutated PPX gene encodes a mutated PPX protein that includes a serine to leucine at a position corresponding to position 305 of SEQ ID NO: 1. In some embodiments, a mutated PPX gene encodes a mutated PPX protein that includes a glutamic acid to arginine at a position corresponding to position 311 of SEQ ID NO: 1. In some embodiments, a mutated PPX gene encodes a mutated PPX protein that includes a threonine to glycine at a position corresponding to position 316 of SEQ ID NO: 1. In some embodiments, a mutated PPX gene encodes a mutated PPX protein that includes a threonine to glycine at a position corresponding to position 318 of SEQ ID NO: 1. In some embodiments, a mutated PPX gene encodes a mutated PPX protein that includes a serine to cysteine at a position corresponding to position 332 of SEQ ID NO: 1. In some embodiments, a mutated PPX gene encodes a mutated PPX protein that includes a serine to leucine at a position corresponding to position 332 of SEQ ID NO: 1. In some embodiments, a mutated PPX gene encodes a mutated PPX protein that includes a leucine to isoleucine at a position corresponding to position 357 of SEQ ID NO: 1. In some embodiments, a mutated PPX gene encodes a mutated PPX protein that includes a lysine to arginine at a position corresponding to position 359 of SEQ ID NO: 1. In some embodiments, a mutated PPX gene encodes a mutated PPX protein that includes a lysine to threonine at a position corresponding to position 359 of SEQ ID NO: 1. In some embodiments, a mutated PPX gene encodes a mutated PPX protein that includes a leucine to lysine at a position corresponding to position 360 of SEQ ID NO 1. In some embodiments, a mutated PPX gene encodes a mutated PPX protein that includes a leucine to aspartic acid at a position corresponding to position 360 of SEQ ID NO: 1. In some embodiments, a mutated PPX gene encodes a mutated PPX protein that includes an alanine to glutamic acid at a position corresponding to position 366 of SEQ ID NO: 1. In some embodiments, a mutated PPX gene encodes a mutated PPX protein that includes a leucine to methionine at a position corresponding to position 393 of SEQ ID NO: 1. In some embodiments, a mutated PPX gene encodes a mutated PPX protein that includes leucine to serine at a position corresponding to position 393 of SEQ ID NO: 1. In some embodiments, a mutated PPX gene encodes a mutated PPX protein that includes a leucine to valine at a position corresponding to position 393 of SEQ ID NO: 1. In some embodiments, a mutated PPX gene encodes a mutated PPX protein that includes a leucine to arginine at a position corresponding to position 403 of SEQ ID NO: 1. In some embodiments, a mutated PPX gene encodes a mutated PPX protein that includes a leucine to serine at a position corresponding to position 403 of SEQ ID NO: 1. In some embodiments, a mutated PPX gene encodes a mutated PPX protein that includes a leucine to serine at a position corresponding to position 424 of SEQ ID NO: 1. In some embodiments, a mutated PPX gene encodes a mutated PPX protein that includes a tyrosine to cysteine at a position corresponding to position 426 of SEQ ID NO: 1. In some embodiments, a mutated PPX gene encodes a mutated PPX protein that includes a tyrosine to phenylalanine at a position corresponding to position 426 of SEQ ID NO: 1. In some embodiments, a mutated PPX gene encodes a mutated PPX protein that includes a tyrosine to histidine at a position corresponding to position 426 of SEQ ID NO: 1. In some embodiments, a mutated PPX gene encodes a mutated PPX protein that includes a tyrosine to isoleucine at a position corresponding to position 426 of SEQ ID NO: 1. In some embodiments, a mutated PPX gene encodes a mutated PPX protein that includes a tyrosine to leucine at a position corresponding to position 426 of SEQ ID NO: 1. In some embodiments, a mutated PPX gene encodes a mutated PPX protein that includes a tyrosine to arginine at a position corresponding to position 426 of SEQ ID NO: 1. In some embodiments, a mutated PPX gene encodes a mutated PPX protein that includes a tyrosine to threonine at a position corresponding to position 426 of SEQ ID NO: 1. In some embodiments, a mutated PPX gene encodes a mutated PPX protein that includes a tyrosine to valine at a position corresponding to position 426 of SEQ ID NO: 1. In some embodiments, a mutated PPX gene encodes a mutated PPX protein that includes a lysine to serine at a position corresponding to position 438 of SEQ ID NO: 1. In some embodiments, a mutated PPX gene encodes a mutated PPX protein that includes a glutamic acid to lysine at a position corresponding to position 440 of SEQ ID NO: 1. In some embodiments, a mutated PPX gene encodes a mutated PPX protein that includes a valine to isoleucine at a position corresponding to position 444 of SEQ ID NO: 1. In some embodiments, a mutated PPX gene encodes a mutated PPX protein that includes a leucine to valine at a position corresponding to position 455 of SEQ ID NO: 1. In some embodiments, a mutated PPX gene encodes a mutated PPX protein that includes a lysine to valine at a position corresponding to position 457 of SEQ ID NO: 1. In some embodiments, a mutated PPX gene encodes a mutated PPX protein that includes a valine to serine at a position corresponding to position 470 of SEQ ID NO: 1. In some embodiments, a mutated PPX gene encodes a mutated PPX protein that includes a valine to tyrosine at a position corresponding to position 470 of SEQ ID NO: 1. In some embodiments, a mutated PPX gene encodes a mutated PPX protein that includes a phenylalanine to serine at a position corresponding to position 478 of SEQ ID NO: 1. In some embodiments, a mutated PPX gene encodes a mutated PPX protein that includes a phenylalanine to glycine at a position corresponding to position 483 of SEQ ID NO: 1. In some embodiments, a mutated PPX gene encodes a mutated PPX protein that includes an aspartic acid to alanine at a position corresponding to position 484 of SEQ ID NO: 1. In some embodiments, a mutated PPX gene encodes a mutated PPX protein that includes an isoleucine to glutamic acid at a position corresponding to position 485 of SEQ ID NO: 1. In some embodiments, a mutated PPX gene encodes a mutated PPX protein that includes a lysine to asparagine at a position corresponding to position 490 of SEQ ID NO: 1. In some embodiments, a mutated PPX gene encodes a mutated PPX protein that includes a leucine to phenylalanine at a position corresponding to position 503 of SEQ ID NO: 1. In some embodiments, a mutated PPX gene encodes a mutated PPX protein that includes a valine to threonine at a position corresponding to position 508 of SEQ ID NO: 1. In some embodiments, a mutated PPX gene encodes a mutated PPX protein that includes an isoleucine to threonine at a position corresponding to position 525 of SEQ ID NO: 1.

**Table 2: Summary of nucleotide/codon mutations in the Arabidopsis plastidal PPX gene that lead to amino acid changes that confer tolerance to PPX inhibitors. Position numbers based on numbering of Arabidopsis plastidal PPX gene number At4g01690 (SEQ ID NO: 1).**

| AA mtn | NA mtn | | AA mtn | NA mtn | | AA mtn | NA mtn | | AA mtn | NA mtn |
|---|---|---|---|---|---|---|---|---|---|---|
| **G52K** | **GGG → AAA** | | **A220I** | **GC**T → **AT**T | | **S332C** | **T**CT → **T**GT | | **Y426C** | T**A**C → T**G**C |
| **N85D** | **A**AT → **G**AT | | **A220L** | **GC**T → **CT**T | | **L357I** | **C**TC → **A**TC | | **Y426F** | **T**AC → **TT**C |
| **R144C** | **AGG → TGC** | | **A220T** | **GC**T → **A**CT | | **K359R** | A**A**A → A**G**A | | **Y426H** | **T**AC → **C**AC |
| | **AGG → TGT** | | | | | | | | | |
| **R144H** | **AGG → CAC** | | **A220V** | **GC**T → **GT**T | | **K359T** | A**A**A → A**C**T | | **Y426I** | **TA**C → **AT**C |
| | **AGG → CAT** | | | | | | | | | |
| **F145L** | **T**TT → **C**TT | | **L226M** | **G**TG → **A**TG | | **L393M** | **T**TG → **A**TG | | **Y426L** | T**AC** → T**TA** |
| | | | | | | | | | | **TA**C → **CT**C |
| **F145Y** | T**T**T → T**A**T | | **M228L** | **A**TG → **C**TG | | **L393S** | T**T**G → T**C**G | | **Y426R** | **TA**C → **CG**C |
| **A180T** | **G**CA → **A**CA | | **S244G** | **A**GC → **G**GC | | **L393V** | **T**TG → **G**TG | | **Y426T** | **TA**C → **AC**C |
| **P185H** | C**CG** → C**AC** | | **S244T** | A**G**C → A**C**C | | **L403R** | **TT**A → **CG**A | | **Y426V** | **TA**C → **GT**C |
| | C**CG** → C**AT** | | | | | | | | | |
| **P185R** | C**C**G → C**G**G | | **Q272F** | **C**A**G** → **TTC** | | **L403S** | **T**TA → **T**CA | | **F478S** | T**T**T → T**C**T |
| | | | | **C**A**G** → **TTT** | | | | | | |
| **A220C** | **GC**T → T**G**T | | **S305L** | **TC**A → **TT**A | | **L424S** | T**T**G → T**C**G | | **S525T** | A**T**T → A**C**T |

| | | | | | | | | | | |
|---|---|---|---|---|---|---|---|---|---|---|
| * "AA mtn" refers to amino acid mutation; "NA mtn" refers to nucleic acid mutation | | | | | | | | | | |

In conjunction with any of the aspects, embodiments, methods and/or compositions disclosed herein, a mutated PPX gene may include a GGG → AAA which encodes a mutated PPX protein that includes an glycine to lysine at a position corresponding to position 52 of SEQ ID NO: 1. In some embodiments, a mutated PPX gene includes a AAT → GAT nucleic acid mutation that encodes a mutated PPX protein that includes an asparagine to aspartic acid at a position corresponding to position 85 of SEQ ID NO: 1. In some embodiments, a mutated PPX gene includes a AGG → TGC or TGT nucleic acid mutation that encodes a mutated PPX protein that includes an arginine to cysteine at a position corresponding to position 144 of SEQ ID NO: 1. In some embodiments, a mutated PPX gene includes a AGG → CAC or CAT nucleic acid mutation that encodes a mutated PPX protein that includes an arginine to histidine at a position corresponding to position 144 of SEQ ID NO: 1. In some embodiments, a mutated PPX gene includes a TTT → CTT nucleic acid mutation that encodes a mutated PPX protein that includes a phenylalanine to leucine at a position corresponding to position 145 of SEQ ID NO: 1. In some embodiments, a mutated PPX gene includes a TTT → TAT nucleic acid mutation that encodes a mutated PPX protein that includes a phenylalanine to tyrosine at a position corresponding to position 145 of SEQ ID NO: 1. In some embodiments, a mutated PPX gene includes a GCA → ACA nucleic acid mutation that encodes a mutated PPX protein that includes an alanine to threonine at a position corresponding to position 180 of SEQ ID NO: 1. In some embodiments, a mutated PPX gene includes a CCG → CAC or CAT nucleic acid mutation that encodes a mutated PPX protein that includes a proline to arginine at a position corresponding to position 185 of SEQ ID NO: 1. In some embodiments, a mutated PPX gene includes a CCG → CGT nucleic acid mutation that encodes a mutated PPX protein that includes a proline to histidine at a position corresponding to position 185 of SEQ ID NO: 1. In some embodiments, a mutated PPX gene includes a CCG → CGG nucleic acid mutation that encodes a mutated PPX protein that includes a proline to arginine at a position corresponding to position 185 of SEQ ID NO: 1. In some embodiments, a mutated PPX gene includes a GCT → TGT nucleic acid mutation that encodes a mutated PPX protein that includes an alanine to cysteine at a position corresponding to position 220 of SEQ ID NO: 1. In some embodiments, a mutated PPX gene includes a GCT → ATT nucleic acid mutation that encodes a mutated PPX protein that includes an alanine to isoleucine at a position corresponding to position 220 of SEQ ID NO: 1. In some embodiments, a mutated PPX gene includes a GCT → CTT nucleic acid mutation that encodes a mutated PPX protein that includes an alanine to leucine at a position corresponding to position 220 of SEQ ID NO: 1. In some embodiments, a mutated PPX gene includes a GCT → ACT nucleic acid mutation that encodes a mutated PPX protein that includes an alanine to threonine at a position corresponding to position 220 of SEQ ID NO: 1. In some embodiments, a mutated PPX gene includes a GCT → GTT nucleic acid mutation that encodes a mutated PPX protein that includes an alanine to valine at a position corresponding to position 220 of SEQ ID NO: 1. In some embodiments, a mutated PPX gene includes a GTG → ATG nucleic acid mutation that encodes a mutated PPX protein that includes a leucine to methionine at a position corresponding to position 226 of SEQ ID NO: 1. In some embodiments, a mutated PPX gene includes a ATG → CTG nucleic acid mutation that encodes a mutated PPX protein that includes a methionine to leucine at a position corresponding to position 228 of SEQ ID NO: 1. In some embodiments, a mutated PPX gene includes a AGC → GGC nucleic acid mutation that encodes a mutated PPX protein that includes a serine to glycine at a position corresponding to position 244 of SEQ ID NO: 1. In some embodiments, a mutated PPX gene includes a AGC → ACC nucleic acid mutation that encodes a mutated PPX protein that includes a serine to threonine at a position corresponding to position 244 of SEQ ID NO: 1. In some embodiments, a mutated PPX gene includes a CAG → TTC or TTT nucleic acid mutation that encodes a mutated PPX protein that includes a glutamine to asparagine at a position corresponding to position 272 of SEQ ID NO: 1. In some embodiments, a mutated PPX gene includes a TCA → TTA nucleic acid mutation that encodes a mutated PPX protein that includes a serine to leucine at a position corresponding to position 305 of SEQ ID NO: 1. In some embodiments, a mutated PPX gene includes a TCT → TGT nucleic acid mutation that encodes a mutated PPX protein that includes a serine to cysteine at a position corresponding to position 332 of SEQ ID NO: 1. In some embodiments, a mutated PPX gene includes a CTC → ATC nucleic acid mutation that encodes a mutated PPX protein that includes a leucine to isoleucine at a position corresponding to position 357 of SEQ ID NO: 1. In some embodiments, a mutated PPX gene includes a AAA → AGA nucleic acid mutation that encodes a mutated PPX protein that includes a lysine to arginine at a position corresponding to position 359 of SEQ ID NO: 1. In some embodiments, a mutated PPX gene includes a AAA → ACT nucleic acid mutation that encodes a mutated PPX protein that includes a lysine to threonine at a position corresponding to position 359 of SEQ ID NO: 1. In some embodiments, a mutated PPX gene includes a TTG → ATG nucleic acid mutation that encodes a mutated PPX protein that includes a leucine to methionine at a position corresponding to position 393 of SEQ ID NO: 1. In some embodiments, a mutated PPX gene includes a TTG → TCG nucleic acid mutation that encodes a mutated PPX protein that includes leucine to serine at a position corresponding to position 393 of SEQ ID NO: 1. In some embodiments, a mutated PPX gene includes a TTG → GTG nucleic acid mutation that encodes a mutated PPX protein that includes a leucine to valine at a position corresponding to position 393 of SEQ ID NO: 1. In some embodiments, a mutated PPX gene includes a TTA → CGA nucleic acid mutation that encodes a mutated PPX protein that includes a leucine to arginine at a position corresponding to position 403 of SEQ ID NO: 1. In some embodiments, a mutated PPX gene includes a TTA → TCA nucleic acid mutation that encodes a mutated PPX protein that includes a leucine to serine at a position corresponding to position 403 of SEQ ID NO: 1. In some embodiments, a mutated PPX gene includes a TTG → TCG nucleic acid mutation that encodes a mutated PPX protein that includes a leucine to serine at a position corresponding to position 424 of SEQ ID NO: 1. In some embodiments, a mutated PPX gene includes a TAC → TGC nucleic acid mutation that encodes a mutated PPX protein that includes a tyrosine to cysteine at a position corresponding to position 426 of SEQ ID NO: 1. In some embodiments, a mutated PPX gene includes a TAC → TTC nucleic acid mutation that encodes a mutated PPX protein that includes a tyrosine to phenylalanine at a position corresponding to position 426 of SEQ ID NO: 1. In some embodiments, a mutated PPX gene includes a TAC → CAC nucleic acid mutation that encodes a mutated PPX protein that includes a tyrosine to histidine at a position corresponding to position 426 of SEQ ID NO: 1. In some embodiments, a mutated PPX gene includes a TAC → ATC nucleic acid mutation that encodes a mutated PPX protein that includes a tyrosine to isoleucine at a position corresponding to position 426 of SEQ ID NO: 1. In some embodiments, a mutated PPX gene includes a TAC → TTA or CTC nucleic acid mutation that encodes a mutated PPX protein that includes a tyrosine to leucine at a position corresponding to position 426 of SEQ ID NO: 1. In some embodiments, a mutated PPX gene includes a TAC → CGC nucleic acid mutation that encodes a mutated PPX protein that includes a tyrosine to arginine at a position corresponding to position 426 of SEQ ID NO: 1. In some embodiments, a mutated PPX gene includes a TAC → ACC nucleic acid mutation that encodes a mutated PPX protein that includes a tyrosine to threonine at a position corresponding to position 426 of SEQ ID NO: 1. In some embodiments, a mutated PPX gene includes a TAC → GTC nucleic acid mutation that encodes a mutated PPX protein that includes a tyrosine to valine at a position corresponding to position 426 of SEQ ID NO: 1. In some embodiments, a mutated PPX gene includes a TTT → TCT nucleic acid mutation that encodes a mutated PPX protein that includes a phenylalanine to serine at a position corresponding to position 478 of SEQ ID NO: 1. In some embodiments, a mutated PPX gene includes a ATT → ACT nucleic acid mutation that encodes a mutated PPX protein that includes an isoleucine to threonine at a position corresponding to position 525 of SEQ ID NO: 1.

**Table 3A: Summary of nucleotide/codon mutations in a potato plastidal PPX gene that lead to amino acid changes that confer tolerance to PPX inhibitors. Position numbers are based on numbering of the Arabidopsis plastidal PPX gene number At4g01690 (SEQ ID NO: 1).**

| AA mtn | NA mtn | | AA mtn | NA mtn | | AA mtn | NA mtn | | AA mtn | NA mtn |
|---|---|---|---|---|---|---|---|---|---|---|
| **N52K** | **AAT** → AAA | | **A220I** | **GC**C → **AT**C | | **S332C** | **A**GT → **T**GT | | **Y426C** | T**A**C → T**G**C |
| **N85D** | **A**AT → **G**AT | | **A220L** | **GC**C → **CT**C | | **L357I** | **C**TT → **A**TT | | **Y426F** | **T**AC → **TT**C |
| **R144C** | **C**GC → **T**GC | | **A220T** | **G**CC → **A**CC | | **S359R** | AG**T** → AG**A** | | **Y426H** | **T**AC → **C**AC |
| **R144H** | C**G**C → C**A**C | | **A220V** | **GC**C → **GT**C | | **S359T** | A**G**T → A**C**T | | **Y426I** | **TA**C → **AT**C |
| **F145L** | **T**TT → **C**TT | | **L226M** | **T**TG → **A**TG | | **L393M** | **T**TG → **A**TG | | **Y426L** | T**AC** → T**TA** |
| | | | | | | | | | | **TA**C → **CT**C |
| **F145Y** | T**T**T → T**A**T | | **M228L** | **A**TG → **C**TG | | **L393S** | T**T**G → T**C**G | | **Y426R** | **TA**C → **CG**C |
| **A180T** | **G**CC → **A**CC | | **S244G** | **A**GC → **G**GC | | **L393V** | **T**TG → **G**TG | | **Y426T** | **TA**C → **AC**C |
| **P185H** | C**C**T → C**A**T | | **S244T** | A**G**C → A**C**C | | **L403R** | C**T**A → C**G**A | | **Y426V** | **TA**C → **GT**C |
| **P185R** | C**C**T → C**G**T | | **K272F** | AA**A** → **TTT** | | **L403S** | **CT**A → **TC**A | | **F478S** | T**T**T → T**C**T |
| | | | | AA**A** → **TTC** | | | | | | |
| **A220C** | G**C**C → **TG**C | | **S305L** | **TC**T → **CT**T | | **L424S** | T**T**G → T**C**G | | **S525T** | **T**CT → **A**CT |

| | | | | | | | | | | |
|---|---|---|---|---|---|---|---|---|---|---|
| * "AA mtn" refers to amino acid mutation; "NA mtn" refers to nucleic acid mutation | | | | | | | | | | |

In some embodiments, in conjunction with any of the aspects, embodiments, methods and/or compositions disclosed herein, the one or more mutations in a mutated PPX gene may encode a mutated PPX protein having one or more mutations, two or more mutations, or three or more mutations selected from the group consisting of a asparagine to lysine at a position corresponding to position 52 of SEQ ID NO: 1; an asparagine to aspartic acid at a position corresponding to position 85 of SEQ ID NO: 1; an arginine to cysteine at a position corresponding to position 144 of SEQ ID NO: 1; an arginine to histidine at a position corresponding to position 144 of SEQ ID NO: 1; a phenylalanine to leucine at a position corresponding to position 145 of SEQ ID NO: 1, a phenylalanine to tyrosine at a position corresponding to position 145 of SEQ ID NO: 1; an alanine to threonine at a position corresponding to position 180 of SEQ ID NO: 1; a proline to histidine at a position corresponding to position 185 of SEQ ID NO: 1; a proline to arginine at a position corresponding to position 185 of SEQ ID NO: 1; an alanine to cysteine at a position corresponding to position 220 of SEQ ID NO: 1; an alanine to isoleucine at a position corresponding to position 220 of SEQ ID NO: 1; an alanine to leucine at a position corresponding to position 220 of SEQ ID NO: 1; an alanine to threonine at a position corresponding to position 220 of SEQ ID NO: 1; an alanine to valine at a position corresponding to position 220 of SEQ ID NO: 1; a leucine to methionine at a position corresponding to position 226 of SEQ ID NO: 1; a methionine to leucine at a position corresponding to position 228 of SEQ ID NO: 1; a serine to glycine at a position corresponding to position 244 of SEQ ID NO: 1; a serine to threonine at a position corresponding to position 244 of SEQ ID NO: 1; a lysine to phenylalanine at a position corresponding to position 272 of SEQ ID NO: 1; a serine to leucine at a position corresponding to position 305 of SEQ ID NO: 1; a serine to cysteine at a position corresponding to position 332 of SEQ ID NO: 1; a leucine to isoleucine at a position corresponding to position 357 of SEQ ID NO: 1; a serine to arginine at a position corresponding to position 359 of SEQ ID NO: 1; a serine to threonine at a position corresponding to position 359 of SEQ ID NO: 1; a leucine to methionine at a position corresponding to position 393 of SEQ ID NO: 1; a leucine to serine at a position corresponding to position 393 of SEQ ID NO: 1; a leucine to valine at a position corresponding to position 393 of SEQ ID NO: 1; a leucine to arginine at a position corresponding to position 403 of SEQ ID NO: 1; a leucine to serine at a position corresponding to position 403 of SEQ ID NO: 1; a leucine to serine at a position corresponding to position 424 of SEQ ID NO: 1; a tyrosine to cysteine at a position corresponding to position 426 of SEQ ID NO: 1; a tyrosine to phenylalanine at a position corresponding to position 426 of SEQ ID NO: 1; a tyrosine to histidine at a position corresponding to position 426 of SEQ ID NO: 1; a tyrosine to isoleucine at a position corresponding to position 426 of SEQ ID NO: 1; a tyrosine to leucine at a position corresponding to position 426 of SEQ ID NO: 1; a tyrosine to arginine at a position corresponding to position 426 of SEQ ID NO: 1; a tyrosine to threonine at a position corresponding to position 426 of SEQ ID NO: 1; a tyrosine to valine at a position corresponding to position 426 of SEQ ID NO: 1; a phenylalanine to serine at a position corresponding to position 478 of SEQ ID NO: 1; and a isoleucine to threonine at a position corresponding to position 525 of SEQ ID NO: 1.

In some embodiments, in conjunction with any of the aspects, embodiments, methods and/or compositions disclosed herein, a mutated PPX gene may encode a mutated PPX protein that includes an asparagine to lysine at a position corresponding to position 52 of SEQ ID NO: 1. In some embodiments, a mutated PPX gene encodes a mutated PPX protein that includes an asparagine to aspartic acid at a position corresponding to position 85 of SEQ ID NO: 1. In some embodiments, a mutated PPX gene encodes a mutated PPX protein that includes an arginine to cysteine at a position corresponding to position 144 of SEQ ID NO: 1. In some embodiments, a mutated PPX gene encodes a mutated PPX protein that includes an arginine to histidine at a position corresponding to position 144 of SEQ ID NO: 1. In some embodiments, a mutated PPX gene encodes a mutated PPX protein that includes a phenylalanine to leucine at a position corresponding to position 145 of SEQ ID NO: 1. In some embodiments, a mutated PPX gene encodes a mutated PPX protein that includes a phenylalanine to tyrosine at a position corresponding to position 145 of SEQ ID NO: 1. In some embodiments, a mutated PPX gene encodes a mutated PPX protein that includes an alanine to threonine at a position corresponding to position 180 of SEQ ID NO: 1. In some embodiments, a mutated PPX gene encodes a mutated PPX protein that includes a proline to histidine at a position corresponding to position 185 of SEQ ID NO: 1. In some embodiments, a mutated PPX gene encodes a mutated PPX protein that includes a proline to arginine at a position corresponding to position 185 of SEQ ID NO: 1. In some embodiments, a mutated PPX gene encodes a mutated PPX protein that includes an alanine to cysteine at a position corresponding to position 220 of SEQ ID NO: 1. In some embodiments, a mutated PPX gene encodes a mutated PPX protein that includes an alanine to isoleucine at a position corresponding to position 220 of SEQ ID NO: 1. In some embodiments, a mutated PPX gene encodes a mutated PPX protein that includes an alanine to leucine at a position corresponding to position 220 of SEQ ID NO: 1. In some embodiments, a mutated PPX gene encodes a mutated PPX protein that includes an alanine to threonine at a position corresponding to position 220 of SEQ ID NO: 1. In some embodiments, a mutated PPX gene encodes a mutated PPX protein that includes an alanine to valine at a position corresponding to position 220 of SEQ ID NO: 1. In some embodiments, a mutated PPX gene encodes a mutated PPX protein that includes a leucine to methionine at a position corresponding to position 226 of SEQ ID NO: 1. In some embodiments, a mutated PPX gene encodes a mutated PPX protein that includes a methionine to leucine at a position corresponding to position 228 of SEQ ID NO: 1. In some embodiments, a mutated PPX gene encodes a mutated PPX protein that includes a serine to glycine at a position corresponding to position 244 of SEQ ID NO: 1. In some embodiments, a mutated PPX gene encodes a mutated PPX protein that includes a serine to threonine at a position corresponding to position 244 of SEQ ID NO: 1. In some embodiments, a mutated PPX gene encodes a mutated PPX protein that includes a lysine to phenylalanine at a position corresponding to position 272 of SEQ ID NO: 1. In some embodiments, a mutated PPX gene encodes a mutated PPX protein that includes a serine to threonine at a position corresponding to position 244 of SEQ ID NO: 1. In some embodiments, a mutated PPX gene encodes a mutated PPX protein that includes a serine to leucine at a position corresponding to position 305 of SEQ ID NO: 1. In some embodiments, a mutated PPX gene encodes a mutated PPX protein that includes a serine to cysteine at a position corresponding to position 332 of SEQ ID NO: 1. In some embodiments, a mutated PPX gene encodes a mutated PPX protein that includes a leucine to isoleucine at a position corresponding to position 357 of SEQ ID NO: 1. In some embodiments, a mutated PPX gene encodes a mutated PPX protein that includes a serine to arginine at a position corresponding to position 359 of SEQ ID NO: 1. In some embodiments, a mutated PPX gene encodes a mutated PPX protein that includes a serine to threonine at a position corresponding to position 359 of SEQ ID NO: 1. In some embodiments, a mutated PPX gene encodes a mutated PPX protein that includes a leucine to methionine at a position corresponding to position 393 of SEQ ID NO: 1. In some embodiments, a mutated PPX gene encodes a mutated PPX protein that includes leucine to serine at a position corresponding to position 393 of SEQ ID NO: 1. In some embodiments, a mutated PPX gene encodes a mutated PPX protein that includes a leucine to valine at a position corresponding to position 393 of SEQ ID NO: 1. In some embodiments, a mutated PPX gene encodes a mutated PPX protein that includes a leucine to arginine at a position corresponding to position 403 of SEQ ID NO: 1. In some embodiments, a mutated PPX gene encodes a mutated PPX protein that includes a leucine to serine at a position corresponding to position 403 of SEQ ID NO: 1. In some embodiments, a mutated PPX gene encodes a mutated PPX protein that includes a leucine to serine at a position corresponding to position 424 of SEQ ID NO: 1. In some embodiments, a mutated PPX gene encodes a mutated PPX protein that includes a tyrosine to cysteine at a position corresponding to position 426 of SEQ ID NO: 1. In some embodiments, a mutated PPX gene encodes a mutated PPX protein that includes a tyrosine to phenylalanine at a position corresponding to position 426 of SEQ ID NO: 1. In some embodiments, a mutated PPX gene encodes a mutated PPX protein that includes a tyrosine to histidine at a position corresponding to position 426 of SEQ ID NO: 1. In some embodiments, a mutated PPX gene encodes a mutated PPX protein that includes a tyrosine to isoleucine at a position corresponding to position 426 of SEQ ID NO: 1. In some embodiments, a mutated PPX gene encodes a mutated PPX protein that includes a tyrosine to leucine at a position corresponding to position 426 of SEQ ID NO: 1. In some embodiments, a mutated PPX gene encodes a mutated PPX protein that includes a tyrosine to arginine at a position corresponding to position 426 of SEQ ID NO: 1. In some embodiments, a mutated PPX gene encodes a mutated PPX protein that includes a tyrosine to threonine at a position corresponding to position 426 of SEQ ID NO: 1. In some embodiments, a mutated PPX gene encodes a mutated PPX protein that includes a tyrosine to valine at a position corresponding to position 426 of SEQ ID NO: 1. In some embodiments, a mutated PPX gene encodes a mutated PPX protein that includes a phenylalanine to serine at a position corresponding to position 478 of SEQ ID NO: 1. In some embodiments, a mutated PPX gene encodes a mutated PPX protein that includes an isoleucine to threonine at a position corresponding to position 525 of SEQ ID NO: 1.

In some embodiments, a mutated PPX gene includes a AAT → AAA nucleic acid mutation that encodes a mutated PPX protein that includes an asparagine to lysine at a position corresponding to position 52 of SEQ ID NO: 7. In some embodiments, a mutated PPX gene includes a AAT → GAT nucleic acid mutation that encodes a mutated PPX protein that includes an asparagine to aspartic acid at a position corresponding to position 85 of SEQ ID NO: 7. In some embodiments, a mutated PPX gene includes a CGC → TGC nucleic acid mutation that encodes a mutated PPX protein that includes an arginine to cysteine at a position corresponding to position 144 of SEQ ID NO: 7. In some embodiments, a mutated PPX gene includes a CGC → CAC nucleic acid mutation that encodes a mutated PPX protein that includes an arginine to histidine at a position corresponding to position 144 of SEQ ID NO: 7. In some embodiments, a mutated PPX gene includes a TTT → CTT nucleic acid mutation that encodes a mutated PPX protein that includes a phenylalanine to leucine at a position corresponding to position 145 of SEQ ID NO: 7. In some embodiments, a mutated PPX gene includes a TTT → TAT nucleic acid mutation that encodes a mutated PPX protein that includes a phenylalanine to tyrosine at a position corresponding to position 145 of SEQ ID NO: 7. In some embodiments, a mutated PPX gene includes a GCC → ACC nucleic acid mutation that encodes a mutated PPX protein that includes an alanine to threonine at a position corresponding to position 180 of SEQ ID NO: 7. In some embodiments, a mutated PPX gene includes a CCT → CAT nucleic acid mutation that encodes a mutated PPX protein that includes a proline to arginine at a position corresponding to position 185 of SEQ ID NO: 7. In some embodiments, a mutated PPX gene includes a CCT → CGT nucleic acid mutation that encodes a mutated PPX protein that includes a proline to histidine at a position corresponding to position 185 of SEQ ID NO: 7. In some embodiments, a mutated PPX gene includes a GCC → TGC nucleic acid mutation that encodes a mutated PPX protein that includes an alanine to cysteine at a position corresponding to position 220 of SEQ ID NO: 7. In some embodiments, a mutated PPX gene includes a GCC → ATC nucleic acid mutation that encodes a mutated PPX protein that includes an alanine to isoleucine at a position corresponding to position 220 of SEQ ID NO: 7. In some embodiments, a mutated PPX gene includes a GCC → CTC nucleic acid mutation that encodes a mutated PPX protein that includes an alanine to leucine at a position corresponding to position 220 of SEQ ID NO: 7. In some embodiments, a mutated PPX gene includes a GCC → ACC nucleic acid mutation that encodes a mutated PPX protein that includes an alanine to threonine at a position corresponding to position 220 of SEQ ID NO: 7. In some embodiments, a mutated PPX gene includes a GCC → GTC nucleic acid mutation that encodes a mutated PPX protein that includes an alanine to valine at a position corresponding to position 220 of SEQ ID NO: 7. In some embodiments, a mutated PPX gene includes a TTG → ATG nucleic acid mutation that encodes a mutated PPX protein that includes a leucine to methionine at a position corresponding to position 226 of SEQ ID NO: 7. In some embodiments, a mutated PPX gene includes a ATG → CTG nucleic acid mutation that encodes a mutated PPX protein that includes a methionine to leucine at a position corresponding to position 228 of SEQ ID NO: 7. In some embodiments, a mutated PPX gene includes a AGC → GGC nucleic acid mutation that encodes a mutated PPX protein that includes a serine to glycine at a position corresponding to position 244 of SEQ ID NO: 7. In some embodiments, a mutated PPX gene includes a AGC → ACC nucleic acid mutation that encodes a mutated PPX protein that includes a serine to threonine at a position corresponding to position 244 of SEQ ID NO: 7. In some embodiments, a mutated PPX gene includes a AAA → AAT nucleic acid mutation that encodes a mutated PPX protein that includes a lysine to phenylalanine at a position corresponding to position 272 of SEQ ID NO: 7. In some embodiments, a mutated PPX gene includes a TCT → CTT nucleic acid mutation that encodes a mutated PPX protein that includes a serine to leucine at a position corresponding to position 305 of SEQ ID NO: 7. In some embodiments, a mutated PPX gene includes a AGT → TGT nucleic acid mutation that encodes a mutated PPX protein that includes a serine to cysteine at a position corresponding to position 332 of SEQ ID NO: 7. In some embodiments, a mutated PPX gene includes a CTT → ATT nucleic acid mutation that encodes a mutated PPX protein that includes a leucine to isoleucine at a position corresponding to position 357 of SEQ ID NO: 7. In some embodiments, a mutated PPX gene includes a AGT → AGA nucleic acid mutation that encodes a mutated PPX protein that includes a serine to arginine at a position corresponding to position 359 of SEQ ID NO: 7. In some embodiments, a mutated PPX gene includes a AGT → ACT nucleic acid mutation that encodes a mutated PPX protein that includes a serine to threonine at a position corresponding to position 359 of SEQ ID NO: 7. In some embodiments, a mutated PPX gene includes a TTG → ATG nucleic acid mutation that encodes a mutated PPX protein that includes a leucine to methionine at a position corresponding to position 393 of SEQ ID NO: 7. In some embodiments, a mutated PPX gene includes a TTG → TCG nucleic acid mutation that encodes a mutated PPX protein that includes leucine to serine at a position corresponding to position 393 of SEQ ID NO: 7. In some embodiments, a mutated PPX gene includes a TTG → GTG nucleic acid mutation that encodes a mutated PPX protein that includes a leucine to valine at a position corresponding to position 393 of SEQ ID NO: 7. In some embodiments, a mutated PPX gene includes a CTA → CGA nucleic acid mutation that encodes a mutated PPX protein that includes a leucine to arginine at a position corresponding to position 403 of SEQ ID NO: 7. In some embodiments, a mutated PPX gene includes a CTA → TCA nucleic acid mutation that encodes a mutated PPX protein that includes a leucine to serine at a position corresponding to position 403 of SEQ ID NO: 7. In some embodiments, a mutated PPX gene includes a TTG → TCG nucleic acid mutation that encodes a mutated PPX protein that includes a leucine to serine at a position corresponding to position 424 of SEQ ID NO: 7. In some embodiments, a mutated PPX gene includes a TAC → TGC nucleic acid mutation that encodes a mutated PPX protein that includes a tyrosine to cysteine at a position corresponding to position 426 of SEQ ID NO: 7. In some embodiments, a mutated PPX gene includes a TAC → AAC nucleic acid mutation that encodes a mutated PPX protein that includes a tyrosine to phenylalanine at a position corresponding to position 426 of SEQ ID NO: 7. In some embodiments, a mutated PPX gene includes a TAC → CAC nucleic acid mutation that encodes a mutated PPX protein that includes a tyrosine to histidine at a position corresponding to position 426 of SEQ ID NO: 7. In some embodiments, a mutated PPX gene includes a TAC → ATC nucleic acid mutation that encodes a mutated PPX protein that includes a tyrosine to isoleucine at a position corresponding to position 426 of SEQ ID NO: 7. In some embodiments, a mutated PPX gene includes a TAC → TTC nucleic acid mutation that encodes a mutated PPX protein that includes a tyrosine to leucine at a position corresponding to position 426 of SEQ ID NO: 7. In some embodiments, a mutated PPX gene includes a TAC → CGC nucleic acid mutation that encodes a mutated PPX protein that includes a tyrosine to arginine at a position corresponding to position 426 of SEQ ID NO: 7. In some embodiments, a mutated PPX gene includes a TAC → ACC nucleic acid mutation that encodes a mutated PPX protein that includes a tyrosine to threonine at a position corresponding to position 426 of SEQ ID NO: 7. In some embodiments, a mutated PPX gene includes a TAC → GTC nucleic acid mutation that encodes a mutated PPX protein that includes a tyrosine to valine at a position corresponding to position 426 of SEQ ID NO: 7. In some embodiments, a mutated PPX gene includes a TTT → TCT nucleic acid mutation that encodes a mutated PPX protein that includes a phenylalanine to serine at a position corresponding to position 478 of SEQ ID NO: 7. In some embodiments, a mutated PPX gene includes a TCT → ACT nucleic acid mutation that encodes a mutated PPX protein that includes an isoleucine to threonine at a position corresponding to position 525 of SEQ ID NO: 7.

**Table 3B: Summary of nucleotide/codon mutations in a potato mitochondrial PPX gene that lead to amino acid changes that confer tolerance to PPX inhibitors. Position numbers are based on numbering of the Solanum tuberosum mitochondrial PPX gene number AJ225108 (SEQ ID NO: 9).**

| **AA mtn** | **NA mtn** | | **AA mtn** | **NA mtn** | | **AA mtn** | **NA mtn** | | **AA mtn** | **NA mtn** |
|---|---|---|---|---|---|---|---|---|---|---|
| D58N | **G**AT -> **A**AT | | S151T | A**G**T -> A**C**T | | K229Q | **A**AG -> **C**AG | | R406K | A**G**G -> A**A**G |
| E64V | G**A**A -> G**T**A | | Q157L | C**A**G -> C**T**G | | K230R | A**A**G -> A**G**G | | K4101 | A**A**A -> A**T**A |
| G74C | **G**GT -> **T**GT | | V164F | **G**TT -> **T**TT | | F283G | G**A**C -> G**G**C | | A423V | G**C**T -> G**T**T |
| G84N | G**G**A -> G**AT** | | D170E | GA**T** -> GA**A** | | A292G | G**C**A -> G**G**A | | C434S | **T**GC -> **A**GC |
| R98C | C**G**C -> C**A**C | | H187Q | **A**AG -> **C**AG | | S296L | T**C**A -> T**T**A | | C434Y | T**G**C -> T**A**C |
| R98H | **C**GC -> **T**GC | | L188F | **C**TT -> **T**TT | | C307S | **T**GT -> **A**GT | | S448A | **T**CA -> **G**CA |
| R98L | C**G**C -> C**T**C | | N195K | AA**T** -> AA**A** | | N324D | **A**AT -> **G**AT | | D451G | G**A**T -> G**G**T |
| N139Y | **CC**T -> **TA**T | | P214H | C**C**T -> C**A**T | | N324K | AA**T** -> AA**A** | | D454N | **G**AC -> **A**AC |
| E150D | GA**A** -> GA**T** | | P214S | **C**CT -> **T**CT | | D330E | GA**T** -> GA**A** | | Y465F | T**A**T -> T**T**T |
| E150K | **G**AA -> **A**AA | | K229E | **A**AG -> **G**AG | | A404S | **G**CC -> **T**CC | | K470T | A**A**G -> A**C**G |
| T500S | A**C**C _> A**G**C | | | | | | | | | |

| | | | | | | | | | | |
|---|---|---|---|---|---|---|---|---|---|---|
| * "AA mtn" refers to amino acid mutation; "NA mtn" refers to nucleic acid mutation | | | | | | | | | | |

In conjunction with any of the aspects, embodiments, methods and/or compositions disclosed herein, a mutated PPX gene may encode a mutated PPX protein that includes an aspartic acid to asparagine at a position corresponding to position 58 of SEQ ID NO: 9. In some embodiments, a mutated PPX gene encodes a mutated PPX protein that includes glutamic acid to valine at a position corresponding to position 64 of SEQ ID NO: 9. In some embodiments, a mutated PPX gene encodes a mutated PPX protein that includes glycine to cysteine at a position corresponding to position 74 of SEQ ID NO: 9. In some embodiments, a mutated PPX gene encodes a mutated PPX protein that includes glycine to asparagine at a position corresponding to position 84 of SEQ ID NO: 9. In some embodiments, a mutated PPX gene encodes a mutated PPX protein that includes leucine to histidine at a position corresponding to position 93 of SEQ ID NO: 9. In some embodiments, a mutated PPX gene encodes a mutated PPX protein that includes lysine to arginine at a position corresponding to position 97 of SEQ ID NO: 9. In some embodiments, a mutated PPX gene encodes a mutated PPX protein that includes arginine to cysteine at a position corresponding to position 98 of SEQ ID NO: 9. In some embodiments, a mutated PPX gene encodes a mutated PPX protein that includes arginine to histidine at a position corresponding to position 98 of SEQ ID NO: 9. In some embodiments, a mutated PPX gene encodes a mutated PPX protein that includes arginine to leucine at a position corresponding to position 98 of SEQ ID NO: 9. In some embodiments, a mutated PPX gene encodes a mutated PPX protein that includes alanine to valine at a position corresponding to position 101 of SEQ ID NO: 9. In some embodiments, a mutated PPX gene encodes a mutated PPX protein that includes serine to asparagine at a position corresponding to position 119 of SEQ ID NO: 9. In some embodiments, a mutated PPX gene encodes a mutated PPX protein that includes phenylalanine to leucine at a position corresponding to position 121 of SEQ ID NO: 9. In some embodiments, a mutated PPX gene encodes a mutated PPX protein that includes threonine to isoleucine at a position corresponding to position 124 of SEQ ID NO: 9. In some embodiments, a mutated PPX gene encodes a mutated PPX protein that includes asparagine to tyrosine at a position corresponding to position 139 of SEQ ID NO: 9. In some embodiments, a mutated PPX gene encodes a mutated PPX protein that includes glutamic acid to aspartic acid at a position corresponding to position 150 of SEQ ID NO: 9. In some embodiments, a mutated PPX gene encodes a mutated PPX protein that includes glutamic acid to lysine at a position corresponding to position 150 of SEQ ID NO: 9. In some embodiments, a mutated PPX gene encodes a mutated PPX protein that includes serine to threonine at a position corresponding to position 151 of SEQ ID NO: 9. In some embodiments, a mutated PPX gene encodes a mutated PPX protein that includes glutamine to leucine at a position corresponding to position 157 of SEQ ID NO: 9. In some embodiments, a mutated PPX gene encodes a mutated PPX protein that includes valine to phenylalanine at a position corresponding to position 164 of SEQ ID NO: 9. In some embodiments, a mutated PPX gene encodes a mutated PPX protein that includes valine to alanine at a position corresponding to position 164 of SEQ ID NO: 9. In some embodiments, a mutated PPX gene encodes a mutated PPX protein that includes aspartic acid to glutamic acid at a position corresponding to position 170 of SEQ ID NO: 9. In some embodiments, a mutated PPX gene encodes a mutated PPX protein that includes cysteine to serine at a position corresponding to position 177 of SEQ ID NO: 9. In some embodiments, a mutated PPX gene encodes a mutated PPX protein that includes histidine to glutamine at a position corresponding to position 187 of SEQ ID NO: 9. In some embodiments, a mutated PPX gene encodes a mutated PPX protein that includes leucine to phenylalanine at a position corresponding to position 188 of SEQ ID NO: 9. In some embodiments, a mutated PPX gene encodes a mutated PPX protein that includes asparagine to lysine at a position corresponding to position 195 of SEQ ID NO: 9. In some embodiments, a mutated PPX gene encodes a mutated PPX protein that includes a proline to histidine at a position corresponding to position 214 of SEQ ID NO: 9. In some embodiments, a mutated PPX gene encodes a mutated PPX protein that includes a proline to serine at a position corresponding to position 214 of SEQ ID NO: 9. In some embodiments, a mutated PPX gene encodes a mutated PPX protein that includes an isoleucine to histidine at a position corresponding to position 215 of SEQ ID NO: 9. In some embodiments, a mutated PPX gene encodes a mutated PPX protein that includes an isoleucine to serine at a position corresponding to position 215 of SEQ ID NO: 9. In some embodiments, a mutated PPX gene encodes a mutated PPX protein that includes lysine to glutamic acid at a position corresponding to position 229 of SEQ ID NO: 9. In some embodiments, a mutated PPX gene encodes a mutated PPX protein that includes lysine to glutamine at a position corresponding to position 229 of SEQ ID NO: 9. In some embodiments, a mutated PPX gene encodes a mutated PPX protein that includes lysine to arginine at a position corresponding to position 230 of SEQ ID NO: 9. In some embodiments, a mutated PPX gene encodes a mutated PPX protein that includes cysteine to arginine at a position corresponding to position 271 of SEQ ID NO: 9. In some embodiments, a mutated PPX gene encodes a mutated PPX protein that includes aspartic acid to glycine at a position corresponding to position 274 of SEQ ID NO: 9. In some embodiments, a mutated PPX gene encodes a mutated PPX protein that includes aspartic acid to glycine at a position corresponding to position 278 of SEQ ID NO: 9. In some embodiments, a mutated PPX gene encodes a mutated PPX protein that includes a phenylalanine to glycine at a position corresponding to position 283 of SEQ ID NO: 9. In some embodiments, a mutated PPX gene encodes a mutated PPX protein that includes alanine to glycine at a position corresponding to position 292 of SEQ ID NO: 9. In some embodiments, a mutated PPX gene encodes a mutated PPX protein that includes serine to leucine at a position corresponding to position 296 of SEQ ID NO: 9. In some embodiments, a mutated PPX gene encodes a mutated PPX protein that includes cysteine to serine at a position corresponding to position 307 of SEQ ID NO: 9. In some embodiments, a mutated PPX gene encodes a mutated PPX protein that includes asparagine to aspartic acid at a position corresponding to position 324 of SEQ ID NO: 9. In some embodiments, a mutated PPX gene encodes a mutated PPX protein that includes asparagine to lysine at a position corresponding to position 324 of SEQ ID NO: 9. In some embodiments, a mutated PPX gene encodes a mutated PPX protein that includes aspartic acid to glutamic acid at a position corresponding to position 330 of SEQ ID NO: 9. In some embodiments, a mutated PPX gene encodes a mutated PPX protein that includes serine to leucine at a position corresponding to position 396 of SEQ ID NO: 9. In some embodiments, a mutated PPX gene encodes a mutated PPX protein that includes alanine to serine at a position corresponding to position 404 of SEQ ID NO: 9. In some embodiments, a mutated PPX gene encodes a mutated PPX protein that includes arginine to lysine at a position corresponding to position 406 of SEQ ID NO: 9. In some embodiments, a mutated PPX gene encodes a mutated PPX protein that includes lysine to isoleucine at a position corresponding to position 410 of SEQ ID NO: 9. In some embodiments, a mutated PPX gene encodes a mutated PPX protein that includes leucine to valine at a position corresponding to position 421 of SEQ ID NO: 9. In some embodiments, a mutated PPX gene encodes a mutated PPX protein that includes alanine to valine at a position corresponding to position 423 of SEQ ID NO: 9. In some embodiments, a mutated PPX gene encodes a mutated PPX protein that includes cysteine to serine at a position corresponding to position 434 of SEQ ID NO: 9. In some embodiments, a mutated PPX gene encodes a mutated PPX protein that includes cysteine to tyrosine at a position corresponding to position 434 of SEQ ID NO: 9. In some embodiments, a mutated PPX gene encodes a mutated PPX protein that includes aspartic acid to glycine at a position corresponding to position 447 of SEQ ID NO: 9. In some embodiments, a mutated PPX gene encodes a mutated PPX protein that includes serine to alanine at a position corresponding to position 448 of SEQ ID NO: 9. In some embodiments, a mutated PPX gene encodes a mutated PPX protein that includes valine to glutamic acid at a position corresponding to position 449 of SEQ ID NO: 9. In some embodiments, a mutated PPX gene encodes a mutated PPX protein that includes aspartic acid to glycine at a position corresponding to position 451 of SEQ ID NO: 9. In some embodiments, a mutated PPX gene encodes a mutated PPX protein that includes aspartic acid to asparagine at a position corresponding to position 454 of SEQ ID NO: 9. In some embodiments, a mutated PPX gene encodes a mutated PPX protein that includes tyrosine to phenylalanine at a position corresponding to position 465 of SEQ ID NO: 9. In some embodiments, a mutated PPX gene encodes a mutated PPX protein that includes lysine to threonine at a position corresponding to position 470 of SEQ ID NO: 9. In some embodiments, a mutated PPX gene encodes a mutated PPX protein that includes threonine to serine at a position corresponding to position 500 of SEQ ID NO: 9.

In conjunction with any of the aspects, embodiments, methods and/or compositions disclosed herein, a mutated PPX gene includes a GAT → AAT nucleic acid mutation that encodes a mutated PPX protein that includes an aspartic acid to asparagine at a position corresponding to position 58 of SEQ ID NO: 9. In some embodiments, a mutated PPX gene includes a GAA → GTA nucleic acid mutation that encodes a mutated PPX protein that includes glutamic acid to valine at a position corresponding to position 64 of SEQ ID NO: 9. In some embodiments, a mutated PPX gene includes a GGT → TGT nucleic acid mutation that encodes a mutated PPX protein that includes glycine to cysteine at a position corresponding to position 74 of SEQ ID NO: 9. In some embodiments, a mutated PPX gene includes a GGA → GAT nucleic acid mutation that encodes a mutated PPX protein that includes glycine to asparagine at a position corresponding to position 84 of SEQ ID NO: 9. In some embodiments, a mutated PPX gene includes a CGC → TGC nucleic acid mutation that encodes a mutated PPX protein that includes arginine to cysteine at a position corresponding to position 98 of SEQ ID NO: 9. In some embodiments, a mutated PPX gene includes a CGC → CAC nucleic acid mutation that encodes a mutated PPX protein that includes arginine to histidine at a position corresponding to position 98 of SEQ ID NO: 9. In some embodiments, a mutated PPX gene includes a CGC → CTC nucleic acid mutation that encodes a mutated PPX protein that includes arginine to leucine at a position corresponding to position 98 of SEQ ID NO: 9. In some embodiments, a mutated PPX gene includes a AAT → TAT nucleic acid mutation that encodes a mutated PPX protein that includes asparagine to tyrosine at a position corresponding to position 139 of SEQ ID NO: 9. In some embodiments, a mutated PPX gene includes a GAA → GAT nucleic acid mutation that encodes a mutated PPX protein that includes glutamic acid to aspartic acid at a position corresponding to position 150 of SEQ ID NO: 9. In some embodiments, a mutated PPX gene includes a GAA → AAA nucleic acid mutation that encodes a mutated PPX protein that includes glutamic acid to lysine at a position corresponding to position 150 of SEQ ID NO: 9. In some embodiments, a mutated PPX gene includes a AGT → ACT nucleic acid mutation that encodes a mutated PPX protein that includes serine to threonine at a position corresponding to position 151 of SEQ ID NO: 9. In some embodiments, a mutated PPX gene includes a CAG → CTG nucleic acid mutation that encodes a mutated PPX protein that includes glutamine to leucine at a position corresponding to position 157 of SEQ ID NO: 9. In some embodiments, a mutated PPX gene includes a GTT → TTT nucleic acid mutation that encodes a mutated PPX protein that includes valine to phenylalanine at a position corresponding to position 164 of SEQ ID NO: 9. In some embodiments, a mutated PPX gene includes a GAT → GAA nucleic acid mutation that encodes a mutated PPX protein that includes aspartic acid to glutamic acid at a position corresponding to position 170 of SEQ ID NO: 9. In some embodiments, a mutated PPX gene includes a CAC → CAG nucleic acid mutation that encodes a mutated PPX protein that includes histidine to glutamine at a position corresponding to position 187 of SEQ ID NO: 9. In some embodiments, a mutated PPX gene includes a CTT → TTT nucleic acid mutation that encodes a mutated PPX protein that includes leucine to phenylalanine at a position corresponding to position 188 of SEQ ID NO: 9. In some embodiments, a mutated PPX gene includes a AAT → AAA nucleic acid mutation that encodes a mutated PPX protein that includes asparagine to lysine at a position corresponding to position 195 of SEQ ID NO: 9. In some embodiments, a mutated PPX gene includes a CCT → CAT nucleic acid mutation that encodes a mutated PPX protein that includes proline to histidine at a position corresponding to position 214 of SEQ ID NO: 9. In some embodiments, a mutated PPX gene includes a CCT → TCT nucleic acid mutation that encodes a mutated PPX protein that includes proline to serine at a position corresponding to position 214 of SEQ ID NO: 9. In some embodiments, a mutated PPX gene includes a AAG → GAG nucleic acid mutation that encodes a mutated PPX protein that includes lysine to glutamic acid at a position corresponding to position 229 of SEQ ID NO: 9. In some embodiments, a mutated PPX gene includes a AAG → CAG nucleic acid mutation that encodes a mutated PPX protein that includes lysine to glutamine at a position corresponding to position 229 of SEQ ID NO: 9. In some embodiments, a mutated PPX gene includes a AAG → AGG nucleic acid mutation that encodes a mutated PPX protein that includes lysine to arginine at a position corresponding to position 230 of SEQ ID NO: 9. In some embodiments, a mutated PPX gene includes a GAC → GGC nucleic acid mutation that encodes a mutated PPX protein that includes aspartic acid to glycine at a position corresponding to position 283 of SEQ ID NO: 9. In some embodiments, a mutated PPX gene includes a TCA → TTA nucleic acid mutation that encodes a mutated PPX protein that includes serine to leucine at a position corresponding to position 296 of SEQ ID NO: 9. In some embodiments, a mutated PPX gene includes a TGT → AGT nucleic acid mutation that encodes a mutated PPX protein that includes cysteine to serine at a position corresponding to position 307 of SEQ ID NO: 9. In some embodiments, a mutated PPX gene includes a AAT → GAT nucleic acid mutation that encodes a mutated PPX protein that includes asparagine to aspartic acid at a position corresponding to position 324 of SEQ ID NO: 9. In some embodiments, a mutated PPX gene includes a AAT → AAA nucleic acid mutation that encodes a mutated PPX protein that includes asparagine to lysine at a position corresponding to position 324 of SEQ ID NO: 9. In some embodiments, a mutated PPX gene includes a GAT → GAA nucleic acid mutation that encodes a mutated PPX protein that includes aspartic acid to glutamic acid at a position corresponding to position 330 of SEQ ID NO: 9. In some embodiments, a mutated PPX gene includes a GCC → TCC nucleic acid mutation that encodes a mutated PPX protein that includes alanine to serine at a position corresponding to position 404 of SEQ ID NO: 9. In some embodiments, a mutated PPX gene includes a AGG → AAG nucleic acid mutation that encodes a mutated PPX protein that includes arginine to lysine at a position corresponding to position 406 of SEQ ID NO: 9. In some embodiments, a mutated PPX gene includes a AAA → ATA nucleic acid mutation that encodes a mutated PPX protein that includes lysine to isoleucine at a position corresponding to position 410 of SEQ ID NO: 9. In some embodiments, a mutated PPX gene includes a XXX GCT → GTT nucleic acid mutation that encodes a mutated PPX protein that includes alanine to valine at a position corresponding to position 423 of SEQ ID NO: 9. In some embodiments, a mutated PPX gene includes a TGC → AGC nucleic acid mutation that encodes a mutated PPX protein that includes cysteine to serine at a position corresponding to position 434 of SEQ ID NO: 9. In some embodiments, a mutated PPX gene includes a TGC → TAC nucleic acid mutation that encodes a mutated PPX protein that includes cysteine to tyrosine at a position corresponding to position 434 of SEQ ID NO: 9. In some embodiments, a mutated PPX gene includes a TCA → GCA nucleic acid mutation that encodes a mutated PPX protein that includes serine to alanine at a position corresponding to position 448 of SEQ ID NO: 9. In some embodiments, a mutated PPX gene includes a GAT → GGT nucleic acid mutation that encodes a mutated PPX protein that includes aspartic acid to glycine at a position corresponding to position 451 of SEQ ID NO: 9. In some embodiments, a mutated PPX gene includes a GAC → AAC nucleic acid mutation that encodes a mutated PPX protein that includes aspartic acid to asparagine at a position corresponding to position 454 of SEQ ID NO: 9. In some embodiments, a mutated PPX gene includes a TAT → TTT nucleic acid mutation that encodes a mutated PPX protein that includes tyrosine to phenylalanine at a position corresponding to position 465 of SEQ ID NO: 9. In some embodiments, a mutated PPX gene includes a AAG → ACG nucleic acid mutation that encodes a mutated PPX protein that includes lysine to threonine at a position corresponding to position 470 of SEQ ID NO: 9. In some embodiments, a mutated PPX gene includes a ACC _> AGC nucleic acid mutation that encodes a mutated PPX protein that includes threonine to serine at a position corresponding to position 500 of SEQ ID NO: 9.

In conjunction with any of the aspects, embodiments, methods and/or compositions disclosed herein, a mutated PPX gene may include a GGG → AAA which encodes a mutated PPX protein that includes an glycine to lysine at a position corresponding to position 52 of SEQ ID NO: 1. In some embodiments, a mutated PPX gene includes a AAT → GAT nucleic acid mutation that encodes a mutated PPX protein that includes an asparagine to aspartic acid at a position corresponding to position 85 of SEQ ID NO: 1.

In some embodiments, in conjunction with any of the aspects, embodiments, methods and/or compositions disclosed herein, a mutated PPX gene may include a combination of mutations, for example, two or more, three or more, four or more, five or more or six or more mutations in a PPX gene. In certain embodiments, the combination of mutations is selected from the combinations of mutations shown in Tables 4a and 4b.

**Table 4A: Combinations of Amino Acid Mutations (each row of each of the three grouped columns represents a combination of mutations). Position numbers are based on numbering of the Arabidopsis plastidal PPX gene number At4g01690 (SEQ ID NO: 1)**

| | | | | | | | | |
|---|---|---|---|---|---|---|---|---|
| R144C | A220T | | | L226M | L424S | | F145L | L424S |
| R144H | S332C | | | L226M | Y426F | | A220T | Y426H |
| R144C | Q272F | | | A220T | Y426F | | F145Y | L393V |
| R144C | K272F | | | A220T | Y426H | | S244T | Y426F |
| G52K | R144H | S244T | | R144C | Y426F | | F145Y | L424S |
| N52K | R144H | S244T | | N85D | Y426H | | A220T | L403R |
| N85D | A220T | | | R144C | Y426H | | L226M | Y426F |
| R144H | S244T | | | S244T | Y426H | | N85D | Y426H |
| R144C | L226M | | | S244G | Y426H | | L226M | L424S |
| N85D | L226M | | | A180T | Y426H | | F145Y | L403R |
| N85D | F145Y | | | L226M | Y426H | | S244G | L393V |
| R144C | M228L | | | F145L | Y426H | | A180T | Y426H |
| N85D | A180T | | | A220T | Y426H | | R144C | Y426H |
| N85D | R144C | | | N85D | Y426H | | N85D | S525T |
| N85D | Q272F | | | F145L | L393V | | L226M | S525T |
| N85D | K272F | | | L226M | L424S | | F145Y | S525T |
| N85D | M228L | | | L226M | Y426F | | F145L | S525T |
| A180T | Y426F | | | A220T | L393V | | S244G | S525T |
| F145L | Y426H | | | A220T | Y426F | | A180T | S525T |
| S244G | Y426F | | | R144C | Y426F | | R144C | S525T |
| F145L | L403R | | | N85D | I525T | | | |
| F145Y | L424S | | | L226M | I525T | | | |
| R144C | L424S | | | F145Y | I525T | | | |
| L226M | Y426H | | | F145L | I525T | | | |
| A220T | L424S | | | R144C | I525T | | | |
| F145Y | Y426F | | | R144C | Y426H | | | |
| R144C | L393V | | | A180T | Y426H | | | |
| S244G | I525T | | | A220T | Y426H | | | |
| A180T | I525T | | | L226M | Y426H | | | |
| S244G | L393V | | | S244T | L393V | | | |
| L226M | L403R | | | F145Y | Y426H | | | |

In conjunction with any of the aspects, embodiments, methods and/or compositions disclosed herein, a mutated PPX protein includes an arginine to cysteine at a position corresponding to position 144 of SEQ ID NO: 1 and an alanine to threonine at a position corresponding to position 220 of SEQ ID NO: 1. In some embodiments, a mutated PPX protein includes an arginine to histidine at a position corresponding to position 144 of SEQ ID NO: 1 and a serine to cysteine at a position corresponding to position 332 of SEQ ID NO: 1. While in other embodiments, a mutated PPX protein includes an arginine to cysteine at a position corresponding to position 144 of SEQ ID NO: 1 and a glutamine to phenylalanine at a position corresponding to position 272 of SEQ ID NO: 1. In some embodiments, a mutated PPX protein includes an glycine to lysine at a position corresponding to position 52 of SEQ ID NO: 1, an arginine to histidine at a position corresponding to position 144 of SEQ ID NO: 1 and a serine to threonine at a position corresponding to position 244 of SEQ ID NO: 1. In some embodiments, a mutated PPX protein includes an asparagine to aspartic acid at a position corresponding to position 85 of SEQ ID NO: 1 and an alanine to threonine at a position corresponding to position 220 of SEQ ID NO: 1. In some embodiments, a mutated PPX protein includes an arginine to histidine at a position corresponding to position 144 of SEQ ID NO: 1 and a serine to threonine at a position corresponding to position 244 of SEQ ID NO: 1. In some embodiments, a mutated PPX protein includes an arginine to cysteine at a position corresponding to position 144 of SEQ ID NO: 1 and a leucine to methionine at a position corresponding to position 226 of SEQ ID NO: 1. In some embodiments, a mutated PPX protein includes an asparagine to aspartic acid at a position corresponding to position 85 of SEQ ID NO: 1 and a leucine to methionine at a position corresponding to position 226 of SEQ ID NO: 1. In some embodiments, a mutated PPX protein includes an asparagine to aspartic acid at a position corresponding to position 85 of SEQ ID NO: 1 and a phenylalanine to tyrosine at a position corresponding to position 145 of SEQ ID NO: 1. In some embodiments, a mutated PPX protein includes an arginine to cysteine at a position corresponding to position 144 of SEQ ID NO: 1 and a methionine to leucine at a position corresponding to position 228 of SEQ ID NO: 1. In some embodiments, a mutated PPX protein includes an asparagine to aspartic acid at a position corresponding to position 85 of SEQ ID NO: 1 and an alanine to threonine at a position corresponding to position 180 of SEQ ID NO: 1. In some embodiments, a mutated PPX protein includes an asparagine to aspartic acid at a position corresponding to position 85 of SEQ ID NO: 1 and an arginine to cysteine at a position corresponding to position 144 of SEQ ID NO: 1. In some embodiments, a mutated PPX protein includes an asparagine to aspartic acid at a position corresponding to position 85 of SEQ ID NO: 1 and a glutamine to phenylalanine at a position corresponding to position 272 of SEQ ID NO: 1. In some embodiments, a mutated PPX protein includes an asparagine to aspartic acid at a position corresponding to position 85 of SEQ ID NO: 1 and a methionine to leucine at a position corresponding to position 228 of SEQ ID NO: 1. In some embodiments, a mutated PPX protein includes an alanine to threonine at a position corresponding to position 180 of SEQ ID NO: 1 and a tyrosine to phenylalanine at a position corresponding to position 426 of SEQ ID NO: 1. In some embodiments, a mutated PPX protein includes a phenylalanine to leucine at a position corresponding to position 145 of SEQ ID NO: 1 and a tyrosine to histidine at a position corresponding to position 426 of SEQ ID NO: 1. In some embodiments, a mutated PPX protein includes a serine to glycine at a position corresponding to position 244 of SEQ ID NO: 1 and a tyrosine to phenylalanine at a position corresponding to position 426 of SEQ ID NO: 1. In some embodiments, a mutated PPX protein includes a phenylalanine to leucine at a position corresponding to position 145 of SEQ ID NO: 1 and a leucine to arginine at a position corresponding to position 403 of SEQ ID NO: 1. In some embodiments, a mutated PPX protein includes a phenylalanine to tyrosine at a position corresponding to position 145 of SEQ ID NO: 1 and a leucine to serine at a position corresponding to position 424 of SEQ ID NO: 1. In some embodiments, a mutated PPX protein includes an arginine to cysteine at a position corresponding to position 144 of SEQ ID NO: 1 and a leucine to serine at a position corresponding to position 424 of SEQ ID NO: 1. In some embodiments, a mutated PPX protein includes a leucine to methionine at a position corresponding to position 226 of SEQ ID NO: 1 and a tyrosine to histidine at a position corresponding to position 426 of SEQ ID NO: 1. In some embodiments, a mutated PPX protein includes an alanine to threonine at a position corresponding to position 220 of SEQ ID NO: 1 and a leucine to serine at a position corresponding to position 424 of SEQ ID NO: 1. In some embodiments, a mutated PPX protein includes a phenylalanine to tyrosine at a position corresponding to position 145 of SEQ ID NO: 1 and a tyrosine to phenylalanine at a position corresponding to position 426 of SEQ ID NO: 1. In some embodiments, a mutated PPX protein includes an arginine to cysteine at a position corresponding to position 144 of SEQ ID NO: 1 and a leucine to valine at a position corresponding to position 393 of SEQ ID NO: 1. In some embodiments, a mutated PPX protein includes a serine to glycine at a position corresponding to position 244 of SEQ ID NO: 1 and a leucine to valine at a position corresponding to position 393 of SEQ ID NO: 1. In some embodiments, a mutated PPX protein includes a leucine to methionine at a position corresponding to position 226 of SEQ ID NO: 1 and a leucine to arginine at a position corresponding to position 403 of SEQ ID NO: 1. In some embodiments, a mutated PPX protein includes a leucine to methionine at a position corresponding to position 226 of SEQ ID NO: 1 and a leucine to serine at a position corresponding to position 424 of SEQ ID NO: 1. In some embodiments, a mutated PPX protein includes a leucine to methionine at a position corresponding to position 226 of SEQ ID NO: 1 and a tyrosine to phenylalanine at a position corresponding to position 426 of SEQ ID NO: 1. In some embodiments, a mutated PPX protein includes an alanine to threonine at a position corresponding to position 220 of SEQ ID NO: 1 and a tyrosine to phenylalanine at a position corresponding to position 426 of SEQ ID NO: 1. In some embodiments, a mutated PPX protein includes an alanine to threonine at a position corresponding to position 220 of SEQ ID NO: 1 and a tyrosine to histidine at a position corresponding to position 426 of SEQ ID NO: 1. In some embodiments, a mutated PPX protein includes an arginine to cysteine at a position corresponding to position 144 of SEQ ID NO: 1 and a tyrosine to phenylalanine at a position corresponding to position 426 of SEQ ID NO: 1. In some embodiments, a mutated PPX protein includes an asparagine to aspartic acid at a position corresponding to position 85 of SEQ ID NO: 1 and and a tyrosine to histidine at a position corresponding to position 426 of SEQ ID NO: 1. In some embodiments, a mutated PPX protein includes an arginine to cysteine at a position corresponding to position 144 of SEQ ID NO: 1 and a tyrosine to histidine at a position corresponding to position 426 of SEQ ID NO: 1. In some embodiments, a mutated PPX protein includes a serine to threonine at a position corresponding to position 244 of SEQ ID NO: 1 and a tyrosine to histidine at a position corresponding to position 426 of SEQ ID NO: 1. In some embodiments, a mutated PPX protein includes a serine to glycine at a position corresponding to position 244 of SEQ ID NO: 1 and a tyrosine to histidine at a position corresponding to position 426 of SEQ ID NO: 1. In some embodiments, a mutated PPX protein includes an alanine to threonine at a position corresponding to position 180 of SEQ ID NO: 1 and a tyrosine to histidine at a position corresponding to position 426 of SEQ ID NO: 1. In some embodiments, a mutated PPX protein includes a leucine to methionine at a position corresponding to position 226 of SEQ ID NO: 1 and a tyrosine to histidine at a position corresponding to position 426 of SEQ ID NO: 1. In some embodiments, a mutated PPX protein includes a phenylalanine to leucine at a position corresponding to position 145 of SEQ ID NO: 1 and a tyrosine to histidine at a position corresponding to position 426 of SEQ ID NO: 1. In some embodiments, a mutated PPX protein includes an alanine to threonine at a position corresponding to position 220 of SEQ ID NO: 1 and a tyrosine to histidine at a position corresponding to position 426 of SEQ ID NO: 1. In some embodiments, a mutated PPX protein includes an asparagine to aspartic acid at a position corresponding to position 85 of SEQ ID NO: 1 and a tyrosine to histidine at a position corresponding to position 426 of SEQ ID NO: 1. In some embodiments, a mutated PPX protein includes a phenylalanine to leucine at a position corresponding to position 145 of SEQ ID NO: 1 and a leucine to valine at a position corresponding to position 393 of SEQ ID NO: 1. In some embodiments, a mutated PPX protein includes a leucine to methionine at a position corresponding to position 226 of SEQ ID NO: 1 and a leucine to serine at a position corresponding to position 424 of SEQ ID NO: 1. In some embodiments, a mutated PPX protein includes a leucine to methionine at a position corresponding to position 226 of SEQ ID NO: 1 and a tyrosine to phenylalanine at a position corresponding to position 426 of SEQ ID NO: 1. In some embodiments, a mutated PPX protein includes an alanine to threonine at a position corresponding to position 220 of SEQ ID NO: 1 and a leucine to valine at a position corresponding to position 393 of SEQ ID NO: 1. In some embodiments, a mutated PPX protein includes an alanine to threonine at a position corresponding to position 220 of SEQ ID NO: 1 and a tyrosine to phenylalanine at a position corresponding to position 426 of SEQ ID NO: 1. In some embodiments, a mutated PPX protein includes an arginine to cysteine at a position corresponding to position 144 of SEQ ID NO: 1 and a tyrosine to phenylalanine at a position corresponding to position 426 of SEQ ID NO: 1. In some embodiments, a mutated PPX protein includes an arginine to cysteine at a position corresponding to position 144 of SEQ ID NO: 1 and a tyrosine to histidine at a position corresponding to position 426 of SEQ ID NO: 1. In some embodiments, a mutated PPX protein includes an alanine to threonine at a position corresponding to position 180 of SEQ ID NO: 1 and a tyrosine to histidine at a position corresponding to position 426 of SEQ ID NO: 1. In some embodiments, a mutated PPX protein includes an alanine to threonine at a position corresponding to position 220 of SEQ ID NO: 1 and a tyrosine to histidine at a position corresponding to position 426 of SEQ ID NO: 1. In some embodiments, a mutated PPX protein includes a leucine to methionine at a position corresponding to position 226 of SEQ ID NO: 1 and a tyrosine to histidine at a position corresponding to position 426 of SEQ ID NO: 1. In some embodiments, a mutated PPX protein includes a serine to threonine at a position corresponding to position 244 of SEQ ID NO: 1 and a leucine to valine at a position corresponding to position 393 of SEQ ID NO: 1. In some embodiments, a mutated PPX protein includes a phenylalanine to tyrosine at a position corresponding to position 145 of SEQ ID NO: 1 and a tyrosine to histidine at a position corresponding to position 426 of SEQ ID NO: 1. In some embodiments, a mutated PPX protein includes an arginine to cysteine at a position corresponding to position 144 of SEQ ID NO: 1 and an isoleucine to threonine at a position corresponding to position 525 of SEQ ID NO: 1. In some embodiments, a mutated PPX protein includes a phenylalanine to leucine at a position corresponding to position 145 of SEQ ID NO: 1 and a leucine to serine at a position corresponding to position 424 of SEQ ID NO: 1. In some embodiments, a mutated PPX protein includes an alanine to threonine at a position corresponding to position 220 of SEQ ID NO: 1 and a tyrosine to histidine at a position corresponding to position 426 of SEQ ID NO: 1. In some embodiments, a mutated PPX protein includes a phenylalanine to tyrosine at a position corresponding to position 145 of SEQ ID NO: 1 and a leucine to valine at a position corresponding to position 393 of SEQ ID NO: 1. In some embodiments, a mutated PPX protein includes a serine to threonine at a position corresponding to position 244 of SEQ ID NO: 1 and a tyrosine to phenylalanine at a position corresponding to position 426 of SEQ ID NO: 1. In some embodiments, a mutated PPX protein includes a phenylalanine to tyrosine at a position corresponding to position 145 of SEQ ID NO: 1 and a leucine to serine at a position corresponding to position 424 of SEQ ID NO: 1. In some embodiments, a mutated PPX protein includes an alanine to threonine at a position corresponding to position 220 of SEQ ID NO: 1 and a leucine to arginine at a position corresponding to position 403 of SEQ ID NO: 1. In some embodiments, a mutated PPX protein includes a leucine to methionine at a position corresponding to position 226 of SEQ ID NO: 1 and a tyrosine to phenylalanine at a position corresponding to position 426 of SEQ ID NO: 1. In some embodiments, a mutated PPX protein includes an asparagine to aspartic acid at a position corresponding to position 85 of SEQ ID NO: 1 and a tyrosine to histidine at a position corresponding to position 426 of SEQ ID NO: 1. In some embodiments, a mutated PPX protein includes a leucine to methionine at a position corresponding to position 226 of SEQ ID NO: 1 and a leucine to serine at a position corresponding to position 424 of SEQ ID NO: 1. In some embodiments, a mutated PPX protein includes a phenylalanine to tyrosine at a position corresponding to position 145 of SEQ ID NO: 1 and a leucine to arginine at a position corresponding to position 403 of SEQ ID NO: 1. In some embodiments, a mutated PPX protein includes a serine to glycine at a position corresponding to position 244 of SEQ ID NO: 1 and a leucine to valine at a position corresponding to position 393 of SEQ ID NO: 1. In some embodiments, a mutated PPX protein includes an alanine to threonine at a position corresponding to position 180 of SEQ ID NO: 1 and a tyrosine to histidine at a position corresponding to position 426 of SEQ ID NO: 1. In some embodiments, a mutated PPX protein includes an arginine to cysteine at a position corresponding to position 144 of SEQ ID NO: 1 and a tyrosine to histidine at a position corresponding to position 426 of SEQ ID NO: 1. In some embodiments, a mutated PPX protein includes a serine to glycine at a position corresponding to position 244 of SEQ ID NO: 1 and an isoleucine to threonine at a position corresponding to position 525 of SEQ ID NO: 1. In some embodiments, a mutated PPX protein includes an alanine to threonine at a position corresponding to position 180 of SEQ ID NO: 1 and an isoleucine to threonine at a position corresponding to position 525 of SEQ ID NO: 1. In some embodiments, a mutated PPX protein includes a phenylalanine to leucine at a position corresponding to position 145 of SEQ ID NO: 1 and an isoleucine to threonine at a position corresponding to position 525 of SEQ ID NO: 1. In some embodiments, a mutated PPX protein includes a phenylalanine to tyrosine at a position corresponding to position 145 of SEQ ID NO: 1 and an isoleucine to threonine at a position corresponding to position 525 of SEQ ID NO: 1. In some embodiments, a mutated PPX protein includes an asparagine to aspartic acid at a position corresponding to position 85 of SEQ ID NO: 1 and an isoleucine to threonine at a position corresponding to position 525 of SEQ ID NO: 1. In some embodiments, a mutated PPX protein includes a leucine to methionine at a position corresponding to position 226 of SEQ ID NO: 1 and an isoleucine to threonine at a position corresponding to position 525 of SEQ ID NO: 1.

In conjunction with any of the aspects, embodiments, methods and/or compositions disclosed herein, a mutated PPX protein includes an arginine to cysteine at a position corresponding to position 144 of SEQ ID NO: 1 and an alanine to threonine at a position corresponding to position 220 of SEQ ID NO: 1. In some embodiments, a mutated PPX protein includes an arginine to histidine at a position corresponding to position 144 of SEQ ID NO: 1 and a serine to cysteine at a position corresponding to position 332 of SEQ ID NO: 1. In some embodiments, a mutated PPX protein includes an arginine to cysteine at a position corresponding to position 144 of SEQ ID NO: 7 and a lysine to phenylalanine at a position corresponding to position 272 of SEQ ID NO: 7. In some embodiments, a mutated PPX protein includes an asparagine to lysine at a position corresponding to position 52 of SEQ ID NO: 7, an arginine to histidine at a position corresponding to position 144 of SEQ ID NO: 7 and a serine to threonine at a position corresponding to position 244 of SEQ ID NO: 7. In some embodiments, a mutated PPX protein includes an asparagine to aspartic acid at a position corresponding to position 85 of SEQ ID NO: 1 and an alanine to threonine at a position corresponding to position 220 of SEQ ID NO: 1. In some embodiments, a mutated PPX protein includes an arginine to histidine at a position corresponding to position 144 of SEQ ID NO: 1 and a serine to threonine at a position corresponding to position 244 of SEQ ID NO: 1. In some embodiments, a mutated PPX protein includes an arginine to cysteine at a position corresponding to position 144 of SEQ ID NO: 1 and a leucine to methionine at a position corresponding to position 226 of SEQ ID NO: 1. In some embodiments, a mutated PPX protein includes an asparagine to aspartic acid at a position corresponding to position 85 of SEQ ID NO: 1 and a leucine to methionine at a position corresponding to position 226 of SEQ ID NO: 1. In some embodiments, a mutated PPX protein includes an asparagine to aspartic acid at a position corresponding to position 85 of SEQ ID NO: 1 and a phenylalanine to tyrosine at a position corresponding to position 145 of SEQ ID NO: 1. In some embodiments, a mutated PPX protein includes an arginine to cysteine at a position corresponding to position 144 of SEQ ID NO: 1 and a methionine to leucine at a position corresponding to position 228 of SEQ ID NO: 1. In some embodiments, a mutated PPX protein includes an asparagine to aspartic acid at a position corresponding to position 85 of SEQ ID NO: 1 and an alanine to threonine at a position corresponding to position 180 of SEQ ID NO: 1. In some embodiments, a mutated PPX protein includes an asparagine to aspartic acid at a position corresponding to position 85 of SEQ ID NO: 1 and an arginine to cysteine at a position corresponding to position 144 of SEQ ID NO: 1. In some embodiments, a mutated PPX protein includes an asparagine to aspartic acid at a position corresponding to position 85 of SEQ ID NO: 1 and an lysine to phenylalanine at a position corresponding to position 272 of SEQ ID NO: 1. In some embodiments, a mutated PPX protein includes an asparagine to aspartic acid at a position corresponding to position 85 of SEQ ID NO: 1 and a methionine to leucine at a position corresponding to position 228 of SEQ ID NO: 1. In some embodiments, a mutated PPX protein includes an alanine to threonine at a position corresponding to position 180 of SEQ ID NO: 1 and a tyrosine to phenylalanine at a position corresponding to position 426 of SEQ ID NO: 1. In some embodiments, a mutated PPX protein includes a phenylalanine to leucine at a position corresponding to position 145 of SEQ ID NO: 1 and a tyrosine to histidine at a position corresponding to position 426 of SEQ ID NO: 1. In some embodiments, a mutated PPX protein includes a serine to glycine at a position corresponding to position 244 of SEQ ID NO: 1 and a tyrosine to phenylalanine at a position corresponding to position 426 of SEQ ID NO: 1. In some embodiments, a mutated PPX protein includes a phenylalanine to leucine at a position corresponding to position 145 of SEQ ID NO: 1 and a leucine to arginine at a position corresponding to position 403 of SEQ ID NO: 1. In some embodiments, a mutated PPX protein includes a phenylalanine to tyrosine at a position corresponding to position 145 of SEQ ID NO: 1 and a leucine to serine at a position corresponding to position 424 of SEQ ID NO: 1. In some embodiments, a mutated PPX protein includes an arginine to cysteine at a position corresponding to position 144 of SEQ ID NO: 1 and a leucine to serine at a position corresponding to position 424 of SEQ ID NO: 1. In some embodiments, a mutated PPX protein includes a leucine to methionine at a position corresponding to position 226 of SEQ ID NO: 1 and a tyrosine to histidine at a position corresponding to position 426 of SEQ ID NO: 1. In some embodiments, a mutated PPX protein includes an alanine to threonine at a position corresponding to position 220 of SEQ ID NO: 1 and a leucine to serine at a position corresponding to position 424 of SEQ ID NO: 1. In some embodiments, a mutated PPX protein includes a phenylalanine to tyrosine at a position corresponding to position 145 of SEQ ID NO: 1 and a tyrosine to phenylalanine at a position corresponding to position 426 of SEQ ID NO: 1. In some embodiments, a mutated PPX protein includes an arginine to cysteine at a position corresponding to position 144 of SEQ ID NO: 1 and a leucine to valine at a position corresponding to position 393 of SEQ ID NO: 1. In some embodiments, a mutated PPX protein includes a serine to glycine at a position corresponding to position 244 of SEQ ID NO: 1 and a leucine to valine at a position corresponding to position 393 of SEQ ID NO: 1. In some embodiments, a mutated PPX protein includes a leucine to methionine at a position corresponding to position 226 of SEQ ID NO: 1 and a leucine to arginine at a position corresponding to position 403 of SEQ ID NO: 1. In some embodiments, a mutated PPX protein includes a leucine to methionine at a position corresponding to position 226 of SEQ ID NO: 1 and a leucine to serine at a position corresponding to position 424 of SEQ ID NO: 1. In some embodiments, a mutated PPX protein includes a leucine to methionine at a position corresponding to position 226 of SEQ ID NO: 1 and a tyrosine to phenylalanine at a position corresponding to position 426 of SEQ ID NO: 1. In some embodiments, a mutated PPX protein includes an alanine to threonine at a position corresponding to position 220 of SEQ ID NO: 1 and a tyrosine to phenylalanine at a position corresponding to position 426 of SEQ ID NO: 1. In some embodiments, a mutated PPX protein includes an alanine to threonine at a position corresponding to position 220 of SEQ ID NO: 1 and a tyrosine to histidine at a position corresponding to position 426 of SEQ ID NO: 1. In some embodiments, a mutated PPX protein includes an arginine to cysteine at a position corresponding to position 144 of SEQ ID NO: 1 and a tyrosine to phenylalanine at a position corresponding to position 426 of SEQ ID NO: 1. In some embodiments, a mutated PPX protein includes an asparagine to aspartic acid at a position corresponding to position 85 of SEQ ID NO: 1 and a tyrosine to histidine at a position corresponding to position 426 of SEQ ID NO: 1. In some embodiments, a mutated PPX protein includes an arginine to cysteine at a position corresponding to position 144 of SEQ ID NO: 1 and a tyrosine to histidine at a position corresponding to position 426 of SEQ ID NO: 1. In some embodiments, a mutated PPX protein includes a serine to threonine at a position corresponding to position 244 of SEQ ID NO: 1 and a tyrosine to histidine at a position corresponding to position 426 of SEQ ID NO: 1. In some embodiments, a mutated PPX protein includes a serine to glycine at a position corresponding to position 244 of SEQ ID NO: 1 and a tyrosine to histidine at a position corresponding to position 426 of SEQ ID NO: 1. In some embodiments, a mutated PPX protein includes an alanine to threonine at a position corresponding to position 180 of SEQ ID NO: 1 and a tyrosine to histidine at a position corresponding to position 426 of SEQ ID NO: 1. In some embodiments, a mutated PPX protein includes a leucine to methionine at a position corresponding to position 226 of SEQ ID NO: 1 and a tyrosine to histidine at a position corresponding to position 426 of SEQ ID NO: 1. In some embodiments, a mutated PPX protein includes a phenylalanine to leucine at a position corresponding to position 145 of SEQ ID NO: 1 and a tyrosine to histidine at a position corresponding to position 426 of SEQ ID NO: 1. In some embodiments, a mutated PPX protein includes an alanine to threonine at a position corresponding to position 220 of SEQ ID NO: 1 and a tyrosine to histidine at a position corresponding to position 426 of SEQ ID NO: 1. In some embodiments, a mutated PPX protein includes an asparagine to aspartic acid at a position corresponding to position 85 of SEQ ID NO: 1 and a tyrosine to histidine at a position corresponding to position 426 of SEQ ID NO: 1. In some embodiments, a mutated PPX protein includes a phenylalanine to leucine at a position corresponding to position 145 of SEQ ID NO: 1 and a leucine to valine at a position corresponding to position 393 of SEQ ID NO: 1. In some embodiments, a mutated PPX protein includes a leucine to methionine at a position corresponding to position 226 of SEQ ID NO: 1 and a leucine to serine at a position corresponding to position 424 of SEQ ID NO: 1. In some embodiments, a mutated PPX protein includes a leucine to methionine at a position corresponding to position 226 of SEQ ID NO: 1 and a tyrosine to phenylalanine at a position corresponding to position 426 of SEQ ID NO: 1. In some embodiments, a mutated PPX protein includes an alanine to threonine at a position corresponding to position 220 of SEQ ID NO: 1 and a leucine to valine at a position corresponding to position 393 of SEQ ID NO: 1. In some embodiments, a mutated PPX protein includes an alanine to threonine at a position corresponding to position 220 of SEQ ID NO: 1 and a tyrosine to phenylalanine at a position corresponding to position 426 of SEQ ID NO: 1. In some embodiments, a mutated PPX protein includes an arginine to cysteine at a position corresponding to position 144 of SEQ ID NO: 1 and a tyrosine to phenylalanine at a position corresponding to position 426 of SEQ ID NO: 1. In some embodiments, a mutated PPX protein includes an arginine to cysteine at a position corresponding to position 144 of SEQ ID NO: 1 and a tyrosine to histidine at a position corresponding to position 426 of SEQ ID NO: 1. In some embodiments, a mutated PPX protein includes an alanine to threonine at a position corresponding to position 180 of SEQ ID NO: 1 and a tyrosine to histidine at a position corresponding to position 426 of SEQ ID NO: 1. In some embodiments, a mutated PPX protein includes an alanine to threonine at a position corresponding to position 220 of SEQ ID NO: 1 and a tyrosine to histidine at a position corresponding to position 426 of SEQ ID NO: 1. In some embodiments, a mutated PPX protein includes a leucine to methionine at a position corresponding to position 226 of SEQ ID NO: 1 and a tyrosine to histidine at a position corresponding to position 426 of SEQ ID NO: 1. In some embodiments, a mutated PPX protein includes a serine to threonine at a position corresponding to position 244 of SEQ ID NO: 1 and a leucine to valine at a position corresponding to position 393 of SEQ ID NO: 1. In some embodiments, a mutated PPX protein includes a phenylalanine to tyrosine at a position corresponding to position 145 of SEQ ID NO: 1 and a tyrosine to histidine at a position corresponding to position 426 of SEQ ID NO: 1. In some embodiments, a mutated PPX protein includes an arginine to cysteine at a position corresponding to position 144 of SEQ ID NO: 7 and an isoleucine to threonine at a position corresponding to position 525 of SEQ ID NO: 7. In some embodiments, a mutated PPX protein includes a phenylalanine to leucine at a position corresponding to position 145 of SEQ ID NO: 1 and a leucine to serine at a position corresponding to position 424 of SEQ ID NO: 1. In some embodiments, a mutated PPX protein includes an alanine to threonine at a position corresponding to position 220 of SEQ ID NO: 1 and a tyrosine to histidine at a position corresponding to position 426 of SEQ ID NO: 1. In some embodiments, a mutated PPX protein includes a phenylalanine to tyrosine at a position corresponding to position 145 of SEQ ID NO: 1 and a leucine to valine at a position corresponding to position 393 of SEQ ID NO: 1. In some embodiments, a mutated PPX protein includes a serine to threonine at a position corresponding to position 244 of SEQ ID NO: 1 and a tyrosine to phenylalanine at a position corresponding to position 426 of SEQ ID NO: 1. In some embodiments, a mutated PPX protein includes a phenylalanine to tyrosine at a position corresponding to position 145 of SEQ ID NO: 1 and a leucine to serine at a position corresponding to position 424 of SEQ ID NO: 1. In some embodiments, a mutated PPX protein includes an alanine to threonine at a position corresponding to position 220 of SEQ ID NO: 1 and a leucine to arginine at a position corresponding to position 403 of SEQ ID NO: 1. In some embodiments, a mutated PPX protein includes a leucine to methionine at a position corresponding to position 226 of SEQ ID NO: 1 and a tyrosine to phenylalanine at a position corresponding to position 426 of SEQ ID NO: 1. In some embodiments, a mutated PPX protein includes an asparagine to aspartic acid at a position corresponding to position 85 of SEQ ID NO: 1 and a tyrosine to histidine at a position corresponding to position 426 of SEQ ID NO: 1. In some embodiments, a mutated PPX protein includes a leucine to methionine at a position corresponding to position 226 of SEQ ID NO: 1 and a leucine to serine at a position corresponding to position 424 of SEQ ID NO: 1. In some embodiments, a mutated PPX protein includes a phenylalanine to tyrosine at a position corresponding to position 145 of SEQ ID NO: 1 and a leucine to arginine at a position corresponding to position 403 of SEQ ID NO: 1. In some embodiments, a mutated PPX protein includes a serine to glycine at a position corresponding to position 244 of SEQ ID NO: 1 and a leucine to valine at a position corresponding to position 393 of SEQ ID NO: 1. In some embodiments, a mutated PPX protein includes an alanine to threonine at a position corresponding to position 180 of SEQ ID NO: 1 and a tyrosine to histidine at a position corresponding to position 426 of SEQ ID NO: 1. In some embodiments, a mutated PPX protein includes an arginine to cysteine at a position corresponding to position 144 of SEQ ID NO: 1 and a tyrosine to histidine at a position corresponding to position 426 of SEQ ID NO: 1. In some embodiments, a mutated PPX protein includes a serine to glycine at a position corresponding to position 244 of SEQ ID NO: 1 and an isoleucine to threonine at a position corresponding to position 525 of SEQ ID NO: 1. In some embodiments, a mutated PPX protein includes a serine to glycine at a position corresponding to position 244 of SEQ ID NO: 7 and an isoleucine to threonine at a position corresponding to position 525 of SEQ ID NO: 7. In some embodiments, a mutated PPX protein includes an alanine to threonine at a position corresponding to position 180 of SEQ ID NO: 7 and an isoleucine to threonine at a position corresponding to position 525 of SEQ ID NO: 7. In some embodiments, a mutated PPX protein includes a phenylalanine to leucine at a position corresponding to position 145 of SEQ ID NO: 7 and an isoleucine to threonine at a position corresponding to position 525 of SEQ ID NO: 7. In some embodiments, a mutated PPX protein includes a phenylalanine to tyrosine at a position corresponding to position 145 of SEQ ID NO: 7 and an isoleucine to threonine at a position corresponding to position 525 of SEQ ID NO: 7. In some embodiments, a mutated PPX protein includes an asparagine to aspartic acid at a position corresponding to position 85 of SEQ ID NO: 7 and an isoleucine to threonine at a position corresponding to position 525 of SEQ ID NO: 7. In some embodiments, a mutated PPX protein includes a leucine to methionine at a position corresponding to position 226 of SEQ ID NO: 7 and an isoleucine to threonine at a position corresponding to position 525 of SEQ ID NO: 7.

**Table 4B: Combinations of Amino Acid Mutations (each row of each of the two grouped columns represents a combination of mutations). Position numbers are based on numbering of the Solanum tuberosum mitochondrial PPX gene number AJ225108 (SEQ ID NO: 9).**

| | | | | | | | | | | |
|---|---|---|---|---|---|---|---|---|---|---|
| G74C | R98C | | | | | R98C | P214H | | | |
| L93H | V164A | | | | | R98C | T124I | L188F | K229Q | |
| R98L | P214H | | | | | R98C | T124I | P214H | K229Q | |
| R98L | T124I | L188F | K229Q | | | R98C | T124I | K229Q | | |
| R98L | T124I | P214H | K229Q | | | R98C | P214H | A423V | | |
| R98L | T124I | K229Q | | | | R98C | T124I | L188F | K229Q | A423V |
| S119N | N139Y | | | | | R98C | T124I | P214H | K229Q | A423V |
| F121L | E150D | | | | | R98C | T124I | K229Q | A423V | |
| S151T | K229E | K230R | | | | R98C | P214H | C307S | | |
| Q157L | H187Q | | | | | R98C | T124I | L188F | K229Q | C307S |
| C271R | D274G | | | | | R98C | T124I | P214H | K229Q | C307S |
| C307S | A423V | | | | | R98C | T124I | K229Q | C307S | |
| S396L | K410I | | | | | R98H | P214H | | | |
| C434S | T500S | | | | | R98H | T124I | L188F | K229Q | |
| D447G | A292G | | | | | R98H | T124I | P214H | K229Q | |
| S448A | N324D | | | | | R98H | T124I | K229Q | | |
| Y465F | K470T | | | | | R98H | P214H | A423V | | |
| R98L | P214H | A243V | | | | R98H | T124I | L188F | K229Q | A423V |
| R98L | T124I | L188F | K229Q | A243V | | R98H | T124I | P214H | K229Q | A423V |
| R98L | T124I | P214H | K229Q | A243V | | R98H | T124I | K229Q | A423V | |
| R98L | T124I | K229Q | A423V | | | R98H | P214H | C307S | | |
| R98L | P214H | C307S | | | | R98H | T124I | L188F | K229Q | C307S |
| R98L | T124I | L188F | K229Q | C307S | | R98H | T124I | P214H | K229Q | C307S |
| R98L | T124I | P214H | K229Q | C307S | | R98H | T124I | K229Q | C307S | |
| R98L | T124I | K229Q | C307S | | | | | | | |

In conjunction with any of the aspects, embodiments, methods and/or compositions disclosed herein, a mutated PPX protein includes a glycine to cysteine at a position corresponding to position 74 of SEQ ID NO: 9 and an arginine to cysteine at a position corresponding to position 98 of SEQ ID NO: 9. In some embodiments, a mutated PPX protein includes an leucine to histidine at a position corresponding to position 93 of SEQ ID NO: 9 and a valine to alanine at a position corresponding to position 164 of SEQ ID NO: 9. In some embodiments, a mutated PPX protein includes an arginine to leucine at a position corresponding to position 98 of SEQ ID NO: 9 and a proline to histidine at a position corresponding to position 214 of SEQ ID NO: 9. In some embodiments, a mutated PPX protein includes an arginine to leucine at a position corresponding to position 98 of SEQ ID NO: 9, a threonine to isoleucine at a position corresponding to position 124 of SEQ ID NO: 9, a leucine to phenylalanine at a position corresponding to position 188 of SEQ ID NO: 9, and a lysine to glutamine at a position corresponding to position 229 of SEQ ID NO: 9. In some embodiments, a mutated PPX protein includes an arginine to leucine at a position corresponding to position 98 of SEQ ID NO: 9, a threonine to isoleucine at a position corresponding to position 124 of SEQ ID NO: 9, a proline to histidine at a position corresponding to position 214, and a lysine to glutamine at a position corresponding to position 229 of SEQ ID NO: 9. In some embodiments, a mutated PPX protein includes an arginine to leucine at a position corresponding to position 98 of SEQ ID NO: 9, a threonine to isoleucine at a position corresponding to position 124 of SEQ ID NO: 9, and a lysine to glutamine at a position corresponding to position 229 of SEQ ID NO: 9. In some embodiments, a mutated PPX protein includes a serine to asparagine at a position corresponding to position 119 of SEQ ID NO: 9 and an asparagine to tyrosine at a position corresponding to position 139 of SEQ ID NO: 9. In some embodiments, a mutated PPX protein includes a phenylalanine to leucine at a position corresponding to position 121 of SEQ ID NO: 9 and a glutamic acid to aspartic acid at a position corresponding to position 150 of SEQ ID NO: 9. In some embodiments, a mutated PPX protein includes a serine to threonine at a position corresponding to position 151 of SEQ ID NO: 9, a lysine to glutamic acid at a position corresponding to position 229 of SEQ ID NO: 9, and a lysine to arginine at a position corresponding to position 230 of SEQ ID NO: 9. In some embodiments, a mutated PPX protein includes a glutamine to leucine at a position corresponding to position 157 of SEQ ID NO: 9 and a histidine to glutamine at a position corresponding to position 187 of SEQ ID NO: 9. In some embodiments, a mutated PPX protein includes a cysteine to arginine at a position corresponding to position 271 of SEQ ID NO: 9 and a aspartic acid to glycine at a position corresponding to position 274 of SEQ ID NO: 9. In some embodiments, a mutated PPX protein includes a cysteine to serine at a position corresponding to position 307 of SEQ ID NO: 9 and an alanine to valine at a position corresponding to position 423 of SEQ ID NO: 9. In some embodiments, a mutated PPX protein includes a serine to leucine at a position corresponding to position 396 of SEQ ID NO: 9 and a lysine to isoleucine at a position corresponding to position 410 of SEQ ID NO: 9. In some embodiments, a mutated PPX protein includes a cysteine to serine at a position corresponding to position 434 of SEQ ID NO: 9 and a threonie to serine at a position corresponding to position 500 of SEQ ID NO: 9. In some embodiments, a mutated PPX protein includes an aspartic acid to glycine at a position corresponding to position 447 of SEQ ID NO: 9 and an alanine to glycine at a position corresponding to position 292 of SEQ ID NO: 9. In some embodiments, a mutated PPX protein includes a serine to alanine at a position corresponding to position 448 of SEQ ID NO: 9 and an asparagine to aspartic acid at a position corresponding to position 324 of SEQ ID NO: 9. In some embodiments, a mutated PPX protein includes a tyrosine to phenylalanine at a position corresponding to position 465 of SEQ ID NO: 9 and a lysine to threonine at a position corresponding to position 470 of SEQ ID NO: 9. In some embodiments, a mutated PPX protein includes an arginine to leucine at a position corresponding to position 98 of SEQ ID NO: 9, a proline to histidine at a position corresponding to position 214 of SEQ ID NO: 9, and an alanine to valine at a position corresponding to position 243 of SEQ ID NO: 9. In some embodiments, a mutated PPX protein includes an arginine to leucine at a position corresponding to position 98 of SEQ ID NO: 9, a threonine to isoleucine at a position corresponding to position 124 of SEQ ID NO: 9, a leucine to phenylalanine at a position corresponding to position 188 of SEQ ID NO: 9, a lysine to glutamine at a position corresponding to position 229 of SEQ ID NO: 9, and an alanine to valine at a position corresponding to position 243 of SEQ ID NO: 9. In some embodiments, a mutated PPX protein includes an arginine to leucine at a position corresponding to position 98 of SEQ ID NO: 9, a threonine to isoleucine at a position corresponding to position 124 of SEQ ID NO: 9, a proline to histidine at a position corresponding to position 214 of SEQ ID NO: 9, a lysine to glutamine at a position corresponding to position 229 of SEQ ID NO: 9, and an alanine to valine at a position corresponding to position 243 of SEQ ID NO: 9. In some embodiments, a mutated PPX protein includes an arginine to leucine at a position corresponding to position 98 of SEQ ID NO: 9, a threonine to isoleucine at a position corresponding to position 124 of SEQ ID NO: 9, a lysine to glutamine at a position corresponding to position 229 of SEQ ID NO: 9, and an alanine to valine at a position corresponding to position 243 of SEQ ID NO: 9. In some embodiments, a mutated PPX protein includes an arginine to leucine at a position corresponding to position 98 of SEQ ID NO: 9, a proline to histidine at a position corresponding to position 214 of SEQ ID NO: 9, and a cysteine to serine at a position corresponding to position 307 of SEQ ID NO: 9. In some embodiments, a mutated PPX protein includes an arginine to leucine at a position corresponding to position 98 of SEQ ID NO: 9, a threonine to isoleucine at a position corresponding to position 124 of SEQ ID NO: 9, a leucine to phenylalanine at a position corresponding to position 188 of SEQ ID NO: 9, a lysine to glutamine at a position corresponding to position 229 of SEQ ID NO: 9, and a cysteine to serine at a position corresponding to position 307 of SEQ ID NO: 9. In some embodiments, a mutated PPX protein includes an arginine to leucine at a position corresponding to position 98 of SEQ ID NO: 9, a threonine to isoleucine at a position corresponding to position 124 of SEQ ID NO: 9, a proline to histidine at a position corresponding to position 214 of SEQ ID NO: 9, a lysine to glutamine at a position corresponding to position 229 of SEQ ID NO: 9, and a cysteine to serine at a position corresponding to position 307 of SEQ ID NO: 9. In some embodiments, a mutated PPX protein includes an arginine to leucine at a position corresponding to position 98 of SEQ ID NO: 9, a threonine to isoleucine at a position corresponding to position 124 of SEQ ID NO: 9, a lysine to glutamine at a position corresponding to position 229 of SEQ ID NO: 9, and a cysteine to serine at a position corresponding to position 307 of SEQ ID NO: 9. In some embodiments, a mutated PPX protein includes an arginine to cysteine at a position corresponding to position 98 of SEQ ID NO: 9 and a proline to histidine at a position corresponding to position 214 of SEQ ID NO: 9. In some embodiments, a mutated PPX protein includes an arginine to cysteine at a position corresponding to position 98 of SEQ ID NO: 9; a threonine to isoleucine at a position corresponding to position 124 of SEQ ID NO: 9; a leucine to phenylalanine at a position corresponding to position 188 of SEQ ID NO: 9 and a lysine to glutamine at a position corresponding to position 229 SEQ ID NO: 9. In some embodiments, a mutated PPX protein includes an arginine to cysteine at a position corresponding to position 98 of SEQ ID NO: 9; a threonine to isoleucine at a position corresponding to position 124 of SEQ ID NO: 9; a proline to hisitidine at a position corresponding to position 214 of SEQ ID NO: 9 and a lysine to glutamine at a position corresponding to position 229 SEQ ID NO: 9. In some embodiments, a mutated PPX protein includes an arginine to cysteine at a position corresponding to position 98 of SEQ ID NO: 9; a threonine to isoleucine at a position corresponding to position 124 of SEQ ID NO: 9; and a lysine to glutamine at a position corresponding to position 229 SEQ ID NO: 9. In some embodiments, a mutated PPX protein includes an arginine to cysteine at a position corresponding to position 98 of SEQ ID NO: 9; a proline to histidine at a position corresponding to position 214 of SEQ ID NO: 9; and an alanine to valine at a position corresponding to position 423 of SEQ ID NO: 9. In some embodiments, a mutated PPX protein includes an arginine to cysteine at a position corresponding to position 98 of SEQ ID NO: 9; a threonine to isoleucine at a position corresponding to position 124 of SEQ ID NO: 9; a leucine to phenylalanine at a position corresponding to position 188 of SEQ ID NO: 9; a lysine to glutamine at a position corresponding to position 229 SEQ ID NO: 9; and an alanine to valine at a position corresponding to position 423 of SEQ ID NO: 9. In some embodiments, a mutated PPX protein includes an arginine to cysteine at a position corresponding to position 98 of SEQ ID NO: 9; a threonine to isoleucine at a position corresponding to position 124 of SEQ ID NO: 9; a proline to histidine at a position corresponding to position 214 of SEQ ID NO: 9; a lysine to glutamine at a position corresponding to position 229 SEQ ID NO: 9; and an alanine to valine at a position corresponding to position 423 of SEQ ID NO: 9. In some embodiments, a mutated PPX protein includes an arginine to cysteine at a position corresponding to position 98 of SEQ ID NO: 9; a threonine to isoleucine at a position corresponding to position 124 of SEQ ID NO: 9; a lysine to glutamine at a position corresponding to position 229 SEQ ID NO: 9; and an alanine to valine at a position corresponding to position 423 of SEQ ID NO: 9. In some embodiments, a mutated PPX protein includes an arginine to cysteine at a position corresponding to position 98 of SEQ ID NO: 9; a proline to histidine at a position corresponding to position 214 of SEQ ID NO: 9; and a cysteine to serine at a position corresponding to position 307 of SEQ ID NO: 9. In some embodiments, a mutated PPX protein includes an arginine to cysteine at a position corresponding to position 98 of SEQ ID NO: 9; a threonine to isoleucine at a position corresponding to position 124 of SEQ ID NO: 9; a leucine to phenylalanine at a position corresponding to position 188 of SEQ ID NO: 9; a lysine to glutamine at a position corresponding to position 229 SEQ ID NO: 9; and a cysteine to serine at a position corresponding to position 307 of SEQ ID NO: 9. In some embodiments, a mutated PPX protein includes an arginine to cysteine at a position corresponding to position 98 of SEQ ID NO: 9; a threonine to isoleucine at a position corresponding to position 124 of SEQ ID NO: 9; a proline to histidine at a position corresponding to position 214 of SEQ ID NO: 9; a lysine to glutamine at a position corresponding to position 229 SEQ ID NO: 9; and a cysteine to serine at a position corresponding to position 307 of SEQ ID NO: 9. In some embodiments, a mutated PPX protein includes an arginine to cysteine at a position corresponding to position 98 of SEQ ID NO: 9; a threonine to isoleucine at a position corresponding to position 124 of SEQ ID NO: 9; a lysine to glutamine at a position corresponding to position 229 SEQ ID NO: 9; and a cysteine to serine at a position corresponding to position 307 of SEQ ID NO: 9. In some embodiments, a mutated PPX protein includes an arginine to histidine at a position corresponding to position 98 of SEQ ID NO: 9 and a proline to histidine at a position corresponding to position 214 of SEQ ID NO: 9. In some embodiments, a mutated PPX protein includes an arginine to histidine at a position corresponding to position 98 of SEQ ID NO: 9; a threonine to isoleucine at a position corresponding to position 124 of SEQ ID NO: 9; a leucine to phenylalanine at a position corresponding to position 188 of SEQ ID NO: 9 and a lysine to glutamine at a position corresponding to position 229 SEQ ID NO: 9. In some embodiments, a mutated PPX protein includes an arginine to histidine at a position corresponding to position 98 of SEQ ID NO: 9; a threonine to isoleucine at a position corresponding to position 124 of SEQ ID NO: 9; a proline to hisitidine at a position corresponding to position 214 of SEQ ID NO: 9 and a lysine to glutamine at a position corresponding to position 229 SEQ ID NO: 9. In some embodiments, a mutated PPX protein includes an arginine to histidine at a position corresponding to position 98 of SEQ ID NO: 9; a threonine to isoleucine at a position corresponding to position 124 of SEQ ID NO: 9; and a lysine to glutamine at a position corresponding to position 229 SEQ ID NO: 9. In some embodiments, a mutated PPX protein includes an arginine to histidine at a position corresponding to position 98 of SEQ ID NO: 9; a proline to histidine at a position corresponding to position 214 of SEQ ID NO: 9; and an alanine to valine at a position corresponding to position 423 of SEQ ID NO: 9. In some embodiments, a mutated PPX protein includes an arginine to histidine at a position corresponding to position 98 of SEQ ID NO: 9; a threonine to isoleucine at a position corresponding to position 124 of SEQ ID NO: 9; a leucine to phenylalanine at a position corresponding to position 188 of SEQ ID NO: 9; a lysine to glutamine at a position corresponding to position 229 SEQ ID NO: 9; and an alanine to valine at a position corresponding to position 423 of SEQ ID NO: 9. In some embodiments, a mutated PPX protein includes an arginine to histidine at a position corresponding to position 98 of SEQ ID NO: 9; a threonine to isoleucine at a position corresponding to position 124 of SEQ ID NO: 9; a proline to histidine at a position corresponding to position 214 of SEQ ID NO: 9; a lysine to glutamine at a position corresponding to position 229 SEQ ID NO: 9; and an alanine to valine at a position corresponding to position 423 of SEQ ID NO: 9. In some embodiments, a mutated PPX protein includes an arginine to histidine at a position corresponding to position 98 of SEQ ID NO: 9; a threonine to isoleucine at a position corresponding to position 124 of SEQ ID NO: 9; a lysine to glutamine at a position corresponding to position 229 SEQ ID NO: 9; and an alanine to valine at a position corresponding to position 423 of SEQ ID NO: 9. In some embodiments, a mutated PPX protein includes an arginine to histidine at a position corresponding to position 98 of SEQ ID NO: 9; a proline to histidine at a position corresponding to position 214 of SEQ ID NO: 9; and a cysteine to serine at a position corresponding to position 307 of SEQ ID NO: 9. In some embodiments, a mutated PPX protein includes an arginine to histidine at a position corresponding to position 98 of SEQ ID NO: 9; a threonine to isoleucine at a position corresponding to position 124 of SEQ ID NO: 9; a leucine to phenylalanine at a position corresponding to position 188 of SEQ ID NO: 9; a lysine to glutamine at a position corresponding to position 229 SEQ ID NO: 9; and a cysteine to serine at a position corresponding to position 307 of SEQ ID NO: 9. In some embodiments, a mutated PPX protein includes an arginine to histidine at a position corresponding to position 98 of SEQ ID NO: 9; a threonine to isoleucine at a position corresponding to position 124 of SEQ ID NO: 9; a proline to histidine at a position corresponding to position 214 of SEQ ID NO: 9; a lysine to glutamine at a position corresponding to position 229 SEQ ID NO: 9; and a cysteine to serine at a position corresponding to position 307 of SEQ ID NO: 9. In some embodiments, a mutated PPX protein includes an arginine to histidine at a position corresponding to position 98 of SEQ ID NO: 9; a threonine to isoleucine at a position corresponding to position 124 of SEQ ID NO: 9; a lysine to glutamine at a position corresponding to position 229 SEQ ID NO: 9; and a cysteine to serine at a position corresponding to position 307 of SEQ ID NO: 9.

### Paralogs

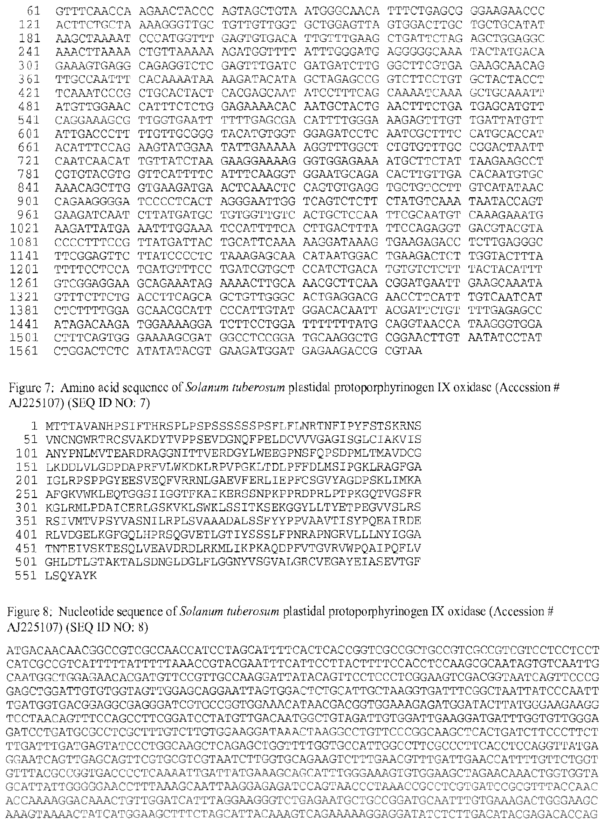

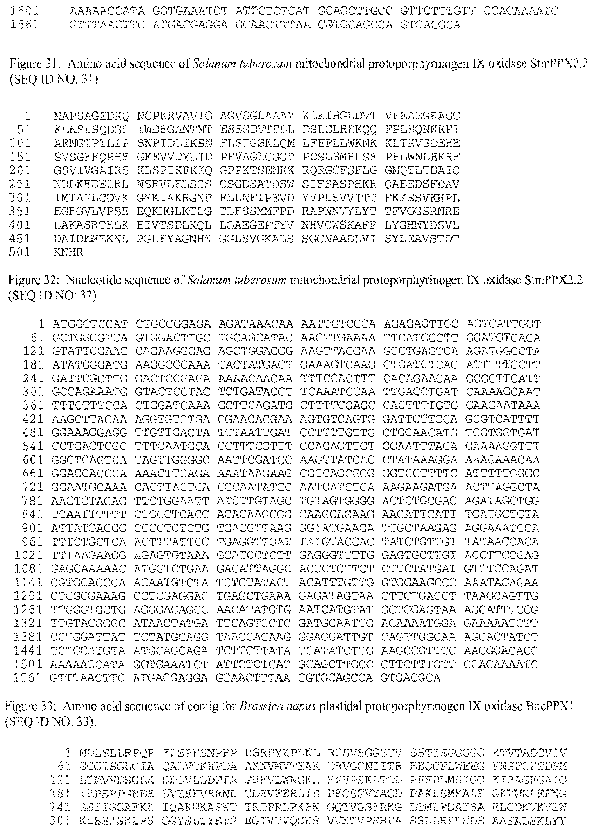

The subject mutations in the PPX gene are generally described herein using the *Solanum tuberosum* plastidal PPX genes and proteins (see e.g., Figures 8 and 7 respectively with amino acid positions referenced to positions in *Arabidopsis thaliana* (SEQ ID NO: 1). The compositions and methods also encompass mutant PPX genes and proteins of other species (paralogs). However, due to variations in the PPX genes of different species, the number of the amino acid residue to be changed in one species may be different in another species. Nevertheless, the analogous position is readily identified by one of skill in the art by sequence homology. For example, Table 6 summarizes the homologous amino acid positions in various plant PPX coding sequence paralogs and Figure 33 shows an amino acid sequence alignment of PPX paralogs from various plants. Thus, analogous positions in these and other paralogs can be identified and mutated.

### Herbicides

The compositions and methods provided herein include PPX genes and PPX proteins that confer resistance to PPX-inhibiting herbicides. In some embodiments, PPX-inhibiting herbicides include the chemical families of diphenylethers, phenylpyrazoles N-phenylphthalimides, thiadiazoles, oxadiazoles, triazolinones, oxazolidinediones, pyrimidindiones. Exemplary PPX-inhibiting herbicide active ingredients and their respective chemical family are summarized in Table 5.

**Table 5: Exemplary PPX-inhibiting Herbicides.**

| **Chemical Family** | **Herbicide Active Ingredient** |
|---|---|
| Diphenylethers | acifluorfen-Na |
| | Bifenox |
| | Chlomethoxyfen |
| | fluoroglycofen-ethyl |
| | Fomesafen |
| | Halosafen |
| | Lactofen |
| | Oxyfluorfen |
| Phenylpyrazoles | Fluazolate |
| | pyraflufen-ethyl |
| N-phenylphthalimides | cinidon-ethyl |
| | Flumioxazin |
| | flumiclorac-pentyl |
| Thiadiazoles | fluthiacet-methyl |
| | Thidiazimin |
| Oxadiazoles | Oxadiazon |
| | Oxadiargyl |
| Triazolinones | Azafenidin |
| | carfentrazone-ethyl |
| | Sulfentrazone |
| Oxazolidinediones | Pentoxazone |
| Pyrimidindiones | Benzfendizone |
| | Butafenacil |
| | Saflufenacil |
| Others | Pyrazogyl |
| | Profluazol |

In some embodiments, PPX-inhibiting herbicide is acifluorfen-Na. In some embodiments, PPX-inhibiting herbicide is bifenox. In some embodiments, PPX-inhibiting herbicide is chlomethoxyfen. In some embodiments, PPX-inhibiting herbicide is fluoroglycofen-ethyl. In some embodiments, PPX-inhibiting herbicide is fomesafen. In some embodiments, PPX-inhibiting herbicide is halosafen. In some embodiments, PPX-inhibiting herbicide is lactofen. In some embodiments, PPX-inhibiting herbicide is oxyfluorfen. In some embodiments, PPX-inhibiting herbicide is fluazolate. In some embodiments, PPX-inhibiting herbicide is pyraflufen-ethyl. In some embodiments, PPX-inhibiting herbicide is cinidon-ethyl. In some embodiments, PPX-inhibiting herbicide is flumioxazin. In some embodiments, PPX-inhibiting herbicide is flumiclorac-pentyl. In some embodiments, PPX-inhibiting herbicide is fluthiacet-methyl. In some embodiments, PPX-inhibiting herbicide is thidiazimin. In some embodiments, PPX-inhibiting herbicide is oxadiazon. In some embodiments, PPX-inhibiting herbicide is oxadiargyl. In some embodiments, PPX-inhibiting herbicide is azafenidin. In some embodiments, PPX-inhibiting herbicide is carfentrazone-ethyl. In some embodiments, PPX-inhibiting herbicide is sulfentrazone. In some embodiments, PPX-inhibiting herbicide is pentoxazone. In some embodiments, PPX-inhibiting herbicide is benzfendizone. In some embodiments, PPX-inhibiting herbicide is butafenacil. In some embodiments, PPX-inhibiting herbicide is saflufenacil. In some embodiments, PPX-inhibiting herbicide is pyrazogyl. In some embodiments, PPX-inhibiting herbicide is profluazol.

Also provided is a transgenic or non-transgenic plant or plant cell having one or more mutations in the PPX gene, for example, such as disclosed herein. In certain embodiments, the plant or plant cell having one or more mutations in a PPX gene has increased resistance or tolerance to a member of PPX-inhibiting herbicides. In certain embodiments, the plant or plant cell having one or more mutations in a PPX gene may exhibit substantially normal growth or development of the plant, its organs, tissues or cells, as compared to the corresponding wild-type plant or cell. In particular aspects and embodiments provided are transgenic or non-transgenic plants having a mutation in a PPX gene, for example, such as disclosed herein, which in certain embodiments has increased resistance or tolerance to one or more members of the PPX-inhibiting herbicide chemical families and may exhibit substantially normal growth or development of the plant, its organs, tissues or cells, as compared to the corresponding wild-type plant or cell, i.e., in the presence of one or more herbicide such as for example, flumioxazin, sulfentrazone or saflufenacil, the mutated PPX protein has substantially the same catalytic activity as compared to the wild-type PPX protein.

Further provided are methods for producing a plant having a mutated PPX gene, for example, having one or more mutations as described herein; preferably the plant substantially maintains the catalytic activity of the wild-type protein irrespective of the presence or absence of a relevant herbicide. In certain embodiments, the methods include introducing into a plant cell a gene repair oligonucleobase with one or more targeted mutations in the PPX gene (for example, such as disclosed herein) and identifying a cell, seed, or plant having a mutated PPX gene.

### Plant Species

In conjunction with any of the aspects, embodiments, methods and/or compositions disclosed herein, plants as disclosed herein can be of any species of dicotyledonous, monocotyledonous or gymnospermous plant, including any woody plant species that grows as a tree or shrub, any herbaceous species, or any species that produces edible fruits, seeds or vegetables, or any species that produces colorful or aromatic flowers. For example, the plant or plant cell may be selected from a species of plant from the group consisting of potato, sunflower, sugar beet, maize, cotton, soybean, wheat, rye, oats, rice, canola, fruits, vegetables, tobacco, barley, sorghum, tomato, mango, peach, apple, pear, strawberry, banana, melon, carrot, lettuce, onion, soya spp, sugar cane, pea, field beans, poplar, grape, citrus, alfalfa, rye, oats, turf and forage grasses, flax, oilseed rape, cucumber, morning glory, balsam, pepper, eggplant, marigold, lotus, cabbage, daisy, carnation, petunia, tulip, iris, lily, and nut-producing plants insofar as they are not already specifically mentioned. The plant or plant cell may also be of a species selected from Table 6. The plant or plant cell may also be of a species selected from the group consisting of *Arabidopsis thaliana, Solanum tuberosum, Solanum phureja, Oryza sativa, Amaranthus tuberculatus, Sorghum bicolor, Ricinus communis* and *Zea mays.*

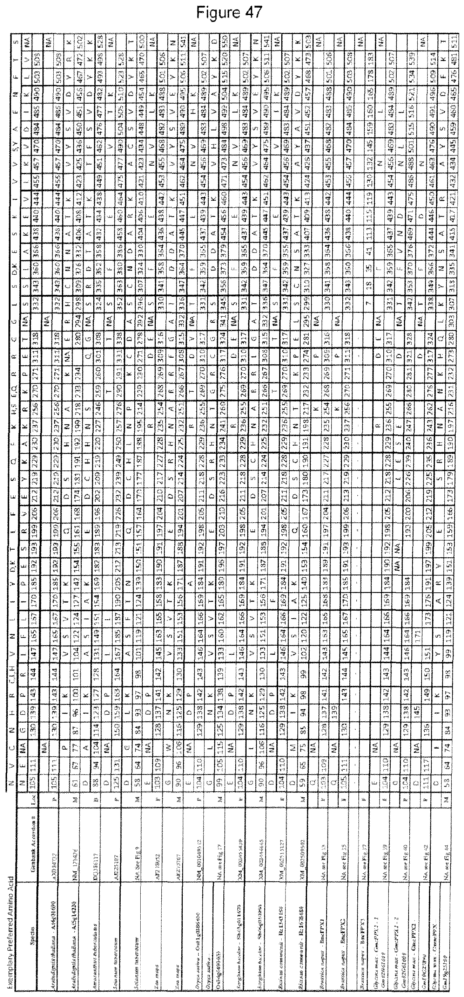

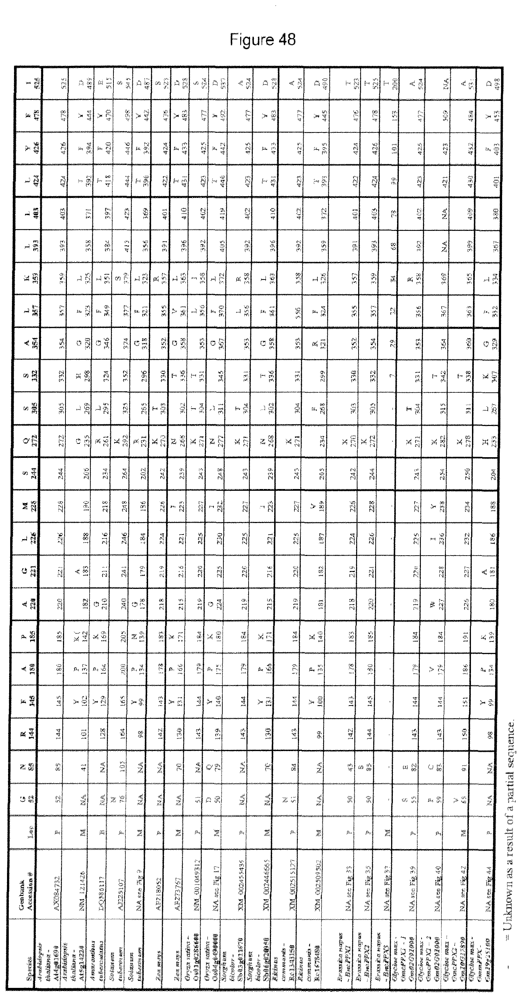

**Table 6. Summary of homologous amino acid positions in plant PPX amino acid sequences of various species. See also Figures 47 and 48 for additional homologous amino acid position summaries.**

| **Species** | **Genbank Accession** # | **Loc** | **G 52** | **N 85** | **R 144** | **F 145** | **A 180** | **P 185** | **A 220** | **L 226** | **M 228** | **S 244** | **Q 272** | **S 305** | **S 332** | **L 357** | **K 359** | **L 393** | **L 403** | **L 424** | **Y 426** | **F 478** | **I 525** |
|---|---|---|---|---|---|---|---|---|---|---|---|---|---|---|---|---|---|---|---|---|---|---|---|
| ***Arabidopsis thaliana-*At4g01690** | AX084732 | P | 52 | 85 | 144 | 145 | 180 | 185 | 220 | 226 | 228 | 244 | 272 | 305 | 332 | 357 | 359 | 393 | 403 | 424 | 426 | 478 | 525 |
| ***Arabidopsis thaliana-*At5g14220** | NM_121426 | M | NA | 41 | 101 | Y 102 | P 137 | K 142 | 182 | 188 | 190 | 206 | G 235 | L 269 | H 298 | F 323 | L 325 | 358 | 371 | T 392 | F 394 | Y 444 | D 489 |
| ***Amaranthus tuberculatus*** | DQ386117 | B | NA | NA | 128 | Y 129 | P 164 | K 169 | G 210 | 216 | 218 | 234 | R 261 | L 295 | 324 | F 349 | L 351 | 384 | 397 | T 418 | F 420 | Y 470 | E 515 |
| ***Solanum tuberosum*** | AJ225107 | P | N 76 | 105 | 164 | 165 | 200 | 205 | 240 | 246 | 248 | 264 | K 292 | 325 | 352 | 377 | S 379 | 413 | 423 | 444 | 446 | 498 | S 545 |
| ***Solanum tuberosum*** | NA see Fig 27. | M | NA | NA | 98 | Y 99 | P 134 | N 139 | G 178 | 184 | 186 | 202 | R 231 | L 265 | 296 | F 321 | L 323 | 356 | 369 | T 390 | F 392 | Y 442 | D 487 |
| ***Zea mays*** | AF218052 | P | NA | NA | 142 | 143 | 178 | P18 3 | 218 | 224 | 226 | 242 | K 270 | T 303 | 330 | 355 | R 357 | 391 | 401 | 422 | 424 | 476 | S 523 |
| ***Zea mays*** | AF273767 | M | NA | 70 | 130 | Y 131 | P 166 | K 171 | 215 | 221 | I 223 | 239 | N 268 | 302 | T 336 | V 361 | L 363 | 396 | 410 | T 431 | F 433 | Y 483 | D 528 |
| ***Oryza sativa** -* **Os01g0286600** | NM_001049312 | P | G 51 | NA | 143 | 144 | 179 | P 184 | 219 | 225 | 227 | 243 | K 271 | T 304 | T 331 | L 356 | I 358 | 392 | 402 | 423 | 425 | 477 | S 524 |
| ***Oryza sativa-*Os04g0490000** | NA see Fig 17. | M | D 50 | Q 79 | 139 | Y 140 | P 175 | K 180 | G 224 | 230 | I 232 | 248 | N 277 | L 311 | 345 | F 370 | L 372 | 405 | 419 | T 440 | F 442 | Y 492 | D 537 |
| ***Sorghum bicolor-*Sb03g011670** | XM_002455439 | P | NA | NA | 143 | 144 | 179 | P 184 | 219 | 225 | 227 | 243 | K 271 | T 304 | 331 | L 356 | R 358 | 392 | 402 | 423 | 425 | 477 | A 524 |
| ***Sorghum bicolor-*Sb06g020950** | XM_002446665 | M | NA | 70 | 130 | Y 131 | P 166 | K 171 | 215 | 221 | I 223 | 239 | N 268 | L 302 | T 336 | F 361 | L 363 | 396 | 410 | T 431 | F 433 | Y 483 | D 528 |
| ***Ricinus communis-*Rc1343150** | XM_002515127 | P | N 51 | 84 | 143 | 144 | 179 | 184 | 219 | 225 | 227 | 243 | K 271 | 304 | 331 | 356 | 358 | 392 | 402 | 423 | 425 | 477 | A 524 |
| ***Ricinus communis-*Rc1678480** | XM_002509502 | M | NA | NA | 99 | Y 100 | P 135 | K 140 | 181 | 187 | V 189 | 205 | 234 | F 268 | 299 | F 324 | L 326 | 359 | 372 | T 393 | F 395 | Y 445 | D 490 |

| | | | | | | | | | | | | | | | | | | | | | | | |
|---|---|---|---|---|---|---|---|---|---|---|---|---|---|---|---|---|---|---|---|---|---|---|---|
| † G210 deleted in DQ386118 leading to tolerance to PPX inhibitor P is plastidal; M is mitochondrial; B is both | | | | | | | | | | | | | | | | | | | | | | | |

The gene repair oligonucleobase can be introduced into a plant cell using any method commonly used in the art, including but not limited to, microcarriers (biolistic delivery), microfibers, polyethylene glycol (PEG)-mediated uptake, electroporation, and microinjection.

Also provided are methods and compositions related to the culture of cells mutated according to methods as disclosed herein in order to obtain a plant that produces seeds, henceforth a "fertile plant", and the production of seeds and additional plants from such a fertile plant.

Also provided are methods of selectively controlling weeds in a field, the field comprising plants with the disclosed PPX gene alterations and weeds, the method comprising application to the field of a herbicide to which the plants have been rendered resistant.

Also provided are mutations in the PPX gene that confer resistance or tolerance to a member of the relevant herbicide to a plant or wherein the mutated PPX gene has substantially the same enzymatic activity as compared to wild-type PPX.

### Selection of Herbicide Resistant Plants and Application of Herbicide

Plants and plant cells can be tested for resistance or tolerance to a herbicide using commonly known methods in the art, e.g., by growing the plant or plant cell in the presence of a herbicide and measuring the rate of growth as compared to the growth rate in the absence of the herbicide.

As used herein, substantially normal growth of a plant, plant organ, plant tissue or plant cell is defined as a growth rate or rate of cell division of the plant, plant organ, plant tissue, or plant cell that is at least 35%, at least 50%, at least 60%, or at least 75% of the growth rate or rate of cell division in a corresponding plant, plant organ, plant tissue or plant cell expressing the wild-type PPX protein.

As used herein, substantially normal development of a plant, plant organ, plant tissue or plant cell is defined as the occurrence of one or more development events in the plant, plant organ, plant tissue or plant cell that are substantially the same as those occurring in a corresponding plant, plant organ, plant tissue or plant cell expressing the wild-type PPX protein.

In conjunction with any of the aspects, embodiments, methods and/or compositions disclosed herein, plant organs provided herein may include, but are not limited to, leaves, stems, roots, vegetative buds, floral buds, meristems, embryos, cotyledons, endosperm, sepals, petals, pistils, carpels, stamens, anthers, microspores, pollen, pollen tubes, ovules, ovaries and fruits, or sections, slices or discs taken therefrom. Plant tissues include, but are not limited to, callus tissues, ground tissues, vascular tissues, storage tissues, meristematic tissues, leaf tissues, shoot tissues, root tissues, gall tissues, plant tumor tissues, and reproductive tissues. Plant cells include, but are not limited to, isolated cells with cell walls, variously sized aggregates thereof, and protoplasts.

Plants are substantially "tolerant" to a relevant herbicide when they are subjected to it and provide a dose/response curve which is shifted to the right when compared with that provided by similarly subjected non-tolerant like plant. Such dose/response curves have "dose" plotted on the X-axis and "percentage kill", "herbicidal effect", etc., plotted on the y-axis. Tolerant plants will require more herbicide than non-tolerant like plants in order to produce a given herbicidal effect. Plants that are substantially "resistant" to the herbicide exhibit few, if any, necrotic, lytic, chlorotic or other lesions, when subjected to herbicide at concentrations and rates which are typically employed by the agrochemical community to kill weeds in the field. Plants which are resistant to a herbicide are also tolerant of the herbicide.

In some embodiments an "increased resistance to a herbicide" or "increased tolerance to a herbicide" refers to a level of resistance or tolerance that a plant, seed, or plant part having a mutated PPX gene or protein as disclosed herein has to plant herbicides above a defined reference level. The defined reference level of resistance to a herbicide is the level of resistance displayed by a plant of the same species without the corresponding mutation(s). In some embodiments, resistance is substantially increased above the defined reference level, e.g., greater than or equal to 20% above, 50% above, 75% above; or 100% above the defined reference level.

### EXAMPLES

The following are examples, which illustrate procedures for practicing the invention. These examples should not be construed as limiting. All percentages are by weight and all solvent mixture proportions are by volume unless otherwise noted.

### Example 1: Plastidal and Mitochondrial PPX gene cloning and characterization

Plastidal and mitochondrial PPX genes were amplified from both cDNA and genomic DNA from a Russet Burbank cultivar. The plastidal PPX clones fall into two classes, given the names StcPPX1 and StcPPX1.1, likely representing alleles of a single PPX gene in potato. Within the amino acid coding sequence, these clones differ by 10 polymorphisms, 3 of which lead to amino acid differences with only two being found in the mature protein. One amino acid difference is in the chloroplast transit peptide. In one of the StcPPX1.1 clones, intron 3 was unspliced.

A full length error-free genomic clone of the plastidal PPX was obtained. The analysis of about 5 Kb of genomic sequence from 5 independent clones and StcPPX cDNA sequencing results indicates that the Russet Burbank variety subject to characterization is heterozygous, with very few polymorphisms existing between the two alleles.

First, five full-length StmPPX genomic DNA clones were cloned and sequenced. These five represented both alleles, having the same SNPs as found in the cDNA. Genomic DNA fragments of a shorter amplicon were cloned and sequenced to test for additional alleles. Cloning this internal amplicon of the mitochondrial PPX indicated that there were three alleles; 6 out of 22 clones had a deletion within one of the introns and the other 16 clones had an even distribution of the two alleles observed in the cDNA clones. Next, another 12 full length StmPPX genomic DNA clones were sequenced.

The completed sequencing of the mitochondrial PPX genes in Russet Burbank potato indicated that there are two genes, which we have termed StmPPX1 and StmPPX2. There are two StmPPX2 alleles with 8 SNPs identified between them. Between StmPPX1 and StmPPX2.1 there is 1 insertion, 4 deletions and 30 SNPs, whereas between StmPPX1 and StmPPX2.2 there is 1 insertion, 4 deletions and 29 SNPs. Additional detail is presented in Table 7.

Gene sequences of the plastidal and mitochondrial potato PPX genes from Russet Burbank (*Solanum tuberosum*) were compared, using the Basic Local Alignment Search Tool (BLAST), with our locally installed database based on scaffolds for the recent release of the first full potato (*Solanum phureja*) genome. Only a single plastidal and a single mitochondrial PPX gene were found.

**Table 7. Allelic differences between the mitochondrial forms of PPX in Russet Burbank potato.**

| **StmPPX nt Position** | **nt Gene 1** | **aa Gene 1** | **nt Gene 2, Allele 1** | **aa Gene 2, Allele 1** | **nt Gene 2, Allele 2** | **aa Gene 2, Allele 2** | **StmPPX aa position** | **AtcPPX aa position** |
|---|---|---|---|---|---|---|---|---|
| 296 | A (TAC) | Y | A (TAC) | Y | T (TTC) | **F** | 99 | 145 |
| 360 | C (AAC) | N | T (AAT) | N | T (AAT) | N | 120 | 166 |
| 402 | T (CCT) | P | A (CCA) | P | A (CCA) | P | 134 | 180 |
| 528 | G (ACG) | T | A (ACA) | T | A (ACA) | T | 176 | 218 |
| 680 | T (GTA) | V | T (GTA) | V | A (GAA) | **E** | 227 | 269\|270 |
| 692 | A (CAC) | **H** | G (CGC) | R | G (CGC) | R | 231 | 272 |

### Example 2: PPX Complementation

StcPPX1, less its chloroplast transit peptide was cloned from cDNA into Cibus' proprietary functional screening vector. This vector may be used both for functional screening, and for GRON QC. The potato PPX genes were used to complement the HemG mutant strain of *E. coli,* which lacks a functional HemG gene, a bacterial homolog of PPX. Without a complementing gene, the media must be supplemented with hematin for *E. coli* growth. Clones for the plastidal PPX gene (pACYStcPPX Col6) and the mitochondrial PPX gene (pACYStmPPX Col 6, 12 and 21) were transformed and all genes/alleles were shown to complement the HemG mutant *E. coli* strain, allowing it to grow in the absence of hematin.

In order to assess mutations that confer tolerance to PPX inhibitors such as Chateau (flumioxazin -Valent/Sumitomo), Naja (diphenylether - MAI) or Kixor (saflufenacil - BASF). PPX inhibiting herbicides are shown in Table 5. Pure active ingredients for the PPX inhibiting herbicides Chateau (flumioxazin -Valent/Sumitomo), Spartan (sulfentrazone - FMC) and Kixor/Sharpen (saflufenacil - BASF) were obtained. The wild-type potato plastidal PPX clone (pACYStcPPX Col6) was transformed to complement the hemG mutant *E. coli* strain and selected with a series of concentrations of the active ingredient for the PPX inhibiting herbicide Spartan (sulfentrazone - FMC) to determine the concentration at which the complemented HemG minus strain does not grow. The wildtype construct did not grow on 2.5 mM sulfentrazone, therefore, selection for tolerant mutants was performed at this concentration. This was further refined, and 0.75 mM sulfentrazone was also used to select for tolerance. The wildtype contruct had limited growth at 10 mM flumioxazin in liquid selection and no growth at 0.3 mM saflufenacil in plate based selection, concentrations used to test for tolerant mutants. All potato mitochondrial PPX genes and alleles were tested for natural tolerance to sulfentrazone and flumioxazin, but none were tolerant.

### Example 3: PPX PCR-Mutagenesis and Selection of Mutagenized clones

Mutagenesis experiments were initially performed on two overlapping fragments (5' and 3') of the potato plastidal PPX gene, to identify mutations in the potato plastidal PPX coding sequence that confer herbicide tolerance.

### Liquid Selection Standardization

Liquid culture selection conditions were developed for both sulfentrazone and flumioxazin. Cultures of 1 mL volume were tested with sulfentrazone and flumioxazin concentrations ranging from 0 to 10 mM. The 0 mM samples had 25 µL DMSO (2.5%) to mimic the concentration of DMSO in the samples containing 10 mM herbicide. Each tube was inoculated with 10 µL of an overnight culture of HemG cells complemented with the wildtype PPX plasmid to ensure uniformity. Spectrophotometric readings (OD600) were taken for each sample (1:4 dilution) and a sample of each plated on LB-Chlor-IPTG plates to determine whether the OD600 correlated with the number of viable colonies. A 10% dilution of the overnight culture was plated for the sulfentrazone-treated cells and a 1% dilution for the cells treated with flumioxazin was plated (see results in Tables 8a-f and 9a-d).

Both sulfentrazone and flumioxazin precipitate out in the liquid media, causing it to appear opaque even before inoculating bacteria. As the ODs for sulfentrazone show, this herbicide eventually goes into solution while flumioxazin does not. Flumioxazin's deficient solubility skews the OD readings, however, flumioxazin demonstrated steadily decreasing colony numbers against the WT gene, indicating the cells ability to absorb the flumioxazin from the media.

### 5' end mutagenesis

The 5' end of the PPX gene was mutagenized using Stratagene GeneMorph II Random Mutagenesis Kit and cloned into XL-1 Blue to check the mutation rate. Results showed 14 out of 16 colonies sequenced were mutants. The 90% XL-1 Blue plates (approximately 4000 colonies) were scraped, plasmid prepped, and transformed into HemG and plated on 2.5 mM sulfentrazone. The sulfentrazone plates grew approximately 200 colonies and the 10% LB-Chlor-IPTG plates grew a lawn of colonies. Tables 9a-d describe the nucleotide and amino acid substitutions found in the tolerant clones.

### Selection of mutagenized clones

Randomly mutagenized plasmids (5' and 3' ends) were transformed into XL1-Blue *E. coli* cells. The resulting colonies were pooled, plasmid DNA isolated and transformed into HemG (PPX mutant *E. coli*) cells. For selection with flumioxazin, cells were recovered for 1 h in liquid minimal media followed by the addition of herbicide and overnight recovery of the cells. The next day, the cells were plated at an appropriate dilution on LB plates containing antibiotic to select for the complementing plasmid. Colonies from each plate were sequenced. After liquid selection in 10 mM flumioxazin, approximately 30 colonies appeared on plates with the wild type (WT) PPX compared to approximately 200-1200 colonies with mutagenized plasmids. For sulfentrazone selection, cultures were grown in minimal media overnight, diluted and plated plates with 0 and 0.75 mM concentration of sulfentrazone. Colony counts were compared between the two and mutation tolerance determined based on the percentage of colonies on the 0.75 mM sulfentrazone plates as compared with those on the 0 mM plates. The number of colonies appearing on the plates served as a method to rank the mutations.

### 3' end mutagenesis

Mutagenesis was performed on the 3' end of the PPX gene. Clones were transformed into HemG and grown overnight in 2.5, 5, and 10 mM flumioxazin for selection. Selection at 5mM flumioxazin had many more colonies than on the 10mM selection plates. Selection for mutagenized clones using 10mM flumioxazin yielded four clones. Selection for mutagenized clones usine 5mM flumioxazin yielded 200 colonies. The top third of the 200 colonies obtained for 3' end mutagenesis were screened in 10mM flumioxazin. All tolerant colonies (approximately 130) were sequenced and the best flumioxazin tolerant mutants, assessed by colony count on flumioxazin, the 3' end assessed for tolerance to sulfentrazone.

### Example 4: Analysis of Amino Acid Substitutions Conferring Tolerance

Tolerance of all possible amino acid substitutions at each position displaying tolerance to sulfentrazone or flumioxazin were tested. Next, single amino acid substiutions were combined in all permutations and combinations to assess complementation and herbicide tolerance. Results of single and multiple mutant combinations to flumioxazin are shown in Tables 8a, 8b, and 8c, where the last column shows the number of colonies reported for each mutation on 10mM flumioxazin. Results of single and multiple mutant combinations to sulfentrazone are shown in Tables 9a and 9b, where the last column shows the number of colonies reported for each mutation on 0.75mM sulfentrazone. Results of single and multiple mutant combinations to saflufenacil are shown in Table 10, where the fourth column shows the number of colonies reported for each mutation on 0.3mM saflufenacil.

**Table 8a. Tolerance of single and multiple mutant combinations in the potato plastidal PPX coding sequence to flumioxazin.**

| **Mutation** | | **Plasmid** | **Avg # of Flumioxazin Resistant Clones** |
|---|---|---|---|
| --- | L393V | F1125 | 0 |
| --- | L403R | F1155 | 2 |
| A180T | Y426F | SD5083 | 2 |
| --- | L424S | F1154 | 2 |
| F145L | Y426H | SD5055 | 3 |
| S244G | Y426F | SD5087 | 10 |
| F145L | L403R | SD5115 | 10 |
| --- | Y426F | F1165 | 11 |
| F145Y | L424S | SD5106 | 13 |
| R144C | L424S | SD5102 | 41 |
| L226M | Y426H | SD5059 | 55 |
| A220T | L403R | SD5114 | 69 |
| --- | Y426H | F1180 | 71 |
| A220T | L424S | SD5104 | 72 |
| F145Y | Y426F | SD5086 | 75 |
| R144C | L393V | SD5092 | 81 |
| A220T | L393V | **SD5094** | 107 |
| S244G | L393V | ***SD5097*** | 114 |
| L226M | L403R | ***SD5119*** | 123 |
| A180T | Y426H | ***SD5053*** | 129 |
| L226M | L424S | ***SD5109*** | 137 |
| L226M | Y426F | ***SD5089*** | 143 |
| A220T | Y426F | **SD5084** | 147 |
| A220T | Y426H | **SD5054** | 151 |
| R144C | Y426F | ***SD5082*** | 155 |
| --- | --- | wt | 0 |

**Table 8b. Tolerance of single and multiple mutant combinations in the potato plastidal PPX coding sequence to flumioxazin.**

| **Mutation** | | | **Plasmid** | **Avg # of Flumioxazin Resistant Clones** |
|---|---|---|---|---|
| R144C | A220T | | SD5011 Col 1 | 701 |
| A220T | | | F113 | 667 |
| R144H | S332C | | F76 | 517 |
| R144C | K272F | | SD5014 Col 1 | 300 |
| N52K | R144H | S244T | F72 | 202 |
| N85D | A220T | | SD5007 Col 1 | 196 |
| R144H | S244T | | F96 | 174 |
| R144C | | | S7 | 139 |
| R144C | L226M | | SD5012 Col 1 | 117 |
| M228L | | | S37 | 107 |
| L226M | | | F114 | 102 |
| N85D | L226M | | SD5008 Col 2 | 50 |
| F145Y | | | S32 | 38 |
| N85D | F145Y | | SD5004 Col 3 | 37 |
| S244G | | | S120 | 31 |
| R144C | M228L | | SD5013 Col 2 | 31 |
| P185R | | | SD5016 Col 1 | 27 |
| N52K | | | SD5001 Col 3 | 23 |
| N85D | A180T | | SD5005 Col 1 | 23 |
| A180T | | | F17 | 22 |
| N85D | R144C | | SD5002 Col 1 | 21 |
| S332C | | | SD5019 Col 1 | 20 |
| F145L | | | S118 | 19 |
| N85D | | | F80 | 16 |
| K272F | | | F7 | 15 |
| S244T | | | SD5018 Col 3 | 10 |
| N85D | K272F | | SD5010 Col 4 | 7 |
| N85D | M228L | | SD5009 Col 3 | 6 |
| | | | WT | 43 |

**Table 8c. Tolerance of single and multiple mutant combinations in the potato plastidal PPX coding sequence to flumioxazin.**

| **Mutation** | | **Plasmid** | **Avg # of Flumioxazin Resistant Clones** |
|---|---|---|---|
| N85D | Y426H | SD5051 | 30 |
| R144C | Y426H | SD5052 | 48 |
| F145Y | Y426H | SD5056 | 52 |
| S244T | Y426H | SD5058 | 73 |
| S244G | Y426H | SD5057 | 80 |
| A180T | Y426H | SD5053 | 128 |
| L226M | Y426H | SD5059 | 228 |
| F145L | Y426H | SD5055 | 305 |
| A220T | Y426H | SD5054 | 391 |
| --- | Y426H | F1180 | 210 |
| --- | --- | wt | 32 |

**Table 8d. Tolerance of single and multiple mutant combinations in the potato mitochondrial PPX coding sequence to 5 mM flumioxazin.**

| | |
|---|---|
| A404S | |
| C271R | D274G |
| C307S | A423V |
| C434S | T500S |
| C434Y | |
| D330E | |
| D447G | A292G |
| D454N | |
| *N324K* | |
| R406K | |
| S396L | K4101 |
| S448A | *N324D* |
| Y465F | K470T |

**Table 8e. Tolerance of single and multiple mutant combinations in the potato mitochondrial PPX coding sequence to 5 mM flumioxazin.**

| | | | |
|---|---|---|---|
| A101V | | | |
| C177S | | | |
| D170E | | | |
| D58N | | | |
| E150K | | | |
| E64V | | | |
| F121L | E150D | | |
| G74C | R98C | | |
| G84N | | | |
| K97R | | | |
| L93H | V164A | | |
| N195K | | | |
| P214S | | | |
| Q157L | H187Q | | |
| R98C | | | |
| R98H | | | |
| R98L | | | |
| R98L | P214H | | |
| R98L | T124I | L188F | K229Q |
| S119N | N139Y | | |
| S151T | K229E | K230R | |
| V164F | | | |

**Table 8f. Tolerance of single and multiple mutant combinations in the potato mitochondrial PPX coding sequence to 10 mM flumioxazin.**

| Mutant | # Colonies with 10 mM Flumioxazin |
|---|---|
| wt | 42 |
| R98L | 83 |
| P214H | 51 |
| R98L/P214H | 88 |
| R98L/P214H/T124I | 110 |
| R98L/P214H/T124I/K229Q | 109 |

**Table 9a. Tolerance of single and multiple mutant combinations in the potato plastidal PPX coding sequence to sulfentrazone.**

| **Mutation** | | **Plasmid** | **Avg 0.75 mM Sulf** | **Avg 0 mM** | **0.75mM/0mM** |
|---|---|---|---|---|---|
| --- | Y426F | F1165 | 8 | 91 | 0.8% |
| --- | L393V | F1125 | 6 | 57 | 1.0% |
| L226M | Y426H | SD5059 | 14 | 79 | 1.8% |
| S244T | L393V | SD5098 | 11 | 53 | 2.1% |
| F145Y | Y426H | SD5056 | 16 | 72 | 2.2% |
| --- | L403R | F1155 | 16 | 72 | 2.2% |
| R144C | S525T | SD5072 | 19 | 76 | 2.6% |
| A220T | L393V | SD5094 | 20 | 55 | 3.6% |
| --- | L424S | F1154 | 21 | 56 | 3.7% |
| F145L | L424S | SD5105 | 18 | 48 | 3.8% |
| --- | Y426H | F1180 | 29 | 68 | 4.2% |
| A220T | Y426H | SD5054 | 19 | 39 | 5.0% |
| F145Y | L393V | SD5096 | 55 | 86 | 6.3% |
| S244T | Y426F | SD5088 | 58 | 88 | 6.5% |
| F145Y | L424S | SD5106 | 49 | 69 | 7.1% |
| A220T | L403R | SD5114 | 50 | 63 | 7.9% |
| L226M | Y426F | SD5089 | 117 | 67 | 17.4% |
| N85D | Y426H | SD5051 | 200 | 92 | 21.8% |
| L226M | L424S | SD5109 | 91 | 40 | 22.6% |
| F145Y | L403R | SD5116 | 315 | 110 | 28.5% |
| F145L | L393V | SD5095 | 381 | 121 | 31.6% |
| L226M | L403R | SD5119 | 252 | 75 | 33.8% |
| R144C | Y426F | SD5082 | 400 | 117 | 34.3% |
| S244G | L393V | SD5097 | 433 | 125 | 34.7% |
| --- | S525T | F1061 | 278 | 79 | 35.3% |
| A180T | Y426H | SD5053 | 331 | 80 | 41.4% |
| R144C | Y426H | SD5052 | 709 | 125 | 56.6% |
| --- | --- | wt | 0 | 85 | 0.0% |

**Table 9b. Tolerance of single and multiple mutant combinations in the potato plastidal PPX coding sequence to sulfentrazone.**

| **Mutation(s)** | | | **Plasmid** | **Avg # of Sulfentrazone Resistant Clones** |
|---|---|---|---|---|
| R144C | M228L | | SD5013 Col 2 | 267 |
| R144C | A220T | | SD5011 Col 1 | 260 |
| F145Y | | | S32 | 233 |
| R144C | L226M | | SD5012 Col 1 | 186 |
| A220T | | | F113 | 149 |
| P185R | | | SD5016 Col 1 | 133 |
| R144C | K272F | | SD5014 Col 1 | 118 |
| N52K | | | SD5001 Col 3 | 90 |
| M228L | | | S37 | 78 |
| N85D | A180T | | SD5005 Col 1 | 75 |
| S244G | | | S120 | 68 |
| R144H | S332C | | F76 | 64 |
| N85D | F145Y | | SD5004 Col 3 | 62 |
| K272F | | | F7 | 61 |
| L226M | | | F114 | 57 |
| R144C | | | S7 | 55 |
| S244T | | | SD5018 Col 3 | 32 |
| R144H | S244T | | F96 | 31 |
| F145L | | | S118 | 29 |
| S332C | | | SD5019 Col 1 | 28 |
| N85D | R144C | | SD5002 Col 1 | 25 |
| N85D | A220T | | SD5007 Col 1 | 19 |
| N85D | L226M | | SD5008 Col 2 | 18 |
| A180T | | | F17 | 18 |
| N52K | R144H | S244T | F72 | 18 |
| N85D | M228L | | SD5009 Col 3 | 12 |
| N85D | | | F80 | 6 |
| N85D | K272F | | SD5010 Col 4 | 6 |
| | | | WT | 23 |

**Table 9c. Tolerance of single and multiple mutant combinations in the potato mitochondrial PPX coding sequence to sulfentrazone.**

| **Sulf Conc** | **Mutant(s)** | **Colonies** | **%age** |
|---|---|---|---|
| 0 mM | R98L/P214H | 1341 | |
| 0.5 mM | R98L/P214H | 349 | 26.0% |
| 0.9 mM | R98L/P214H | 127 | 9.5% |
| 1.0 mM | R98L/P214H | 77 | 5.7% |
| 1.1 mM | R98L/P214H | 67 | 5.0% |
| 1.2 mM | R98L/P214H | 48 | 3.6% |
| 0 mM | R98L | 1541 | |
| 0.5 mM | R98L | 339 | 22.0% |
| 0.9 mM | R98L | 145 | 9.4% |
| 1.0 mM | R98L | 110 | 7.1% |
| 1.1 mM | R98L | 76 | 4.9% |
| 1.2 mM | R98L | 54 | 3.5% |
| 0 mM | R98L/T124I/K229Q | 1220 | |
| 0.5 mM | R98L/T124I/K229Q | 312 | 25.6% |
| 0.9 mM | R98L/T124I/K229Q | 88 | 7.2% |
| 1.0 mM | R98L/T124I/K229Q | 66 | 5.4% |
| 1.1 mM | R98L/T124I/K229Q | 40 | 3.3% |

**Table 9d. Tolerance of single and multiple mutant combinations in the potato mitochondrial PPX coding sequence to sulfentrazone.**

| **Sulf Conc** | **Mutant(s)** | **Colonies** | **%age** |
|---|---|---|---|
| 0 mM | R98L/P214H | 1088 | |
| 0.4 mM | R98L/P214H | 251 | 23.1% |
| 0.5 mM | R98L/P214H | 150 | 13.8% |
| 0.6 mM | R98L/P214H | 105 | 9.7% |
| 0.7 mM | R98L/P214H | 108 | 9.9% |
| 0.8 mM | R98L/P214H | 51 | 4.7% |
| 0 mM | R98L | 1171 | |
| 0.4 mM | R98L | 174 | 14.9% |
| 0.5 mM | R98L | 104 | 8.9% |
| 0.6 mM | R98L | 98 | 8.4% |
| 0.7 mM | R98L | 92 | 7.9% |
| 0.8 mM | R98L | 51 | 4.4% |
| 0 mM | R98L/T124I/P214H/K229Q | 1134 | |
| 0.4 mM | R98L/T124I/P214H/K229Q | 724 | 63.8% |
| 0.5 mM | R98L/T124I/P214H/K229Q | 654 | 57.7% |
| 0.6 mM | R98L/T124I/P214H/K229Q | 402 | 35.4% |
| 0.7 mM | R98L/T124I/P214H/K229Q | 302 | 26.6% |
| 0.8 mM | R98L/T124I/P214H/K229Q | 280 | 24.7% |
| 0 mM | P214H | 1184 | |
| 0.4 mM | P214H | 0 | 0.0% |
| 0.5 mM | P214H | 0 | 0.0% |
| 0.6 mM | P214H | 0 | 0.0% |
| 0.7 mM | P214H | 0 | 0.0% |
| 0.8 mM | P214H | 0 | 0.0% |

**Table 10. Tolerance of single and multiple mutant combinations in the potato plastidal PPX coding sequence to saflufenacil measured by number of colonies reported.**

| **Mutation** | | **Plasmid** | **Avg # of saflufenacilresistant clones** | **Avg 0 mM** | **0.3mM/0mM** |
|---|---|---|---|---|---|
| --- | S525T | F1061 | 0 | 69 | 0.0% |
| N85D | Y426H | SD5051 | 0 | 64 | 0.0% |
| F145L | L393V | SD5095 | 0 | 114 | 0.0% |
| S244G | L393V | SD5097 | 0 | 111 | 0.0% |
| F145Y | L403R | SD5116 | 0 | 104 | 0.0% |
| L226M | L424S | SD5109 | 6 | 50 | 1.3% |
| L226M | Y426F | SD5089 | 15 | 96 | 1.5% |
| A220T | L393V | SD5094 | 116 | 99 | 11.7% |
| A220T | Y426F | SD5084 | 190 | 87 | 22.0% |
| L226M | L403R | SD5119 | 225 | 69 | 32.5% |
| R144C | Y426F | SD5082 | 319 | 61 | 52.6% |
| R144C | Y426H | SD5052 | 415 | 75 | 55.4% |
| A180T | Y426H | SD5053 | 394 | 60 | 65.7% |
| A220T | Y426H | SD5054 | 356 | 46 | 77.5% |
| --- | --- | wt | 0 | 96 | 0.0% |

### Example 5: Plant Cell Culture - Herbicide Kill Curves

### Flumioxazin kill curves

Herbicide selection experiments were performed to determine the concentration of herbicide necessary to kill protoplast derived microcalli in a defined treatment period. In light of an initial kill curve result where a concentration of 125 µM was sufficient to kill all cells within a week, a new kill curve was designed using lower concentrations of flumioxazin aimed at determining the concentration at which 99% of the cells are killed (see Table 11). The herbicide was suspended in DMSO, with the final concentration of DMSO in the herbicide treatments being 1%. Development of cells was evaluated under the microscope once a week. Excepting the control treatments, division in all treatments with flumioxazin was prevented after one week and after one month no microcalli developed at any concentration tested. A flumioxazin concentration of 0.032 mM is sufficient to prevent microcallus development from potato protoplasts.

**Table 11. Summary of results of flumioxazin kill curve experiments with cell suspension and shoot tip-derived protoplasts. Protoplasts were exposed to flumioxazin for a period of one month.**

| Non treated protoplast + PEG | # Of calli in 3 beads | | Stop-GFP + Correcting GRON | # of calli in 3 beads | |
|---|---|---|---|---|---|
| Flumioxazin Conc. µM | # | % | Flumioxazin Conc. µM | # | % |
| Control #1* | 185 | | Control | 230 | |
| Control #2** | 150 | | Control | 190 | |
| Av. Cont 1 + Cont 2 | 157.5 | 100 | Av. Cont 1 + Cont 2 | 210 | 100 |
| 0.0156 | 40 | 25 | 0.0156 | 83 | 39 |
| 0.0312 | 8 | 5 | 0.0312 | 11 | 4 |
| 0.0468 | 0.0 | 0.0 | 0.0468 | 0.0 | 0.0 |
| 0.0624 | 0 | 0 | 0.0624 | 0 | 0 |

| | | | | | |
|---|---|---|---|---|---|
| * Culture medium, no herbicide ** Culture medium with 1% DMSO, no herbicide | | | | | |

### Sulfentrazone kill curves

Kill curves with sulfentrazone on shoot tip-derived protoplasts and cell suspension showed concentrations of 7.8 µM sulfentrazone are sufficient to kill all protoplast-derived cells (see Table 12). Therefore, new kill curves were initiated with lower concentrations of 0, 0.5, 0.6, 0.7 and 0.8 µM of sulfentrazone, shown in Table 13. The results suggest that GRON treated protoplasts may be selected at concentrations between 0.6 µM and 0.7 µM of the herbicide.

**Table 12. Summary of results of sulfentrazone kill curve experiments with cell suspension and shoot tip-derived protoplasts. Protoplasts were exposed to sulfentrazone for a period of one month.**

| **Sulfentrazone treatment [µM]** | **Microcallus formation** |
|---|---|
| Control: 1* | Yes; abundant |
| Control: 2** | Yes; abundant |
| 62.5 | No |
| 31.25 | No |
| 15.6 | No |
| 7.8 | No |

| | |
|---|---|
| * Culture medium, no herbicide ** Culture medium with 1% DMSO, no herbicide | |

**Table 13. Summary of results of sulfentrazone kill curve experiments with cell suspension and shoot tip-derived protoplasts. Protoplasts were exposed to the herbicide for a period of one month.**

| Non treated protoplast + PEG | # Of calli in 3 beads | | Stop-GFP + Correcting GRON | # of calli in beads | |
|---|---|---|---|---|---|
| Sulfentrazon Conc. µM | # | % | Sulfentrazonel Conc. µM | # | % |
| 0.0 | 115 | 100 | 0.0 | 125 | 100% |
| 0.5 | 45 | 30 | 0.5 | 38 | 30.2 |
| 0.6 | 8 | 6.9 | 0.6 | 12 | 9.6 |
| 0.7 | 2 | 1.7 | 0.7 | 7 | 5.6 |
| 0.8 | 0.0 | 0.0 | 0.8 | 0.0 | 0.0 |

| | | | | | |
|---|---|---|---|---|---|
| * Culture medium, no herbicide ** Culture medium with 1% DMSO, no herbicide | | | | | |

### Example 6: Leaf disk kill curves.

Aimed at establish the concentration of sulfentrazone that will inhibit callus formation in leaf disc explants, leaf discs were punched with a sterile hole punch from 5 weeks old in vitro grown potato plants. The leaf discs were cultured in petri dishes containing solid Haberlach culture medium containing various concentrations of Sulfentrazone in a final concentration of 1% DMSO. Six leaf discs cultured at each herbicide concentration. Plates were sealed with micropore tape and incubated at room temperature (approximately 23°C). In an initial experiment, 7.8 µM sulfentrazone, the lowest concentration tested in that experiment, was sufficient to stop callus formations and bleach all leaf discs within 20 days. Results of kill curve with lower concentrations of sulfentrazone showed that a concentration of 3.0 µM of Sulfentrazone was sufficient to inhibit callus formation in almost all leaf discs after 20 days, whereas callus initiated after 13 days on the leaf veins of some leaf discs grown on 2.0 µM of Sulfentrazone. Similar leaf disc kill curve experiments were performed using saflufenacil where 0.5 µM of this herbicide was sufficient to inhibit callus formation in almost all leaf discs after 20 days.

### Example 7: Materials and Methods for Cell Culture and GRON Introduction

**Table 14. GRON Sequences**

| **GRON** | **Sequence** |
|---|---|
| StcPPX1144/C/47/5'Cy3/3'idC | VGTTGGGAGATCCTGATGCGCCTTGCTTTGTCTTG TGGAAGGATAAACH (SEQ ID NO: 33) |
| StcPPX1144/NC/47/5'Cy3/3'idC | VGTTTATCCTTCCACAAGACAAAGCAAGGCGCATC AGGATCTCCCAACH (SEQ ID NO: 34) |
| StcPPX1220/C/47/5'Cy3/3'idC | VCATCATTTTACAGGTGTTTACACCGGTGACCCCT CAAAATTGH (SEQ ID NO: 35) |
| StcPPX1220/NC/47/5'Cy3/3'idC | VCAATTTTGAGGGGTCACCGGTGTAAACACCTGTA AAATGATGH (SEQ ID NO: 36) |

| | |
|---|---|
| The converting base is shown in bold. V=CY3; H=3'DMT dC CPG | |

**Cell Culture Work Description.** Shoots, for example, derived from seeds, tubers, axillary buds, leaves, steams, roots, callus, or from microspore-derived embryos, are propagated under sterile conditions *in vitro.* Explants are subcultured, for example, every 3-4 weeks and cultured in Magenta GA7 culture vessels (Phytotechnology Laboratories, Shawnee Mission, KS, USA) with vented lids in a volume of about 100 mL culture medium, for example MS medium, according to Murashige and Skoog (A revised medium for rapid growth and bioassays with tobacco cultures. Physiol. Plant 15 (1962) 473-49), or modifications thereof. The vessels may be sealed with Micropore tape (3M Company). Young leaves, shoot tips, roots, microtubers or long stem segments possessing a leaf and axillary bud, as well as callus derived from these tissues, may be used for protoplast isolation. Protoplasts may also be isolated from suspension culture cells derived from young leaves, shoot tips, roots, microtubers or long stem segments possessing a leaf and axillary bud, as well as callus derived from these tissues.

### Protoplast isolation from shoot tips

About 200 shoot tips of 2 - 8 week-old *in vitro* shoots that have been cultured under a regular day/night regime, or, preferably were kept for two days before protoplast isolation in the dark, shoots may be cut into small pieces with a scalpel in a petri dish with sterile water. After all tips have been cut, the water is replaced with protoplast culture medium, preferably BN (B5 Salts and Vitamins (Phytotechnology Laboratories), glucose 20 g/L, mannitol 70 g/L, alpha naphthalene acetic acid 5 mg/L, additional CaCl₂ x 2H₂O 600 mg/L, casein hydrolysate 250 mg/L, cysteine-HCL 10 mg/L, polyvinylpyrrolidone (MW 10,000) 5 g/L. After approximately 1 - 2 h, the protoplast culture medium is replaced with enzyme solution, for example consisting of medium BN, in which 0.5% (w/v) Cellulase YC and 0.75% (w/v) Macerozyme R10 (both from Karlan Research Products, Cottonwood, Arizona), 1 g/L bovine serum albumin, and 1 g/L 2-morpholinoethanesulfonic acid are dissolved. The ratio of the number of shoot tips over the volume of enzyme solution can be between 10 and 16, preferably 13. The dish with shoot tip pieces in enzyme solution is incubated for at 25°C-30°C, preferably 28°C, in darkness on a shaker set to about 50 rpm. After overnight incubation the protoplast suspension is purified using an iodixanol density gradient (adapted from Optiprep Application Sheet C18; Purification of Intact Plant Protoplasts; Axis-Shield USA, 10 Commerce Way, Norton, MA 02776). After the density gradient centrifugation, the band with purified protoplasts is removed together with about 5 mL W5 medium (Frigerio *et al.,* 1998). The protoplast density and yield are determined with a hemocytometer. The protoplasts density is adjusted to1 x 10⁶/mL in BN medium containing 2 mg/L 2,6-dichlorobenzonitrile (cellulose synthase inhibitor), and the protoplasts are cultured in darkness at 30°C for about 16 h.

### Protoplast isolation from cell suspensions

The isolation of protoplasts from cell suspensions follows the same protocol as described for the isolation of protoplasts from shoot tips, with the following exceptions: 1. Fast growing cell suspensions are used, preferably three days after their last subculture. 1.5 mL settled cell volume is transferred to about 15 ml BN medium, which after 2h is replaced with enzyme solution. 2. The protoplast purification is followed immediately by the GRON/PEG treatment.

### Gene Repair Oligonucleotide (GRON) introduction

The protoplast suspension is mixed with an equal volume of W5 medium, transferred to a 50 mL centrifuge tube, and centrifuged for 10 min at the lowest setting of a clinical centrifuge (about 50 x g). The supernatant is removed and replaced with TM medium (Klaus, S. Markerfreie transplastome Tabakpflanzen (Marker-free transplastomic tobacco plants). PhD Dissertation, 2002, Ludwig-Maximilians-Universität München, 109 pp), adjusting the protoplast density to 5 × 10⁶/mL. Aliquots of 100 µL containing 5 × 10⁵ protoplasts each are distributed into 12 mL round bottom centrifuge tubes. GRONs (such as those shown in Table 14) targeted at one or more mutations in one or both of the mitochondrial and plastidal PPX genes are then introduced into the protoplasts using a PEG treatment. To introduce the GRONs into the protoplasts, 12.5 µg GRON dissolved in 25 µL purified water and 125 µL of a polyethylene glycol solution (5 g PEG MW 1500, 638 mg mannitol, 207 mg CaNO₃ x 4H₂O and 8.75 mL purified water; pH adjusted to about 9.0) is added. After a 10-30 min incubation on ice, the protoplast-PEG suspension is washed with W5 medium and resuspended in medium BN. The suspension is kept overnight in darkness at room temperature.

GRONs may be introduced into protoplasts by electroporation, cationic lipids, nanoparticles, polycations such as hexadimethrine bromide (polybrene) or spermidine, or by using GRONs complexed to a variety of cell penetrating peptides (CPPs) including but not limited to TAT, pVEC, transportan, nona-arginine, BAX inhibiting peptide (VPMLK), or such as those listed in Patel et al. Cell Penetrating Peptides: Intracellular Pathways and Pharmaceutical Perspectives. Pharmaceutical Research, 24 (2007) 1977-1992, or Veldhoen et al. Recent developments in peptide-based nucleic acid delivery. International Journal of Molecular Science (2008) 1276-1320. In another embodiment, GRONs are introduced into protoplasts through negatively charged polymers including, but not limited to dendrimers such as Polyamidoamine (PAMAM).

GRONs may also be delivered into whole tissues or cells using methods that may include microinjection, biolistics with the GRONs coated on carriers such as gold or directly in the form of droplets of a GRON suspension, GRON coated whiskers or using GRONs complexed to a variety of cell penetrating peptides (CPPs) negatively charged polymers as mentioned in the preceding paragraph. Other embodiments envision the use of ultrasound, imbibition in GRON containing solutions, or permeabilization of cell walls, for example through agents such as toluene or saponin.

### Embedding of protoplasts in calcium alginate

One day after the GRON introduction, protoplasts are embedded in calcium alginate. The embedding of protoplasts in gel substrates (e.g., agarose, alginate) has been shown to enhance protoplast survival and to increase division frequencies of protoplast-derived cells. The method applied is based on that described in Dovzhenko et al. (Thin-alginate-layer technique for protoplast culture of tobacco leaf protoplasts: shoot formation in less than two weeks. Protoplasma 204 (1998) 114-118).

### Protoplast culture and selection of herbicide-resistant calli

The selection of herbicide-resistant calli is carried out using sequential subcultures of the alginates in liquid media according to Pelletier *et al.* (1983). Selection may be started one week after the PEG/GRON treatment at an appropriate concentration of PPX-inhibiting herbicide; for example, 32 µM flumioxazin, or 0.25 µM, 0.5 µM, 1 µM, 2 µM, 3 µM, 4 µM, 5 µM, 6 µM, 7 µM, 7.8 µM, 15.6 µM, 31.2 µM or 62.5 µM sulfentrazone.

Before the end of the selection phase in liquid medium, cells and colonies are released from the alginate by treating them for 30-45 min with culture medium containing 50 mM sodium citrate. At the moment of transferring released colonies from liquid to solid medium Cul (Haberlach et al. Isolation, culture and regeneration of protoplasts from potato and several related Solanum species. Plant Science, 39 (1985) 67-74), the majority of colonies may be either dead, or consist of a greenish center, covered with outer layers of dead cells. On the solidified selection medium (Cul + herbicide) the majority of microcalli that still contain living cells may stop growing and turn brownish. Limited growth of individual calli continues occasionally, but all non-resistant calli eventually turn brown and die. Two to three weeks after the transfer to solidified selection medium (occasionally earlier), actively growing calli may appear among a background of brownish cells and microcalli

Regeneration of plants from protoplast-derived, herbicide-tolerant calli with a confirmed mutation in a PPX gene is performed. PPX-inhibiting herbicide-tolerant calli that develop on solidified selection medium and whose DNA upon analysis shows the presence of a mutation are transferred to herbicide-free medium Cul to accelerate development. Individual callus lines vary in their growth rates and morphologies. In general, the development towards shoot regeneration follows these steps:
Undifferentiated, green callus → callus with dark green areas → development of shoot initials → development of a plant.

The development of individual callus lines is variable, but through continuous subculture and multiplication on Cul medium or by changing the media formulation to differentiation medium including but not limited to Haberlach differentiation medium, for an acceptable period of time (1-6 months) followed by transfer of the callus lines to regeneration media including but not limited to Bokelmann regeneration medium (Bokelmann G.S. and Roest S., Z. Pflanzenphysiol. vol. 109, p. 259-265 (1983)) eventually many produce shoots.

Once shoots with three to four leaves are formed on regeneration medium, they are transferred to propagation medium including but not limited to MS medium. On this medium, over time, shoots and leaves develop that are morphologically 'normal'. After *in vitro* plantlets produce roots, standard protocols are used for the adaptation to greenhouse conditions.

### Molecular screening

Using standard molecular techniques and more sensitive PCR based technologies can be used to monitor the frequency of PPX mutations following an RTDS treatment. These molecular techniques include and are not limited to, allele specific PCR, DNA sequencing and other SNP identification technologies using non-PCR techniques. These techniques allow for monitoring the frequency of PPX targeted mutations early in the procedure. In certain embodiments, the mutations can be measured in populations of single cells. These techniques can then be applicable throughout the culture process to confirm and monitor mutations are present in selected calli and regenerated plants.

### Example 8: Herbicide Spray

*Solanum tuberosum* or Russet Burbank potato cultivar plants when they are 2-6" tall (generally the 5-6 leaf stage) are sprayed with various PPX-inhibiting herbicides. Herbicides are sprayed in the presence of 0.25% AU391 surfactant. The herbicides are sprayed, for example, at the following rates:

| | |
|---|---|
| Flumioxazin | 2 oz active ingredient/Acre (ai/A) |
| Sulfentrazone | 4.5 oz ai/A |
| Saflufenacil | 1-13 oz ai/A |

Herbicides are applied by foliar spray with control plants being left unsprayed. PPX-inhibiting herbicide trials are evaluated 14 days post spraying using a damage scale of 1-10 with 1 being dead, and 10 being the undamaged unsprayed controls. Individual plant lines are scored at each spray rate compared to the performance of the controls at that particular rate. PPX inhibiting herbicides have a potentially wide window of application and can be used as a "pre" or "post" application for crops including potato. Herbicide evaluations include both greenhouse and field applications to monitor plant (crop) damage and/or weed control. Products from RTDS work can allow farmers to plant crops like potato and apply PPX inhibiting herbicides to eliminate or control weeds in the fields while not damaging crops.

Unless otherwise defined, all technical and scientific terms used herein have the same meaning as commonly understood by one of ordinary skill in the art to which this invention belongs.

The inventions illustratively described herein may suitably be practiced in the absence of any element or elements, limitation or limitations, not specifically disclosed herein. Thus, for example, the terms "comprising," "including," "containing," etc. shall be read expansively and without limitation. Additionally, the terms and expressions employed herein have been used as terms of description and not of limitation, and there is no intention in the use of such terms and expressions of excluding any equivalents of the features shown and described or portions thereof. It is recognized that various modifications are possible within the scope of the invention claimed.

Thus, it should be understood that although the present invention has been specifically disclosed by preferred embodiments and optional features, modification, improvement, and variation of the inventions disclosed may be resorted to by those skilled in the art, and that such modifications, improvements and variations are considered to be within the scope of this invention. The materials, methods, and examples provided here are representative of preferred embodiments, are exemplary, and are not intended as limitations on the scope of the invention.

The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein.

In addition, where features or aspects of the invention are described in terms of Markush groups, those skilled in the art will recognize that the invention is also thereby described in terms of any individual member or subgroup of members of the Markush group.

All publications, patent applications, patents, and other references mentioned herein are expressly incorporated by reference in their entirety, to the same extent as if each were incorporated by reference individually. In case of conflict, the present specification, including definitions, will control.

The following statements further describe the present invention.
**1.** A non-transgenic plant cell comprising a mutated protoporphyrinogen IX oxidase (PPX) gene, wherein said gene encodes a protein comprising a mutation at one or more amino acid positions corresponding to a position selected from the group consisting of 52, 85, 105, 111, 130, 139, 143, 144, 145, 147, 165, 167, 170, 180, 185, 192, 193, 199, 206, 212, 219, 220, 221, 226, 228, 229, 230, 237, 244, 256, 257, 270, 271, 272, 305, 311, 316, 318, 332, 343, 354, 357, 359, 360, 366, 393, 403, 424, 426, 430, 438, 440, 444, 455, 457, 470, 478, 483, 484, 485, 487, 490, 503, 508 and 525 of SEQ ID NO: 1 or wherein said gene encodes a protein comprising a mutation at one or more amino acid positions corresponding to a position selected from the group consisting of 58, 64, 74, 84, 93, 97, 98, 101, 119, 121, 124, 139, 150 151, 157, 164, 170, 177, 187, 188, 195, 214, 215, 229, 230, 271, 274, 278, 283, 292, 296, 307, 324, 330, 396, 404, 406, 410, 421, 423, 434, 447, 448, 449, 451, 454, 465, 470 and 500 of SEQ ID NO: 9.
**2.** The non-transgenic plant cell of statement 1, wherein the mutations at one or more amino acid positions are selected from the group consisting of
   a glycine to lysine at a position corresponding to position 52 of SEQ ID NO: 1;
   an asparagine to lysine at a position corresponding to position 52 of SEQ ID NO: 7;
   an asparagine to aspartic acid at a position corresponding to position 85 of SEQ ID NO: 1;
   a glutamic acid to valine at a position corresponding to position 111 of SEQ ID NO: 1;
   a glycine to asparagine at a position corresponding to position 130 of SEQ ID NO: 1;
   an aspartic acid to histidine at a position corresponding to position 139 of SEQ ID NO: 1;
   a proline to arginine at a position corresponding to position 143 of SEQ ID NO: 1;
   an arginine to cysteine at a position corresponding to position 144 of SEQ ID NO: 1;
   ; an arginine to leucine at a position corresponding to position 144 of SEQ ID NO: 1;
   an arginine to histidine at a position corresponding to position 144 of SEQ ID NO: 1;
   a phenylalanine to leucine at a position corresponding to position 145 of SEQ ID NO: 1, ;
   a phenylalanine to tyrosine at a position corresponding to position 145 of SEQ ID NO: 1;
   a leucine to valine at a position corresponding to position 147 of SEQ ID NO: 1;
   a phenylalanine to asparagine at a position corresponding to position 165 of SEQ ID NO: 1;
   an alanine to threonine at a position corresponding to position 180 of SEQ ID NO: 1;
   a proline to histidine at a position corresponding to position 185 of SEQ ID NO: 1;
   a proline to arginine at a position corresponding to position 185 of SEQ ID NO: 1;
   a proline to tyrosine at a position corresponding to position 185 of SEQ ID NO: 1;
   a glutamic acid to aspartic acid at a position corresponding to position 192 of SEQ ID NO: 1;
   a glutamic acid to lysine at a position corresponding to position 192 of SEQ ID NO: 1;
   a serine to threonine at a position corresponding to position 193 of SEQ ID NO: 1;
   an arginine to leucine at a position corresponding to position 199 of SEQ ID NO: 1;
   a valine to phenylalanine at a position corresponding to position 206 of SEQ ID NO: 1;
   a tyrosine to serine at a position corresponding to position 219 of SEQ ID NO: 1;
   an alanine to threonine at a position corresponding to position 220 of SEQ ID NO: 1;
   a leucine to methionine at a position corresponding to position 226 of SEQ ID NO: 1;
   a methionine to leucine at a position corresponding to position 228 of SEQ ID NO: 1;
   a lysine to glutamine at a position corresponding to position 229 of SEQ ID NO: 1;
   an alanine to phenylalanine at a position corresponding to position 230 of SEQ ID NO: 1;
   a serine to glycine at a position corresponding to position 244 of SEQ ID NO: 1;
   a serine to threonine at a position corresponding to position 244 of SEQ ID NO: 1;
   an arginine to histidine at a position corresponding to position 256 of SEQ ID NO: 1;
   an arginine to serine at a position corresponding to position 256 of SEQ ID NO: 1;
   a lysine to glutamic acid at a position corresponding to position 270 of SEQ ID NO: 1;
   a lysine to glutamine at a position corresponding to position 270 of SEQ ID NO: 1;
   a proline to arginine at a position corresponding to position 271 of SEQ ID NO: 1;
   a glutamine to phenylalanine at a position corresponding to position 272 of SEQ ID NO: 1;
   a lysine to phenylalanine at a position corresponding to position 272 of SEQ ID NO: 7;
   a serine to leucine at a position corresponding to position 305 of SEQ ID NO: 1;
   a glutamic acid arginine at a position corresponding to position 311 of SEQ ID NO: 1;
   a threonine to glycine at a position corresponding to position 316 of SEQ ID NO: 1;a threonine to glycine at a position corresponding to position 318 of SEQ ID NO: 1;
   a serine to cysteine at a position corresponding to position 332 of SEQ ID NO: 1;
   a leucine to isoleucine at a position corresponding to position 357 of SEQ ID NO: 1;
   a lysine to arginine at a position corresponding to position 359 of SEQ ID NO: 1;
   a lysine to threonine at a position corresponding to position 359 of SEQ ID NO: 1;
   a serine to arginine at a position corresponding to position 359 of SEQ ID NO: 7;
   a serine to threonine at a position corresponding to position 359 of SEQ ID NO: 7;
   a leucine to aspartic acid at a position corresponding to position 360 of SEQ ID NO: 1;
   a leucine to lysine at a position corresponding to position 360 of SEQ ID NO: 1;
   an alanine to glutamic acid at a position corresponding to position 366 of SEQ ID NO: 1;
   a leucine to methionine at a position corresponding to position 393 of SEQ ID NO: 1;
   a leucine to serine at a position corresponding to position 393 of SEQ ID NO: 1;
   a leucine to valine at a position corresponding to position 393 of SEQ ID NO: 1;
   a leucine to arginine at a position corresponding to position 403 of SEQ ID NO: 1;
   a leucine to serine at a position corresponding to position 424 of SEQ ID NO: 1;
   a tyrosine to cysteine at a position corresponding to position 426 of SEQ ID NO: 1;
   a tyrosine to phenylalanine at a position corresponding to position 426 of SEQ ID NO: 1;
   a tyrosine to histidine at a position corresponding to position 426 of SEQ ID NO: 1;
   a tyrosine to isoleucine at a position corresponding to position 426 of SEQ ID NO: 1;
   a tyrosine to leucine at a position corresponding to position 426 of SEQ ID NO: 1;
   a tyrosine to arginine at a position corresponding to position 426 of SEQ ID NO: 1;
   a tyrosine to threonine at a position corresponding to position 426 of SEQ ID NO: 1;
   a tyrosine to valine at a position corresponding to position 426 of SEQ ID NO: 1;
   a serine to leucine at a position corresponding to position 430 of SEQ ID NO: 1;
   a lysine to serine at a position corresponding to position 438 of SEQ ID NO: 1;
   a glutamic acid to lysine at a position corresponding to position 440 of SEQ ID NO: 1;
   a valine to isoleucine at a position corresponding to position 444 of SEQ ID NO: 1;
   a leucine to valine at a position corresponding to position 455 of SEQ ID NO: 1;
   a lysine to valine at a position corresponding to position 457 of SEQ ID NO: 1;
   a valine to serine at a position corresponding to position 470 of SEQ ID NO: 1;
   a valine to tyrosine at a position corresponding to position 470 of SEQ ID NO: 1;
   a phenylalanine to serine at a position corresponding to position 478 of SEQ ID NO: 1;
   a phenylalanine to glycine at a position corresponding to position 483 of SEQ ID NO: 1;
   an aspartic acid to alanine at a position corresponding to position 484 of SEQ ID NO: 1;
   an isoleucine to glutamic acid at a position corresponding to position 485 of SEQ ID NO: 1;
   an aspartic acid to glycine at a position corresponding to position 487 of SEQ ID NO: 1;
   a lysine to asparagine at a position corresponding to position 490 of SEQ ID NO: 1;
   a leucine to phenylalanine at a position corresponding to position 503 of SEQ ID NO: 1;
   a valine to threonine at a position corresponding to position 508 of SEQ ID NO: 1;
   an isoleucine to threonine at a position corresponding to position 525 of SEQ ID NO: 1.
   an isoleucine to threonine at a position corresponding to position 525 of SEQ ID NO: 7;
   an aspartic acid to asparagine at a position corresponding to position 58 of SEQ ID NO: 9;
   a glutamic acid to valine at a position corresponding to position 64 of SEQ ID NO: 9;
   a glycine to cysteine at a position corresponding to position 74 of SEQ ID NO: 9;
   a glycine to asparagine at a position corresponding to position 84 of SEQ ID NO: 9;
   a leucine to histidine at a position corresponding to position 93 of SEQ ID NO: 9;
   a lysine to arginine at a position corresponding to position 97 of SEQ ID NO: 9;
   an arginine to histidine at a position corresponding to position 98 of SEQ ID NO: 9;
   an arginine to cysteine at a position corresponding to position 98 of SEQ ID NO: 9;
   an arginine to leucine at a position corresponding to position 98 of SEQ ID NO:9;
   an alanine to valine at a position corresponding to position 101 of SEQ ID NO: 9;
   a serine to asparagine at a position corresponding to position 119 of SEQ ID NO: 9;
   a phenylalanine to leucine at a position corresponding to position 121 of SEQ ID NO: 9;
   a threonine to isoleucine at a position corresponding to position 124 of SEQ ID NO: 9;
   an asparagine to tyrosine at a position corresponding to position 139 of SEQ ID NO: 9;
   an asparagine to arginine at a position corresponding to position 139 of SEQ ID NO: 9;
   an asparagine to histidine at a position corresponding to position 139 of SEQ ID NO: 9;
   a glutamic acid to aspartic acid at a position corresponding to position 150 of SEQ ID NO: 9;
   a glutamic acid to lysine at a position corresponding to position 150 of SEQ ID NO: 9;
   a serine to threonine at a position corresponding to position 151 of SEQ ID NO: 9;
   a glutamine to leucine at a position corresponding to position 157 of SEQ ID NO: 9;
   a valine to phenylalanine at a position corresponding to position 164 of SEQ ID NO: 9;
   a valine to alanine at a position corresponding to position 164 of SEQ ID NO: 9;
   an aspartic acid to glutamic acid at a position corresponding to position 170 of SEQ ID NO: 9;
   a cysteine to serine at a position corresponding to position 177 of SEQ ID NO: 9;
   a histidine to glutamine at a position corresponding to position 187 of SEQ ID NO: 9;
   a leucine to phenylalanine at a position corresponding to position 188 of SEQ ID NO: 9;
   an asparagine to lysine at a position corresponding to position 195 of SEQ ID NO: 9;
   a proline to serine at a position corresponding to position 214 of SEQ ID NO: 9;
   a proline to histidine at a position corresponding to position 214 of SEQ ID NO: 9;
   an isoleucine to serine at a position corresponding to position 215 of SEQ ID NO: 9;
   an isoleucine to histidine at a position corresponding to position 215 of SEQ ID NO: 9;
   a lysine to glutamic acid at a position corresponding to position 229 of SEQ ID NO: 9;
   a lysine to glutamine at a position corresponding to position 229 of SEQ ID NO: 9;
   a lysine to arginine at a position corresponding to position 230 of SEQ ID NO: 9;
   a cysteine to arginine at a position corresponding to position 271 of SEQ ID NO: 9;
   an aspartic acid to glycine at a position corresponding to position 274 of SEQ ID NO: 9;
   a phenylalanine to glycine at a position corresponding to position 283 of SEQ ID NO: 9;
   an alanine to glycine at a position corresponding to position 292 of SEQ ID NO: 9;
   a serine to leucine at a position corresponding to position 296 of SEQ ID NO: 9;
   a cysteine to serine at a position corresponding to position 307 of SEQ ID NO: 9;
   an asparagine to aspartic acid at a position corresponding to position 324 of SEQ ID NO: 9;
   an asparagine to lysine at a position corresponding to position 324 of SEQ ID NO: 9;
   an aspartic acid to glutamic acid at a position corresponding to position 330 of SEQ ID NO: 9;
   a serine to leucine at a position corresponding to position 396 of SEQ ID NO: 9;
   an alanine to serine at a position corresponding to position 404 of SEQ ID NO: 9;
   an arginine to lysine at a position corresponding to position 406 of SEQ ID NO: 9;
   a lysine to isoleucine at a position corresponding to position 410 of SEQ ID NO: 9;
   a leucine to valine at a position corresponding to position 421 of SEQ ID NO: 9;
   an alanine to valine at a position corresponding to position 423 of SEQ ID NO: 9;
   a cysteine to serine at a position corresponding to position 434 of SEQ ID NO: 9;
   a cysteine to tyrosine at a position corresponding to position 434 of SEQ ID NO: 9;
   an aspartic acid to glycine at a position corresponding to position 447 of SEQ ID NO: 9;
   a serine to alanine at a position corresponding to position 448 of SEQ ID NO: 9;
   a valine to glutamic acid at a position corresponding to position 449 of SEQ ID NO: 9;
   an aspartic acid to glycine at a position corresponding to position 451 of SEQ ID NO: 9;
   an aspartic acid to asparagine at a position corresponding to position 454 of SEQ ID NO: 9;
   a tyrosine to phenylalanine at a position corresponding to position 465 of SEQ ID NO: 9;
   a lysine to threonine at a position corresponding to position 470 of SEQ ID NO: 9; and
   a threonine to serine at a position corresponding to position 500 of SEQ ID NO: 9.
**3.** The non-transgenic plant cell of any of statements 1-2, wherein said mutated PPX gene comprises one or more nucleic acid sequence mutations selected from Tables 2, 3a and 3b.
**4.** The non-transgenic plant cell of any of statements 1-3, wherein said mutated PPX gene encodes a protein comprising a combination of two or more mutations selected from Tables 4a and 4b.
**5.** A non-transgenic plant comprising a mutated protoporphyrinogen IX oxidase (PPX) gene wherein said gene encodes a protein comprising a mutation at one or more amino acid positions corresponding to a position selected from the group consisting of 52, 85, 105, 111, 130, 139, 143, 144, 145, 147, 165, 167, 170, 180, 185, 192, 193, 199, 206, 212, 219, 220, 221, 226, 228, 229, 230, 237, 244, 256, 257, 270, 271, 272, 305, 311, 316, 318, 332, 343, 354, 357, 359, 360, 366, 393, 403, 424, 426, 430, 438, 440, 444, 455, 457, 470, 478, 483, 484, 485, 487, 490, 503, 508 and 525 of SEQ ID NO: 1 or wherein said gene encodes a protein comprising a mutation at one or more amino acid positions corresponding to a position selected from the group consisting of 58, 64, 74, 84, 93, 97, 98, 101, 119, 121, 124, 139, 150 151, 157, 164, 170, 177, 187, 188, 195, 214, 215, 229, 230, 271, 274, 278, 283, 292, 296, 307, 324, 330, 396, 404, 406, 410, 421, 423, 434, 447, 448, 449, 451, 454, 465, 470and 500 of SEQ ID NO: 9.
**6.** The non-transgenic plant of statement 5, wherein said mutations at one or more amino acid positions are selected from the group consisting of
   a glycine to lysine at a position corresponding to position 52 of SEQ ID NO: 1;
   an asparagine to lysine at a position corresponding to position 52 of Figure 7 SEQ ID NO: 7;
   an asparagine to aspartic acid at a position corresponding to position 85 of SEQ ID NO: 1;
   a glutamic acid to valine at a position corresponding to position 111 of SEQ ID NO: 1;
   a glycine to asparagine at a position corresponding to position 130 of SEQ ID NO: 1;
   an aspartic acid to histidine at a position corresponding to position 139 of SEQ ID NO: 1;
   a proline to arginine at a position corresponding to position 143 of SEQ ID NO: 1;
   an arginine to cysteine at a position corresponding to position 144 of SEQ ID NO: 1;
   ; an arginine to leucine at a position corresponding to position 144 of SEQ ID NO: 1;
   an arginine to histidine at a position corresponding to position 144 of SEQ ID NO: 1;
   a phenylalanine to leucine at a position corresponding to position 145 of SEQ ID NO: 1, ;
   a phenylalanine to tyrosine at a position corresponding to position 145 of SEQ ID NO: 1;
   a leucine to valine at a position corresponding to position 147 of SEQ ID NO: 1;
   a phenylalanine to asparagine at a position corresponding to position 165 of SEQ ID NO: 1;
   an alanine to threonine at a position corresponding to position 180 of SEQ ID NO: 1;
   a proline to histidine at a position corresponding to position 185 of SEQ ID NO: 1;
   a proline to arginine at a position corresponding to position 185 of SEQ ID NO: 1;
   a proline to tyrosine at a position corresponding to position 185 of SEQ ID NO: 1;
   a glutamic acid to aspartic acid at a position corresponding to position 192 of SEQ ID NO: 1;
   a glutamic acid to lysine at a position corresponding to position 192 of SEQ ID NO: 1;
   a serine to threonine at a position corresponding to position 193 of SEQ ID NO: 1;
   an arginine to leucine at a position corresponding to position 199 of SEQ ID NO: 1;
   a valine to phenylalanine at a position corresponding to position 206 of SEQ ID NO: 1;
   a tyrosine to serine at a position corresponding to position 219 of SEQ ID NO: 1;
   an alanine to threonine at a position corresponding to position 220 of SEQ ID NO: 1;
   a leucine to methionine at a position corresponding to position 226 of SEQ ID NO: 1;
   a methionine to leucine at a position corresponding to position 228 of SEQ ID NO: 1;
   a lysine to glutamine at a position corresponding to position 229 of SEQ ID NO: 1;
   an alanine to phenylalanine at a position corresponding to position 230 of SEQ ID NO: 1;
   a serine to glycine at a position corresponding to position 244 of SEQ ID NO: 1;
   a serine to threonine at a position corresponding to position 244 of SEQ ID NO: 1;
   an arginine to histidine at a position corresponding to position 256 of SEQ ID NO: 1;
   an arginine to serine at a position corresponding to position 256 of SEQ ID NO: 1;
   a lysine to glutamic acid at a position corresponding to position 270 of SEQ ID NO: 1;
   a lysine to glutamine at a position corresponding to position 270 of SEQ ID NO: 1;
   a proline to arginine at a position corresponding to position 271 of SEQ ID NO: 1;
   a glutamine to phenylalanine at a position corresponding to position 272 of SEQ ID NO: 1;
   a lysine to phenylalanine at a position corresponding to position 272 of SEQ ID NO: 7;
   a serine to leucine at a position corresponding to position 305 of SEQ ID NO: 1;
   a glutamic acid arginine at a position corresponding to position 311 of SEQ ID NO: 1;
   a threonine to glycine at a position corresponding to position 316 of SEQ ID NO: 1;
   a threonine to glycine at a position corresponding to position 318 of SEQ ID NO: 1;
   a serine to cysteine at a position corresponding to position 332 of SEQ ID NO: 1;
   a leucine to isoleucine at a position corresponding to position 357 of SEQ ID NO: 1;
   a lysine to arginine at a position corresponding to position 359 of SEQ ID NO: 1;
   a lysine to threonine at a position corresponding to position 359 of SEQ ID NO: 1;
   a serine to arginine at a position corresponding to position 359 of SEQ ID NO: 7;
   a serine to threonine at a position corresponding to position 359 of SEQ ID NO: 7;
   a leucine to aspartic acid at a position corresponding to position 360 of SEQ ID NO: 1;
   a leucine to lysine at a position corresponding to position 360 of SEQ ID NO: 1;
   an alanine to glutamic acid at a position corresponding to position 366 of SEQ ID NO: 1;
   a leucine to methionine at a position corresponding to position 393 of SEQ ID NO: 1;
   a leucine to serine at a position corresponding to position 393 of SEQ ID NO: 1;
   a leucine to valine at a position corresponding to position 393 of SEQ ID NO: 1;
   a leucine to arginine at a position corresponding to position 403 of SEQ ID NO: 1;
   a leucine to serine at a position corresponding to position 424 of SEQ ID NO: 1;
   a tyrosine to cysteine at a position corresponding to position 426 of SEQ ID NO: 1;
   a tyrosine to phenylalanine at a position corresponding to position 426 of SEQ ID NO: 1;
   a tyrosine to histidine at a position corresponding to position 426 of SEQ ID NO: 1;
   a tyrosine to isoleucine at a position corresponding to position 426 of SEQ ID NO: 1;
   a tyrosine to leucine at a position corresponding to position 426 of SEQ ID NO: 1;
   a tyrosine to arginine at a position corresponding to position 426 of SEQ ID NO: 1;
   a tyrosine to threonine at a position corresponding to position 426 of SEQ ID NO: 1;
   a tyrosine to valine at a position corresponding to position 426 of SEQ ID NO: 1;
   a serine to leucine at a position corresponding to position 430 of SEQ ID NO: 1;
   a lysine to serine at a position corresponding to position 438 of SEQ ID NO: 1;
   a glutamic acid to lysine at a position corresponding to position 440 of SEQ ID NO: 1;
   a valine to isoleucine at a position corresponding to position 444 of SEQ ID NO: 1;
   a leucine to valine at a position corresponding to position 455 of SEQ ID NO: 1;
   a lysine to valine at a position corresponding to position 457 of SEQ ID NO: 1;
   a valine to serine at a position corresponding to position 470 of SEQ ID NO: 1;
   a valine to tyrosine at a position corresponding to position 470 of SEQ ID NO: 1;
   a phenylalanine to serine at a position corresponding to position 478 of SEQ ID NO: 1;
   a phenylalanine to glycine at a position corresponding to position 483 of SEQ ID NO: 1;
   an aspartic acid to alanine at a position corresponding to position 484 of SEQ ID NO: 1;
   an isoleucine to glutamic acid at a position corresponding to position 485 of SEQ ID NO: 1;
   an aspartic acid to glycine at a position corresponding to position 487 of SEQ ID NO: 1;
   a lysine to asparagine at a position corresponding to position 490 of SEQ ID NO: 1;
   a leucine to phenylalanine at a position corresponding to position 503 of SEQ ID NO: 1;
   a valine to threonine at a position corresponding to position 508 of SEQ ID NO: 1;
   an isoleucine to threonine at a position corresponding to position 525 of SEQ ID NO: 1.
   and an isoleucine to threonine at a position corresponding to position 525 of SEQ ID NO: 7;
   an aspartic acid to asparagine at a position corresponding to position 58 of SEQ ID NO: 9;
   a glutamic acid to valine at a position corresponding to position 64 of SEQ ID NO: 9;
   a glycine to cysteine at a position corresponding to position 74 of SEQ ID NO: 9;
   a glycine to asparagine at a position corresponding to position 84 of SEQ ID NO: 9;
   a leucine to histidine at a position corresponding to position 93 of SEQ ID NO: 9;
   a lysine to arginine at a position corresponding to position 97 of SEQ ID NO: 9;
   an arginine to histidine at a position corresponding to position 98 of SEQ ID NO: 9;
   an arginine to cysteine at a position corresponding to position 98 of SEQ ID NO: 9;
   an arginine to leucine at a position corresponding to position 98 of SEQ ID NO:9;
   an alanine to valine at a position corresponding to position 101 of SEQ ID NO: 9;
   a serine to asparagine at a position corresponding to position 119 of SEQ ID NO: 9;
   a phenylalanine to leucine at a position corresponding to position 121 of SEQ ID NO: 9;
   a threonine to isoleucine at a position corresponding to position 124 of SEQ ID NO: 9;
   an asparagine to tyrosine at a position corresponding to position 139 of SEQ ID NO: 9;
   an asparagine to arginine at a position corresponding to position 139 of SEQ ID NO: 9;
   an asparagine to histidine at a position corresponding to position 139 of SEQ ID NO: 9;
   a glutamic acid to aspartic acid at a position corresponding to position 150 of SEQ ID NO: 9;
   a glutamic acid to lysine at a position corresponding to position 150 of SEQ ID NO: 9;
   a serine to threonine at a position corresponding to position 151 of SEQ ID NO: 9;
   a glutamine to leucine at a position corresponding to position 157 of SEQ ID NO: 9;
   a valine to phenylalanine at a position corresponding to position 164 of SEQ ID NO: 9;
   a valine to alanine at a position corresponding to position 164 of SEQ ID NO: 9;
   an aspartic acid to glutamic acid at a position corresponding to position 170 of SEQ ID NO: 9;
   a cysteine to serine at a position corresponding to position 177 of SEQ ID NO: 9;
   a histidine to glutamine at a position corresponding to position 187 of SEQ ID NO: 9;
   a leucine to phenylalanine at a position corresponding to position 188 of SEQ ID NO: 9;
   an asparagine to lysine at a position corresponding to position 195 of SEQ ID NO: 9;
   a proline to serine at a position corresponding to position 214 of SEQ ID NO: 9;
   a proline to histidine at a position corresponding to position 214 of SEQ ID NO: 9;
   an isoleucine to serine at a position corresponding to position 215 of SEQ ID NO: 9;
   an isoleucine to histidine at a position corresponding to position 215 of SEQ ID NO: 9;
   a lysine to glutamic acid at a position corresponding to position 229 of SEQ ID NO: 9;
   a lysine to glutamine at a position corresponding to position 229 of SEQ ID NO: 9;
   a lysine to arginine at a position corresponding to position 230 of SEQ ID NO: 9;
   a cysteine to arginine at a position corresponding to position 271 of SEQ ID NO: 9;
   an aspartic acid to glycine at a position corresponding to position 274 of SEQ ID NO: 9;
   a phenylalanine to glycine at a position corresponding to position 283 of SEQ ID NO: 9;
   an alanine to glycine at a position corresponding to position 292 of SEQ ID NO: 9;
   a serine to leucine at a position corresponding to position 296 of SEQ ID NO: 9;
   a cysteine to serine at a position corresponding to position 307 of SEQ ID NO: 9;
   an asparagine to aspartic acid at a position corresponding to position 324 of SEQ ID NO: 9;
   an asparagine to lysine at a position corresponding to position 324 of SEQ ID NO: 9;
   an aspartic acid to glutamic acid at a position corresponding to position 330 of SEQ ID NO: 9;
   a serine to leucine at a position corresponding to position 396 of SEQ ID NO: 9;
   an alanine to serine at a position corresponding to position 404 of SEQ ID NO: 9;
   an arginine to lysine at a position corresponding to position 406 of SEQ ID NO: 9;
   a lysine to isoleucine at a position corresponding to position 410 of SEQ ID NO: 9;
   a leucine to valine at a position corresponding to position 421 of SEQ ID NO: 9;
   an alanine to valine at a position corresponding to position 423 of SEQ ID NO: 9;
   a cysteine to serine at a position corresponding to position 434 of SEQ ID NO: 9;
   a cysteine to tyrosine at a position corresponding to position 434 of SEQ ID NO: 9;
   an aspartic acid to glycine at a position corresponding to position 447 of SEQ ID NO: 9;
   a serine to alanine at a position corresponding to position 448 of SEQ ID NO: 9;
   a valine to glutamic acid at a position corresponding to position 449 of SEQ ID NO: 9;
   an aspartic acid to glycine at a position corresponding to position 451 of SEQ ID NO: 9;
   an aspartic acid to asparagine at a position corresponding to position 454 of SEQ ID NO: 9;
   a tyrosine to phenylalanine at a position corresponding to position 465 of SEQ ID NO: 9;
   a lysine to threonine at a position corresponding to position 470 of SEQ ID NO: 9; and
   a threonine to serine at a position corresponding to position 500 of SEQ ID NO: 9.
**7.** The non-transgenic plant of any of statements 5-6, wherein said mutated PPX gene comprises one or more nucleic acid sequence mutations selected from Tables 2 and 3.
**8.** The non-transgenic plant of any of statements 5-7, wherein said mutated PPX gene encodes a protein comprising a combination of two or more mutations selected from Tables 4a and 4b.
**9.** The non-transgenic plant of any of statements 5-8, wherein said plant is selected from the group consisting of potato, sunflower, sugar beet, maize, cotton, soybean, wheat, rye, oats, rice, canola, fruits, vegetables, tobacco, barley, sorghum, tomato, mango, peach, apple, pear, strawberry, banana, melon, carrot, lettuce, onion, soya spp, sugar cane, pea, field beans, poplar, grape, citrus, alfalfa, rye, oats, turf and forage grasses, flax, oilseed rape, cucumber, morning glory, balsam, pepper, eggplant, marigold, lotus, cabbage, daisy, carnation, petunia, tulip, iris, lily, and nut-producing plants.
**10.** The non-transgenic plant of any of statements 5-9, wherein said plant is a species selected from the group consisting of *Solanum tuberosum, Oryza sativa,* and *Zea mays.*
**11.** The non-transgenic plant of any of statements 5-10, wherein said plant is a Russet Burbank potato cultivar.
**12.** The non-transgenic plant of any of statements 5-11, wherein said plant is asexually reproduced.
**13.** The non-transgenic plant of any of statements 5-12, wherein said plant is produced from a tuber.
**14.** The non-transgenic plant of any of statements 5-13, wherein said plant is resistant to one or more herbicides.
**15.** The non-transgenic plant of any of statements 5-14, wherein said plant is resistant to one or more PPX-inhibiting herbidicides.
**16.** The non-transgenic plant of any of statements 5-15, wherein said plant is resistant to one or more herbicides selected from Table 5.
**17.** The non-transgenic plant of any of statements 5-16, wherein said plant is resistant to one or more herbicides selected from the group consisting of flumioxazin, sulfentrazone, and saflufenacil.
**18.** A method for producing a non-transgenic plant cell with a mutated PPX gene, comprising introducing into a plant cell a gene repair oligonucleobase (GRON) with a targeted mutation in a protoporphyrinogen IX oxidase (PPX) gene to produce a plant cell with a PPX gene that expresses a PPX protein comprising a mutation at one or more amino acid positions corresponding to a position selected from the group consisting of 52, 85, 105, 111, 130, 139, 143, 144, 145, 147, 165, 167, 170, 180, 185, 192, 193, 199, 206, 212, 219, 220, 221, 226, 228, 229, 230, 237, 244, 256, 257, 270, 271, 272, 305, 311, 316, 318, 332, 343, 354, 357, 359, 360, 366, 393, 403, 424, 426, 430, 438, 440, 444, 455, 457, 470, 478, 483, 484, 485, 487, 490, 503, 508 and 525 of SEQ ID NO: 1 or wherein said gene encodes a protein comprising a mutation at one or more amino acid positions corresponding to a position selected from the group consisting of 58, 64, 74, 84, 93, 97, 98, 101, 119, 121, 124, 139, 150 151, 157, 164, 170, 177, 187, 188, 195, 214, 215, 229, 230, 271, 274, 278, 283, 292, 296, 307, 324, 330, 396, 404, 406, 410, 421, 423, 434, 447, 448, 449, 451, 454, 465, 470 and 500 of SEQ ID NO: 9.
**19.** The method of statement 18, wherein said mutation comprises one or more mutations at one or more amino acid position selected from the group consisting of a glycine to lysine at a position corresponding to position 52 of SEQ ID NO: 1;
   an asparagine to lysine at a position corresponding to position 52 of Figure 7 SEQ ID NO: 7;
   an asparagine to aspartic acid at a position corresponding to position 85 of SEQ ID NO: 1;
   a glutamic acid to valine at a position corresponding to position 111 of SEQ ID NO: 1;
   a glycine to asparagine at a position corresponding to position 130 of SEQ ID NO: 1;
   an aspartic acid to histidine at a position corresponding to position 139 of SEQ ID NO: 1;
   a proline to arginine at a position corresponding to position 143 of SEQ ID NO: 1;
   an arginine to cysteine at a position corresponding to position 144 of SEQ ID NO: 1;
   ; an arginine to leucine at a position corresponding to position 144 of SEQ ID NO: 1;
   an arginine to histidine at a position corresponding to position 144 of SEQ ID NO: 1;
   a phenylalanine to leucine at a position corresponding to position 145 of SEQ ID NO: 1, ;
   a phenylalanine to tyrosine at a position corresponding to position 145 of SEQ ID NO: 1;
   a leucine to valine at a position corresponding to position 147 of SEQ ID NO: 1;
   a phenylalanine to asparagine at a position corresponding to position 165 of SEQ ID NO: 1;
   an alanine to threonine at a position corresponding to position 180 of SEQ ID NO: 1;
   a proline to histidine at a position corresponding to position 185 of SEQ ID NO: 1;
   a proline to arginine at a position corresponding to position 185 of SEQ ID NO: 1;
   a proline to tyrosine at a position corresponding to position 185 of SEQ ID NO: 1;
   a glutamic acid to aspartic acid at a position corresponding to position 192 of SEQ ID NO: 1;
   a glutamic acid to lysine at a position corresponding to position 192 of SEQ ID NO: 1;
   a serine to threonine at a position corresponding to position 193 of SEQ ID NO: 1;
   an arginine to leucine at a position corresponding to position 199 of SEQ ID NO: 1;
   a valine to phenylalanine at a position corresponding to position 206 of SEQ ID NO: 1;
   a tyrosine to serine at a position corresponding to position 219 of SEQ ID NO: 1;
   an alanine to threonine at a position corresponding to position 220 of SEQ ID NO: 1;
   a leucine to methionine at a position corresponding to position 226 of SEQ ID NO: 1;
   a methionine to leucine at a position corresponding to position 228 of SEQ ID NO: 1;
   a lysine to glutamine at a position corresponding to position 229 of SEQ ID NO: 1;
   an alanine to phenylalanine at a position corresponding to position 230 of SEQ ID NO: 1;
   a serine to glycine at a position corresponding to position 244 of SEQ ID NO: 1;
   a serine to threonine at a position corresponding to position 244 of SEQ ID NO: 1;
   an arginine to histidine at a position corresponding to position 256 of SEQ ID NO: 1;
   an arginine to serine at a position corresponding to position 256 of SEQ ID NO: 1;
   a lysine to glutamic acid at a position corresponding to position 270 of SEQ ID NO: 1;
   a lysine to glutamine at a position corresponding to position 270 of SEQ ID NO: 1;
   a proline to arginine at a position corresponding to position 271 of SEQ ID NO: 1;
   a glutamine to phenylalanine at a position corresponding to position 272 of SEQ ID NO: 1;
   a lysine to phenylalanine at a position corresponding to position 272 of SEQ ID NO: 7;
   a serine to leucine at a position corresponding to position 305 of SEQ ID NO: 1;
   a glutamic acid arginine at a position corresponding to position 311 of SEQ ID NO: 1;
   a threonine to glycine at a position corresponding to position 316 of SEQ ID NO: 1;
   a threonine to glycine at a position corresponding to position 318 of SEQ ID NO: 1;
   a serine to cysteine at a position corresponding to position 332 of SEQ ID NO: 1;
   a leucine to isoleucine at a position corresponding to position 357 of SEQ ID NO: 1;
   a lysine to arginine at a position corresponding to position 359 of SEQ ID NO: 1;
   a lysine to threonine at a position corresponding to position 359 of SEQ ID NO: 1;
   a serine to arginine at a position corresponding to position 359 of SEQ ID NO: 7;
   a serine to threonine at a position corresponding to position 359 of SEQ ID NO: 7;
   a leucine to aspartic acid at a position corresponding to position 360 of SEQ ID NO: 1;
   a leucine to lysine at a position corresponding to position 360 of SEQ ID NO: 1;
   an alanine to glutamic acid at a position corresponding to position 366 of SEQ ID NO: 1;
   a leucine to methionine at a position corresponding to position 393 of SEQ ID NO: 1;
   a leucine to serine at a position corresponding to position 393 of SEQ ID NO: 1;
   a leucine to valine at a position corresponding to position 393 of SEQ ID NO: 1;
   a leucine to arginine at a position corresponding to position 403 of SEQ ID NO: 1;
   a leucine to serine at a position corresponding to position 424 of SEQ ID NO: 1;
   a tyrosine to cysteine at a position corresponding to position 426 of SEQ ID NO: 1;
   a tyrosine to phenylalanine at a position corresponding to position 426 of SEQ ID NO: 1;
   a tyrosine to histidine at a position corresponding to position 426 of SEQ ID NO: 1;
   a tyrosine to isoleucine at a position corresponding to position 426 of SEQ ID NO: 1;
   a tyrosine to leucine at a position corresponding to position 426 of SEQ ID NO: 1;
   a tyrosine to arginine at a position corresponding to position 426 of SEQ ID NO: 1;
   a tyrosine to threonine at a position corresponding to position 426 of SEQ ID NO: 1;
   a tyrosine to valine at a position corresponding to position 426 of SEQ ID NO: 1;
   a serine to leucine at a position corresponding to position 430 of SEQ ID NO: 1;
   a lysine to serine at a position corresponding to position 438 of SEQ ID NO: 1;
   a glutamic acid to lysine at a position corresponding to position 440 of SEQ ID NO: 1;
   a valine to isoleucine at a position corresponding to position 444 of SEQ ID NO: 1;
   a leucine to valine at a position corresponding to position 455 of SEQ ID NO: 1;
   a lysine to valine at a position corresponding to position 457 of SEQ ID NO: 1;
   a valine to serine at a position corresponding to position 470 of SEQ ID NO: 1;
   a valine to tyrosine at a position corresponding to position 470 of SEQ ID NO: 1;
   a phenylalanine to serine at a position corresponding to position 478 of SEQ ID NO: 1;
   a phenylalanine to glycine at a position corresponding to position 483 of SEQ ID NO: 1;
   an aspartic acid to alanine at a position corresponding to position 484 of SEQ ID NO: 1;
   an isoleucine to glutamic acid at a position corresponding to position 485 of SEQ ID NO: 1;
   an aspartic acid to glycine at a position corresponding to position 487 of SEQ ID NO: 1;
   a lysine to asparagine at a position corresponding to position 490 of SEQ ID NO: 1;
   a leucine to phenylalanine at a position corresponding to position 503 of SEQ ID NO: 1;
   a valine to threonine at a position corresponding to position 508 of SEQ ID NO: 1;
   an isoleucine to threonine at a position corresponding to position 525 of SEQ ID NO: 1.
   and an isoleucine to threonine at a position corresponding to position 525 of SEQ ID NO: 7;
   an aspartic acid to asparagine at a position corresponding to position 58 of SEQ ID NO: 9;
   a glutamic acid to valine at a position corresponding to position 64 of SEQ ID NO: 9;
   a glycine to cysteine at a position corresponding to position 74 of SEQ ID NO: 9;
   a glycine to asparagine at a position corresponding to position 84 of SEQ ID NO: 9;
   a leucine to histidine at a position corresponding to position 93 of SEQ ID NO: 9;
   a lysine to arginine at a position corresponding to position 97 of SEQ ID NO: 9;
   an arginine to histidine at a position corresponding to position 98 of SEQ ID NO: 9;
   an arginine to cysteine at a position corresponding to position 98 of SEQ ID NO: 9;
   an arginine to leucine at a position corresponding to position 98 of SEQ ID NO:9;
   an alanine to valine at a position corresponding to position 101 of SEQ ID NO: 9;
   a serine to asparagine at a position corresponding to position 119 of SEQ ID NO: 9;
   a phenylalanine to leucine at a position corresponding to position 121 of SEQ ID NO: 9;
   a threonine to isoleucine at a position corresponding to position 124 of SEQ ID NO: 9;
   an asparagine to tyrosine at a position corresponding to position 139 of SEQ ID NO: 9;
   an asparagine to arginine at a position corresponding to position 139 of SEQ ID NO: 9;
   an asparagine to histidine at a position corresponding to position 139 of SEQ ID NO: 9;
   a glutamic acid to aspartic acid at a position corresponding to position 150 of SEQ ID NO: 9;
   a glutamic acid to lysine at a position corresponding to position 150 of SEQ ID NO: 9;
   a serine to threonine at a position corresponding to position 151 of SEQ ID NO: 9;
   a glutamine to leucine at a position corresponding to position 157 of SEQ ID NO: 9;
   a valine to phenylalanine at a position corresponding to position 164 of SEQ ID NO: 9;
   a valine to alanine at a position corresponding to position 164 of SEQ ID NO: 9;
   an aspartic acid to glutamic acid at a position corresponding to position 170 of SEQ ID NO: 9;
   a cysteine to serine at a position corresponding to position 177 of SEQ ID NO: 9;
   a histidine to glutamine at a position corresponding to position 187 of SEQ ID NO: 9;
   a leucine to phenylalanine at a position corresponding to position 188 of SEQ ID NO: 9;
   an asparagine to lysine at a position corresponding to position 195 of SEQ ID NO: 9;
   a proline to serine at a position corresponding to position 214 of SEQ ID NO: 9;
   a proline to histidine at a position corresponding to position 214 of SEQ ID NO: 9;
   an isoleucine to serine at a position corresponding to position 215 of SEQ ID NO: 9;
   an isoleucine to histidine at a position corresponding to position 215 of SEQ ID NO: 9;
   a lysine to glutamic acid at a position corresponding to position 229 of SEQ ID NO: 9;
   a lysine to glutamine at a position corresponding to position 229 of SEQ ID NO: 9;
   a lysine to arginine at a position corresponding to position 230 of SEQ ID NO: 9;
   a cysteine to arginine at a position corresponding to position 271 of SEQ ID NO: 9;
   an aspartic acid to glycine at a position corresponding to position 274 of SEQ ID NO: 9;
   a phenylalanine to glycine at a position corresponding to position 283 of SEQ ID NO: 9;
   an alanine to glycine at a position corresponding to position 292 of SEQ ID NO: 9;
   a serine to leucine at a position corresponding to position 296 of SEQ ID NO: 9;
   a cysteine to serine at a position corresponding to position 307 of SEQ ID NO: 9;
   an asparagine to aspartic acid at a position corresponding to position 324 of SEQ ID NO: 9;
   an asparagine to lysine at a position corresponding to position 324 of SEQ ID NO: 9;
   an aspartic acid to glutamic acid at a position corresponding to position 330 of SEQ ID NO: 9;
   a serine to leucine at a position corresponding to position 396 of SEQ ID NO: 9;
   an alanine to serine at a position corresponding to position 404 of SEQ ID NO: 9;
   an arginine to lysine at a position corresponding to position 406 of SEQ ID NO: 9;
   a lysine to isoleucine at a position corresponding to position 410 of SEQ ID NO: 9;
   a leucine to valine at a position corresponding to position 421 of SEQ ID NO: 9;
   an alanine to valine at a position corresponding to position 423 of SEQ ID NO: 9;
   a cysteine to serine at a position corresponding to position 434 of SEQ ID NO: 9;
   a cysteine to tyrosine at a position corresponding to position 434 of SEQ ID NO: 9;
   an aspartic acid to glycine at a position corresponding to position 447 of SEQ ID NO: 9;
   a serine to alanine at a position corresponding to position 448 of SEQ ID NO: 9;
   a valine to glutamic acid at a position corresponding to position 449 of SEQ ID NO: 9;
   an aspartic acid to glycine at a position corresponding to position 451 of SEQ ID NO: 9;
   an aspartic acid to asparagine at a position corresponding to position 454 of SEQ ID NO: 9;
   a tyrosine to phenylalanine at a position corresponding to position 465 of SEQ ID NO: 9;
   a lysine to threonine at a position corresponding to position 470 of SEQ ID NO: 9; and
   a threonine to serine at a position corresponding to position 500 of SEQ ID NO: 9.
**20.** The method of any of statements 18-19, wherein said mutated PPX gene comprises one or more nucleic acid sequence mutations selected from Table 2.
**21.** The method of any of statements 18-20, wherein said mutated PPX gene encodes a protein comprising a combination of two or more mutations selected from Tables 3a and 3b.
**22.** The method of any of statements 18-21, wherein said plant cell is of a plant selected from the group consisting of potato, sunflower, sugar beet, maize, cotton, soybean, wheat, rye, oats, rice, canola, fruits, vegetables, tobacco, barley, sorghum, tomato, mango, peach, apple, pear, strawberry, banana, melon, carrot, lettuce, onion, soya spp, sugar cane, pea, field beans, poplar, grape, citrus, alfalfa, rye, oats, turf and forage grasses, flax, oilseed rape, cucumber, morning glory, balsam, pepper, eggplant, marigold, lotus, cabbage, daisy, carnation, petunia, tulip, iris, lily, and nut-producing plants.
**23.** The method of any of statements 18-22, wherein said plant cell is a species selected from the group consisting of *Solanum tuberosum, Oryza sativa, Sorghum bicolor, Ricinus communis, Brassica napus, Glycine max,* and *Zea mays.*
**24.** The method of any of statements 18-23, wherein said plant cell is a Russet Burbank potato cultivar.
**25.** The method of any of statements 18-24, wherein said plant is resistant to one or more herbicides.
**26.** The method of any of statements 18-25, wherein said plant is resistant to one or more PPX-inhibiting herbidicides.
**27.** The method of any of statements 18-26, wherein said plant is resistant to one or more herbicides selected from Table 5.
**28.** The method of any of statements 18-27, wherein said plant is resistant to one or more herbicides selected from the group consisting of flumioxazin, sulfentrazone, and saflufenacil.
**29.** A method for producing a herbicide-resistant plant, comprising
   (a) introducing into a plant cell a gene repair oligonucleobase (GRON) with a targeted mutation in a protoporphyrinogen IX oxidase (PPX) gene to produce a plant cell with a PPX gene that expresses a PPX protein comprising a mutation at one or more amino acid positions corresponding to a position selected from the group consisting of 52, 85, 105, 111, 130, 139, 143, 144, 145, 147, 165, 167, 170, 180, 185, 192, 193, 199, 206, 212, 219, 220, 221, 226, 228, 229, 230, 237, 244, 256, 257, 270, 271, 272, 305, 311, 316, 318, 332, 343, 354, 357, 359, 360, 366, 393, 403, 424, 426, 430, 438, 440, 444, 455, 457, 470, 478, 483, 484, 485, 487, 490, 503, 508 and 525 of SEQ ID NO: 1 or wherein said gene encodes a protein comprising a mutation at one or more amino acid positions corresponding to a position selected from the group consisting of 58, 64, 74, 84, 93, 97, 98, 101, 119, 121, 124, 139, 150 151, 157, 164, 170, 177, 187, 188, 195, 214, 215, 229, 230, 271, 274, 278, 283, 292, 296, 307, 324, 330, 396, 404, 406, 410, 421, 423, 434, 447, 448, 449, 451, 454, 465, 470 and 500 of SEQ ID NO: 9.
   (b) identifying a plant cell having substantially normal growth and catalytic activity as compared to a corresponding wild-type plant cell in the presence of a herbicide; and
   (c) regenerating a non-transgenic herbicide-resistant plant having a mutated PPX gene from said plant cell.
**30.** The method of statement 29, wherein the herbicide is a PPX-inhibiting herbicide.
**31.** The method of any of statements 29-30, wherein said mutation comprises one or more mutations selected from the group consisting of
   a glycine to lysine at a position corresponding to position 52 of SEQ ID NO: 1;
   an asparagine to lysine at a position corresponding to position 52 of Figure 7 SEQ ID NO: 7;
   an asparagine to aspartic acid at a position corresponding to position 85 of SEQ ID NO: 1;
   a glutamic acid to valine at a position corresponding to position 111 of SEQ ID NO: 1;
   a glycine to asparagine at a position corresponding to position 130 of SEQ ID NO: 1;
   an aspartic acid to histidine at a position corresponding to position 139 of SEQ ID NO: 1;
   a proline to arginine at a position corresponding to position 143 of SEQ ID NO: 1;
   an arginine to cysteine at a position corresponding to position 144 of SEQ ID NO: 1;
   ; an arginine to leucine at a position corresponding to position 144 of SEQ ID NO: 1;
   an arginine to histidine at a position corresponding to position 144 of SEQ ID NO: 1;
   a phenylalanine to leucine at a position corresponding to position 145 of SEQ ID NO: 1, ;
   a phenylalanine to tyrosine at a position corresponding to position 145 of SEQ ID NO: 1;
   a leucine to valine at a position corresponding to position 147 of SEQ ID NO: 1;
   a phenylalanine to asparagine at a position corresponding to position 165 of SEQ ID NO: 1;
   an alanine to threonine at a position corresponding to position 180 of SEQ ID NO: 1;
   a proline to histidine at a position corresponding to position 185 of SEQ ID NO: 1;
   a proline to arginine at a position corresponding to position 185 of SEQ ID NO: 1;
   a proline to tyrosine at a position corresponding to position 185 of SEQ ID NO: 1;
   a glutamic acid to aspartic acid at a position corresponding to position 192 of SEQ ID NO: 1;
   a glutamic acid to lysine at a position corresponding to position 192 of SEQ ID NO: 1;
   a serine to threonine at a position corresponding to position 193 of SEQ ID NO: 1;
   an arginine to leucine at a position corresponding to position 199 of SEQ ID NO: 1;
   a valine to phenylalanine at a position corresponding to position 206 of SEQ ID NO: 1;
   a tyrosine to serine at a position corresponding to position 219 of SEQ ID NO: 1;
   an alanine to threonine at a position corresponding to position 220 of SEQ ID NO: 1;
   a leucine to methionine at a position corresponding to position 226 of SEQ ID NO: 1;
   a methionine to leucine at a position corresponding to position 228 of SEQ ID NO: 1;
   a lysine to glutamine at a position corresponding to position 229 of SEQ ID NO: 1;
   an alanine to phenylalanine at a position corresponding to position 230 of SEQ ID NO: 1;
   a serine to glycine at a position corresponding to position 244 of SEQ ID NO: 1;
   a serine to threonine at a position corresponding to position 244 of SEQ ID NO: 1;
   an arginine to histidine at a position corresponding to position 256 of SEQ ID NO: 1;
   an arginine to serine at a position corresponding to position 256 of SEQ ID NO: 1;
   a lysine to glutamic acid at a position corresponding to position 270 of SEQ ID NO: 1;
   a lysine to glutamine at a position corresponding to position 270 of SEQ ID NO: 1;
   a proline to arginine at a position corresponding to position 271 of SEQ ID NO: 1;
   a glutamine to phenylalanine at a position corresponding to position 272 of SEQ ID NO: 1;
   a lysine to phenylalanine at a position corresponding to position 272 of SEQ ID NO: 7;
   a serine to leucine at a position corresponding to position 305 of SEQ ID NO: 1;
   a glutamic acid arginine at a position corresponding to position 311 of SEQ ID NO: 1;
   a threonine to glycine at a position corresponding to position 316 of SEQ ID NO: 1;
   a threonine to glycine at a position corresponding to position 318 of SEQ ID NO: 1;
   a serine to cysteine at a position corresponding to position 332 of SEQ ID NO: 1;
   a leucine to isoleucine at a position corresponding to position 357 of SEQ ID NO: 1;
   a lysine to arginine at a position corresponding to position 359 of SEQ ID NO: 1;
   a lysine to threonine at a position corresponding to position 359 of SEQ ID NO: 1;
   a serine to arginine at a position corresponding to position 359 of SEQ ID NO: 7;
   a serine to threonine at a position corresponding to position 359 of SEQ ID NO: 7;
   a leucine to aspartic acid at a position corresponding to position 360 of SEQ ID NO: 1;
   a leucine to lysine at a position corresponding to position 360 of SEQ ID NO: 1;
   an alanine to glutamic acid at a position corresponding to position 366 of SEQ ID NO: 1;
   a leucine to methionine at a position corresponding to position 393 of SEQ ID NO: 1;
   a leucine to serine at a position corresponding to position 393 of SEQ ID NO: 1;
   a leucine to valine at a position corresponding to position 393 of SEQ ID NO: 1;
   a leucine to arginine at a position corresponding to position 403 of SEQ ID NO: 1;
   a leucine to serine at a position corresponding to position 424 of SEQ ID NO: 1;
   a tyrosine to cysteine at a position corresponding to position 426 of SEQ ID NO: 1;
   a tyrosine to phenylalanine at a position corresponding to position 426 of SEQ ID NO: 1;
   a tyrosine to histidine at a position corresponding to position 426 of SEQ ID NO: 1;
   a tyrosine to isoleucine at a position corresponding to position 426 of SEQ ID NO: 1;
   a tyrosine to leucine at a position corresponding to position 426 of SEQ ID NO: 1;
   a tyrosine to arginine at a position corresponding to position 426 of SEQ ID NO: 1;
   a tyrosine to threonine at a position corresponding to position 426 of SEQ ID NO: 1;
   a tyrosine to valine at a position corresponding to position 426 of SEQ ID NO: 1;
   a serine to leucine at a position corresponding to position 430 of SEQ ID NO: 1;
   a lysine to serine at a position corresponding to position 438 of SEQ ID NO: 1;
   a glutamic acid to lysine at a position corresponding to position 440 of SEQ ID NO: 1;
   a valine to isoleucine at a position corresponding to position 444 of SEQ ID NO: 1;
   a leucine to valine at a position corresponding to position 455 of SEQ ID NO: 1;
   a lysine to valine at a position corresponding to position 457 of SEQ ID NO: 1;
   a valine to serine at a position corresponding to position 470 of SEQ ID NO: 1;
   a valine to tyrosine at a position corresponding to position 470 of SEQ ID NO: 1;
   a phenylalanine to serine at a position corresponding to position 478 of SEQ ID NO: 1;
   a phenylalanine to glycine at a position corresponding to position 483 of SEQ ID NO: 1;
   an aspartic acid to alanine at a position corresponding to position 484 of SEQ ID NO: 1;
   an isoleucine to glutamic acid at a position corresponding to position 485 of SEQ ID NO: 1;
   an aspartic acid to glycine at a position corresponding to position 487 of SEQ ID NO: 1;
   a lysine to asparagine at a position corresponding to position 490 of SEQ ID NO: 1;
   a leucine to phenylalanine at a position corresponding to position 503 of SEQ ID NO: 1;
   a valine to threonine at a position corresponding to position 508 of SEQ ID NO: 1;
   an isoleucine to threonine at a position corresponding to position 525 of SEQ ID NO: 1.
   and an isoleucine to threonine at a position corresponding to position 525 of SEQ ID NO: 7;
   an aspartic acid to asparagine at a position corresponding to position 58 of SEQ ID NO: 9;
   a glutamic acid to valine at a position corresponding to position 64 of SEQ ID NO: 9;
   a glycine to cysteine at a position corresponding to position 74 of SEQ ID NO: 9;
   a glycine to asparagine at a position corresponding to position 84 of SEQ ID NO: 9;
   a leucine to histidine at a position corresponding to position 93 of SEQ ID NO: 9;
   a lysine to arginine at a position corresponding to position 97 of SEQ ID NO: 9;
   an arginine to histidine at a position corresponding to position 98 of SEQ ID NO: 9;
   an arginine to cysteine at a position corresponding to position 98 of SEQ ID NO: 9;
   an arginine to leucine at a position corresponding to position 98 of SEQ ID NO:9;
   an alanine to valine at a position corresponding to position 101 of SEQ ID NO: 9;
   a serine to asparagine at a position corresponding to position 119 of SEQ ID NO: 9;
   a phenylalanine to leucine at a position corresponding to position 121 of SEQ ID NO: 9;
   a threonine to isoleucine at a position corresponding to position 124 of SEQ ID NO: 9;
   an asparagine to tyrosine at a position corresponding to position 139 of SEQ ID NO: 9;
   an asparagine to arginine at a position corresponding to position 139 of SEQ ID NO: 9;
   an asparagine to histidine at a position corresponding to position 139 of SEQ ID NO: 9;
   a glutamic acid to aspartic acid at a position corresponding to position 150 of SEQ ID NO: 9;
   a glutamic acid to lysine at a position corresponding to position 150 of SEQ ID NO: 9;
   a serine to threonine at a position corresponding to position 151 of SEQ ID NO: 9;
   a glutamine to leucine at a position corresponding to position 157 of SEQ ID NO: 9;
   a valine to phenylalanine at a position corresponding to position 164 of SEQ ID NO: 9;
   a valine to alanine at a position corresponding to position 164 of SEQ ID NO: 9;
   an aspartic acid to glutamic acid at a position corresponding to position 170 of SEQ ID NO: 9;
   a cysteine to serine at a position corresponding to position 177 of SEQ ID NO: 9;
   a histidine to glutamine at a position corresponding to position 187 of SEQ ID NO: 9;
   a leucine to phenylalanine at a position corresponding to position 188 of SEQ ID NO: 9;
   an asparagine to lysine at a position corresponding to position 195 of SEQ ID NO: 9;
   a proline to serine at a position corresponding to position 214 of SEQ ID NO: 9;
   a proline to histidine at a position corresponding to position 214 of SEQ ID NO: 9;
   an isoleucine to serine at a position corresponding to position 215 of SEQ ID NO: 9;
   an isoleucine to histidine at a position corresponding to position 215 of SEQ ID NO: 9;
   a lysine to glutamic acid at a position corresponding to position 229 of SEQ ID NO: 9;
   a lysine to glutamine at a position corresponding to position 229 of SEQ ID NO: 9;
   a lysine to arginine at a position corresponding to position 230 of SEQ ID NO: 9;
   a cysteine to arginine at a position corresponding to position 271 of SEQ ID NO: 9;
   an aspartic acid to glycine at a position corresponding to position 274 of SEQ ID NO: 9;
   a phenylalanine to glycine at a position corresponding to position 283 of SEQ ID NO: 9;
   an alanine to glycine at a position corresponding to position 292 of SEQ ID NO: 9;
   a serine to leucine at a position corresponding to position 296 of SEQ ID NO: 9;
   a cysteine to serine at a position corresponding to position 307 of SEQ ID NO: 9;
   an asparagine to aspartic acid at a position corresponding to position 324 of SEQ ID NO: 9;
   an asparagine to lysine at a position corresponding to position 324 of SEQ ID NO: 9;
   an aspartic acid to glutamic acid at a position corresponding to position 330 of SEQ ID NO: 9;
   a serine to leucine at a position corresponding to position 396 of SEQ ID NO: 9;
   an alanine to serine at a position corresponding to position 404 of SEQ ID NO: 9;
   an arginine to lysine at a position corresponding to position 406 of SEQ ID NO: 9;
   a lysine to isoleucine at a position corresponding to position 410 of SEQ ID NO: 9;
   a leucine to valine at a position corresponding to position 421 of SEQ ID NO: 9;
   an alanine to valine at a position corresponding to position 423 of SEQ ID NO: 9;
   a cysteine to serine at a position corresponding to position 434 of SEQ ID NO: 9;
   a cysteine to tyrosine at a position corresponding to position 434 of SEQ ID NO: 9;
   an aspartic acid to glycine at a position corresponding to position 447 of SEQ ID NO: 9;
   a serine to alanine at a position corresponding to position 448 of SEQ ID NO: 9;
   a valine to glutamic acid at a position corresponding to position 449 of SEQ ID NO: 9;
   an aspartic acid to glycine at a position corresponding to position 451 of SEQ ID NO: 9;
   an aspartic acid to asparagine at a position corresponding to position 454 of SEQ ID NO: 9;
   a tyrosine to phenylalanine at a position corresponding to position 465 of SEQ ID NO: 9;
   a lysine to threonine at a position corresponding to position 470 of SEQ ID NO: 9; and
   a threonine to serine at a position corresponding to position 500 of SEQ ID NO: 9.
**32.** The method of any of statements 29-31, wherein said mutated PPX gene comprises one or more nucleic acid sequence mutations selected from Table 2.
**33.** The method of any of statements 29-32, wherein said mutated PPX gene encodes a protein comprising a combination of two or more mutations selected from Tables 3a and 3b.
**34.** The method of any of statements 29-33, wherein said plant is selected from the group consisting of potato, sunflower, sugar beet, maize, cotton, soybean, wheat, rye, oats, rice, canola, fruits, vegetables, tobacco, barley, sorghum, tomato, mango, peach, apple, pear, strawberry, banana, melon, carrot, lettuce, onion, soya spp, sugar cane, pea, field beans, poplar, grape, citrus, alfalfa, rye, oats, turf and forage grasses, flax, oilseed rape, cucumber, morning glory, balsam, pepper, eggplant, marigold, lotus, cabbage, daisy, carnation, petunia, tulip, iris, lily, and nut-producing plants.
**35.** The method of any of statements 29-34, wherein said plant is a species selected from the group consisting of *Solanum tuberosum, Oryza sativa,* and *Zea mays.*
**36.** The method of any of statements 29-35, wherein said plant is a Russet Burbank potato cultivar.
**37.** The method of any of statements 29-36, wherein said plant is asexually reproduced.
**38.** The method of any of statements 29-37, wherein said plant is produced from a tuber.
**39.** The method of any of statements 29-38, wherein said plant is resistant to one or more herbicides.
**40.** The method of any of statements 29-39, wherein said plant is resistant to one or more PPX-inhibiting herbidicides.
**41.** The method of any of statements 29-40, wherein said plant is resistant to one or more herbicides selected from Table 5.
**42.** The method of any of statements 29-41, wherein said plant is resistant to one or more herbicides selected from the group consisting of flumioxazin, sulfentrazone, and saflufenacil.
**43.** A plant cell comprising a mutated protoporphyrinogen IX oxidase (PPX) gene, wherein said gene encodes a protein comprising a mutation at one or more amino acid positions corresponding to a position selected from the group consisting of 52, 85, 105, 111, 130, 139, 143, 144, 145, 147, 165, 167, 170, 180, 185, 192, 193, 199, 206, 212, 219, 220, 221, 226, 228, 229, 230, 237, 244, 256, 257, 270, 271, 272, 305, 311, 316, 318, 332, 343, 354, 357, 359, 360, 366, 393, 403, 424, 426, 430, 438, 440, 444, 455, 457, 470, 478, 483, 484, 485, 487, 490, 503, 508 and 525 of SEQ ID NO: 1 or wherein said gene encodes a protein comprising a mutation at one or more amino acid positions corresponding to a position selected from the group consisting of 58, 64, 74, 84, 93, 97, 98, 101, 119, 121, 124, 139, 150 151, 157, 164, 170, 177, 187, 188, 195, 214, 215, 229, 230, 271, 274, 278, 283, 292, 296, 307, 324, 330, 396, 404, 406, 410, 421, 423, 434, 447, 448, 449, 451, 454, 465, 470 and 500 of SEQ ID NO: 9.
**44.** The plant cell of statement 43, wherein the mutations at one or more amino acid positions are selected from the group consisting of
   a glycine to lysine at a position corresponding to position 52 of SEQ ID NO: 1;
   an asparagine to lysine at a position corresponding to position 52 of Figure 7 SEQ ID NO: 7;
   an asparagine to aspartic acid at a position corresponding to position 85 of SEQ ID NO: 1;
   a glutamic acid to valine at a position corresponding to position 111 of SEQ ID NO: 1;
   a glycine to asparagine at a position corresponding to position 130 of SEQ ID NO: 1;
   an aspartic acid to histidine at a position corresponding to position 139 of SEQ ID NO: 1;
   a proline to arginine at a position corresponding to position 143 of SEQ ID NO: 1;
   an arginine to cysteine at a position corresponding to position 144 of SEQ ID NO: 1;
   ; an arginine to leucine at a position corresponding to position 144 of SEQ ID NO: 1;
   an arginine to histidine at a position corresponding to position 144 of SEQ ID NO: 1;
   a phenylalanine to leucine at a position corresponding to position 145 of SEQ ID NO: 1, ;
   a phenylalanine to tyrosine at a position corresponding to position 145 of SEQ ID NO: 1;
   a leucine to valine at a position corresponding to position 147 of SEQ ID NO: 1;
   a phenylalanine to asparagine at a position corresponding to position 165 of SEQ ID NO: 1;
   an alanine to threonine at a position corresponding to position 180 of SEQ ID NO: 1;
   a proline to histidine at a position corresponding to position 185 of SEQ ID NO: 1;
   a proline to arginine at a position corresponding to position 185 of SEQ ID NO: 1;
   a proline to tyrosine at a position corresponding to position 185 of SEQ ID NO: 1;
   a glutamic acid to aspartic acid at a position corresponding to position 192 of SEQ ID NO: 1;
   a glutamic acid to lysine at a position corresponding to position 192 of SEQ ID NO: 1;
   a serine to threonine at a position corresponding to position 193 of SEQ ID NO: 1;
   an arginine to leucine at a position corresponding to position 199 of SEQ ID NO: 1;
   a valine to phenylalanine at a position corresponding to position 206 of SEQ ID NO: 1;
   a tyrosine to serine at a position corresponding to position 219 of SEQ ID NO: 1;
   an alanine to threonine at a position corresponding to position 220 of SEQ ID NO: 1;
   a leucine to methionine at a position corresponding to position 226 of SEQ ID NO: 1;
   a methionine to leucine at a position corresponding to position 228 of SEQ ID NO: 1;
   a lysine to glutamine at a position corresponding to position 229 of SEQ ID NO: 1;
   an alanine to phenylalanine at a position corresponding to position 230 of SEQ ID NO: 1;
   a serine to glycine at a position corresponding to position 244 of SEQ ID NO: 1;
   a serine to threonine at a position corresponding to position 244 of SEQ ID NO: 1;
   an arginine to histidine at a position corresponding to position 256 of SEQ ID NO: 1;
   an arginine to serine at a position corresponding to position 256 of SEQ ID NO: 1;
   a lysine to glutamic acid at a position corresponding to position 270 of SEQ ID NO: 1;
   a lysine to glutamine at a position corresponding to position 270 of SEQ ID NO: 1;
   a proline to arginine at a position corresponding to position 271 of SEQ ID NO: 1;
   a glutamine to phenylalanine at a position corresponding to position 272 of SEQ ID NO: 1;
   a lysine to phenylalanine at a position corresponding to position 272 of SEQ ID NO: 7;
   a serine to leucine at a position corresponding to position 305 of SEQ ID NO: 1;
   a glutamic acid arginine at a position corresponding to position 311 of SEQ ID NO: 1;
   a threonine to glycine at a position corresponding to position 316 of SEQ ID NO: 1;
   a threonine to glycine at a position corresponding to position 318 of SEQ ID NO: 1;
   a serine to cysteine at a position corresponding to position 332 of SEQ ID NO: 1;
   a leucine to isoleucine at a position corresponding to position 357 of SEQ ID NO: 1;
   a lysine to arginine at a position corresponding to position 359 of SEQ ID NO: 1;
   a lysine to threonine at a position corresponding to position 359 of SEQ ID NO: 1;
   a serine to arginine at a position corresponding to position 359 of SEQ ID NO: 7;
   a serine to threonine at a position corresponding to position 359 of SEQ ID NO: 7;
   a leucine to aspartic acid at a position corresponding to position 360 of SEQ ID NO: 1;
   a leucine to lysine at a position corresponding to position 360 of SEQ ID NO: 1;
   an alanine to glutamic acid at a position corresponding to position 366 of SEQ ID NO: 1;
   a leucine to methionine at a position corresponding to position 393 of SEQ ID NO: 1;
   a leucine to serine at a position corresponding to position 393 of SEQ ID NO: 1;
   a leucine to valine at a position corresponding to position 393 of SEQ ID NO: 1;
   a leucine to arginine at a position corresponding to position 403 of SEQ ID NO: 1;
   a leucine to serine at a position corresponding to position 424 of SEQ ID NO: 1;
   a tyrosine to cysteine at a position corresponding to position 426 of SEQ ID NO: 1;
   a tyrosine to phenylalanine at a position corresponding to position 426 of SEQ ID NO: 1;
   a tyrosine to histidine at a position corresponding to position 426 of SEQ ID NO: 1;
   a tyrosine to isoleucine at a position corresponding to position 426 of SEQ ID NO: 1;
   a tyrosine to leucine at a position corresponding to position 426 of SEQ ID NO: 1;
   a tyrosine to arginine at a position corresponding to position 426 of SEQ ID NO: 1;
   a tyrosine to threonine at a position corresponding to position 426 of SEQ ID NO: 1;
   a tyrosine to valine at a position corresponding to position 426 of SEQ ID NO: 1;
   a serine to leucine at a position corresponding to position 430 of SEQ ID NO: 1;
   a lysine to serine at a position corresponding to position 438 of SEQ ID NO: 1;
   a glutamic acid to lysine at a position corresponding to position 440 of SEQ ID NO: 1;
   a valine to isoleucine at a position corresponding to position 444 of SEQ ID NO: 1;
   a leucine to valine at a position corresponding to position 455 of SEQ ID NO: 1;
   a lysine to valine at a position corresponding to position 457 of SEQ ID NO: 1;
   a valine to serine at a position corresponding to position 470 of SEQ ID NO: 1;
   a valine to tyrosine at a position corresponding to position 470 of SEQ ID NO: 1;
   a phenylalanine to serine at a position corresponding to position 478 of SEQ ID NO: 1;
   a phenylalanine to glycine at a position corresponding to position 483 of SEQ ID NO: 1;
   an aspartic acid to alanine at a position corresponding to position 484 of SEQ ID NO: 1;
   an isoleucine to glutamic acid at a position corresponding to position 485 of SEQ ID NO: 1;
   an aspartic acid to glycine at a position corresponding to position 487 of SEQ ID NO: 1;
   a lysine to asparagine at a position corresponding to position 490 of SEQ ID NO: 1;
   a leucine to phenylalanine at a position corresponding to position 503 of SEQ ID NO: 1;
   a valine to threonine at a position corresponding to position 508 of SEQ ID NO: 1;
   an isoleucine to threonine at a position corresponding to position 525 of SEQ ID NO: 1.
   and an isoleucine to threonine at a position corresponding to position 525 of SEQ ID NO: 7;
   an aspartic acid to asparagine at a position corresponding to position 58 of SEQ ID NO: 9;
   a glutamic acid to valine at a position corresponding to position 64 of SEQ ID NO: 9;
   a glycine to cysteine at a position corresponding to position 74 of SEQ ID NO: 9;
   a glycine to asparagine at a position corresponding to position 84 of SEQ ID NO: 9;
   a leucine to histidine at a position corresponding to position 93 of SEQ ID NO: 9;
   a lysine to arginine at a position corresponding to position 97 of SEQ ID NO: 9;
   an arginine to histidine at a position corresponding to position 98 of SEQ ID NO: 9;
   an arginine to cysteine at a position corresponding to position 98 of SEQ ID NO: 9;
   an arginine to leucine at a position corresponding to position 98 of SEQ ID NO:9;
   an alanine to valine at a position corresponding to position 101 of SEQ ID NO: 9;
   a serine to asparagine at a position corresponding to position 119 of SEQ ID NO: 9;
   a phenylalanine to leucine at a position corresponding to position 121 of SEQ ID NO: 9;
   a threonine to isoleucine at a position corresponding to position 124 of SEQ ID NO: 9;
   an asparagine to tyrosine at a position corresponding to position 139 of SEQ ID NO: 9;
   an asparagine to arginine at a position corresponding to position 139 of SEQ ID NO: 9;
   an asparagine to histidine at a position corresponding to position 139 of SEQ ID NO: 9;
   a glutamic acid to aspartic acid at a position corresponding to position 150 of SEQ ID NO: 9;
   a glutamic acid to lysine at a position corresponding to position 150 of SEQ ID NO: 9;
   a serine to threonine at a position corresponding to position 151 of SEQ ID NO: 9;
   a glutamine to leucine at a position corresponding to position 157 of SEQ ID NO: 9;
   a valine to phenylalanine at a position corresponding to position 164 of SEQ ID NO: 9;
   a valine to alanine at a position corresponding to position 164 of SEQ ID NO: 9;
   an aspartic acid to glutamic acid at a position corresponding to position 170 of SEQ ID NO: 9;
   a cysteine to serine at a position corresponding to position 177 of SEQ ID NO: 9;
   a histidine to glutamine at a position corresponding to position 187 of SEQ ID NO: 9;
   a leucine to phenylalanine at a position corresponding to position 188 of SEQ ID NO: 9;
   an asparagine to lysine at a position corresponding to position 195 of SEQ ID NO: 9;
   a proline to serine at a position corresponding to position 214 of SEQ ID NO: 9;
   a proline to histidine at a position corresponding to position 214 of SEQ ID NO: 9;
   an isoleucine to serine at a position corresponding to position 215 of SEQ ID NO: 9;
   an isoleucine to histidine at a position corresponding to position 215 of SEQ ID NO: 9;
   a lysine to glutamic acid at a position corresponding to position 229 of SEQ ID NO: 9;
   a lysine to glutamine at a position corresponding to position 229 of SEQ ID NO: 9;
   a lysine to arginine at a position corresponding to position 230 of SEQ ID NO: 9;
   a cysteine to arginine at a position corresponding to position 271 of SEQ ID NO: 9;
   an aspartic acid to glycine at a position corresponding to position 274 of SEQ ID NO: 9;
   a phenylalanine to glycine at a position corresponding to position 283 of SEQ ID NO: 9;
   an alanine to glycine at a position corresponding to position 292 of SEQ ID NO: 9;
   a serine to leucine at a position corresponding to position 296 of SEQ ID NO: 9;
   a cysteine to serine at a position corresponding to position 307 of SEQ ID NO: 9;
   an asparagine to aspartic acid at a position corresponding to position 324 of SEQ ID NO: 9;
   an asparagine to lysine at a position corresponding to position 324 of SEQ ID NO: 9;
   an aspartic acid to glutamic acid at a position corresponding to position 330 of SEQ ID NO: 9;
   a serine to leucine at a position corresponding to position 396 of SEQ ID NO: 9;
   an alanine to serine at a position corresponding to position 404 of SEQ ID NO: 9;
   an arginine to lysine at a position corresponding to position 406 of SEQ ID NO: 9;
   a lysine to isoleucine at a position corresponding to position 410 of SEQ ID NO: 9;
   a leucine to valine at a position corresponding to position 421 of SEQ ID NO: 9;
   an alanine to valine at a position corresponding to position 423 of SEQ ID NO: 9;
   a cysteine to serine at a position corresponding to position 434 of SEQ ID NO: 9;
   a cysteine to tyrosine at a position corresponding to position 434 of SEQ ID NO: 9;
   an aspartic acid to glycine at a position corresponding to position 447 of SEQ ID NO: 9;
   a serine to alanine at a position corresponding to position 448 of SEQ ID NO: 9;
   a valine to glutamic acid at a position corresponding to position 449 of SEQ ID NO: 9;
   an aspartic acid to glycine at a position corresponding to position 451 of SEQ ID NO: 9;
   an aspartic acid to asparagine at a position corresponding to position 454 of SEQ ID NO: 9;
   a tyrosine to phenylalanine at a position corresponding to position 465 of SEQ ID NO: 9;
   a lysine to threonine at a position corresponding to position 470 of SEQ ID NO: 9; and
   a threonine to serine at a position corresponding to position 500 of SEQ ID NO: 9.
**45.** The plant cell of any of statements 43-44, wherein said mutated PPX gene comprises one or more nucleic acid sequence mutations selected from Table 2.
**46.** The plant cell of any of statements 43-45, wherein said mutated PPX gene encodes a protein comprising a combination of two or more mutations selected from Tables 3a and 3b.
**47.** A plant comprising a mutated protoporphyrinogen IX oxidase (PPX) gene
   wherein said gene encodes a protein comprising a mutation at one or more amino acid positions corresponding to a position selected from the group consisting of 52, 85, 105, 111, 130, 139, 143, 144, 145, 147, 165, 167, 170, 180, 185, 192, 193, 199, 206, 212, 219, 220, 221, 226, 228, 229, 230, 237, 244, 256, 257, 270, 271, 272, 305, 311, 316, 318, 332, 343, 354, 357, 359, 360, 366, 393, 403, 424, 426, 430, 438, 440, 444, 455, 457, 470, 478, 483, 484, 485, 487, 490, 503, 508 and 525 of SEQ ID NO: 1 or wherein said gene encodes a protein comprising a mutation at one or more amino acid positions corresponding to a position selected from the group consisting of 58, 64, 74, 84, 93, 97, 98, 101, 119, 121, 124, 139, 150 151, 157, 164, 170, 177, 187, 188, 195, 214, 215, 229, 230, 271, 274, 278, 283, 292, 296, 307, 324, 330, 396, 404, 406, 410, 421, 423, 434, 447, 448, 449, 451, 454, 465, 470 and 500 of SEQ ID NO: 9.
**48.** The plant of statement 47, wherein said mutations at one or more amino acid positions are selected from the group consisting of
   a glycine to lysine at a position corresponding to position 52 of SEQ ID NO: 1;
   an asparagine to lysine at a position corresponding to position 52 of Figure 7 SEQ ID NO: 7;
   an asparagine to aspartic acid at a position corresponding to position 85 of SEQ ID NO: 1;
   a glutamic acid to valine at a position corresponding to position 111 of SEQ ID NO: 1;
   a glycine to asparagine at a position corresponding to position 130 of SEQ ID NO: 1;
   an aspartic acid to histidine at a position corresponding to position 139 of SEQ ID NO: 1;
   a proline to arginine at a position corresponding to position 143 of SEQ ID NO: 1;
   an arginine to cysteine at a position corresponding to position 144 of SEQ ID NO: 1;
   ; an arginine to leucine at a position corresponding to position 144 of SEQ ID NO: 1;
   an arginine to histidine at a position corresponding to position 144 of SEQ ID NO: 1;
   a phenylalanine to leucine at a position corresponding to position 145 of SEQ ID NO: 1, ;
   a phenylalanine to tyrosine at a position corresponding to position 145 of SEQ ID NO: 1;
   a leucine to valine at a position corresponding to position 147 of SEQ ID NO: 1;
   a phenylalanine to asparagine at a position corresponding to position 165 of SEQ ID NO: 1;
   an alanine to threonine at a position corresponding to position 180 of SEQ ID NO: 1;
   a proline to histidine at a position corresponding to position 185 of SEQ ID NO: 1;
   a proline to arginine at a position corresponding to position 185 of SEQ ID NO: 1;
   a proline to tyrosine at a position corresponding to position 185 of SEQ ID NO: 1;
   a glutamic acid to aspartic acid at a position corresponding to position 192 of SEQ ID NO: 1;
   a glutamic acid to lysine at a position corresponding to position 192 of SEQ ID NO: 1;
   a serine to threonine at a position corresponding to position 193 of SEQ ID NO: 1;
   an arginine to leucine at a position corresponding to position 199 of SEQ ID NO: 1;
   a valine to phenylalanine at a position corresponding to position 206 of SEQ ID NO: 1;
   a tyrosine to serine at a position corresponding to position 219 of SEQ ID NO: 1;
   an alanine to threonine at a position corresponding to position 220 of SEQ ID NO: 1;
   a leucine to methionine at a position corresponding to position 226 of SEQ ID NO: 1;
   a methionine to leucine at a position corresponding to position 228 of SEQ ID NO: 1;
   a lysine to glutamine at a position corresponding to position 229 of SEQ ID NO: 1;
   an alanine to phenylalanine at a position corresponding to position 230 of SEQ ID NO: 1;
   a serine to glycine at a position corresponding to position 244 of SEQ ID NO: 1;
   a serine to threonine at a position corresponding to position 244 of SEQ ID NO: 1;
   an arginine to histidine at a position corresponding to position 256 of SEQ ID NO: 1;
   an arginine to serine at a position corresponding to position 256 of SEQ ID NO: 1;
   a lysine to glutamic acid at a position corresponding to position 270 of SEQ ID NO: 1;
   a lysine to glutamine at a position corresponding to position 270 of SEQ ID NO: 1;
   a proline to arginine at a position corresponding to position 271 of SEQ ID NO: 1;
   a glutamine to phenylalanine at a position corresponding to position 272 of SEQ ID NO: 1;
   a lysine to phenylalanine at a position corresponding to position 272 of SEQ ID NO: 7;
   a serine to leucine at a position corresponding to position 305 of SEQ ID NO: 1;
   a glutamic acid arginine at a position corresponding to position 311 of SEQ ID NO: 1;
   a threonine to glycine at a position corresponding to position 316 of SEQ ID NO: 1;
   a threonine to glycine at a position corresponding to position 318 of SEQ ID NO: 1;
   a serine to cysteine at a position corresponding to position 332 of SEQ ID NO: 1;
   a leucine to isoleucine at a position corresponding to position 357 of SEQ ID NO: 1;
   a lysine to arginine at a position corresponding to position 359 of SEQ ID NO: 1;
   a lysine to threonine at a position corresponding to position 359 of SEQ ID NO: 1;
   a serine to arginine at a position corresponding to position 359 of SEQ ID NO: 7;
   a serine to threonine at a position corresponding to position 359 of SEQ ID NO: 7;
   a leucine to aspartic acid at a position corresponding to position 360 of SEQ ID NO: 1;
   a leucine to lysine at a position corresponding to position 360 of SEQ ID NO: 1;
   an alanine to glutamic acid at a position corresponding to position 366 of SEQ ID NO: 1;
   a leucine to methionine at a position corresponding to position 393 of SEQ ID NO: 1;
   a leucine to serine at a position corresponding to position 393 of SEQ ID NO: 1;
   a leucine to valine at a position corresponding to position 393 of SEQ ID NO: 1;
   a leucine to arginine at a position corresponding to position 403 of SEQ ID NO: 1;
   a leucine to serine at a position corresponding to position 424 of SEQ ID NO: 1;
   a tyrosine to cysteine at a position corresponding to position 426 of SEQ ID NO: 1;
   a tyrosine to phenylalanine at a position corresponding to position 426 of SEQ ID NO: 1;
   a tyrosine to histidine at a position corresponding to position 426 of SEQ ID NO: 1;
   a tyrosine to isoleucine at a position corresponding to position 426 of SEQ ID NO: 1;
   a tyrosine to leucine at a position corresponding to position 426 of SEQ ID NO: 1;
   a tyrosine to arginine at a position corresponding to position 426 of SEQ ID NO: 1;
   a tyrosine to threonine at a position corresponding to position 426 of SEQ ID NO: 1;
   a tyrosine to valine at a position corresponding to position 426 of SEQ ID NO: 1;
   a serine to leucine at a position corresponding to position 430 of SEQ ID NO: 1;
   a lysine to serine at a position corresponding to position 438 of SEQ ID NO: 1;
   a glutamic acid to lysine at a position corresponding to position 440 of SEQ ID NO: 1;
   a valine to isoleucine at a position corresponding to position 444 of SEQ ID NO: 1;
   a leucine to valine at a position corresponding to position 455 of SEQ ID NO: 1;
   a lysine to valine at a position corresponding to position 457 of SEQ ID NO: 1;
   a valine to serine at a position corresponding to position 470 of SEQ ID NO: 1;
   a valine to tyrosine at a position corresponding to position 470 of SEQ ID NO: 1;
   a phenylalanine to serine at a position corresponding to position 478 of SEQ ID NO: 1;
   a phenylalanine to glycine at a position corresponding to position 483 of SEQ ID NO: 1;
   an aspartic acid to alanine at a position corresponding to position 484 of SEQ ID NO: 1;
   an isoleucine to glutamic acid at a position corresponding to position 485 of SEQ ID NO: 1;
   an aspartic acid to glycine at a position corresponding to position 487 of SEQ ID NO: 1;
   a lysine to asparagine at a position corresponding to position 490 of SEQ ID NO: 1;
   a leucine to phenylalanine at a position corresponding to position 503 of SEQ ID NO: 1;
   a valine to threonine at a position corresponding to position 508 of SEQ ID NO: 1;
   an isoleucine to threonine at a position corresponding to position 525 of SEQ ID NO: 1.
   and an isoleucine to threonine at a position corresponding to position 525 of SEQ ID NO: 7;
   an aspartic acid to asparagine at a position corresponding to position 58 of SEQ ID NO: 9;
   a glutamic acid to valine at a position corresponding to position 64 of SEQ ID NO: 9;
   a glycine to cysteine at a position corresponding to position 74 of SEQ ID NO: 9;
   a glycine to asparagine at a position corresponding to position 84 of SEQ ID NO: 9;
   a leucine to histidine at a position corresponding to position 93 of SEQ ID NO: 9;
   a lysine to arginine at a position corresponding to position 97 of SEQ ID NO: 9;
   an arginine to histidine at a position corresponding to position 98 of SEQ ID NO: 9;
   an arginine to cysteine at a position corresponding to position 98 of SEQ ID NO: 9;
   an arginine to leucine at a position corresponding to position 98 of SEQ ID NO:9;
   an alanine to valine at a position corresponding to position 101 of SEQ ID NO: 9;
   a serine to asparagine at a position corresponding to position 119 of SEQ ID NO: 9;
   a phenylalanine to leucine at a position corresponding to position 121 of SEQ ID NO: 9;
   a threonine to isoleucine at a position corresponding to position 124 of SEQ ID NO: 9;
   an asparagine to tyrosine at a position corresponding to position 139 of SEQ ID NO: 9;
   an asparagine to arginine at a position corresponding to position 139 of SEQ ID NO: 9;
   an asparagine to histidine at a position corresponding to position 139 of SEQ ID NO: 9;
   a glutamic acid to aspartic acid at a position corresponding to position 150 of SEQ ID NO: 9;
   a glutamic acid to lysine at a position corresponding to position 150 of SEQ ID NO: 9;
   a serine to threonine at a position corresponding to position 151 of SEQ ID NO: 9;
   a glutamine to leucine at a position corresponding to position 157 of SEQ ID NO: 9;
   a valine to phenylalanine at a position corresponding to position 164 of SEQ ID NO: 9;
   a valine to alanine at a position corresponding to position 164 of SEQ ID NO: 9;
   an aspartic acid to glutamic acid at a position corresponding to position 170 of SEQ ID NO: 9;
   a cysteine to serine at a position corresponding to position 177 of SEQ ID NO: 9;
   a histidine to glutamine at a position corresponding to position 187 of SEQ ID NO: 9;
   a leucine to phenylalanine at a position corresponding to position 188 of SEQ ID NO: 9;
   an asparagine to lysine at a position corresponding to position 195 of SEQ ID NO: 9;
   a proline to serine at a position corresponding to position 214 of SEQ ID NO: 9;
   a proline to histidine at a position corresponding to position 214 of SEQ ID NO: 9;
   an isoleucine to serine at a position corresponding to position 215 of SEQ ID NO: 9;
   an isoleucine to histidine at a position corresponding to position 215 of SEQ ID NO: 9;
   a lysine to glutamic acid at a position corresponding to position 229 of SEQ ID NO: 9;
   a lysine to glutamine at a position corresponding to position 229 of SEQ ID NO: 9;
   a lysine to arginine at a position corresponding to position 230 of SEQ ID NO: 9;
   a cysteine to arginine at a position corresponding to position 271 of SEQ ID NO: 9;
   an aspartic acid to glycine at a position corresponding to position 274 of SEQ ID NO: 9;
   a phenylalanine to glycine at a position corresponding to position 283 of SEQ ID NO: 9;
   an alanine to glycine at a position corresponding to position 292 of SEQ ID NO: 9;
   a serine to leucine at a position corresponding to position 296 of SEQ ID NO: 9;
   a cysteine to serine at a position corresponding to position 307 of SEQ ID NO: 9;
   an asparagine to aspartic acid at a position corresponding to position 324 of SEQ ID NO: 9;
   an asparagine to lysine at a position corresponding to position 324 of SEQ ID NO: 9;
   an aspartic acid to glutamic acid at a position corresponding to position 330 of SEQ ID NO: 9;
   a serine to leucine at a position corresponding to position 396 of SEQ ID NO: 9;
   an alanine to serine at a position corresponding to position 404 of SEQ ID NO: 9;
   an arginine to lysine at a position corresponding to position 406 of SEQ ID NO: 9;
   a lysine to isoleucine at a position corresponding to position 410 of SEQ ID NO: 9;
   a leucine to valine at a position corresponding to position 421 of SEQ ID NO: 9;
   an alanine to valine at a position corresponding to position 423 of SEQ ID NO: 9;
   a cysteine to serine at a position corresponding to position 434 of SEQ ID NO: 9;
   a cysteine to tyrosine at a position corresponding to position 434 of SEQ ID NO: 9;
   an aspartic acid to glycine at a position corresponding to position 447 of SEQ ID NO: 9;
   a serine to alanine at a position corresponding to position 448 of SEQ ID NO: 9;
   a valine to glutamic acid at a position corresponding to position 449 of SEQ ID NO: 9;
   an aspartic acid to glycine at a position corresponding to position 451 of SEQ ID NO: 9;
   an aspartic acid to asparagine at a position corresponding to position 454 of SEQ ID NO: 9;
   a tyrosine to phenylalanine at a position corresponding to position 465 of SEQ ID NO: 9;
   a lysine to threonine at a position corresponding to position 470 of SEQ ID NO: 9; and
   a threonine to serine at a position corresponding to position 500 of SEQ ID NO: 9.
**49.** The plant cell of any of statements 47-48, wherein said mutated PPX gene encodes a protein comprising a combination of two or more mutations selected from Tables 4a and 4b.
**50.** The plant of any of statements 47-49, wherein said mutated PPX gene comprises one or more nucleic acid sequence mutations selected from Tables 2, 3a and 3b.
**51.** The plant of any of statements 47-50, wherein said mutated PPX gene encodes a protein comprising a combination of two or more mutations selected from Tables 4a and 4b.
**52.** The plant of any of statements 47-51, wherein said plant is selected from the group consisting of potato, sunflower, sugar beet, maize, cotton, soybean, wheat, rye, oats, rice, canola, fruits, vegetables, tobacco, barley, sorghum, tomato, mango, peach, apple, pear, strawberry, banana, melon, carrot, lettuce, onion, soya spp, sugar cane, pea, field beans, poplar, grape, citrus, alfalfa, rye, oats, turf and forage grasses, flax, oilseed rape, cucumber, morning glory, balsam, pepper, eggplant, marigold, lotus, cabbage, daisy, carnation, petunia, tulip, iris, lily, and nut-producing plants.
**53.** The plant of any of statements 47-52, wherein said plant is a species selected from the group consisting of *Solanum tuberosum, Oryza sativa,* and *Zea mays.*
**54.** The plant of any of statements 47-53, wherein said plant is a Russet Burbank potato cultivar.
**55.** The plant of any of statements 47-54, wherein said plant is asexually reproduced.
**56.** The plant of any of statements 47-55, wherein said plant is produced from a tuber.
**57.** The plant of any of statements 47-56, wherein said plant is resistant to one or more herbicides.
**58.** The plant of any of statements 47-57, wherein said plant is resistant to one or more PPX-inhibiting herbidicides.
**59.** The plant of any of statements 47-58, wherein said plant is resistant to one or more herbicides selected from Table 5.
**60.** The plant of any of statements 47-59, wherein said plant is resistant to one or more herbicides selected from the group consisting of flumioxazin, sulfentrazone, and saflufenacil.
**61.** The plant cell or plant of any of one of statements 1-17 or 43-60, wherein said mutations is one or more mutations selected from the group consisting of an arginine to cysteine at a position corresponding to position 98 of SEQ ID NO: 9 (R98C), an arginine to histidine at a position corresponding to position 98 of SEQ ID NO: 9 (R98H), an arginine to lysine at a position corresponding to position 98 of SEQ ID NO: 9 (R98L), a threonine to isoleucine at a position corresponding to position 124 of SEQ ID NO: 9 (T124I), an arginine to cysteine at a position corresponding to position 144 of SEQ ID NO: 9 (R144C), an arginine to histidine at a position corresponding to position 144 of SEQ ID NO: 9 (R144H), an arginine to leucine at a position corresponding to position 144 of SEQ ID NO: 9 (R144L), a proline to tyrosine at a position corresponding to position 185 of SEQ ID NO: 9 (P185Y), a proline to arginine at a position corresponding to position 185 of SEQ ID NO: 9 (P185R), a proline to histidine at a position corresponding to position 185 of SEQ ID NO: 9 (P185H), a proline to serine at a position corresponding to position 214 of SEQ ID NO: 9 (P214S), an alanine to threonine at a position corresponding to position 220 of SEQ ID NO: 9 (A220T), a proline to histidine at a position corresponding to position 214 of SEQ ID NO: 9 (P214H), a lysine to glutamine at a position corresponding to position 229 of SEQ ID NO: 9 (K229Q), a cysteine to serine at a position corresponding to position 307 of SEQ ID NO: 9 (C307S), a serine to cysteine at a position corresponding to position 332 of SEQ ID NO: 9 (S332C), a serine to leucine at a position corresponding to position 332 of SEQ ID NO: 9 (S332L), and an alanine to valine at a position corresponding to position 423 of SEQ ID NO: 9 (A423V).
**62.** The method of any one of statements 18-42, wherein said mutations is one or more mutations selected from the group consisting of an arginine to cysteine at a position corresponding to position 98 of SEQ ID NO: 9 (R98C), an arginine to histidine at a position corresponding to position 98 of SEQ ID NO: 9 (R98H), an arginine to lysine at a position corresponding to position 98 of SEQ ID NO: 9 (R98L), a threonine to isoleucine at a position corresponding to position 124 of SEQ ID NO: 9 (T124I), a proline to serine at a position corresponding to position 214 of SEQ ID NO: 9 (P214S), a proline to histidine at a position corresponding to position 214 of SEQ ID NO: 9 (P214H), a lysine to glutamine at a position corresponding to position 229 of SEQ ID NO: 9 (K229Q), a cysteine to serine at a position corresponding to position 307 of SEQ ID NO: 9 (C307S), and an alanine to valine at a position corresponding to position 423 of SEQ ID NO: 9 (A423V).

Other embodiments are set forth within the following claims.

## Claims

1. A plant resistant to one or more PPX-inhibiting herbicides, said plant comprising a mutated protoporphyrinogen IX oxidase (PPX) gene wherein said gene encodes a protein comprising a leucine to methionine substitution at the amino acid position corresponding to position 226 of SEQ ID NO: 1.

2. The plant of claim 1, wherein said plant is selected from the group consisting of potato, sunflower, sugar beet, maize, cotton, soybean, wheat, rye, oats, rice, canola, fruits, vegetables, tobacco, barley, sorghum, tomato, mango, peach, apple, pear, strawberry, banana, melon, carrot, lettuce, onion, soya spp, sugar cane, pea, field beans, poplar, grape, citrus, alfalfa, rye, oats, turf and forage grasses, flax, oilseed rape, cucumber, morning glory, balsam, pepper, eggplant, marigold, lotus, cabbage, daisy, carnation, petunia, tulip, iris, lily, and nut-producing plants, in particular wherein said plant is a species selected from the group consisting of *Solanum tuberosum, Oryza sativa,* and *Zea mays,* or wherein said plant is a Russet Burbank potato cultivar.

3. The plant of claim 1 or 2, wherein said plant is canola.

4. The plant of any of the preceding claims, wherein said plant is resistant to one or more PPX-inhibiting herbicides selected from acifluorfen-Na, Bifenox, Chlomethoxyfen, fluoroglycofen-ethyl, Fomesafen, Halosafen, Lactofen, Oxyfluorfen, Fluazolate, pyraflufen-ethyl, cinidon-ethyl, Flumioxazin, flumiclorac-pentyl, fluthiacet-methyl, Thidiazimin, Oxadiazon, Oxadiargyl, Azafenidin, carfentrazone-ethyl, Sulfentrazone, Pentoxazone, Benzfendizone, Butafenacil, Saflufenacil, Pyrazogyl, Profluazol, in particular wherein said plant is resistant to one or more herbicides selected from the group consisting of flumioxazin, sulfentrazone, and saflufenacil.

5. The plant of any of the preceding claims, wherein the plant is a non-transgenic plant.

6. A plant cell comprising a mutated protoporphyrinogen IX oxidase (PPX) gene, wherein said gene encodes a protein comprising a leucine to methionine substitution at the amino acid position corresponding to position 226 of SEQ ID NO: 1.

7. The plant cell of claim 6, wherein the plant cell is a non-transgenic plant cell.

8. A method for producing a non-transgenic plant cell with a mutated PPX gene, comprising introducing into a plant cell a gene repair oligonucleobase (GRON) with a targeted mutation in a protoporphyrinogen IX oxidase (PPX) gene to produce a plant cell with a PPX gene that expresses a PPX protein comprising a leucine to methionine substitution at the amino acid position corresponding to position 226 of SEQ ID NO: 1.

9. The method of claim 8, for producing a plant resistant to one or more PPX-inhibiting herbicides, further comprising
identifying a plant cell having a growth rate or rate of cell division in the presence of a herbicide that is at least 35% of the growth rate or rate of cell division in a corresponding plant expressing the wild-type PPX protein in the absence of the herbicide; and
regenerating a non-transgenic plant resistant to one or more PPX-inhibiting herbicides, the plant having a mutated PPX gene from said plant cell.

10. The method of claim 8 or 9, wherein said plant cell is of a plant selected from the group consisting of potato, sunflower, sugar beet, maize, cotton, soybean, wheat, rye, oats, rice, canola, fruits, vegetables, tobacco, barley, sorghum, tomato, mango, peach, apple, pear, strawberry, banana, melon, carrot, lettuce, onion, soya spp, sugar cane, pea, field beans, poplar, grape, citrus, alfalfa, rye, oats, turf and forage grasses, flax, oilseed rape, cucumber, morning glory, balsam, pepper, eggplant, marigold, lotus, cabbage, daisy, carnation, petunia, tulip, iris, lily, and nut-producing plants, in particular wherein said plant cell is a species selected from the group consisting of *Solanum tuberosum, Oryza sativa, Sorghum bicolor, Ricinus communis, Brassica napus, Glycine max,* and *Zea mays,* or wherein said plant cell is a Russet Burbank potato cultivar.

11. The method of any of claims 8-10, wherein said plant cell is a canola cell.

12. The method of any of claims 8-11, wherein said plant cell is resistant to one or more PPX-inhibiting herbicides selected from acifluorfen-Na, Bifenox, Chlomethoxyfen, fluoroglycofen-ethyl, Fomesafen, Halosafen, Lactofen, Oxyfluorfen, Fluazolate, pyraflufen-ethyl, cinidon-ethyl, Flumioxazin, flumiclorac-pentyl, fluthiacet-methyl, Thidiazimin, Oxadiazon, Oxadiargyl, Azafenidin, carfentrazone-ethyl, Sulfentrazone, Pentoxazone, Benzfendizone, Butafenacil, Saflufenacil, Pyrazogyl, Profluazol, in particular wherein said plant cell is resistant to one or more herbicides selected from the group consisting of flumioxazin, sulfentrazone, and saflufenacil.
